# EUROPEAN PATENT SPECIFICATION

(11) **EP 2 576 831 B1**
(45) Date of publication and mention of the grant of the patent: **08.04.2015**
(21) Application number: 11746614.4
(22) Date of filing: 25.05.2011
(51) Int. Cl.: C12Q 1/68

(54) **DIAGNOSTIC METHOD**
DIAGNOSEVERFAHREN
MÉTHODE DIAGNOSTIQUE

(30) Priority: 25.05.2010 GB 201008719
(43) Date of publication of application: 10.04.2013
(73) Proprietor: National University of Ireland, Galway, Galway (IE)
(72) Inventor: REDDINGTON, Kate, Mary, Westport Co. Mayo (IE); BARRY, Thomas, Gerard, Kinvara County Galway (IE); O'GRADY, Justin, Joseph, Galway (IE); SMITH, Terence, James, Galway (IE)
(74) Representative: Carpmaels & Ransford LLP
(86) International application number: PCT/IB2011/001719
(87) International publication number: WO 2011/148269

(56) References cited:
- DATABASE EMBL [Online] 29 August 2000 (2000-08-29), "Mycobacterium tuberculosis strain H2255 (W) insertion sequence IS6110, partial sequence; and flanking insertion site 21-9.", XP002663004, retrieved from EBI accession no. EM_PRO:AF228674 Database accession no. AF228674
- DATABASE Geneseq [Online] 30 March 2001 (2001-03-30), "IGFBP2 oligonucleotide #89.", XP002663005, retrieved from EBI accession no. GSN:AAF45250 Database accession no. AAF45250
- WARREN R M ET AL: "Differentiation of Mycobacterium tuberculosis complex by PCR amplification of genomic regions of difference.", THE INTERNATIONAL JOURNAL OF TUBERCULOSIS AND LUNG DISEASE : THE OFFICIAL JOURNAL OF THE INTERNATIONAL UNION AGAINST TUBERCULOSIS AND LUNG DISEASE JUL 2006 LNKD- PUBMED:16850559, vol. 10, no. 7, July 2006 (2006-07), pages 818-822, XP002663006, ISSN: 1027-3719
- HERRERA-LEÓN LAURA ET AL: "Aplicación de métodos moleculares para la identificación de las especies del complejo Mycobacterium tuberculosis [Differentiation of species within the Mycobacterium tuberculosis complex by molecular techniques]", ENFERMEDADES INFECCIOSAS Y MICROBIOLOGIA CLINICA, DOYMA, BARCELONA, ES, vol. 27, no. 9, 1 November 2009 (2009-11-01), pages 496-502, XP008144998, ISSN: 0213-005X, DOI: 10.1016/J.EIMC.2009.01.008 [retrieved on 2009-05-01]
- B. A. PINSKY ET AL: "Multiplex Real-Time PCR Assay for Rapid Identification of Mycobacterium tuberculosis Complex Members to the Species Level", JOURNAL OF CLINICAL MICROBIOLOGY, vol. 46, no. 7, 1 July 2008 (2008-07-01), pages 2241-2246, XP055011475, ISSN: 0095-1137, DOI: 10.1128/JCM.00347-08
- POUNDER J I ET AL: "Mycobacterium tuberculosis complex differentiation by genomic deletion patterns with multiplex polymerase chain reaction and melting analysis", DIAGNOSTIC MICROBIOLOGY AND INFECTIOUS DISEASES, ELSEVIER SCIENCE PUBLISHING CO., AMSTERDAM, NL, vol. 67, no. 1, 12 March 2010 (2010-03-12) , pages 101-105, XP026995896, ISSN: 0732-8893 [retrieved on 2010-04-09]
- K. REDDINGTON ET AL: "Novel Multiplex Real-Time PCR Diagnostic Assay for Identification and Differentiation of Mycobacterium tuberculosis, Mycobacterium canettii, and Mycobacterium tuberculosis Complex Strains", JOURNAL OF CLINICAL MICROBIOLOGY, vol. 49, no. 2, 1 February 2011 (2011-02-01), pages 651-657, XP055011278, ISSN: 0095-1137, DOI: 10.1128/JCM.01426-10

### SEQUENCE LISTING

<110> NATIONAL UNIVERSITY OF IRELAND, GALWAY
<120> DIAGNOSTIC METHOD
<130> P054g03WO
<140>
   <141> 2011-05-25
<150> GB 1008719.5
   <151> 2010-05-25
<160> 173
<170> SeqWin2010, version 1.0
<210> 1
   <211> 906
   <212> DNA
   <213> Mycobacterium tuberculosis
<400> 1
   tcagtgccgc ccttctacca gcttcagttt ccgtctgcgg gacctgcgca gtgaactgcg 60
   caccatgagg tgggaacgca gcgccagtga tccccgcagg gtccagcgca gcggagcccg 120
   ccaccaacca gaatgtcggt cggctaagaa gatataggtg cttttgtgat gggcggccag 180
   atggcttgcc gggtcgcgac ccgtcgaatg cgccttgtgg tgcagaacct cggctgacgg 240
   cacatacacc gacagccaac cggctttgcc aagccggtcg ccaaggtcga cgtcctccat 300
   gtacatgaag taacgttcgt cgaatccgcc gacctggcca aacgccgacc ggcgcaccag 360
   taggcaagac cccgacaacc aacccaccgg ccgttcactg ggctccagcc gctcctgccg 420
   gtaggccgtc gtccacggat tgcgcggcca gaacggcccg agcactgcgt gcatgccgcc 480
   gcggatcagg ctgggcatct gccgcgccga cgggtacacc gacccgtcgg ggtcccgaat 540
   cagcgggccc agcgcgcccg cgcggggcca gcgggaggcg gcgtccagta gtgcatcgat 600
   actgcccggg ccccattgca cgtccgggtt ggccacgatc acccagtcat cgacccaggg 660
   ttcgccggca tcgcccgcca tttcaccgag ctgggcgatc gtccgattca ccgcggttcc 720
   gtacccgagg ttggcccctg tgggcagcag ccgcacgttg gggtagcgct gcaccgcggc 780
   ctgcggggtg ccgtcggtgg agccgttgtc tgccaacagc acgctgaccg gccgctcggt 840
   ggccagcgac aacgacgcca ggaaccgctc tagatggggc cccggcgagt aggtcaccgc 900
   taccac 906
<210> 2
   <211> 906
   <212> DNA
   <213> Mycobacterium tuberculosis
<400> 2
   tcagtgccgc ccttctacca gcttcagttt ccgtctgcgg gacctgcgca gtgaactgcg   60
   caccatgagg tgggaacgca gcgccagtga tccccgcagg gtccagcgca gcggagcccg  120
   ccaccaacca gaatgtcggt cggctaagaa gatataggtg cttttgtgat gggcggccag  180
   atggcttgcc gggtcgcgac ccgtcgaatg cgccttgtgg tgcagaacct cggctgacgg  240
   cacatacacc gacagccaac cggctttgcc aagccggtcg ccaaggtcga cgtcctccat  300
   gtacatgaag taacgttcgt cgaatccgcc gacctggcca aacgccgacc ggcgcaccag  360
   taggcaagac cccgacaacc aacccaccgg ccgttcactg ggctccagcc gctcctgccg  420
   gtaggccgtc gtccacggat tgcgcggcca gaacggcccg agcactgcgt gcatgccgcc  480
   gcggatcagg ctgggcatct gccgcgccga cgggtacacc gacccgtcgg ggtcccgaat  540
   cagcgggccc agcgcgcccg cgcggggcca gcgggaggcg gcgtccagta gtgcatcgat  600
   actgcccggg ccccattgca cgtccgggtt ggccacgatc acccagtcat cgacccaggg  660
   ttcgccggca tcgcccgcca tttcaccgag ctgggcgatc gtccgattca ccgcggttcc  720
   gtacccgagg ttggcccctg tgggcagcag ccgcacgttg gggtagcgct gcaccgcggc  780
   ctgcggggtg ccgtcggtgg agccgttgtc tgccaacagc acgctgaccg gccgctcggt  840
   ggccagcgac aacgacgcca ggaaccgctc tagatggggc cccggcgagt aggtcaccgc  900
   taccac                                                             906
<210> 3
   <211> 906
   <212> DNA
   <213> Mycobacterium tuberculosis
<400> 3
   tcagtgccgc ccttctacca gcttcagttt ccgtctgcgg gacctgcgca gtgaactgcg   60
   caccatgagg tgggaacgca gcgccagtga tccccgcagg gtccagcgca gcggagcccg  120
   ccaccaacca gaatgtcggt cggctaagaa gatataggtg cttttgtgat gggcggccag  180
   atggcttgcc gggtcgcgac ccgtcgaatg cgccttgtgg tgcagaacct cggctgacgg  240
   cacatacacc gacagccaac cggctttgcc aagccggtcg ccaaggtcga cgtcctccat  300
   gtacatgaag taacgttcgt cgaatccgcc gacctggcca aacgccgacc ggcgcaccag  360
   taggcaagac cccgacaacc aacccaccgg ccgttcactg ggctccagcc gctcctgccg  420
   gtaggccgtc gtccacggat tgcgcggcca gaacggcccg agcactgcgt gcatgccgcc  480
   gcggatcagg ctgggcatct gccgcgccga cgggtacacc gacccgtcgg ggtcccgaat  540
   cagcgggccc agcgcgcccg cgcggggcca gcgggaggcg gcgtccagta gtgcatcgat  600
   actgcccggg ccccattgca cgtccgggtt ggccacgatc acccagtcat cgacccaggg  660
   ttcgccggca tcgcccgcca tttcaccgag ctgggcgatc gtccgattca ccgcggttcc  720
   gtacccgagg ttggcccctg tgggcagcag ccgcacgttg gggtagcgct gcaccgcggc  780
   ctgcggggtg ccgtcggtgg agccgttgtc tgccaacagc acgctgaccg gccgctcggt  840
   ggccagcgac aacgacgcca ggaaccgctc tagatggggc cccggcgagt aggtcaccgc  900
   taccac                                                             906
<210> 4
   <211> 924
   <212> DNA
   <213> Mycobacterium tuberculosis
<400> 4
   tcagtgccgc ccttctacca gcttcagttt ccgtctgcgg gacctgcgca gtgaactgcg   60
   caccatgagg tgggaacgca gcgccagtga tccccgcagg gtccagcgca gcggagcccg  120
   ccaccaacca gaatgtcggt cggctaagaa gatataggtg cttttgtgat gggcggccag  180
   atggcttgcc gggtcgcgac ccgtcgaatg cgccttgtgg tgcagaacct cggctgacgg  240
   cacatacacc gacagccaac cggctttgcc aagccggtcg ccaaggtcga cgtcctccat  300
   gtacatgaag taacgttcgt cgaatccgcc gacctggcca aacgccgacc ggcgcaccag  360
   taggcaagac cccgacaacc aacccaccgg ccgttcactg ggctccagcc gctcctgccg  420
   gtaggccgtc gtccacggat tgcgcggcca gaacggcccg agcactgcgt gcatgccgcc  480
   gcggatcagg ctgggcatct gccgcgccga cgggtacacc gacccgtcgg ggtcccgaat  540
   cagcgggccc agcgcgcccg cgcggggcca gcgggaggcg gcgtccagta gtgcatcgat  600
   actgcccggg ccccattgca cgtccgggtt ggccacgatc acccagtcat cgacccaggg  660
   ttcgccggca tcgcccgcca tttcaccgag ctgggcgatc gtccgattca ccgcggttcc  720
   gtacccgagg ttggcccctg tgggcagcag ccgcacgttg gggtagcgct gcaccgcggc  780
   ctgcggggtg ccgtcggtgg agccgttgtc tgccaacagc acgctgaccg gccgctcggt  840
   ggccagcgac aacgacgcca ggaaccgctc tagatggggc cccggcgagt aggtcaccgc  900
   taccaccggc aggacgtcag tcac                                         924
<210> 5
   <211> 906
   <212> DNA
   <213> Mycobacterium tuberculosis
<400> 5
   tcagtgccgc ccttctacca gcttcagttt ccgtctgcgg gacctgcgca gtgaactgcg   60
   caccatgagg tgggaacgca gcgccagtga tccccgcagg gtccagcgca gcggagcccg  120
   ccaccaacca gaatgtcggt cggctaagaa gatataggtg cttttgtgat gggcggccag  180
   atggcttgcc gggtcgcgac ccgtcgaatg cgccttgtgg tgcagaacct cggctgacgg  240
   cacatacacc gacagccaac cggctttgcc aagccggtcg ccaaggtcga cgtcctccat  300
   gtacatgaag taacgttcgt cgaatccgcc gacctggcca aacgccgacc ggcgcaccag  360
   taggcaagac cccgacaacc aacccaccgg ccgttcactg ggctccagcc gctcctgccg  420
   gtaggccgtc gtccacggat tgcgcggcca gaacggcccg agcactgcgt gcatgccgcc  480
   gcggatcagg ctgggcatct gccgcgccga cgggtacacc gacccgtcgg ggtcccgaat  540
   cagcgggccc agcgcgcccg cgcggggcca gcgggaggcg gcgtccagta gtgcatcgat  600
   actgcccggg ccccattgca cgtccgggtt ggccacgatc acccagtcat cgacccaggg  660
   ttcgccggca tcgcccgcca tttcaccgag ctgggcgatc gtccgattca ccgcggttcc  720
   gtacccgagg ttggcccctg tgggcagcag ccgcacgttg gggtagcgct gcaccgcggc  780
   ctgcggggtg ccgtcggtgg agccgttgtc tgccaacagc acgctgaccg gccgctcggt  840
   ggccagcgac aacgacgcca ggaaccgctc tagatggggc cccggcgagt aggtcaccgc  900
   taccac                                                             906
<210> 6
   <211> 894
   <212> DNA
   <213> Mycobacterium bovis BCG
<400> 6
   tcagtgccgc ccttctacca gcttcagttt ccgtctgcgg gacctgcgca ccatgaggtg   60
   ggaacgcagc gccagtgatc cccgcagggt ccagcgcagc ggagcccgcc accaaccaga  120
   atgtcggtcg gctaagaaga tataggtgct tttgtgatgg gcggccagat ggcttgccgg  180
   gtcgcgaccc gtcgaatgcg ccttgtggtg cagaacctcg gctgacggca catacaccga  240
   cagccaaccg gctttgccaa gccggtcgcc aaggtcgacg tcctccatgt acatgaagta  300
   acgttcgtcg aatccgccga cctggccaaa cgccgaccgg cgcaccagta ggcaagaccc  360
   cgacaaccaa cccaccggcc gttcactggg ctccagccgc tcctgccggt aggccgtcgt  420
   ccacggattg cgcggccaga acggcccgag cactgcgtgc atgccgccgc ggatcaggct  480
   gggcatctgc cgcgccgacg ggtacaccga cccgtcgggg tcccgaatca gcgggcccag  540
   cgcgcccgcg cggggccagc gggaggcggc gtccagtagt gcatcgatac tgcccgggcc  600
   ccattgcacg tccgggttgg ccacgatcac ccagtcatcg acccagggtt cgccggcatc  660
   gcccgccatt tcaccgagct gggcgatcgt ccgattcacc gcggttccgt acccgaggtt  720
   ggcccctgtg ggcagcagcc gcacgttggg gtagcgctgc accgcggcct gcggggtgcc  780
   gtcggtggag ccgttgtctg ccaacagcac gctgaccggc cgctcggtgg ccagcgacaa  840
   cgacgccagg aaccgctcta gatggggccc cggcgagtag gtcaccgcta ccac        894
<210> 7
   <211> 894
   <212> DNA
   <213> Mycobacterium bovis BCG
<400> 7
   tcagtgccgc ccttctacca gcttcagttt ccgtctgcgg gacctgcgca ccatgaggtg   60
   ggaacgcagc gccagtgatc cccgcagggt ccagcgcagc ggagcccgcc accaaccaga  120
   atgtcggtcg gctaagaaga tataggtgct tttgtgatgg gcggccagat ggcttgccgg  180
   gtcgcgaccc gtcgaatgcg ccttgtggtg cagaacctcg gctgacggca catacaccga  240
   cagccaaccg gctttgccaa gccggtcgcc aaggtcgacg tcctccatgt acatgaagta  300
   acgttcgtcg aatccgccga cctggccaaa cgccgaccgg cgcaccagta ggcaagaccc  360
   cgacaaccaa cccaccggcc gttcactggg ctccagccgc tcctgccggt aggccgtcgt  420
   ccacggattg cgcggccaga acggcccgag cactgcgtgc atgccgccgc ggatcaggct  480
   gggcatctgc cgcgccgacg ggtacaccga cccgtcgggg tcccgaatca gcgggcccag  540
   cgcgcccgcg cggggccagc gggaggcggc gtccagtagt gcatcgatac tgcccgggcc  600
   ccattgcacg tccgggttgg ccacgatcac ccagtcatcg acccagggtt cgccggcatc  660
   gcccgccatt tcaccgagct gggcgatcgt ccgattcacc gcggttccgt acccgaggtt  720
   ggcccctgtg ggcagcagcc gcacgttggg gtagcgctgc accgcggcct gcggggtgcc  780
   gtcggtggag ccgttgtctg ccaacagcac gctgaccggc cgctcggtgg ccagcgacaa  840
   cgacgccagg aaccgctcta gatggggccc cggcgagtag gtcaccgcta ccac        894
<210> 8
   <211> 894
   <212> DNA
   <213> Mycobacterium bovis
<400> 8
   tcagtgccgc ccttctacca gcttcagttt ccgtctgcgg gacctgcgca ccatgaggtg 60
   ggaacgcagc gccagtgatc cccgcagggt ccagcgcagc ggagcccgcc accaaccaga 120
   atgtcggtcg gctaagaaga tataggtgct tttgtgatgg gcggccagat ggcttgccgg 180
   gtcgcgaccc gtcgaatgcg ccttgtggtg cagaacctcg gctgacggca catacaccga 240
   cagccaaccg gctttgccaa gccggtcgcc aaggtcgacg tcctccatgt acatgaagta 300
   acgttcgtcg aatccgccga cctggccaaa cgccgaccgg cgcaccagta ggcaagaccc 360
   cgacaaccaa cccaccggcc gttcactggg ctccagccgc tcctgccggt aggccgtcgt 420
   ccacggattg cgcggccaga acggcccgag cactgcgtgc atgccgccgc ggatcaggct 480
   gggcatctgc cgcgccgacg ggtacaccga cccgtcgggg tcccgaatca gcgggcccag 540
   cgcgcccgcg cggggccagc gggaggcggc gtccagtagt gcatcgatac tgcccgggcc 600
   ccattgcacg tccgggttgg ccacgatcac ccagtcatcg acccaggstt cgccggcatc 660
   gcccgccatt tcaccgagct gggcgatcgt ccgattcacc gcggttccgt acccgaggtt 720
   ggcccctgtg ggcagcagcc gcacgttggg gtagcgctgc accgcggcct gcggggtgcc 780
   gtcggtggag ccgttgtctg ccaacagcac gctgaccggc cgctcggtgg ccagcgacaa 840
   cgacgccagg aaccgctcta gatggggccc cggcgagtag gtcaccgcta ccac 894
<210> 9
   <211> 881
   <212> DNA
   <213> Mycobacterium africanum
<400> 9
   tcagtgccgc ccttctacca gcttcagttt ccgtctgcgg gacctgcgca ccatgaggtg 60
   ggaacgcagc gccagtgatc cccgcagggt ccagcgcagc ggagcccgcc accaaccaga 120
   atgtcggtcg gctaagaaga tataggtgct tttgtgatgg gcggccagat ggcttgccgg 180
   gtcgcgaccc gtcgaatgcg ccttgtggtg cagaacctcg gctgacggca catacaccga 240
   cagccaaccg gctttgccaa gccggtcgcc aaggtcgacg tcctccatgt acatgaagta 300
   acgttcgtcg aatccgccga cctggccaaa cgccgaccgg cgcaccagta ggcaagaccc 360
   cgacaaccaa cccaccggcc gttcactggg ctccagccgc tcctgccggt aggccgtcgt 420
   ccacggattg cgcggccaga acggcccgag cactgcgtgc atgccgccgc ggatcaggct 480
   gggcatctgc cgcgccgacg ggtacaccga cccgtcgggg tcccgaatca gcgggcccag 540
   cgcgcccgcg cggggccagc gggaggcggc gtccagtagt gcatcgatac tgcccgggcc 600
   ccattgcacg tccgggttgg ccacgatcac ccagtcatcg acccagggtt cgccggcatc 660
   gcccgccatt tcaccgagct gggcgatcgt ccgattcacc gcggttccgt acccgaggtt 720
   ggcccctgtg ggcagcagcc gcacgttggg gtagcgctgc accgcggcct gcggggtgcc 780
   gtcggtggag ccgttgtctg ccaacagcac gctgaccggc cgctcggtgg ccagcgacaa 840
   cgacgccagg aaccgctcta gatggggccc cggcgagtag g 881
<210> 10
   <211> 905
   <212> DNA
   <213> Mycobacterium microti
<400> 10
   tcagtgccgc ccttctacca gcttcagttt ccgtctgcgg gacctgcgca ccatgaggtg 60
   ggaacgcagc gccagtgatc cccgcagggt ccagcgcagc ggagcccgcc accaaccaga 120
   atgtcggtcg gctaagaaga tataggtgct tttgtgatgg gcggccagat ggcttgccgg 180
   gtcgcgaccc gtcgaatgcg ccttgtggtg cagaacctcg gctgacggca catacaccga 240
   cagccaaccg gctttgccaa gccggtcgcc aaggtcgacg tcctccatgt acatgaagta 300
   acgttcgtcg aatccgccga cctggccaaa cgccgaccgg cgcaccagta ggcaagaccc 360
   cgacaaccaa cccaccggcc gttcactggg ctccagccgc tcctgccggt aggccgtcgt 420
   ccacggattg cgcggccaga acggcccgag cactgcgtgc atgccgccgc ggatcaggct 480
   gggcatctgc cgcgccgacg ggtacaccga cccgtcgggg tcccgaatca gcgggcccag 540
   cgcgcccgcg cggggccagc gggaggcggc gtccagtagt gcatcgatac tgcccgggcc 600
   ccattgcacg tccgggttgg ccacgatcac ccagtcatcg acccagggtt cgccggcatc 660
   gcccgccatt tcaccgagct gggcgatcgt ccgattcacc gcggttccgt acccgaggtt 720
   ggcccctgtg ggcagcagcc gcacgttggg gtagcgctgc accgcggcct gcggggtgcc 780
   gtcggtggag ccgttgtctg ccaacagcac gctgaccggc cgctcggtgg ccagcgacaa 840
   cgacgccagg aaccgctcta gatggggccc cggcgagtag gtcaccgcta ccacaccgct 900
   accac                                                             905
<210> 11
   <211> 876
   <212> DNA
   <213> Mycobacterium avium
<400> 11
   tcagtgccgc ccttctaccc gccgcccgga ctcgcgcagc gcgctgcgca ccatcagccg 60
   ggcgcgcacc gccagcgacg cgcgcagcgc ccagcgcagc ggcgcgcgca tccaaccgga 120
   ataccggtcg gccagaaaca tataggtgct gcggtggtgg gcggccaggt ggctggccgg 180
   gtcggccccg gtggagtggc ccttgtggtg cagcacctcg gccgacggca cgtagaccga 240
   cagccagccg gccttgccca gccggtcgcc gaggtcgacg tcctccatgt acatgaagta 300
   gcgctcgtcg aacccgccga cccgctcgaa tgccgagcgg cgcaccagca ggcacgaacc 360
   cgacagccag cccaccggcc gctcgctggg ctccagccgc tcctggcgat aggccgtcga 420
   ccacgggttg ttcttccaga acggcccgac gaccgcgtgc atgccgccgc gcaccaggct 480
   gggcaggtgc cgcgccgacg ggtacaccga cccgtcgggg tcgcgcacca gcgggcccag 540
   cgcgccggcc cgcggccagc gcgccgcggc gtccagcagc gcgtcgatgc tgccgggccc 600
   ccactgcacg tccgggttgg cgacgatcac ccactcgccg ccgcgatcca gctgtgccac 660
   agcacgattc accgcggtgc cgtagccgag gttggcgccg gtgtgaaaca gccgcacgtt 720
   ggggtagcgc tcgacggcgg cctgcggcgt cccgtcggtg gagccgttgt cggccagcag 780
   cacgcacacc tcgcgttcgg tggccagcga cagcgacgcc aggaagcgct ccaggtgcgg 840
   gcccggtgaa taggtcaccg tcaccaccgg cagcac                           876
<210> 12
   <211> 867
   <212> DNA
   <213> Mycobacterium avium
<400> 12
   tcagtgccgc ccttctaccc gccgcccgga ctcgcgcagc gcgctgcgca ccatcagccg   60
   ggcgcgcacc gccagcgacg cgcgcagcgc ccagcgcagc ggcgcgcgca tccaaccgga  120
   ataccggtcg gccagaaaca tataggtgct gcggtggtgg gcggccaggt ggctggccgg  180
   gtcggccccg gtggagtggc ccttgtggtg cagcacctcg gccgacggca cgtagaccga  240
   cagccagccg gccttgccca gccggtcgcc gaggtcgacg tcctccatgt acatgaagta  300
   gcgctcgtcg aacccgccga cccgctcgaa tgccgagcgg cgcaccagca ggcacgaacc  360
   cgacagccag cccaccggcc gctcgctggg ctccagccgc tcctggcggt aggccgtcga  420
   ccacgggttg ttcttccaga acggcccgac gaccgcgtgc atgccgccgc gcaccaggct  480
   gggcaggtgc cgcgccgacg ggtacaccga cccgtcgggg tcgcgcacca gcgggcccag  540
   cgcgccggcc cgcggccagc gcgccgcggc gtccagcagc gcgtcgatgc tgccgggccc  600
   ccactgcacg tccgggttgg cgacgatcac ccactcgccg ccgcgatcca gctgtgccac  660
   agcacgattc accgcggtgc cgtagccgag gttggcgccg gtgtgaaaca gccgcacgtt  720
   ggggtagcgc tcgacggcgg cctgcggcgt cccgtcggtg gagccgttgt cggccagcag  780
   cacgcacacc tcgcgttcgg tggccagcga cagcgacgcc aggaagcgct ccaggtgcgg  840
   gcccggtgaa taggtcaccg tcaccac                                      867
<210> 13
   <211> 885
   <212> DNA
   <213> Mycobacterium marinum
<400> 13
   tcagtgccgc ccttctacca gctttcggcg cgaactgcgc accatcagac gggagcgtac   60
   cgccagcgat ccccgcaaag tccagcgcaa tggggcccgc caccagccgg catggcgatc  120
   cgccaggaaa atgtaggtgc ttcggtggtg tgccgccagg tgattggccg gatcgcgtcc  180
   ggtggagtgg cctttgtggt gcagcacttc cgccgacggc acatagacgc tgagccagcc  240
   ggcctggccc agccggtcgc cgaggtccac gtcttccata tacatgaagt agcgctcgtc  300
   gaaaccgccg atctggcgga acgcggagcg gcgcaccaac aggcacgaac ccgatagcca  360
   gcccaccggc cgttcgctgg gctcgaggtg ttcctggcga taggccttgg tccagggatt  420
   gcggggccat accggcccga gcaccgcgtg cataccgccg cggaccaggc tgggcagatg  480
   gcgcgccgag gggtacaccg atccatcggg atcgcggatc agcgggccca acgccccggc  540
   ctggggccag cgctcgacgg cctcgagcag cgcgtcgatg ctgcccggac cccactgcac  600
   gtccgggttg gccacgatca gccagtcgtc tggctccggt tgttcggtga gctgggcgac  660
   ggcccggttc accgcggtgc catacccgag gttggccccg gtgtggaaga tccgcacgtt  720
   ggggtagcgc tcgacagccg cctgaggtgt tccgtcggtg gagccgttgt cggccagcag  780
   cacactcacc ggacggtcgg tggccagcga caaagacgcc aagaagcgct ccaggtgggg  840
   gcccggcgag taggtcaccg ccacgaccgg caggacgtca gtcac                  885
<210> 14
   <211> 885
   <212> DNA
   <213> Mycobacterium ulcerans
<400> 14
   tcagtgccgc ccttctacca gctttcggcg cgaactgcgc accatcagac gggagcgtac   60
   cgccagcgat ccccgcaaag tccagcgcaa tggggcccgc caccagccgg catggcgatc  120
   cgccaggaaa atgtaggtgc ttcggtggtg taccgccagg tgagtggccg gatcgcgtcc  180
   ggtggagtgg cctttgtggt gcagcacttc cgccgacggc acatagacgc tgagccagcc  240
   ggcctggccc agccggtcgc cgaggtccac gtcttccata tacatgaagt agcgctcgtc  300
   gaaaccgccg atctggcgga acgcggagcg gcgcaccaac aggcacgaac ccgatagcca  360
   gcccaccggc cgttcgctgg gctcgaggtg ttcctggcga taggccttgg tccagggatt  420
   gcggggccat accggcccga gcaccgcgtg cataccgccg cggaccaggc tgggcagatg  480
   gcgcgccgag gggtacaccg atccatcggg atcgcggatc agcgggccca acgccccggc  540
   ctggggccag cgctcgacgg cctcgagcag cgcgtcgatg ctgcccggac cccactgcac  600
   gtccgggttg gccacgatca gccagtcgtc tggctccggt tgttcggtga gttgggcgac  660
   ggcccggttc accgcggtgc cgtacccgag gttggccccg gtgtggaaga tccgcacgtt  720
   ggggtagcgc tcgacggccg cctgaggtgt tccgtcggtg gagccgttgt cggccagcag  780
   cacactcacc ggacggtcgg tggccagcga caaagacgcc aagaagcgct ccaagtgggg  840
   gcccggcgag taggtcaccg ccacgaccgg caggacgtca gtcac                  885
<210> 15
   <211> 125
   <212> DNA
   <213> Artificial Sequence
<220>
   <223> Mycobacterium tuberculosis DNA fragment
<400> 15
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 16
   <211> 125
   <212> DNA
   <213> Artificial Sequence
<220>
   <223> Mycobacterium tuberculosis DNA fragment
<400> 16
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 17
   <211> 125
   <212> DNA
   <213> Artificial Sequence
<220>
   <223> Mycobacterium tuberculosis DNA fragment
<400> 17
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 18
   <211> 125
   <212> DNA
   <213> Artificial Sequence
<220>
   <223> Mycobacterium tuberculosis DNA fragment
<400> 18
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 19
   <211> 125
   <212> DNA
   <213> Artificial Sequence
<220>
   <223> Mycobacterium tuberculosis DNA fragment
<400> 19
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 20
   <211> 125
   <212> DNA
   <213> Artificial Sequence
<220>
   <223> Mycobacterium tuberculosis DNA fragment
<400> 20
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 21
   <211> 125
   <212> DNA
   <213> Artificial Sequence
<220>
   <223> Mycobacterium tuberculosis DNA fragment
<400> 21
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 22
   <211> 125
   <212> DNA
   <213> Artificial Sequence
<220>
   <223> Mycobacterium tuberculosis DNA fragment
<400> 22
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 23
   <211> 125
   <212> DNA
   <213> Artificial Sequence
<220>
   <223> Mycobacterium tuberculosis DNA fragment
<400> 23
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 24
   <211> 125
   <212> DNA
   <213> Artificial Sequence
<220>
   <223> Mycobacterium tuberculosis DNA fragment
<400> 24
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 25
   <211> 125
   <212> DNA
   <213> Artificial Sequence
<220>
   <223> Mycobacterium tuberculosis DNA fragment
<400> 25
   taccagcttc agtttccgtc tgcgggacct gcgcagtgaa ctgcgcacca tgaggtggga  60
   acgcagcgcc agtgatcccc gcagggtcca gcgcagcgga gcccgccacc aaccagaatg  120
   tcggt                                                              125
<210> 26
   <211> 125
   <212> DNA
   <213> Artificial Sequence
<220>
   <223> Mycobacterium tuberculosis DNA fragment
<400> 26
   taccagcttc agtttccgtc tgcgggacct gcgcagtgaa ctgcgcacca tgaggtggga  60
   acgcagcgcc agtgatcccc gcagggtcca gcgcagcgga gcccgccacc aaccagaatg  120
   tcggt                                                              125
<210> 27
   <211> 125
   <212> DNA
   <213> Artificial Sequence
<220>
   <223> Mycobacterium tuberculosis DNA fragment
<400> 27
   taccagcttc agtttccgtc tgcgggacct gcgcagtgaa ctgcgcacca tgaggtggga  60
   acgcagcgcc agtgatcccc gcagggtcca gcgcagcgga gcccgccacc aaccagaatg  120
   tcggt                                                              125
<210> 28
   <211> 125
   <212> DNA
   <213> Artificial Sequence
<220>
   <223> Mycobacterium tuberculosis DNA fragment
<400> 28
   taccagcttc agtttccgtc tgcgggacct gcgcagtgaa ctgcgcacca tgaggtggga  60
   acgcagcgcc agtgatcccc gcagggtcca gcgcagcgga gcccgccacc aaccagaatg  120
   tcggt                                                              125
<210> 29
   <211> 125
   <212> DNA
   <213> Artificial Sequence
<220>
   <223> Mycobacterium tuberculosis DNA fragment
<400> 29
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 30
   <211> 125
   <212> DNA
   <213> Artificial Sequence
<220>
   <223> Mycobacterium tuberculosis DNA fragment
<400> 30
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 31
   <211> 125
   <212> DNA
   <213> Artificial Sequence
<220>
   <223> Mycobacterium tuberculosis DNA fragment
<400> 31
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 32
   <211> 125
   <212> DNA
   <213> Artificial Sequence
<220>
   <223> Mycobacterium tuberculosis DNA fragment
<400> 32
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 33
   <211> 125
   <212> DNA
   <213> Artificial Sequence
<220>
   <223> Mycobacterium tuberculosis DNA fragment
<400> 33
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 34
   <211> 125
   <212> DNA
   <213> Artificial Sequence
<220>
   <223> Mycobacterium tuberculosis DNA fragment
<400> 34
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 35
   <211> 125
   <212> DNA
   <213> Artificial Sequence
<220>
   <223> Mycobacterium tuberculosis DNA fragment
<400> 35
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 36
   <211> 125
   <212> DNA
   <213> Artificial Sequence
<220>
   <223> Mycobacterium tuberculosis DNA fragment
<400> 36
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 37
   <211> 125
   <212> DNA
   <213> Artificial Sequence
<220>
   <223> Mycobacterium tuberculosis DNA fragment
<400> 37
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 38
   <211> 125
   <212> DNA
   <213> Artificial Sequence
<220>
   <223> Mycobacterium tuberculosis DNA fragment
<400> 38
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 39
   <211> 125
   <212> DNA
   <213> Artificial Sequence
<220>
   <223> Mycobacterium tuberculosis DNA fragment
<400> 39
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 40
   <211> 125
   <212> DNA
   <213> Artificial Sequence
<220>
   <223> Mycobacterium canettii DNA fragment
<400> 40
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 41
   <211> 125
   <212> DNA
   <213> Artificial Sequence
<220>
   <223> Mycobacterium canettii DNA fragment
<400> 41
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 42
   <211> 125
   <212> DNA
   <213> Artificial Sequence
<220>
   <223> Mycobacterium canettii DNA fragment
<400> 42
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 43
   <211> 125
   <212> DNA
   <213> Artificial Sequence
<220>
   <223> Mycobacterium canettii DNA fragment
<400> 43
   taccagcttc agtttccgtc tgcgggacct gcgcagtgaa ctgcgcacca tgaggtggga   60
   acgcagcgcc agtgatcccc gcagggtcca gcgcagcgga gcccgccacc aaccagaatg  120
   tcggt                                                              125
<210> 44
   <211> 125
   <212> DNA
   <213> Artificial Sequence
<220>
   <223> Mycobacterium canettii DNA fragment
<400> 44
   taccagcttc agtttccgtc tgcgggacct gcgcagtgaa ctgcgcacca tgaggtggga 60
   acgcagcgcc agtgatcccc gcagggtcca gcgcagcgga gcccgccacc aaccagaatg 120
   tcggt 125
<210> 45
   <211> 113
   <212> DNA
   <213> Artificial Sequence
<220>
   <223> Mycobacterium pinnipedii DNA fragment
<400> 45
   taccagcttc agtttccgtc tgcgggacct gcgcaccatg aggtgggaac gcagcgccag 60
   tgatccccgc agggtccagc gcagcggagc ccgccaccaa ccagaatgtc ggt 113
<210> 46
   <211> 113
   <212> DNA
   <213> Artificial Sequence
<220>
   <223> Mycobacterium caprae DNA fragment
<400> 46
   taccagcttc agtttccgtc tgcgggacct gcgcaccatg aggtgggaac gcagcgccag 60
   tgatccccgc agggtccagc gcagcggagc ccgccaccaa ccagaatgtc ggt 113
<210> 47
   <211> 1962
   <212> DNA
   <213> Mycobacterium tuberculosis
<400> 47
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtggcgca tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                  1962
<210> 48
   <211> 1962
   <212> DNA
   <213> Mycobacterium tuberculosis
<400> 48
   tcacttcttg cctttgtccc cggcggcatc ggtggacaat gccgcgacga aagcctcctg   60
   tggcacctcg acgcgcccga tggtcttcat ccgcttcttg ccttccttct gcttctccag   120
   cagcttgcgt ttgcgcgtga tgtcgccgcc gtagcacttg gacaacacgt ccttgcggat   180
   cgcgcggatg ttttcgcggg caatgatttt cgatccgatg gcggcctgca ccggcacctc   240
   gaactgctgg cgcgggatca gctccttgag tttggtggtc atcttgttgc cgtaggcata   300
   cgccgtgtcc ttgtgcacga tcgcgctgaa cgcatccacc gcctcgccct gcagcaggat   360
   gtcgaccttg accagcgcgg cctcctgttc gccggcctcc tcgtagtcga ggctggcata   420
   gccgcgggtg cgcgatttca gtgcgtcgaa gaagtcgaag atgatctcgc cgagcggcat   480
   ggtgtagcgc agttccaccc gctcggggga gagatagtcc atgccgccca actcgccgcg   540
   gcgcgactgg cacagctcca tgatggtgcc gatgaactcg ctgggcgcga tgatggtggt   600
   cttgacgacg ggctcgtaga ccgtgcggat cttgccctcc ggccagtccg acggattggt   660
   cacccggatt tcggtgccgt cgtctttgtg cacccgatac accacattgg gtgaggtcga   720
   gatcaggtcc aggccgaact cgcgctcaag gcgctcacgg gtgatctcca tgtgcagcag   780
   gcccaagaaa ccgcaccgga acccaaaacc cagcgccacc gaggtttccg gctcataggt   840
   caaggccgcg tcgttgagct gcagcttgtc cagggcgtcg cgcaggttcg ggtagtccga   900
   accgtcgacc ggatacaacc ccgagtagac catcggtttg ggctcacggt agccggtcaa   960
   cgcttcggcg gcagccccgc gggcccggga gaggctggtc acggtgtcgc ccaccttgga   1020
   ctggcggacg tccttgacgc cggtgatcag gtaacccacc tcgccgacac cgaggccctc   1080
   acacggtttc ggctcgggtg agacgatgcc gacctcaagc agctcgtggg tggcgccggt   1140
   ggacatcatc atgatgcgct cacgggggct gatcttgccg tcgacgacgc ggacgtaggt   1200
   caccactccg cggtagatgt cgtaaacgga gtcgaaaatc attgcgcggg taggtgcctc   1260
   ggcgtcgccc tgagggggcg gcacctgtcg gaccacctcg tcgagcaggt cggacacgcc   1320
   ttcgccggtt ttgccggaca cccgcaacac ctcggccggc tcgcagccga tgatgtgtgc   1380
   catctcggcg gcgtaacggt ccgggtcggc cgcgggcagg tcgatcttgt tgagcaccgg   1440
   gatgatgtgc aggtcgcggt ccaacgccag gtagaggttc gccagcgtct gcgcctcgat   1500
   gccttgcgcg gcatcgacca acagcaccgc accctcgcaa gcctccagcg cacgcgagac   1560
   ttcgtaggtg aagtcgacat ggcccggggt gtcgatcaga tgcagcacgt agtcggtctt   1620
   gtcgacccgc cagggtagcc gcacattctg ggccttgatg gtgatgccgc gttcccgctc   1680
   gatgtccatc cgatccaagt actgggcccg catagagcgt tcgtcgacca cgccggtgag   1740
   ctgcagcatc cggtcggcca acgttgactt gccgtggtcg atgtgggcga tgatgcaaaa   1800
   gttcctaatc tgcgccggcg cagtgaaggt tttgtcggcg aaactgctga tgggaatctc   1860
   ctggagcggg ggttgacggg tatccagggt atccgcgtcg ggcagctgcg acccaatcgc   1920
   gctcggtcga tcgcgtctat gctgcgagca tggcgtccgc ac                      1962
<210> 49
   <211> 1962
   <212> DNA
   <213> Mycobacterium tuberculosis
<400> 49
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                   1962
<210> 50
   <211> 1962
   <212> DNA
   <213> Mycobacterium tuberculosis
<400> 50
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                    1962
<210> 51
   <211> 1962
   <212> DNA
   <213> Mycobacterium tuberculosis
<400> 51
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc 240
   gaactgctgg cgcgggatca gctccttgag tttggtggtc atcttgttgc cgtaggcata 300
   cgccgtgtcc ttgtgcacga tcgcgctgaa cgcatccacc gcctcgccct gcagcaggat 360
   gtcgaccttg accagcgcgg cctcctgttc gccggcctcc tcgtagtcga ggctggcata 420
   gccgcgggtg cgcgatttca gtgcgtcgaa gaagtcgaag atgatctcgc cgagcggcat 480
   ggtgtagcgc agttccaccc gctcggggga gagatagtcc atgccgccca actcgccgcg 540
   gcgcgactgg cacagctcca tgatggtgcc gatgaactcg ctgggcgcga tgatggtggt 600
   cttgacgacg ggctcgtaga ccgtgcggat cttgccctcc ggccagtccg acggattggt 660
   cacccggatt tcggtgccgt cgtctttgtg cacccgatac accacattgg gtgaggtcga 720
   gatcaggtcc aggccgaact cgcgctcaag gcgctcacgg gtgatctcca tgtgcagcag 780
   gcccaagaaa ccgcaccgga acccaaaacc cagcgccacc gaggtttccg gctcataggt 840
   caaggccgcg tcgttgagct gcagcttgtc cagggcgtcg cgcaggttcg ggtagtccga 900
   accgtcgacc ggatacaacc ccgagtagac catcggtttg ggctcacggt agccggtcaa 960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac 1962
<210> 52
   <211> 1962
   <212> DNA
   <213> Mycobacterium tuberculosis
<400> 52
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg   540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                   1962
<210> 53
   <211> 1971
   <212> DNA
   <213> Mycobacterium tuberculosis
<400> 53
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc acggaagtca c          1971
<210> 54
   <211> 1962
   <212> DNA
   <213> Mycobacterium bovis
<400> 54
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga  1020
   ctggcggacg tccttgacgc cggtgatcag gtaacccacc tcgccgacac cgaggccctc  1080
   acacggtttc ggctcgggtg agacgatgcc gacctcaagc agctcgtggg tggcgccggt  1140
   ggacatcatc atgatgcgct cacgggggct gatcttgccg tcgacgacgc ggacgtaggt  1200
   caccactccg cggtagatgt cgtaaacgga gtcgaaaatc attgcgcggg taggtgcctc  1260
   ggcgtcgccc tgagggggcg gcacctgtcg gaccacctcg tcgagcaggt cggacacgcc  1320
   ttcgccggtt ttgccggaca cccgcaacac ctcggccggc tcgcagccga tgatgtgtgc  1380
   catctcggcg gcgtaacggt ccgggtcggc cgcgggcagg tcgatcttgt tgagcaccgg  1440
   gatgatgtgc aggtcgcggt ccaacgccag gtagaggttc gccagcgtct gcgcctcgat  1500
   gccttgcgcg gcatcgacca acagcaccgc accctcgcaa gcctccagcg cacgcgagac  1560
   ttcgtaggtg aagtcgacat ggcccggggt gtcgatcaga tgcagcacgt agtcggtctt  1620
   gtcgacccgc cagggtagcc gcacattctg ggccttgatg gtgatgccgc gttcccgctc  1680
   gatgtccatc cgatccaagt actgggcccg catagagcgt tcgtcgacca ctccggtgag  1740
   ctgcagcatc cggtcggcca acgttgactt gccgtggtcg atgtgggcga tgatgcaaaa  1800
   gttcctaatc tgcgccggcg cagtgaaggt tttgtcggcg aaactgctga tgggaatctc  1860
   ctggagcggg ggttgacggg tatccagggt atccgcgtcg ggcagctgcg acccaatcgc  1920
   gctcggtcga tcgcgtctat gctgcgagca tggcgtccgc ac                     1962
<210> 55
   <211> 1962
   <212> DNA
   <213> Mycobacterium bovis BCG
<400> 55
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca ctccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                    1962
<210> 56
   <211> 1962
   <212> DNA
   <213> Mycobacterium bovis BCG
<400> 56
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgagggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcgggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca ctccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                   1962
<210> 57
   <211> 1962
   <212> DNA
   <213> Mycobacterium microti
<400> 57
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtgtcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccgatac accacattgg gtgaggtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggcg gcagccccgc gggcccggga gaggctggtc acggtgtcgc ccaccttgga  1020
   ctggcggacg tccttgacgc cggtgatcag gtaacccacc tcgccgacac cgaggccctc  1080
   acacggtttc ggctcgggtg agacgatgcc gacctcaagc agctcgtggg tggcgccggt  1140
   ggacatcatc atgatgcgct cacgggggct gatcttgccg tcgacgacgc ggacgtaggt  1200
   caccactccg cggtagatgt cgtaaacgga gtcgaaaatc attgcgcggg taggtgcctc  1260
   ggcgtcgccc tgagggggcg gcacctgtcg gaccacctcg tcgagcaggt cggacacgcc  1320
   ttcgccggtt ttgccggaca cccgcaacac ctcggccggc tcgcagccga tgatgtgtgc  1380
   catctcggcg gcgtaacggt ccgggtcggc cgcgggcagg tcgatcttgt tgagcaccgg  1440
   gatgatgtgc aggtcgcggt ccaacgccag gtagaggttc gccagcgtct gcgcctcgat  1500
   gccttgcgcg gcatcgacca acagcaccgc accctcgcaa gcctccagcg cacgcgagac  1560
   ttcgtaggtg aagtcgacat ggcccggggt gtcgatcaga tgcagcacgt agtcggtctt  1620
   gtcgacccgc cagggtagcc gcacattctg ggccttgatg gtgatgccgc gttcccgctc  1680
   gatgtccatc cgatccaagt actgggcccg catagagcgt tcgtcgacca ctccggtgag  1740
   ctgcagcatc cggtcggcca acgttgactt gccgtggtcg atgtgggcga tgatgcaaaa  1800
   gttcctaatc tgcgccggcg cagtgaaggt tttgtcggcg aaactgctga tgggaatctc  1860
   ctggagcggg ggttgacggg tatccagggt atccgcgtcg ggcagctgcg acccaatcgc  1920
   gctcggtcga tcgcgtctat gctgcgagca tggcgtccgc ac                      1962
<210> 58
   <211> 1962
   <212> DNA
   <213> Mycobacterium canettii
<400> 58
   tcacttcttg cctttgtccc cggcggcatc ggtggacaat gccgcgacga aagcctcctg   60
   tggcacctcg acgcgcccga tggtcttcat ccgcttcttg ccttccttct gcttctccag  120
   cagcttgcgt ttgcgcgtga tgtcgccgcc gtagcacttg gacaacacgt ccttgcggat  180
   cgcgcggatg ttttcgcggg caatgatttt cgatccgatg gcggcctgca ccggcacctc  240
   gaactgctgg cgcgggatca gctccttgag tttggtggtc atcttgttgc cgtaggcata  300
   cgccgtatcc ttgtgcacga tcgcgctgaa cgcatccacc gcctcgccct gcagcaggat  360
   gtcgaccttg accagcgcgg cctcctgttc gccggcctcc tcgtagtcga ggctggcata  420
   gccgcgggtg cgcgatttca gtgcgtcgaa gaagtcgaag atgatctcgc cgagcggcat  480
   ggtgtagcgc agttccaccc gctcggggga gagatagtcc atgccgccca actcgccgcg  540
   gcgcgactgg cacagctcca tgatggtgcc gatgaactcg ctgggcgcga tgatggtggt  600
   cttgacgacg ggctcgtaga ccgtgcggat cttgccctcc ggccagtccg acggattggt  660
   cacccggatt tcggtgccgt cgtctttgtg cacccggtac accacattgg gtgaagtcga  720
   gatcaggtcc aggccgaact cgcgctcaag gcgctcacgg gtgatctcca tgtgcagcag  780
   gcccaagaaa ccgcaccgga acccaaaacc cagcgccacc gaggtttccg gctcataggt  840
   caaggccgcg tcgttgagct gcagcttgtc cagggcgtcg cgcaggttcg ggtagtccga  900
   accgtcgacc ggatacaacc ccgagtagac catcggtttg ggctcacggt agccggtcaa  960
   cgcttcggct gcagccccgc gggcccggga gaggctggtc acggtgtcgc ccaccttgga 1020
   ctggcggacg tccttgacgc cggtgatcag gtaacccacc tcgccgacac cgaggccctc 1080
   acacggtttc ggctcgggtg agacgatgcc gacctcaagc agctcgtggg tggcgccggt 1140
   ggacatcatc atgatgcgct cacgggggct gatcttgccg tcgacgacgc ggacgtaggt 1200
   caccactccg cggtagatgt cgtaaacgga gtcgaaaatc attgcgcggg taggtgcctc 1260
   ggcgtcgccc tgcgggggcg gcacctgtcg gaccacctcg tcgagcaggt cggacacgcc 1320
   ttcgccggtt ttgccggaca cccgcaacac ctcggccggc tcgcagccga tgatgtgtgc 1380
   catctcggcg gcgtaacggt ccgggtcggc cgcaggcagg tcgatcttgt tgagcaccgg 1440
   gatgatgtgc aggtcgcggt ccaacgccag gtagaggttc gccagcgtct gcgcctcgat 1500
   gccttgcgcg gcatcgacca acagcaccgc accctcgcaa gcctccagcg cacgcgagac 1560
   ttcgtaggtg aagtcgacat ggcccggggt gtcgatcaga tgcagcacgt agtcggtctt 1620
   gtcgacccgc cagggtagcc gcacattctg ggccttgatg gtgatgccgc gttcccgctc 1680
   gatgtccatc cgatccaagt actgggcccg catagagcgt tcgtcgacca cgccggtgag 1740
   ctgcagcatc cggtcggcca acgttgactt gccgtggtcg atgtgggcga tgatgcaaaa 1800
   gttcctaatc tgcgccggcg cagtgaaggt tttgtcggcg aaactgctga tgggaatctc 1860
   ctggagcggg ggttgacggg tatccagggt atccgcgtcg ggcagctgcg acccaatcgc 1920
   gctcggtcga tcgcgtctat gctgcgagca tggcgtccgc ac                    1962
<210> 59
   <211> 627
   <212> DNA
   <213> Mycobacterium africanum
<400> 59
   catccaccgc ctcgccctgc agcaggatgt cgaccttgac cagcgcggca tcctgttcgc   60
   cggcctccta gtagtcgagg ctggcatagc cgcgggtgcg cgatttcagt gcatcgaaga  120
   agtcgaagat gatctcgccg agcggcatgg tgtagcgcag ttccacccgc tcggcggaga  180
   gatagtccat gccgcccaac tcgccgcggc gcgactggca cagctccatg atggtgccga  240
   tgaactcgct gggcgcgatg atggtggtct caacgacggg ctcgtagacc ttgcggatct  300
   tgccctccgg accagtccga cggattggtc acccggattt cggtgccgtc gtcttggtgc  360
   acccgataca ccacattggg tgaggtcgag atcaggtcca ggccgaactc gcgctcaagg  420
   cgctcacggg tgatctccat gtgcatgcag gcccaagaaa ccgcaccgga acccaaaacc  480
   cagcgccacc gaggtttccg gctcataggt caaggccgcg tcgttgagct gcagcttgtc  540
   cagggcgtcg cgcaggttcg ggtagtccga accgtcgacc ggatacaacc ccgagtagac  600
   catcggtttg ggctcacggt agccggg                                      627
<210> 60
   <211> 1983
   <212> DNA
   <213> Mycobacterium avium
<400> 60
   ttacttcttg gatttgtcgc ccgcggcgtc ggcggacagc gcggcgacga acgcctcctg   60
   cggcacgtcg acccggccga tggtcttcat ccgcttcttg ccctccttct gcttttccag  120
   aagtttgcgc ttgcgggtga tgtcaccgcc gtaacacttg gacagcacgt ccttgcggat  180
   ggcccgaatg ttctcgcggg caatgatttt cgagccgatc gcggcctgga cgggcacctc  240
   gaactgctgg cgcgggatca gctccttgag cttggtggtc atcttgttgc cgtacgcgaa  300
   cgccgcatcc ttgtgcacga tcgcgctgaa cgcgtcgacg gcctccccct gcaacaggat  360
   gtccaccttg accagctggg cctcctgctc gccggcctcc tcgtagtcca ggctggcgta  420
   gccgcgggtc cgcgacttca gcgagtcgaa gaagtcgaag atgatctcgc ccaacggcat  480
   ggtgtagcgc agctcaaccc gttccggcga caggtaatcc atgccgccca gctcgccgcg  540
   gcgggactgg cacagctcca tgatggtgcc gatgaattcg ctgggcgcga tgatggtggt  600
   cttgaccacc ggctcgtaca ccgtgcgcac cttgccctcg ggccagtccg acgggttggt  660
   caccacgatc tcggtgccgt cctctttgat cacccggtac accacgttgg gcgacgtcga  720
   gatcaggtcg aggtcgaact cgcgctccag gcgctcgcgg gtgatctcca tgtgcagcaa  780
   acccaaaaag ccacaacgga atccgcagcc cagcgccacc gatgtctccg gttcgtaggt  840
   gagcgcggcg tcgttgagcc gcagccggtc cagcgcgtcg cgcagatccg gatagtccga  900
   gccgtccacc ggatacagac ccgaatagac catcggcttg ggttcgcggt atccggtcag  960
   cgcctcttgc gcgccgtggc gcgcgctggt gacggtgtcg cccactttgg actggcggac 1020
   gtccttcacc ccggtgatca ggtagcccac ctcgccgacg ccaaggccgt cgctggcctt 1080
   cggctcgggt gagacgatgc cgacctcgag cagttcgtgg gtggcgccgg tggacatcat 1140
   cgcgatgcgt tcgcgcgggg tgatcttgcc gtcgaccacc cgcacgtagg tcaccacgcc 1200
   gcggtagatg tcgtagaccg agtcgaagat catcgcgcgc agcggcgcat cggcctgccc 1260
   ctgcggcggc ggcacctggc gcaccacctc gtcgagcagc cgcgccacgc cctccccggt 1320
   tttgccggac acccacagca cgtcgtcggg ttcgcacccg atgatgtggg cgagctcgcc 1380
   ggcgtagcga tccgggtcgg cggccggcag gtcgatcttg ttgaggaccg ggatgatggt 1440
   cagatcgcgg tccagcgcca ggtagaggtt ggccagcgtc tgggcctcga tgccctgcgc 1500
   ggcgtcgacc agcagcacgg caccttcgca ggcctccagt gcgcgcgaca cctcgtaggt 1560
   gaagtcgacg tggcccgggg tgtcgatcag gtgcaggaca aattctttgc cggcgtcctc 1620
   gccgccggag acctgccagg gcagccgcac gttctgcgcc ttgatggtga tgccgcgctc 1680
   ccgctcgatg tccatccggt ccaggtactg ggcgcgcatc gaccgctcgt cgacgacgcc 1740
   ggtgagctgc agcatccggt cggccagcgt cgacttgccg tgatcgatgt gggcgatgat 1800
   gcagaagttg cgaatctgcg ccggcgcggt gaaggtcttg tcggcgaaac tgctgatggg 1860
   tatctcctgg tccgggcctg ctagacggcg gttcgcaagt gtgtccagcg tatcggcgcg 1920
   gccggactgc ggcacaatcg gcgcgtctat gctgcgaata tggcgtccgg ccggaagtcg 1980
   cag                                                               1983
<210> 61
   <211> 1941
   <212> DNA
   <213> Mycobacterium leprae
<400> 61
   ttatttcttg tccttgtctc ctgcggcatc ggcggacaac gcggcgacaa acgcttcctg   60
   cggcacctcg acccgcccaa tggtcttcat ccgtttcttg ccttccttct gcttttccag  120
   aagcttacgt ttgcgggtga tatcgccgcc ataacatttc gacagcacat ccttgcgtat  180
   cgccctaata ttttcgcgcg caatgacttt cgatccaata gccgcctgta ctggcacctc  240
   aaactgctga cgtgggatca gttctttgag cttgttggtc atcttgttgc cataggcaga  300
   ggctgaatcc ttgtgcacaa tcgcgctgaa tgcgtcgacg gcctcgcctt gcagcaggat  360
   gtcaaccttg accagttggg cctcctgctc gccagcctcc tcatagtcga ggctagcgta  420
   gccccgggtg cgtgacttca gcgaatcgaa gaaatcgaag atgatttccc cgagcggcat  480
   aatgtagcgt aactcgactc gctcaggtga aagatagtcc atgccaccta attcgccacg  540
   gcgcgactgg cacagctcca tgatcgttcc gatgaactcg ctgggcgcaa tgatggtgat  600
   cttcaccact ggctcgtaca ccgttcggat cttgccctcc ggccagtctg acgggttggt  660
   caccacaatc tcggtgttat cttctgtcac cacacggtat acgacgttgg gcgacgtgga  720
   gatcaggtcc aggtcgaact cgcgctctaa gcgttcgcgg gttatatcta tgtgcagcaa  780
   accgaggaag ccgcaccggt acccaacgcc cagcgccacc gatgtttccg gctcgtaggt  840
   cagcgccgcg tcgttgagct gtaacttacc tagagcgtca cgcaaactcg ggtagtccga  900
   actgtcgacg ggatacagcc cggagtacac catgggcttg ggttctcggt agccagttaa  960
   cggttcagtg gcaccataac gaaccgtcgt tacagtgtcg ccgactttgg attggcggac 1020
   gtctttaacc ccagtaatca ggtagcccac ctcccccacg cccaggcccg cgctggcctt 1080
   cggttcaggc gacacgatgc cgacctcgag cagttcgtac gtcgcaccgg tggacatcat 1140
   cgcgatgcgc tcacgcgggc tgatcttgcc gtcgaccaca cggacgtagg tgaccacgcc 1200
   tcggtagatg tcgtagacgg agtcgaagat catcgcgcgg gtaggcgcat cagggtcacc 1260
   ttgcggatgc ggcacccgac ggaccacctc gtcaagaagg tcagaaaccc cctcgccggt 1320
   tttgccggac acccgaagca catcgcctga ctcataacca atgatgtggg cgatctcagc 1380
   ggcgtaacgg tccggatcgg cagccggcag gtcgattttg tttagcaccg gaataatcgt 1440
   caagtcacgc tccagagcga gatagagatt ggccaaggtc tgagcttcga tgccctggac 1500
   ggcgtctacc agcagcaccg caccctcaca ggcttccaat gctcgcgata cctcgtaggt 1560
   gaagtccaca tggccggggg tgtcgatcaa gtgcaacaca taattctcag tcgtcccacc 1620
   agctgtgaca ctccaagaca gccgcacgtt ctgcgcttta atcgtgattc cgcgctcacg 1680
   ttcgatgtcc atccggtcca ggtactgggc acgcatcgac cgctcatcga cgacaccagt 1740
   cagctgaagc atccggtccg ccagcgtgga tttgccgtga tcaatatgag cgattatgca 1800
   gaagttccta atctgcgccg gcgcggtaaa ggtcttgtca gcgaaactgc tgatgggaat 1860
   ctcctgggct ccagttacta gagaatgttt gaacggcgat tcgccggtgt ccggcttatc 1920
   cacgcgaagt gaccaagaca c                                           1941
<210> 62
   <211> 1740
   <212> DNA
   <213> Mycobacterium ulcerans
<400> 62
   ctacttcttg cccttatccc ccgcggcgtc ggtggacagt gccgcgacaa acgcctcctg   60
   cggcacctcg acccggccga tggacttcat ccgcttcttg ccctctttct gcttttccag  120
   cagcttgcgt ttgcgggtga tgtcaccgcc gtagcacttc gacaacacat ccttgaggat  180
   cgcgcggatg ttctcgcgcg cgatgatctt ggaaccgatg gccgcctgca ccggcacctc  240
   gaactgctga cgcggaatca gctccttgag cttggtggtc atcttgttgc cgtaggcgaa  300
   cgccgaatcc ttgtggacga tagcgctgaa cgcatcgacg gcctcgcctt gcagcaggat  360
   gtcgaccttg accagttggg cttcctgctc gccggactcc tcgtaatcga gactggcgta  420
   gccacgggtc cgcgatatga gcgagtcgaa gaagtcaaag atgatctccc ccaacggcat  480
   tgtgtatcgc agttccaccc gttcgggcga caaatagtcc atgcccccca gctcgccgcg  540
   ccgcgactgg cacagctcca tgatggtgcc gatgaattcg ctgggcgcga tgatggtggt  600
   cttcaccacc ggctcgtaga cggtgcggat cttgccctcg ggccagtccg acgggttggt  660
   cacctgcatt tcggtgccgt cgtccttgat gacgcggtac acgacgttgg gcgaggtcga   720
   gatcaggtcg aggtcgaact cgcgctccag acgttcccga ctgatctcca tgtgcagcag   780
   gcccaggaag ccgcaccgga atccgaaacc cagcgccacc gacgtttcgg gctcataggt   840
   cagggccgcg tcgttgagct gcagcttgtc cagggcgtcc cgcaggttcg gatagtccga   900
   tccgtcaacg ggatacagtc ccgaatacac catcggcttg ggttcgcggt agccggtcag   960
   tgcctcggtg gcaccttttc gggcggtcgt gacggtgtcg ccgaccttgg actgccacac  1020
   gtccttgacc ccggtgataa gataacccac ctcgccgaca ccaaggccgt cgctggcctt  1080
   gggttcgggt gagacgatgc cgacctcgag cagttcgtgg gtggcgccgg tggacatcat  1140
   ggcgatgcgc tcgcgggggg tgatcttgcc gtcgacgacg cggacgtagg tgaccacacc  1200
   gcggtagatg tcatagacgg agtcgaagat cattgcgcga gtgggtgcgt cggcatcgcc  1260
   ctgcggtggc ggcacctcgc gcaccacgtg gtcgagcagg tctgcgacgc cttccccggt  1320
   tttgccggaa acccgcagca cgtcgccggg ctcgcagccg atgatgtgag caatctcgcc  1380
   cgcgtagcgg tccgggtcgg cggccggcag gtcgatcttg ttcagcaccg gaatgatgtg  1440
   caggtcgcgg tccagtgcca ggtagaggtt ggccagcgtc tgggcctcga tgccctgggc  1500
   ggcgtcaacc agcagcaccg cgccctcgca ggcctccagc gcacgtgaca cctcgtaggt  1560
   gaagtcgacg tgtcctggcg tgtcgatgag atgcaggacg tactcggttc catcgagctg  1620
   ccagggcagc cgcacattct gcgccttgat ggtgatcccg cgttcgcgtt cgatatccat  1680
   ccggtccagg tactgggccc gcatcgagcg ctcgtcaacg accccggtca actgcagcat  1740
<210> 63
   <211> 1935
   <212> DNA
   <213> Mycobacterium avium
<400> 63
   ttacttcttg gatttgtcgc ccgcggcgtc ggcggacagc gccgcgacga acgcctcctg    60
   cggcacgtcg acccggccga tggtcttcat ccgcttcttg ccctccttct gcttttccag   120
   aagtttgcgc ttacgggtga tgtcaccgcc gtaacacttg gacagcacgt ccttgcggat   180
   ggcccgaatg ttctcgcggg caatgatttt cgagccgatc gcggcctgga cgggcacctc   240
   gaactgctga cgggggatca gctccttgag cttggtggtc atcttgttgc cgtaggcgaa   300
   cgccgcatcc ttgtgcacga tcgcgctgaa cgcgtcgacg gcctccccct gcaacaggat   360
   gtccaccttg accagctggg cctcctgctc gccggcctcc tcgtagtcca ggctggcgta   420
   gccgcgggtc cgcgacttca gcgagtcgaa gaagtcgaag atgatctcgc ccaacggcat   480
   ggtgtagcgc agctcgaccc gttccggcga caggtaatcc atgccgccca gctcgccgcg   540
   gcgggactgg cacagctcca tgatggtgcc gatgaattcg ctgggcgcga tgatggtggt   600
   cttgaccacc ggctcgtaca ccgtgcgcac cttgccctcg ggccagtccg acgggttggt   660
   caccacgatc tcggtgccgt cctctttgat cacccggtac accacgttgg gcgacgtcga   720
   gatcaggtcg aggtcgaact cgcgctccag gcgctcgcgg gtgatctcca tgtgcagcaa   780
   acccaaaaag ccgcaacgga atccgaagcc cagcgccacc gacgtctccg gttcgtaggt   840
   gagcgcggcg tcgttgagcc gcagccggtc cagcgcgtcg cgcagatccg gatagtccga   900
   gccgtccacc ggatacagac ccgaatagac catcggcttg ggttcgcgat atccggtcag   960
   cgcctcttgc gcgccgtggc gcgcgctggt cacggtgtcg cccactttgg actggcggac  1020
   gtccttcacc ccggtgatca ggtagcccac ctcgccgacg cccaggccgt cgctggcctt  1080
   cggctcgggt gagacgatgc cgacttcgag cagttcgtgg gtggcgccgg tggacatcat  1140
   cgcgatgcgt tcgcgcgggg tgatcttgcc gtcgaccacc cgcacgtagg tcaccacgcc  1200
   gcggtagatg tcgtagaccg agtcgaagat catcgcgcgc agcggcgcat cggcctgccc  1260
   ctgcggcggc ggcacctggc gcaccacctc gtcgagcagc cgcgccacgc cctccccggt  1320
   tttgccggac acccgcagca cgtcgtcggg ttcgcacccg atgatgtggg cgagctcgcc  1380
   ggcgtaccgg tccgggtcgg cggccggcag gtcgatcttg ttgaggaccg ggatgatggt  1440
   cagatcgcgg tccagcgcca ggtagaggtt ggccagcgtc tgggcctcga tgccctgcgc  1500
   ggcgtcgacc agcagcacgg caccttcgca ggcctccagt gcgcgcgaca cctcgtaggt  1560
   gaagtcgacg tggcccgggg tgtcgatcag gtgcaggaca aattctttgc cggcgtcctc  1620
   gccgccggag acctgccagg gcagccgcac gttctgcgcc ttgatggtga tgccgcgctc  1680
   ccgctcgatg tccatccggt ccaggtactg ggcgcgcatc gaccgctcgt cgacgacgcc  1740
   ggtgagctgc agcatccggt cggccagcgt cgacttgccg tgatcgatgt gggcgatgat  1800
   gcagaagttg cgaatctgcg ccggcgcggt gaaggtcttg tcggcgaaac tgctgatggg  1860
   tatctcctgg tccgggcctg ctagacggcg gttcgcaagt gtgtccagcg tatcggcgcg  1920
   gccggactgc ggcac                                                   1935
<210> 64
   <211> 1959
   <212> DNA
   <213> Mycobacterium vanbaalenii
<400> 64
   tcatttcttc ggcttgtccg cggtggattc ggtggacagc gcggcgacga aggcctcctg   60
   cgggacgtcg acccggccga tggtcttcat ccgcttcttg ccttccttct gcttttccag  120
   cagcttgcgc ttacgggtga tgtcaccgcc gtagcacttg gacagcacat ccttgcggat  180
   cgcccgaatg ttctcgcggg caatgattct cgagccgacg gcggcctgca cgggcacctc  240
   gaactgctgg cgcgggatga gctccttgag cttggtggtc atcttgttgc cgtaggccga  300
   ggccccgtcc ttgtgcacga tcgccgagaa cgcgtcgacg gcctcgccct gcagcaggat  360
   gtcgaccttg accaggtcgg cctcctgctc gcctgcctcc tcgtagtcca ggctggcgta  420
   gccgcgggtg cgcgatttca gcgagtcgaa gaagtcgaag atgatctcgc ccagcggcat  480
   ggtgtagcgc agctcgacgc gctcgggcga caggtagtcc attccgccga gttcaccgcg  540
   ccgcgactgg cacagctcca tgatcgtgcc gatgaactcg ctgggcgcga tcaccgtcgt  600
   cttgaccacc ggctcgaaca ccgatcgcac cttgccctcg ggccagtccg aggggttggt  660
   gacgatgatc tcggacccgt cgtccttgac gacgcggtag accacgttgg gcgcggtcga  720
   gatcaggtcc aggttgaact cgcgctccag gcgttcccgg gtgatctcca tgtgcagcag  780
   cccgaggaac ccgcagcgga acccgaaccc gagcgccacc gacgtctcgg gttcgtacgt  840
   cagcgccgcg tcgttgagtt gcagcttgtc cagcgcctcg cgcagcaccg ggtagtccga  900
   gccgtcgacc ggatacaggc ccgagtagac catcggcctg ggctcccggt agccggtcaa  960
   cgcttccttg gcaccgttac gcgccgtcgt caccgtgtcg ccgaccttgg actggcgcac 1020
   gtccttcaca ccggtgatga ggtagccgac ctcgccgacg ccgaggccgt cggagggctt 1080
   gggctcgggt gagacgattc ccacctcgag cagttcgtgg gtggcgccgg tcgacatcat 1140
   cgcgatgcgc tcgcgcgggg tgatcttccc gtccaccacc cgcacgtagg tcaccacgcc 1200
   gcggtagatg tcgtagaccg agtcgaagat catcgcgcgg gcaggcgcgt ccgggtcgcc 1260
   ctgcggcgcc gggatctccc gcaccacgtg gtcgagcagc tcggccacac cctcacccgt 1320
   cttgcccgac acccgcaaca cgtcctcggg ctcgcagccg atgatgtggg cgatctcggc 1380
   ggcgtaccgg tccgggtctg cggcgggcag atcgatcttg ttcagcaccg ggatgatcgt 1440
   caggtcgcgg tccagcgcca gatacaggtt cgccagcgtc tgcgcttcga tgccctgggc 1500
   ggcgtcgacc agcagcaccg cgccctcgca cgcctccagc gcgcgggaca cctcataggt 1560
   gaaatcaaca tggccgggcg tgtcaatcaa atgcagcacg aactcctcac cgttgaccac 1620
   ccacggcagc cgcacgttct gcgccttgat cgtgatcccg cgctcacgct cgatgtccat 1680
   ccggtccagg tactgcgccc gcatcgagcg ctcgtcgacc acaccggtga gctgcagcat 1740
   ccggtcggcc agggtggact ttccgtggtc gatgtgggcg atgatgcaga agttccgaat 1800
   ctgcgccggc gcagtgaacg tcttgtcggc gaagctgctg atgggaatct cctggtgagc 1860
   gggtcgtggc ggcctgaaca ggcctgtcca gagtatcgag cgcacacccc cgcgacacaa 1920
   tcgagccgtg atcgaggcgg cttcggggca ccggggcac                        1959
<210> 65
   <211> 1905
   <212> DNA
   <213> Mycobacterium gilvum
<400> 65
   ctacttcttc ggcttgtccg cggcggactc ggtcgacagt gccgcgacga aggcctcctg 60
   cggcacctcg acccggccga tcgtcttcat ccgcttcttg ccttccttct gcttctccag 120
   cagcttgcgc ttacgagtga tgtcaccgcc gtagcacttc gacagcacgt ccttgcggat 180
   cgcccggatg ttctctcgcg caatgattct cgagccgatc gcggcctgca cgggcacctc 240
   gaactgctgg cgtgggatca gttccttcag cttggtcgtc atcttgttgc cgtacgccgc 300
   ggcaccgtcc ttgtggacga tggcgctgaa cgcgtcgacg gcttcgccct gcagcaggat 360
   gtcgaccttg accaggtcgg cctcctgctc gcccgcctcc tcgtagtcga ggctcgcgta 420
   gccgcgggtg cgggacttca gcgagtcgaa gaagtcgaag atgatctcgc ccagcggcat 480
   cgtgtagcgc agctcgaccc gctcgggtga caggtagtcc atgccgccga gctcgccacg 540
   gcgcgactgg cacagctcca tgatcgttcc gatgaactca ctcggcgcga tcaccgtcgt 600
   cttgacgacc ggctcgaaca ccgaacggac cttgccctcg ggccagtccg acgggttggt 660
   gaccgtgagc tcgctgttgt cctctttgat gacgcggtag acgacgttgg gcgcggtcga 720
   gatcaggtcg aggttgaact cgcgttcgag ccgctcgcgc gtgatctcca tgtgcagcaa 780
   gcccaggaag ccgcagcgga agccgaaccc gagcgcgacc gacgtctccg gctcgtaagt 840
   cagtgccgcg tcgttcagct gcagtttgtc gagcgcctcg cgcaacaccg ggtagtcgga 900
   accgtccacg ggatacaggc ccgagtagac catcggcttg ggctcgcggt agccggtcag 960
   cgcttcggtg gcacccttgc gtgccgtcgt caccgtgtcg ccgaccttgg actgacgcac 1020
   gtccttcacg ccggtgatca ggtagccgac ctcgccgaca ccgagaccga ccgaaggctt 1080
   ggggtccggc gagacgatgc ccacttcgag gagttcgtgc gtcgcgccgg tcgacatcat 1140
   cgcgatgcgc tcacgcgggg tgatcctgcc gtcgacgacg cgcacatagg tgacgacgcc 1200
   gcggtagatg tcgtacaccg agtcgaagat catcgcgcgc gccggagcgt ccgggtcgcc 1260
   ctgcggcggc gggatctccc ggacgacgtg gtcgagcagg tcgccgacgc cggcgccggt 1320
   cttgcccgac acccgcagca catcctccgg ttcgcagccg atgatgtgcg cgatctcacc 1380
   ggcgtagcgg tcggggtcgg cggcgggcag gtcgatcttg ttgagcaccg ggatgattgt 1440
   caggtcgcga tccagcgcca ggtacaggtt cgccagggtc tgcgcctcga tgccctgggc 1500
   ggcgtcgacc agcagcaccg caccctcgca ggcctccagc gcgcgcgaca cctcgtaggt 1560
   gaaatcgacg tggccagggg tgtcgatcag atgcaggacg aactcttcgc cgttgacgac 1620
   ccacggcagg cgcacgttct gcgccttgat cgtgatgccg cgttcccgct cgatgtccat 1680
   ccggtccagg tactgcgccc gcatgtccct gtccgcgacc acaccggtga gctgcagcat 1740
   ccgatcggcc agggtggact tgccgtggtc gatgtgggcg atgatgcaga agttcctgat 1800
   ctgcgccggc gcagtgaacg tcttgtcggc gaagctggcg atgggaatct cctggtgagc 1860
   ggggtctgtc ggcctgagca ggccagtcca gagtatcgag cgcat 1905
<210> 66
   <211> 1854
   <212> DNA
   <213> Mycobacterium abscessus
<400> 66
   ctacttcttc ggtttgtccg ccgtcgactc ggtggacagt gccgccacaa acgcctcctg 60
   cggcacctcg acgcgaccga tggtcttcat gcgcttcttg ccctccttct gcttctcgag 120
   cagcttgcgc ttacgggtga tatcaccgcc gtagcacttg gagagcacat ccttacggat 180
   ggcccgaata ttctcgcgcg caatgattct cgatccgaca gcggcctgca ccggcacctc 240
   gaactgctgg cgcgggatga gttccttgag cttgacggtc atcttgttgc cataggccga 300
   ggccccgtcc ttgtggacga tggcgctgaa cgcgtcgacc gcctcgccct gcagcaggat 360
   gtcgaccttg accagatcgg cctcctgctc gccggcctcc tcgtaatcca ggctggcgta 420
   gccgcgcgta cgcgacttca acgagtcgaa gaagtcgaag atgatctcgc ccaacggcat 480
   cgtgtagcgc agctcgacgc gttcgggtga caggtagtcc atgccgccga gctcgccgcg 540
   ccgcgattgg cacagctcca tgatcgaacc gatgaattca ctcggcgcga tcaccgtcgt 600
   cttgaccacc ggctcgaaca ccgaccggat cttgccctcg ggccagatgg acggattggt 660
   caccatcatc cctggatcat cggccgtcat tccttcggta atcacccggt acaccacgtt 720
   gggagccgtc gagatgaggt cgaggttgaa ctcgcgttcg aggcgctcac gggtgatctc 780
   catgtgcagc aatcccagga agccacaacg gaatccgaag cccagcgcca ccgaggtctc 840
   cggctcgtag gtgagcgcgg cgtcgttgag ctggagtttg tccagcgcct cgcgcaggtt 900
   cgggtagtcg gatccgtcca ccgggtacag cccggaatag accatcggct tgggttcgcg 960
   gtagccggtc agcggctcct tggcgccgtt gcgcgcggcg gtgacggtgt caccgacctt 1020
   cgactggcgc acatccttca cgccggtgat caggtagccc acctcgccga ccccgagtcc 1080
   cgcggagggt ttgggctccg gcgagacgat gcccacttcc agcagttcgt gggtggcgcc 1140
   cgtcgacatc atcgcgatct tctcgcgcgg agtgatcttg ccgtccacga cgcgcacgta 1200
   ggtgaccaca ccgcggtaga tgtcgtagac cgagtcgaag atcatcgccc gcgccggcgc 1260
   atccggatca ccttgcggcg ctgggatgag ccgcaccacc tcgtcgagca gcgccgcgac 1320
   gccctccccg gtcttaccgg acacccgcag cacatcggag ggctcgcagc cgatgatgtg 1380
   cgcgatctcc tcggcgtaac gctccggatc ggccgcgggc aggtcgatct tgttcaggac 1440
   cgggatgatc gtcaggtcct tgtccagcgc caggtacagg ttggccagcg tctgcgcttc 1500
   gatgccctgc gcggcgtcga ccagcagcac tgccccctcg cacgcctcca gggcgcgcga 1560
   cacctcgtag gtgaagtcga cgtgcccggg ggtgtcgatc aggtgtagca catggtcctg 1620
   gccattgagc tgccacggca gccgcacgtt ctgtgccttg atggtgatgc cgcgctcacg 1680
   ctcgatatcc atgcgatcca ggtactgcgc gcgcatggaa cgctcgtcga ccacaccggt 1740
   cagctgcagc atccggtcgg ccagggtcga cttcccgtgg tcgatgtggg cgatgatgca 1800
   gaagttacga atcagcgccg gatccgtgaa cgtcgtgtcg gcaaaacttg gcac 1854
<210> 67
   <211> 1917
   <212> DNA
   <213> Mycobacterium marinum
<400> 67
   ctacttcttg cccttatccc ccgcggcgtc ggtggacagt gccgcgacaa acgcctcctg 60
   cggcacctcg acccggccga tggtcttcat ccgcttcttg ccctctttct gcttttccag 120
   cagcttgcgt ttgcgggtga tgtcaccgcc gtagcacttc gacaacacat ccttgcggat 180
   cgcgcggatg ttctcgcgcg cgatgatctt ggaaccgatg gccgcctgca ccggcacctc 240
   gaactgctga cgcggaatca gctccttgag cttggtggtc atcttgttgc cgtaggcgaa 300
   cgccgaatcc ttgtggacga tagcgctgaa cgcatcgacg gcctcgcctt gcagcaggat 360
   gtcgaccttg accagttggg cttcctgctc gccggactcc tcgtaatcga gactggcgta 420
   gccacgggtc cgcgacttga gcgagtcgaa gaagtcaaag atgatctccc ccaacggcat 480
   tgtgtatcgc agttccaccc gttcgggcga caaatagtcc atgcccccca gctcgccgcg 540
   ccgcgactgg cacagctcca tgatggtgcc gatgaattcg ctgggcgcga tgatggtggt 600
   cttcaccacc ggctcgtaga cggtgcggat cttgccctcg ggccagtccg acgggttggt 660
   cacctgcatt tcggtgccgt cgtccttgat gacgcggtac acgacgttgg gcgaggtcga 720
   gatcaggtcg aggtcgaact cgcgctccag acgttcccga ctgatctcca tgtgcagcag 780
   gcccaggaag ccgcaccgga atccgaaacc cagcgccacc gacgtttcgg gctcataggt 840
   cagggccgcg tcgttgagct gcagcttgtc cagggcgtcc cgcaggttcg gatagtccga 900
   tccgtcaacg ggatacagtc ccgaatacac catcggcttg ggttcgcggt agccggtcag 960
   tgcctcggtg gcaccttttc gggcggtcgt gacggtgtcg ccgaccttgg actgccgcac 1020
   gtccttgacc ccggtgatca gataacccac ctcgccgaca ccaaggccgt cgctggcctt 1080
   gggttcgggt gagacgatgc cgacctcgag cagttcgtgg gtggcgccgg tggacatcat 1140
   ggcgatgcgc tcgcgggggg tgatcttgcc gtcgacgacg cggacgtagg tgaccacacc 1200
   gcggtagatg tcatagacgg agtcgaagat cattgcgcga gtgggtgcgt cggcatcgcc 1260
   ctgcggtggc ggcacctcgc gcaccacgtg gtcgagcagg tctgcgacgc cttccccggt 1320
   tttgccggaa acccgcagca cgtcgccggg ctcgcagccg atgatgtgag caatctcgcc 1380
   cgcgtagcgg tccgggtcgg cggccggcag gtcgatcttg ttcagcaccg gaatgatgtg 1440
   caggtcgcgg tccagtgcca ggtagaggtt ggccagcgtc tgggcctcga tgccctgggc 1500
   ggcgtcaacc agcagcaccg cgccctcgca ggcctccagc gcacgtgaca cctcgtaggt 1560
   gaagtcgacg tgtcctggcg tgtcgatgag atgcaggacg tactcggttc catcgagctg 1620
   ccagggcagc cgcacattct gcgccttgat ggtgatcccg cgttcgcgtt cgatatccat 1680
   ccggtccagg tactgggccc gcatcgagcg ctcgtcaacg accccggtca actgcagcat 1740
   tcggtccgcc agcgtcgact ttccgtggtc gatgtgagcg atgatgcaga agttccgaat 1800
   ctgcgccggc ggggtgaagg ttttgtcggc gaaactgctg atgggactct cctgaagcgg 1860
   gggtttgcgg gtttccagcc tatctgtgca gcgccgcccg gacctacttg aggccaa 1917
<210> 68
   <211> 1896
   <212> DNA
   <213> Mycobacterium smegmatis
<400> 68
   ctacttcttg ggcttgtcgc ccgccgcgtc ggtggacagc gccgcgacga atgcctcctg 60
   cggcacgtcg acccggccga tcgtcttcat gcgcttcttg ccctctttct gcttctcgag 120
   cagcttgcgc ttacgggtga tgtcaccgcc gtagcacttc gagagcacgt ccttgcggat 180
   ggcccggatg ttttcgcgcg caatgattct cgagccgatc gcggcctgca ccgggacctc 240
   gaactgctgt cgcgggatca gttctttgag cttggaggtc atcttgttgc cgtaggccga 300
   cgcaccgtcc ttgtggacga tagccgagaa cgcgtcgacg gcctcgccct gcagcaggat 360
   gtcgaccttg accagatcgg cctcctgctc accggcctcc tcgtagtcga ggctcgcgta 420
   gccgcgggtg cgggacttca gcgagtcgaa gaagtcgaag atgatctcgc ccaacggcat 480
   gatgtagcgc agttcgacac gctcgggcga caggtagtcc atgccctgca gttcgccgcg 540
   acgggactgg cacagctcca tgatggtgcc gatgaactcg ctgggcgcga tgatcgtggt 600
   cttcacgacg ggctcgaaca ccgtgcggat cttgccttcc ggccagtccg acgggttcgt 660
   cacgaccttc tcggaaccgt cgtcctgcac gacgcggtac accacgttgg gtgaggtgga 720
   gatcaggtcc aggccgaact cgcgctccag gcgttcacgg gtgatctcca tgtgcagcag 780
   tccgaggaag ccgcagcgga acccgaagcc cagggccacc gaggtctccg gctcatacgt 840
   cagtgcggcg tcgttgagtt gcagcttgtc cagcgcgtca cgcagatccg ggtagtccga 900
   accgtcgacg ggatacaggc ccgagtacac catcggcttg ggctcgcgat aacccgtgag 960
   cgcctcggtc gcgcccttgc gcgccgtggt caccgtgtca ccgaccttcg actggcggac 1020
   gtccttcaca cccgtgatca ggtaaccgac ctcaccgacg cccaggcccg cactggcctt 1080
   cggttccggt gagacgatgc cgacctcgag cagttcatgg gtggcgccgg tggacatcat 1140
   cgcgatgcgt tcgcgcggca cgatcttgcc gtcgaccaca cggacgtagg tcaccacgcc 1200
   gcggtagatg tcgtacacgg agtcgaagat catcgcgcgc gtcggggcgt cggggtcacc 1260
   gaccggcggc ggcaccttac gcaccacctc gtcgagcagc tcggccacgc cttcgccggt 1320
   cttgcccgag acacgcagca cgtccgacgg ctcacacccg atgatgtggg cgagctcgtc 1380
   ggcatagcgg tccgggtcag cggcgggcag gtcgatcttg ttgagcaccg ggatgatcgc 1440
   caggtcgcgg tccagcgcca ggtacaggtt ggccagcgtc tgcgcctcga tgccctgcgc 1500
   cgcgtcgacc agcagcaccg cgccctcgca ggcctccagc gcgcgcgaca cctcgtaggt 1560
   gaagtcgacg tggcccgggg tgtcgatcag gtgcagcacg taatcacccg cgtccgcgcc 1620
   gtcttggccg tccttcagcg tccacggaag ccggacgttc tgagccttga tggtgatccc 1680
   gcgctcacgt tcgatgtcca tgcggtcgag gtactgggcc cgcatcgacc gctcatcgac 1740
   aacaccggtg agctgcagca tccggtcggc cagcgtcgac ttgccgtggt cgatgtgggc 1800
   gatgatgcag aagttccgaa tctgcgccgg cgcagtgaac gtcttgtcgg cgaagctgct 1860
   gatgggaatc tcctggtgag cgtgggtcaa gcgcac 1896
<210> 69
   <211> 475
   <212> DNA
   <213> Artificial Sequence
<220>
   <223> Mycobacterium tuberculosis DNA fragment
<400> 69
   cggcatggtg tagcgcagtt ccacccgctc gggggagaga tagtccatgc cgcccaactc 60
   gccgcggcgc gactggcaca gctccatgat ggtgccgatg aactcgctgg gcgcgatgat 120
   ggtggtcttg acgacgggct cgtagaccgt gcggatcttg ccctccggcc agtccgacgg 180
   attggtcacc cggatttcgg tgccgtcgtc tttgtgcacc cgatacacca cattgggtga 240
   ggtcgagatc aggtccaggc cgaactcgcg ctcaaggcgc tcacgggtga tctccatgtg 300
   cagcaggccc aagaaaccgc accggaaccc aaaacccagc gccaccgagg tttccggctc 360
   ataggtcaag gccgcgtcgt tgagctgcag cttgtccagg gcgtcgcgca ggttcgggta 420
   gtccgaaccg tcgaccggat acaaccccga gtagaccatc ggtttgggct cacgg 475
<210> 70
   <211> 475
   <212> DNA
   <213> Artificial Sequence
<220>
   <223> Mycobacterium bovis DNA fragment
<400> 70
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgcacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcggata  420
   gtccgaaccg tcgaccggat acnaccccga gtagaccatc ggtttgggct cacgg       475
<210> 71
   <211> 475
   <212> DNA
   <213> Artificial Sequence
<220>
   <223> Mycobacterium canettii DNA fragment
<400> 71
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgcacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg       475
<210> 72
   <211> 475
   <212> DNA
   <213> Artificial Sequence
<220>
   <223> Mycobacterium canettii DNA fragment
<400> 72
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgcacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg       475
<210> 73
   <211> 475
   <212> DNA
   <213> Artificial Sequence
<220>
   <223> Mycobacterium africanum DNA fragment
<400> 73
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgcacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg       475
<210> 74
   <211> 475
   <212> DNA
   <213> Artificial Sequence
<220>
   <223> Mycobacterium microti DNA fragment
<400> 74
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgcacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg       475
<210> 75
   <211> 475
   <212> DNA
   <213> Artificial Sequence
<220>
   <223> Mycobacterium pinipedii DNA fragment
<400> 75
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgcacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg       475
<210> 76
   <211> 475
   <212> DNA
   <213> Artificial Sequence
<220>
   <223> Mycobacterium caprae DNA fragment
<400> 76
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgtacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg        475
<210> 77
   <211> 475
   <212> DNA
   <213> Artificial Sequence
<220>
   <223> Mycobacterium caprae DNA fragment
<400> 77
   cggcatggtg tagcgcagtt ccacccgctc gggggagaga tagtccatgc cgcccaactc   60
   gccgcggcgc gactggcaca gctccatgat ggtgccgatg aactcgctgg gcgcgatgat  120
   ggtggtcttg acgacgggct cgtagaccgt gcggatcttg ccctccggcc agtccgacgg  180
   attggtcacc cggatttcgg tgccgtcgtc tttgtgtacc cgatacacca cattgggtga  240
   ggtcgagatc aggtccaggc cgaactcgcg ctcaaggcgc tcacgggtga tctccatgtg  300
   cagcaggccc aagaaaccgc accggaaccc aaaacccagc gccaccgagg tttccggctc  360
   ataggtcaag gccgcgtcgt tgagctgcag cttgtccagg gcgtcgcgca ggttcgggta  420
   gtccgaaccg tcgaccggat acaaccccga gtagaccatc ggtttgggct cacgg        475
<210> 78
   <211> 2869
   <212> DNA
   <213> Mycobacterium canettii
<400> 78
   tgtcggcggc cacgtcagac tgcccagtga tggccatata agtgcccgct ggcggtcatg   60
   aaaactgccc gctggcggtc acgagatctg cccagttgat ttgttcgtcc cgcgtgcctg  120
   cgaggtgcgg gggcccctcc tcgggtgcgc tgaacggtgc caaccgttgt tcagctcccg  180
   aggaggggtg aagtgaagtc tgccgaggag atcatggaaa ttctggaagc ctacgatttg  240
   accggttcgt tgcgtgatgc ggcggaactg gcggggtgct cgcatcacac ggtcgccgag  300
   tatgtggccg cgcgggagcg gggcgagttg acgcccgggc gcgcggcgcg gcgggagatg   360
   ctggtggatc cgtatctgga caagctcgag gagtgggtcg accgctcgcg gggcaaggtc   420
   cgcggcgatg tcgcccacga gaagctggtc gcgttggggt atgcgggttc gcagcgtacg   480
   acacggcggg cggtcgccga ggtcaaggca gcgtatcggg cggggcgacg gcgggtgcac   540
   cgtccgtgga tcaccgagcc ggggatgtgg tttcagtacg acttcgggga tggcccgcgc   600
   gtgagaggtg ttggcacgca attgttttgc gcgtggctgg cgtggtgccg gtttagggtg   660
   gtgctggcgc ttttggataa gacactgcca tcggtgatgg ccgcgatcga tacaacgcta   720
   cgcgtcttcg gtggggtgcc cacctacgcg ttgaccgata acgagaagac cgtgaccagc   780
   gagcatgtcg cggggatacc ggtgcgcaac gccaagatgc tggacttcgc ccgccattac   840
   gggctcacga tcgccacctg cgtgccggcc gatccggcca gcaagggcgg ctcggaaaac   900
   gcggtcaaga tcgccaaggc cgatctggtg ccctgtgagg ccaacctgct accggaatac   960
   cacagtttcg ccgagctgca agcggcgtgc gcgacgtttt gccagcaggt caacaatcgc   1020
   ccgcatcggg tcacgcggcg catcccggcg gagatgctgg ccgaggaacg cgcccggtta   1080
   cacccgttac ccgcccatcc ctacaccgcg gcgttcgggg tgacccgcac ggtgccgccc   1140
   aacaccgcga tgatcacctt tgagcacggg tcgtattcgg tgccgcacac cttgtgcggg   1200
   cagacggtgt gggtgcgggc ccacgaccag caggtagtgg tcgtgcacct cggccatgcc   1260
   ggcccagtcg aagtcgcccg ccaccagcgc accacgccgg gtaacccgcg ggtggatgac   1320
   gcgcatttcc caccccggcc caaggggccg ctgggccgaa caccacgcgc gaaaactgtt   1380
   gctgaggcgc agtttttggc tctcggtgac ggggcagcat tgtggttgac cgaggccgcc   1440
   gccgcgggat gctcgcggat tcgggccaag atggccgggg cggtcgattt ggccgcactg   1500
   cacgatcggg gcagcgtgga ccgcgccttg ggccaagccg cgaccgcggg ccggttcggc   1560
   cacggcgatc tggccgccat cgtggcccat caggccggcg accccgacca ccacagcgcc   1620
   tcgcagcccg cccacgcggg cgagtacaac agcctggccc aaggcaccgg tggctgggcg   1680
   aagctcggtg agcaggaggc caactaacga tggcctacct cgacatctcc tttaagacca   1740
   catcgatcct gctcaacacc tgcggcgccg acgatcccga ccaactggtc ggcgtggccg   1800
   ccgcagaact gttccagtgg gcgtggctga tcggcgagct cgccagctgg ctcgccgacg   1860
   ctgacgagca cacccacgcc gacttcgacc ggttcttcag cagctaccgc ggcgtcgaca   1920
   agaccgcagg gttggccacc cacatcgcgc agcgcatcgc cgcgctgctc gacggggacc   1980
   ggagccagcc atgagcacaa caacaccgcc accaccaccg ctagacgacg agctgatgcg   2040
   gctgctcaag cggatgcggc tgccttacat ccgcaacgcc gcacccgagc tgctggccac   2100
   cgccaaggcg caacgctggg accctgccga ggtgctcaaa gcgcttctga ccgaagaggt   2160
   caacgggcga gaccgctccg cgctggccat ccgccgcacg cgggccggtt ttcccaccgg   2220
   taagaccttc gccgcgtggg accccgcact gtcgtccatc cccgcaccca cccaggccgc   2280
   gttgcgcacc ctggaatgga tccaccgacg cgaaaacctg gtggtctgcg ggccgtcggg   2340
   caccggcaag acgttcctgc tcgaagccct cggccaacaa gccgtcgaaa ccgggctcca   2400
   cgtcgcgtgg tttagcctcg aacaactcgg cgccctggtg cgccggcacc gcgccgacga   2460
   caccgtcacc aaagccatca gccgtatcct gcgtgccgat ctggtcgcgg ttgatgacat   2520
   cggcctgctg ccggtcggca ccgacgccgc cgaagggctc taccggcttg tcgacgccgc   2580
   ttacgagaaa cgctccatcg cactatcaag caatcttcac ccggcaggat tcgacgagtt   2640
   gatgcccaag acactggcca ccgccaccgt cgaccggctc ctgcaccacg cccacgtctg   2700
   ccagaccagt ggcgacagca tccgacttac ccaagccatg gccggcaagg gggtgaacgc   2760
   cttgacctaa cccccagcca cgtctggtgg ccgccagcag gcagatctcg tggccgccag   2820
   cgggcagttc tcatgaccgc cactgggcag ttccccatgt cccttgaca             2869
<210> 79
   <211> 2832
   <212> DNA
   <213> Artificial Sequence
<220>
   <223> Mycobacterium canetti DNA fragment
<400> 79
   tataagtgcc cgctggcggt catgaaaact gcccgctggc ggtcacgaga tctgcccagt 60
   tgatttgttc gtcccgcgtg cctgcgaggt gcgggggccc ctcctcgggt gcgctgaacg 120
   gtgccaaccg ttgttcagct cccgaggagg ggtgaagtga agtctgccga ggagatcatg 180
   gaaattctgg aagcctacga tttgaccggt tcgttgcgtg atgcggcgga actggcgggg 240
   tgctcgcatc acacggtcgc cgagtatgtg gccgcgcggg agcggggcga gttgacgccc 300
   gggcgcgcgg cgcggcggga gatgctggtg gatccgtatc tggacaagct cgaggagtgg 360
   gtcgaccgct cgcggggcaa ggtccgcggc gatgtcgccc acgagaagct ggtcgcgttg 420
   gggtatgcgg gttcgcagcg tacgacacgg cgggcggtcg ccgaggtcaa ggcagcgtat 480
   cgggcggggc gacggcgggt gcaccgtccg tggatcaccg agccggggat gtggtttcag 540
   tacgacttcg gggatggccc gcgcgtgaga ggtgttggca cgcaattgtt ttgcgcgtgg 600
   ctggcgtggt gccggtttag ggtggtgctg gcgcttttgg ataagacact gccatcggtg 660
   atggccgcga tcgatacaac gctacgcgtc ttcggtgggg tgcccaccta cgcgttgacc 720
   gataacgaga agaccgtgac cagcgagcat gtcgcgggga taccggtgcg caacgccaag 780
   atgctggact tcgcccgcca ttacgggctc acgatcgcca cctgcgtgcc ggccgatccg 840
   gccagcaagg gcggctcgga aaacgcggtc aagatcgcca aggccgatct ggtgccctgt 900
   gaggccaacc tgctaccgga ataccacagt ttcgccgagc tgcaagcggc gtgcgcgacg 960
   ttttgccagc aggtcaacaa tcgcccgcat cgggtcacgc ggcgcatccc ggcggagatg 1020
   ctggccgagg aacgcgcccg gttacacccg ttacccgccc atccctacac cgcggcgttc 1080
   ggggtgaccc gcacggtgcc gcccaacacc gcgatgatca cctttgagca cgggtcgtat 1140
   tcggtgccgc acaccttgtg cgggcagacg gtgtgggtgc gggcccacga ccagcaggta 1200
   gtggtcgtgc acctcggcca tgccggccca gtcgaagtcg cccgccacca gcgcaccacg 1260
   ccgggtaacc cgcgggtgga tgacgcgcat ttcccacccc ggcccaaggg gccgctgggc 1320
   cgaacaccac gcgcgaaaac tgttgctgag gcgcagtttt tggctctcgg tgacggggca 1380
   gcattgtggt tgaccgaggc cgccgccgcg ggatgctcgc ggattcgggc caagatggcc 1440
   ggggcggtcg atttggccgc actgcacgat cggggcagcg tggaccgcgc cttgggccaa 1500
   gccgcgaccg cgggccggtt cggccacggc gatctggccg ccatcgtggc ccatcaggcc 1560
   ggcgaccccg accaccacag cgcctcgcag cccgcccacg cgggcgagta caacagcctg 1620
   gcccaaggca ccggtggctg ggcgaagctc ggtgagcagg aggccaacta acgatggcct 1680
   acctcgacat ctcctttaag accacatcga tcctgctcaa cacctgcggc gccgacgatc 1740
   ccgaccaact ggtcggcgtg gccgccgcag aactgttcca gtgggcgtgg ctgatcggcg 1800
   agctcgccag ctggctcgcc gacgctgacg agcacaccca cgccgacttc gaccggttct 1860
   tcagcagcta ccgcggcgtc gacaagaccg cagggttggc cacccacatc gcgcagcgca 1920
   tcgccgcgct gctcgacggg gaccggagcc agccatgagc acaacaacac cgccaccacc 1980
   accgctagac gacgagctga tgcggctgct caagcggatg cggctgcctt acatccgcaa 2040
   cgccgcaccc gagctgctgg ccaccgccaa ggcgcaacgc tgggaccctg ccgaggtgct 2100
   caaagcgctt ctgaccgaag aggtcaacgg gcgagaccgc tccgcgctgg ccatccgccg 2160
   cacgcgggcc ggttttccca ccggtaagac cttcgccgcg tgggaccccg cactgtcgtc 2220
   catccccgca cccacccagg ccgcgttgcg caccctggaa tggatccacc gacgcgaaaa 2280
   cctggtggtc tgcgggccgt cgggcaccgg caagacgttc ctgctcgaag ccctcggcca 2340
   acaagccgtc gaaaccgggc tccacgtcgc gtggtttagc ctcgaacaac tcggcgccct 2400
   ggtgcgccgg caccgcgccg acgacaccgt caccaaagcc atcagccgta tcctgcgtgc 2460
   cgatctggtc gcggttgatg acatcggcct gctgccggtc ggcaccgacg ccgccgaagg 2520
   gctctaccgg cttgtcgacg ccgcttacga gaaacgctcc atcgcactat caagcaatct 2580
   tcacccggca ggattcgacg agttgatgcc caagacactg gccaccgcca ccgtcgaccg 2640
   gctcctgcac cacgcccacg tctgccagac cagtggcgac agcatccgac ttacccaagc 2700
   catggccggc aagggggtga acgccttgac ctaaccccca gccacgtctg gtggccgcca 2760
   gcaggcagat ctcgtggccg ccagcgggca gttctcatga ccgccactgg gcagttcccc 2820
   atgtcccttg ac 2832
<210> 80
   <211> 2832
   <212> DNA
   <213> Artificial Sequence
<220>
   <223> Mycobacterium canettii DNA fragment
<400> 80
   tataagtgcc cgctggcggt catgaaaact gcccgctggc ggtcacgaga tctgcccagt   60
   tgatttgttc gtcccgcgtg cctgcgaggt gcgggggccc ctcctcgggt gcgctgaacg  120
   gtgccaaccg ttgttcagct cccgaggagg ggtgaagtga agtctgccga ggagatcatg  180
   gaaattctgg aagcctacga tttgaccggt tcgttgcgtg atgcggcgga actggcgggg  240
   tgctcgcatc acacggtcgc cgagtatgtg gccgcgcggg agcggggcga gttgacgccc  300
   gggcgcgcgg cgcggcggga gatgctggtg gatccgtatc tggacaagct cgaggagtgg  360
   gtcgaccgct cgcggggcaa ggtccgcggc gatgtcgccc acgagaagct ggtcgcgttg  420
   gggtatgcgg gttcgcagcg tacgacacgg cgggcggtcg ccgaggtcaa ggcagcgtat  480
   cgggcggggc gacggcgggt gcaccgtccg tggatcaccg agccggggat gtggtttcag  540
   tacgacttcg gggatggccc gcgcgtgaga ggtgttggca cgcaattgtt ttgcgcgtgg  600
   ctggcgtggt gccggtttag ggtggtgctg gcgcttttgg ataagacact gccatcggtg  660
   atggccgcga tcgatacaac gctacgcgtc ttcggtgggg tgcccaccta cgcgttgacc  720
   gataacgaga agaccgtgac cagcgagcat gtcgcgggga taccggtgcg caacgccaag  780
   atgctggact tcgcccgcca ttacgggctc acgatcgcca cctgcgtgcc ggccgatccg  840
   gccagcaagg gcggctcgga aaacgcggtc aagatcgcca aggccgatct ggtgccctgt  900
   gaggccaacc tgctaccgga ataccacagt ttcgccgagc tgcaagcggc gtgcgcgacg  960
   ttttgccagc aggtcaacaa tcgcccgcat cgggtcacgc ggcgcatccc ggcggagatg 1020
   ctggccgagg aacgcgcccg gttacacccg ttacccgccc atccctacac cgcggcgttc 1080
   ggggtgaccc gcacggtgcc gcccaacacc gcgatgatca cctttgagca cgggtcgtat 1140
   tcggtgccgc acaccttgtg cgggcagacg gtgtgggtgc gggcccacga ccagcaggta 1200
   gtggtcgtgc acctcggcca tgccggccca gtcgaagtcg cccgccacca gcgcaccacg 1260
   ccgggtaacc cgcgggtgga tgacgcgcat ttcccacccc ggcccaaggg gccgctgggc 1320
   cgaacaccac gcgcgaaaac tgttgctgag gcgcagtttt tggctctcgg tgacggggca 1380
   gcattgtggt tgaccgaggc cgccgccgcg ggatgctcgc ggattcgggc caagatggcc 1440
   ggggcggtcg atttggccgc actgcacgat cggggcagcg tggaccgcgc cttgggccaa 1500
   gccgcgaccg cgggccggtt cggccacggc gatctggccg ccatcgtggc ccatcaggcc 1560
   ggcgaccccg accaccacag cgcctcgcag cccgcccacg cgggcgagta caacagcctg 1620
   gcccaaggca ccggtggctg ggcgaagctc ggtgagcagg aggccaacta acgatggcct 1680
   acctcgacat ctcctttaag accacatcga tcctgctcaa cacctgcggc gccgacgatc 1740
   ccgaccaact ggtcggcgtg gccgccgcag aactgttcca gtgggcgtgg ctgatcggcg 1800
   agctcgccag ctggctcgcc gacgctgacg agcacaccca cgccgacttc gaccggttct 1860
   tcagcagcta ccgcggcgtc gacaagaccg cagggttggc cacccacatc gcgcagcgca 1920
   tcgccgcgct gctcgacggg gaccggagcc agccatgagc acaacaacac cgccaccacc 1980
   accgctagac gacgagctga tgcggctgct caagcggatg cggctgcctt acatccgcaa 2040
   cgccgcaccc gagctgctgg ccaccgccaa ggcgcaacgc tgggaccctg ccgaggtgct 2100
   caaagcgctt ctgaccgaag aggtcaacgg gcgagaccgc tccgcgctgg ccatccgccg 2160
   cacgcgggcc ggtttcccca ccggtaagac cttcgccgcg tgggaccccg cactgtcgtc 2220
   catccccgca cccacccagg ccgcgttgcg caccctggaa tggatccacc gacgcgaaaa 2280
   cctggtggtc tgcgggccgt cgggcaccgg caagacgttc ctgctcgaag ccctcggcca 2340
   acaagccgtc gaaaccgggc tccacgtcgc gtggtttagc ctcgaacaac tcggcgccct 2400
   ggtgcgccgg caccgcgccg acgacaccgt caccaaagcc atcagccgta tcctgcgtgc 2460
   cgatctggtc gcggttgatg acatcggcct gctgccggtc ggcaccgacg ccgccgaagg 2520
   gctctaccgg cttgtcgacg ccgcttacga gaaacgctcc atcgcactat caagcaatct 2580
   tcacccggca ggattcgacg agttgatgcc caagacactg gccaccgcca ccgtcgaccg 2640
   gctcctgcac cacgcccacg tctgccagac cagtggcgac agcatccgac ttacccaagc 2700
   catggccggc aagggggtga acgccttgac ctaaccccca gccacgtctg gtggccgcca 2760
   gcaggcagat ctcgtggccg ccagcgggca gttctcatga ccgccactgg gcagttcccc 2820
   atgtcccttg ac                                                      2832
<210> 81
   <211> 2832
   <212> DNA
   <213> Artificial Sequence
<220>
   <223> Mycobacterium canettii DNA fragment
<400> 81
   tataagtgcc cgctggcggt catgaaaact gcccgctggc ggtcacgaga tctgcccagt 60
   tgatttgttc gtcccgcgtg cctgcgaggt gcgggggccc ctcctcgggt gcgctgaacg 120
   gtgccaaccg ttgttcagct cccgaggagg ggtgaagtga agtctgccga ggagatcatg 180
   gaaattctgg aagcctacga tttgaccggt tcgttgcgtg atgcggcgga actggcgggg 240
   tgctcgcatc acacggtcgc cgagtatgtg gccgcgcggg agcggggcga gttgacgccc 300
   gggcgcgcgg cgcggcggga gatgctggtg gatccgtatc tggacaagct cgaggagtgg 360
   gtcgaccgct cgcggggcaa ggtccgcggc gatgtcgccc acgagaagct ggtcgcgttg 420
   gggtatgcgg gttcgcagcg tacgacacgg cgggcggtcg ccgaggtcaa ggcagcgtat 480
   cgggcggggc gacggcgggt gcaccgtccg tggatcaccg agccggggat gtggtttcag 540
   tacgacttcg gggatggccc gcgcgtgaga ggtgttggca cgcaattgtt ttgcgcgtgg 600
   ctggcgtggt gccggtttag ggtggtgctg gcgcttttgg ataagacact gccatcggtg 660
   atggccgcga tcgatacaac gctacgcgtc ttcggtgggg tgcccaccta cgcgttgacc 720
   gataacgaga agaccgtgac cagcgagcat gtcgcgggga taccggtgcg caacgccaag 780
   atgctggact tcgcccgcca ttacgggctc acgatcgcca cctgcgtgcc ggccgatccg 840
   gccagcaagg gcggctcgga aaacgcggtc aagatcgcca aggccgatct ggtgccctgt 900
   gaggccaacc tgctaccgga ataccacagt ttcgccgagc tgcaagcggc gtgcgcgacg 960
   ttttgccagc aggtcaacaa tcgcccgcat cgggtcacgc ggcgcatccc ggcggagatg 1020
   ctggccgagg aacgcgcccg gttacacccg ttacccgccc atccctacac cgcggcgttc 1080
   ggggtgaccc gcacggtgcc gcccaacacc gcgatgatca cctttgagca cgggtcgtat 1140
   tcggtgccgc acaccttgtg cgggcagacg gtgtgggtgc gggcccacga ccagcaggta 1200
   gtggtcgtgc acctcggcca tgccggccca gtcgaagtcg cccgccacca gcgcaccacg 1260
   ccgggtaacc cgcgggtgga tgacgcgcat ttcccacccc ggcccaaggg gccgctgggc 1320
   cgaacaccac gcgcgaaaac tgttgctgag gcgcagtttt tggctctcgg tgacggggca 1380
   gcattgtggt tgaccgaggc cgccgccgcg ggatgctcgc ggattcgggc caagatggcc 1440
   ggggcggtcg atttggccgc actgcacgat cggggcagcg tggaccgcgc cttgggccaa 1500
   gccgcgaccg cgggccggtt cggccacggc gatctggccg ccatcgtggc ccatcaggcc 1560
   ggcgaccccg accaccacag cgcctcgcag cccgcccacg cgggcgagta caacagcctg 1620
   gcccaaggca ccggtggctg ggcgaagctc ggtgagcagg aggccaacta acgatggcct 1680
   acctcgacat ctcctttaag accacatcga tcctgctcaa cacctgcggc gccgacgatc 1740
   ccgaccaact ggtcggcgtg gccgccgcag aactgttcca gtgggcgtgg ctgatcggcg 1800
   agctcgccag ctggctcgcc gacgctgacg agcacaccca cgccgacttc gaccggttct 1860
   tcagcagcta ccgcggcgtc gacaagaccg cagggttggc cacccacatc gcgcagcgca 1920
   tcgccgcgct gctcgacggg gaccggagcc agccatgagc acaacaacac cgccaccacc 1980
   accgctagac gacgagctga tgcggctgct caagcggatg cggctgcctt acatccgcaa 2040
   cgccgcaccc gagctgctgg ccaccgccaa ggcgcaacgc tgggaccctg ccgaggtgct 2100
   caaagcgctt ctgaccgaag aggtcaacgg gcgagaccgc tccgcgctgg ccatccgccg 2160
   cacgcgggcc ggtttcccca ccggtaagac cttcgccgcg tgggaccccg cactgtcgtc 2220
   catccccgca cccacccagg ccgcgttgcg caccctggaa tggatccacc gacgcgaaaa 2280
   cctggtggtc tgcgggccgt cgggcaccgg caagacgttc ctgctcgaag ccctcggcca 2340
   acaagccgtc gaaaccgggc tccacgtcgc gtggtttagc ctcgaacaac tcggcgccct 2400
   ggtgcgccgg caccgcgccg acgacaccgt caccaaagcc atcagccgta tcctgcgtgc 2460
   cgatctggtc gcggttgatg acatcggcct gctgccggtc ggcaccgacg ccgccgaagg 2520
   gctctaccgg cttgtcgacg ccgcttacga gaaacgctcc atcgcactat caagcaatct 2580
   tcacccggca ggattcgacg agttgatgcc caagacactg gccaccgcca ccgtcgaccg 2640
   gctcctgcac cacgcccacg tctgccagac cagtggcgac agcatccgac ttacccaagc 2700
   catggccggc aagggggtga acgccttgac ctaaccccca gccacgtctg gtggccgcca 2760
   gcaggcagat ctcgtggccg ccagcgggca gttctcatga ccgccactgg gcagttcccc 2820
   atgtcccttg ac 2832
<210> 82
   <211> 2832
   <212> DNA
   <213> Artificial Sequence
<220>
   <223> Mycobacterium canettii DNA fragment
<400> 82
   tataagtgcc cgctggcggt catgaaaact gcccgctggc ggtcacgaga tctgcccagt   60
   tgatttgttc gtcccgcgtg cctgcgaggt gcgggggccc ctcctcgggt gcgctgaacg  120
   gtgccaaccg ttgttcagct cccgaggagg ggtgaagtga agtctgccga ggagatcatg  180
   gaaattctgg aagcctacga tttgaccggt tcgttgcgtg atgcggcgga actggcgggg  240
   tgctcgcatc acacggtcgc cgagtatgtg gccgcgcggg agcggggcga gttgacgccc  300
   gggcgcgcgg cgcggcggga gatgctggtg gatccgtatc tggacaagct cgaggagtgg  360
   gtcgaccgct cgcggggcaa ggtccgcggc gatgtcgccc acgagaagct ggtcgcgttg  420
   gggtatgcgg gttcgcagcg tacgacacgg cgggcggtcg ccgaggtcaa ggcagcgtat  480
   cgggcggggc gacggcgggt gcaccgtccg tggatcaccg agccggggat gtggtttcag  540
   tacgacttcg gggatggccc gcgcgtgaga ggtgttggca cgcaattgtt ttgcgcgtgg  600
   ctggcgtggt gccggtttag ggtggtgctg gcgcttttgg ataagacact gccatcggtg  660
   atggccgcga tcgatacaac gctacgcgtc ttcggtgggg tgcccaccta cgcgttgacc  720
   gataacgaga agaccgtgac cagcgagcat gtcgcgggga taccggtgcg caacgccaag  780
   atgctggact tcgcccgcca ttacgggctc acgatcgcca cctgcgtgcc ggccgatccg  840
   gccagcaagg gcggctcgga aaacgcggtc aagatcgcca aggccgatct ggtgccctgt  900
   gaggccaacc tgctaccgga ataccacagt ttcgccgagc tgcaagcggc gtgcgcgacg  960
   ttttgccagc aggtcaacaa tcgcccgcat cgggtcacgc ggcgcatccc ggcggagatg 1020
   ctggccgagg aacgcgcccg gttacacccg ttacccgccc atccctacac cgcggcgttc 1080
   ggggtgaccc gcacggtgcc gcccaacacc gcgatgatca cctttgagca cgggtcgtat 1140
   tcggtgccgc acaccttgtg cgggcagacg gtgtgggtgc gggcccacga ccagcaggta 1200
   gtggtcgtgc acctcggcca tgccggccca gtcgaagtcg cccgccacca gcgcaccacg 1260
   ccgggtaacc cgcgggtgga tgacgcgcat ttcccacccc ggcccaaggg gccgctgggc 1320
   cgaacaccac gcgcgaaaac tgttgctgag gcgcagtttt tggctctcgg tgacggggca 1380
   gcattgtggt tgaccgaggc cgccgccgcg ggatgctcgc ggattcgggc caagatggcc 1440
   ggggcggtcg atttggccgc actgcacgat cggggcagcg tggaccgcgc cttgggccaa 1500
   gccgcgaccg cgggccggtt cggccacggc gatctggccg ccatcgtggc ccatcaggcc 1560
   ggcgaccccg accaccacag cgcctcgcag cccgcccacg cgggcgagta caacagcctg 1620
   gcccaaggca ccggtggctg ggcgaagctc ggtgagcagg aggccaacta acgatggcct 1680
   acctcgacat ctcctttaag accacatcga tcctgctcaa cacctgcggc gccgacgatc 1740
   ccgaccaact ggtcggcgtg gccgccgcag aactgttcca gtgggcgtgg ctgatcggcg 1800
   agctcgccag ctggctcgcc gacgctgacg agcacaccca cgccgacttc gaccggttct 1860
   tcagcagcta ccgcggcgtc gacaagaccg cagggttggc cacccacatc gcgcagcgca 1920
   tcgccgcgct gctcgacggg gaccggagcc agccatgagc acaacaacac cgccaccacc 1980
   accgctagac gacgagctga tgcggctgct caagcggatg cggctgcctt acatccgcaa 2040
   cgccgcaccc gagctgctgg ccaccgccaa ggcgcaacgc tgggaccctg ccgaggtgct 2100
   caaagcgctt ctgaccgaag aggtcaacgg gcgagaccgc tccgcgctgg ccatccgccg 2160
   cacgcgggcc ggttttccca ccggtaagac cttcgccgcg tgggaccccg cactgtcgtc 2220
   catccccgca cccacccagg ccgcgttgcg caccctggaa tggatccacc gacgcgaaaa 2280
   cctggtggtc tgcgggccgt cgggcaccgg caagacgttc ctgctcgaag ccctcggcca 2340
   acaagccgtc gaaaccgggc tccacgtcgc gtggtttagc ctcgaacaac tcggcgccct 2400
   ggtgcgccgg caccgcgccg acgacaccgt caccaaagcc atcagccgta tcctgcgtgc 2460
   cgatctggtc gcggttgatg acatcggcct gctgccggtc ggcaccgacg ccgccgaagg 2520
   gctctaccgg cttgtcgacg ccgcttacga gaaacgctcc atcgcactat caagcaatct 2580
   tcacccggca ggattcgacg agttgatgcc caagacactg gccaccgcca ccgtcgaccg 2640
   gctcctgcac cacgcccacg tctgccagac cagtggcgac agcatccgac ttacccaagc 2700
   catggccggc aagggggtga acgccttgac ctaaccccca gccacgtctg gtggccgcca 2760
   gcaggcagat ctcgtggccg ccagcgggca gttctcatga ccgccactgg gcagttcccc 2820
   atgtcccttg ac                                                     2832
<210> 83
   <211> 2832
   <212> DNA
   <213> Artificial Sequence
<220>
   <223> Mycobacterium canettii DNA fragment
<400> 83
   tataagtgcc cgctggcggt catgaaaact gcccgctggc ggtcacgaga tctgcccagt   60
   tgatttgttc gtcccgcgtg cctgcgaggt gcgggggccc ctcctcgggt gcgctgaacg  120
   gtgccaaccg ttgttcagct cccgaggagg ggtgaagtga agtctgccga ggagatcatg  180
   gaaattctgg aagcctacga tttgaccggt tcgttgcgtg atgcggcgga actggcgggg  240
   tgctcgcatc acacggtcgc cgagtatgtg gccgcgcggg agcggggcga gttgacgccc  300
   gggcgcgcgg cgcggcggga gatgctggtg gatccgtatc tggacaagct cgaggagtgg  360
   gtcgaccgct cgcggggcaa ggtccgcggc gatgtcgccc acgagaagct ggtcgcgttg  420
   gggtatgcgg gttcgcagcg tacgacacgg cgggcggtcg ccgaggtcaa ggcagcgtat  480
   cgggcggggc gacggcgggt gcaccgtccg tggatcaccg agccggggat gtggtttcag  540
   tacgacttcg gggatggccc gcgcgtgaga ggtgttggca cgcaattgtt ttgcgcgtgg  600
   ctggcgtggt gccggtttag ggtggtgctg gcgcttttgg ataagacact gccatcggtg  660
   atggccgcga tcgatacaac gctacgcgtc ttcggtgggg tgcccaccta cgcgttgacc  720
   gataacgaga agaccgtgac cagcgagcat gtcgcgggga taccggtgcg caacgccaag  780
   atgctggact tcgcccgcca ttacgggctc acgatcgcca cctgcgtgcc ggccgatccg  840
   gccagcaagg gcggctcgga aaacgcggtc aagatcgcca aggccgatct ggtgccctgt  900
   gaggccaacc tgctaccgga ataccacagt ttcgccgagc tgcaagcggc gtgcgcgacg  960
   ttttgccagc aggtcaacaa tcgcccgcat cgggtcacgc ggcgcatccc ggcggagatg 1020
   ctggccgagg aacgcgcccg gttacacccg ttacccgccc atccctacac cgcggcgttc 1080
   ggggtgaccc gcacggtgcc gcccaacacc gcgatgatca cctttgagca cgggtcgtat 1140
   tcggtgccgc acaccttgtg cgggcagacg gtgtgggtgc gggcccacga ccagcaggta 1200
   gtggtcgtgc acctcggcca tgccggccca gtcgaagtcg cccgccacca gcgcaccacg 1260
   ccgggtaacc cgcgggtgga tgacgcgcat ttcccacccc ggcccaaggg gccgctgggc 1320
   cgaacaccac gcgcgaaaac tgttgctgag gcgcagtttt tggctctcgg tgacggggca 1380
   gcattgtggt tgaccgaggc cgccgccgcg ggatgctcgc ggattcgggc caagatggcc 1440
   ggggcggtcg atttggccgc actgcacgat cggggcagcg tggaccgcgc cttgggccaa 1500
   gccgcgaccg cgggccggtt cggccacggc gatctggccg ccatcgtggc ccatcaggcc 1560
   ggcgaccccg accaccacag cgcctcgcag cccgcccacg cgggcgagta caacagcctg 1620
   gcccaaggca ccggtggctg ggcgaagctc ggtgagcagg aggccaacta acgatggcct 1680
   acctcgacat ctcctttaag accacatcga tcctgctcaa cacctgcggc gccgacgatc 1740
   ccgaccaact ggtcggcgtg gccgccgcag aactgttcca gtgggcgtgg ctgatcggcg 1800
   agctcgccag ctggctcgcc gacgctgacg agcacaccca cgccgacttc gaccggttct 1860
   tcagcagcta ccgcggcgtc gacaagaccg cagggttggc cacccacatc gcgcagcgca 1920
   tcgccgcgct gctcgacggg gaccggagcc agccatgagc acaacaacac cgccaccacc 1980
   accgctagac gacgagctga tgcggctgct caagcggatg cggctgcctt acatccgcaa 2040
   cgccgcaccc gagctgctgg ccaccgccaa ggcgcaacgc tgggaccctg ccgaggtgct 2100
   caaagcgctt ctgaccgaag aggtcaacgg gcgagaccgc tccgcgctgg ccatccgccg 2160
   cacgcgggcc ggttttccca ccggtaagac cttcgccgcg tgggaccccg cactgtcgtc 2220
   catccccgca cccacccagg ccgcgttgcg caccctggaa tggatccacc gacgcgaaaa 2280
   cctggtggtc tgcgggccgt cgggcaccgg caagacgttc ctgctcgaag ccctcggcca 2340
   acaagccgtc gaaaccgggc tccacgtcgc gtggtttagc ctcgaacaac tcggcgccct 2400
   ggtgcgccgg caccgcgccg acgacaccgt caccaaagcc atcagccgta tcctgcgtgc 2460
   cgatctggtc gcggttgatg acatcggcct gctgccggtc ggcaccgacg ccgccgaagg 2520
   gctctaccgg cttgtcgacg ccgcttacga gaaacgctcc atcgcactat caagcaatct 2580
   tcacccggca ggattcgacg agttgatgcc caagacactg gccaccgcca ccgtcgaccg 2640
   gctcctgcac cacgcccacg tctgccagac cagtggcgac agcatccgac ttacccaagc 2700
   catggccggc aagggggtga acgccttgac ctaaccccca gccacgtctg gtggccgcca 2760
   gcaggcagat ctcgtggccg ccagcgggca gttctcatga ccgccactgg gcagttcccc 2820
   atgtcccttg ac                                                     2832
<210> 84
   <211> 1896
   <212> DNA
   <213> Mycobacterium smegmatis
<400> 84
   ctacttcttg ggcttgtcgc ccgccgcgtc ggtggacagc gccgcgacga atgcctcctg   60
   cggcacgtcg acccggccga tcgtcttcat gcgcttcttg ccctctttct gcttctcgag  120
   cagcttgcgc ttacgggtga tgtcaccgcc gtagcacttc gagagcacgt ccttgcggat  180
   ggcccggatg ttttcgcgcg caatgattct cgagccgatc gcggcctgca ccgggacctc  240
   gaactgctgt cgcgggatca gttctttgag cttggaggtc atcttgttgc cgtaggccga  300
   cgcaccgtcc ttgtggacga tagccgagaa cgcgtcgacg gcctcgccct gcagcaggat  360
   gtcgaccttg accagatcgg cctcctgctc accggcctcc tcgtagtcga ggctcgcgta  420
   gccgcgggtg cgggacttca gcgagtcgaa gaagtcgaag atgatctcgc ccaacggcat  480
   gatgtagcgc agttcgacac gctcgggcga caggtagtcc atgccctgca gttcgccgcg  540
   acgggactgg cacagctcca tgatggtgcc gatgaactcg ctgggcgcga tgatcgtggt  600
   cttcacgacg ggctcgaaca ccgtgcggat cttgccttcc ggccagtccg acgggttcgt  660
   cacgaccttc tcggaaccgt cgtcctgcac gacgcggtac accacgttgg gtgaggtgga  720
   gatcaggtcc aggccgaact cgcgctccag gcgttcacgg gtgatctcca tgtgcagcag  780
   tccgaggaag ccgcagcgga acccgaagcc cagggccacc gaggtctccg gctcatacgt  840
   cagtgcggcg tcgttgagtt gcagcttgtc cagcgcgtca cgcagatccg ggtagtccga  900
   accgtcgacg ggatacaggc ccgagtacac catcggcttg ggctcgcgat aacccgtgag  960
   cgcctcggtc gcgcccttgc gcgccgtggt caccgtgtca ccgaccttcg actggcggac 1020
   gtccttcaca cccgtgatca ggtaaccgac ctcaccgacg cccaggcccg cactggcctt 1080
   cggttccggt gagacgatgc cgacctcgag cagttcatgg gtggcgccgg tggacatcat 1140
   cgcgatgcgt tcgcgcggca cgatcttgcc gtcgaccaca cggacgtagg tcaccacgcc 1200
   gcggtagatg tcgtacacgg agtcgaagat catcgcgcgc gtcggggcgt cggggtcacc 1260
   gaccggcggc ggcaccttac gcaccacctc gtcgagcagc tcggccacgc cttcgccggt 1320
   cttgcccgag acacgcagca cgtccgacgg ctcacacccg atgatgtggg cgagctcgtc 1380
   ggcatagcgg tccgggtcag cggcgggcag gtcgatcttg ttgagcaccg ggatgatcgc 1440
   caggtcgcgg tccagcgcca ggtacaggtt ggccagcgtc tgcgcctcga tgccctgcgc 1500
   cgcgtcgacc agcagcaccg cgccctcgca ggcctccagc gcgcgcgaca cctcgtaggt 1560
   gaagtcgacg tggcccgggg tgtcgatcag gtgcagcacg taatcacccg cgtccgcgcc 1620
   gtcttggccg tccttcagcg tccacggaag ccggacgttc tgagccttga tggtgatccc 1680
   gcgctcacgt tcgatgtcca tgcggtcgag gtactgggcc cgcatcgacc gctcatcgac 1740
   aacaccggtg agctgcagca tccggtcggc cagcgtcgac ttgccgtggt cgatgtgggc 1800
   gatgatgcag aagttccgaa tctgcgccgg cgcagtgaac gtcttgtcgg cgaagctgct 1860
   gatgggaatc tcctggtgag cgtgggtcaa gcgcac                          1896
<210> 85
   <211> 19
   <212> DNA
   <213> Artificial Sequence
<220>
   <223> Primer
<400> 85
   taccagcttc agtttccgt 19
<210> 86
   <211> 21
   <212> DNA
   <213> Artificial Sequence
<220>
   <223> Primer
<400> 86
   gcacctatat cttcttagcc g 21
<210> 87
   <211> 18
   <212> DNA
   <213> Artificial Sequence
<220>
   <223> Primer
<400> 87
   ggatgtcgac cttgacca 18
<210> 88
   <211> 17
   <212> DNA
   <213> Artificial Sequence
<220>
   <223> Primer
<400> 88
   ctgatcaccg gcgtcaa 17
<210> 89
   <211> 15
   <212> DNA
   <213> Artificial Sequence
<220>
   <223> Primer
<400> 89
   tgtcggcggc cacgt 15
<210> 90
   <211> 21
   <212> DNA
   <213> Artificial Sequence
<220>
   <223> Primer
<400> 90
   gaagtccagc atcttggcgt t 21
<210> 91
   <211> 15
   <212> DNA
   <213> Artificial Sequence
<220>
   <223> Primer
<400> 91
   tgtcggcggc cacgt 15
<210> 92
   <211> 17
   <212> DNA
   <213> Artificial Sequence
<220>
   <223> Primer
<400> 92
   atcgtgcagt gcggcca 17
<210> 93
   <211> 20
   <212> DNA
   <213> Artificial Sequence
<220>
   <223> Primer
<400> 93
   gcagcattgt ggttgaccga 20
<210> 94
   <211> 17
   <212> DNA
   <213> Artificial Sequence
<220>
   <223> Primer
<400> 94
   tcccagcgtt gcgcctt 17
<210> 95
   <211> 18
   <212> DNA
   <213> Artificial Sequence
<220>
   <223> Primer
<400> 95
   tgatgcggct gctcaagc 18
<210> 96
   <211> 21
   <212> DNA
   <213> Artificial Sequence
<220>
   <223> Primer
<400> 96
   tgtcaaggga catggggaac t 21
<210> 97
   <211> 19
   <212> DNA
   <213> Artificial Sequence
<220>
   <223> Primer
<400> 97
   taccagcttc agtttccgt 19
<210> 98
   <211> 19
   <212> DNA
   <213> Artificial Sequence
<220>
   <223> Probe
<400> 98
   atggtgcgca gttcactgc 19
<210> 99
   <211> 21
   <212> DNA
   <213> Artificial Sequence
<220>
   <223> Probe
<400> 99
   gcacctatat cttcttagcc g 21
<210> 100
   <211> 17
   <212> DNA
   <213> Artificial Sequence
<220>
   <223> Primer
<400> 100
   agaccgtgcg gatcttg 17
<210> 101
   <211> 20
   <212> DNA
   <213> Artificial Sequence
<220>
   <223> Probe
<400> 101
   acggattggt cacccggatt 20
<210> 102
   <211> 18
   <212> DNA
   <213> Artificial Sequence
<220>
   <223> Primer
<400> 102
   catggagatc acccgtga 18
<210> 103
   <211> 21
   <212> DNA
   <213> Artificial Sequence
<220>
   <223> Primer
<400> 103
   atgtggtttc agtacgactt c 21
<210> 104
   <211> 20
   <212> DNA
   <213> Artificial Sequence
<220>
   <223> Probe
<400> 104
   tgagaggtgt tggcacgcaa 20
<210> 105
   <211> 20
   <212> DNA
   <213> Artificial Sequence
<220>
   <223> Primer
<400> 105
   gatggcagtg tcttatccaa 20
<210> 106
   <211> 17
   <212> DNA
   <213> Artificial Sequence
<220>
   <223> Primer
<400> 106
   agaccgtgcg gatcttg 17
<210> 107
   <211> 19
   <212> DNA
   <213> Artificial Sequence
<220>
   <223> Probe
<400> 107
   acgaccttct cggaaccgt 19
<210> 108
   <211> 18
   <212> DNA
   <213> Artificial Sequence
<220>
   <223> Primer
<400> 108
   catggagatc acccgtga 18
<210> 109
   <211> 681
   <212> DNA
   <213> Mycobacterium tuberculosis
<400> 109
   ttactttgcc gcgacgacga atccggcgat gatcgcctcg atgtcggaag cgtgcttgac 60
   ggcctcgttg gccagactcg tgatggtgag ctgcaccagg tagcgctgct tggccggcgg 120
   tgcgccggtt gggaagacga tccggttcca ggtgtgcagt cgcctgccgt gcaggtcata 180
   actgccctga atcatcgagg acggaaaccc gttgaagtct gccgtcgagg agtccaattc 240
   ggtgaagttc gtcgacagcc gggcatcggc agtgccatgc ttgagcgctt cggcgatatc 300
   gaagtcccgg tgcagcttga acaccatgag catggccgtt ggatagcttt cgcccttggc 360
   gatcatctcc gtgttcgggg tgatgttcgg atttttcatc ggtgcccagc ccggtggtgt 420
   cggaatcgac acggtcaggt cggtcaggct gctcggtgcc accggctctc cggtgacgcc 480
   gacgctttcc agatacttcc acagcgggac cggcacttcc gtcgtggtcg agacggcgct 540
   ggtggttggg ctcgtggaca aaatcgactg gaagtcaggc gatttcggtc cgcaagcgac 600
   cgctgacatt gccagcgtgg ctaccgcgac cgcgaccgcc aagggtctca cagaatcttg 660
   cggacagcgt cgaccggcca a 681
<210> 110
   <211> 681
   <212> DNA
   <213> Mycobacterium tuberculosis
<400> 110
   ttactttgcc gcgacgacga atccggcgat gatcgcctcg atgtcggaag cgtgcttgac 60
   ggcctcgttg gccagactcg tgatggtgag ctgcaccagg tagcgctgct tggccggcgg 120
   tgcgccggtt gggaagacga tccggttcca ggtgtgcagt cgcctgccgt gcaggtcata 180
   actgccctga atcatcgagg acggaaaccc gttgaagtct gccgtcgagg agtccaattc 240
   ggtgaagttc gtcgacagcc gggcatcggc agtgccatgc ttgagcgctt cggcgatatc 300
   gaagtcccgg tgcagcttga acaccatgag catggccgtt ggatagcttt cgcccttggc 360
   gatcatctcc gtgttcgggg tgatgttcgg atttttcatc ggtgcccagc ccggtggtgt 420
   cggaatcgac acggtcaggt cggtcaggct gctcggtgcc accggctctc cggtgacgcc 480
   gacgctttcc agatacttcc acagcgggac cggcacttcc gtcgtggtcg agacggcgct 540
   ggtggttggg ctcgtggaca aaatcgactg gaagtcaggc gatttcggtc cgcaagcgac 600
   cgctgacatt gccagcgtgg ctaccgcgac cgcgaccgcc aagggtctca cagaatcttg 660
   cggacagcgt cgaccggcca a 681
<210> 111
   <211> 681
   <212> DNA
   <213> Mycobacterium tuberculosis
<400> 111
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgg  120
   tgcgccggtt gggaagacga tccggttcca ggtgtgcagt cgcctgccgt gcaggtcata  180
   actgccctga atcatcgagg acggaaaccc gttgaagtct gccgtcgagg agtccaattc  240
   ggtgaagttc gtcgacagcc gggcatcggc agtgccatgc ttgagcgctt cggcgatatc  300
   gaagtcccgg tgcagcttga acaccatgag catggccgtt ggatagcttt cgcccttggc  360
   gatcatctcc gtgttcgggg tgatgttcgg atttttcatc ggtgcccagc ccggtggtgt  420
   cggaatcgac acggtcaggt cggtcaggct gctcggtgcc accggctctc cggtgacgcc  480
   gacgctttcc agatacttcc acagcgggac cggcacttcc gtcgtggtcg agacggcgct  540
   ggtggttggg ctcgtggaca aaatcgactg gaagtcaggc gatttcggtc cgcaagcgac  600
   cgctgacatt gccagcgtgg ctaccgcgac cgcgaccgcc aagggtctca cagaatcttg  660
   cggacagcgt cgaccggcca a                                            681
<210> 112
   <211> 681
   <212> DNA
   <213> Mycobacterium tuberculosis
<400> 112
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgg  120
   tgcgccggtt gggaagacga tccggttcca ggtgtgcagt cgcctgccgt gcaggtcata  180
   actgccctga atcatcgagg acggaaaccc gttgaagtct gccgtcgagg agtccaattc  240
   ggtgaagttc gtcgacagcc gggcatcggc agtgccatgc ttgagcgctt cggcgatatc  300
   gaagtcccgg tgcagcttga acaccatgag catggccgtt ggatagcttt cgcccttggc  360
   gatcatctcc gtgttcgggg tgatgttcgg atttttcatc ggtgcccagc ccggtggtgt  420
   cggaatcgac acggtcaggt cggtcaggct gctcggtgcc accggctctc cggtgacgcc  480
   gacgctttcc agatacttcc acagcgggac cggcacttcc gtcgtggtcg agacggcgct  540
   ggtggttggg ctcgtggaca aaatcgactg gaagtcaggc gatttcggtc cgcaagcgac  600
   cgctgacatt gccagcgtgg ctaccgcgac cgcgaccgcc aagggtctca cagaatcttg  660
   cggacagcgt cgaccggcca a                                            681
<210> 113
   <211> 759
   <212> DNA
   <213> Mycobacterium bovis BCG
<400> 113
   ctaggctaac ccatggctac tgcattgggg aaattcgatc cttgtgagct gctcggatag   60
   ctgtgcccca accgtgcgga caattacttt gccgcgacga cgaatccggc gatgatcgcc  120
   tcgatgtcgg aagcgtgctt gacggcctcg ttggccagac tcgtgatggt gagctgcacc  180
   aggtagcgct gcttggccgg cgccggttgg gaagacgatc cggttccagg tgtgcagtcg  240
   cctgccgtgc aggtcataac tgccctgaat catcgaggac ggaaacccgt tgaagtctgc  300
   cgtcgaggag tccaattcgg tgaagttcgt cgacagccgg gcatcggcag tgccatgctt  360
   gagcgcttcg gcgatatcga agtcccggtg cagcttgaac accatgagca tggccgttgg  420
   atagctttcg cccttggcga tcatctccgt gttcggggtg atgttcggat ttttcatcgg  480
   tgcccagccc ggtggtgtcg gaatcgacac ggtcaggtcg gtcaggctgc tcggtgccac  540
   cggctctccg gtgacgccga cgctttccag atacttccac agcgggaccg gcacttccgt  600
   cgtggtcgag acggcgctgg tggttgggct cgtggacaaa atcgactgga agtcaggcga  660
   tttcggtccg caagcgaccg ctgacattgc cagcgtggct accgcgaccg cgaccgccaa  720
   gggtctcaca gaatcttgcg gacagcgtcg accggccaa                         759
<210> 114
   <211> 759
   <212> DNA
   <213> Mycobacterium bovis BCG
<400> 114
   ctaggctaac ccatggctac tgcattgggg aaattcgatc cttgtgagct gctcggatag 60
   ctgtgcccca accgtgcgga caattacttt gccgcgacga cgaatccggc gatgatcgcc 120
   tcgatgtcgg aagcgtgctt gacggcctcg ttggccagac tcgtgatggt gagctgcacc 180
   aggtagcgct gcttggccgg cgccggttgg gaagacgatc cggttccagg tgtgcagtcg 240
   cctgccgtgc aggtcataac tgccctgaat catcgaggac ggaaacccgt tgaagtctgc 300
   cgtcgaggag tccaattcgg tgaagttcgt cgacagccgg gcatcggcag tgccatgctt 360
   gagcgcttcg gcgatatcga agtcccggtg cagcttgaac accatgagca tggccgttgg 420
   atagctttcg cccttggcga tcatctccgt gttcggggtg atgttcggat ttttcatcgg 480
   tgcccagccc ggtggtgtcg gaatcgacac ggtcaggtcg gtcaggctgc tcggtgccac 540
   cggctctccg gtgacgccga cgctttccag atacttccac agcgggaccg gcacttccgt 600
   cgtggtcgag acggcgctgg tggttgggct cgtggacaaa atcgactgga agtcaggcga 660
   tttcggtccg caagcgaccg ctgacattgc cagcgtggct accgcgaccg cgaccgccaa 720
   gggtctcaca gaatcttgcg gacagcgtcg accggccaa 759
<210> 115
   <211> 759
   <212> DNA
   <213> Mycobacterium bovis
<400> 115
   ctaggctaac ccatggctac tgcattgggg aaattcgatc cttgtgagct gctcggatag 60
   ctgtgcccca accgtgcgga caattacttt gccgcgacga cgaatccggc gatgatcgcc 120
   tcgatgtcgg aagcgtgctt gacggcctcg ttggccagac tcgtgatggt gagctgcacc 180
   aggtagcgct gcttggccgg cgccggttgg gaagacgatc cggttccagg tgtgcagtcg 240
   cctgccgtgc aggtcataac tgccctgaat catcgaggac ggaaacccgt tgaagtctgc 300
   cgtcgaggag tccaattcgg tgaagttcgt cgacagccgg gcatcggcag tgccatgctt 360
   gagcgcttcg gcgatatcga agtcccggtg cagcttgaac accatgagca tggccgttgg 420
   atagctttcg cccttggcga tcatctccgt gttcggggtg atgttcggat ttttcatcgg 480
   tgcccagccc ggtggtgtcg gaatcgacac ggtcaggtcg gtcaggctgc tcggtgccac 540
   cggctctccg gtgacgccga cgctttccag atacttccac agcgggaccg gcacttccgt 600
   cgtggtcgag acggcgctgg tggttgggct cgtggacaaa atcgactgga agtcaggcga 660
   tttcggtccg caagcgaccg ctgacattgc cagcgtggct accgcgaccg cgaccgccaa 720
   gggtctcaca gaatcttgcg gacagcgtcg accggccaa 759
<210> 116
   <211> 681
   <212> DNA
   <213> Mycobacterium africanum
<400> 116
   ttactttgcc gcgacgacga atccggcgat gatcgcctcg atgtcggaag cgtgcttgac 60
   ggcctcgttg gccagactcg tgatggtgag ctgcaccagg tagcgctgct tggccggcgg 120
   tgcgccggtt gggaagacga tccggttcca ggtgtgcagt cgcctgccgt gcaggtcata 180
   actgccctga atcatcgagg acggaaaccc gttgaagtct gccgtcgagg agtccaattc 240
   ggtgaagttc gtcgacagcc gggcatcggc agtgccatgc ttgagcgctt cggcgatatc 300
   gaagtcccgg tgcagcttga acaccatgag catggccgtt ggatagcttt cgcccttggc 360
   gatcatctcc gtgttcgggg tgatgttcgg atttttcatc ggtgcccagc ccggtggtgt 420
   cggaatcgac acggtcaggt cggtcaggct gctcggtgcc accggctctc cggtgacgcc 480
   gacgctttcc agatacttcc acagcgggac cggcacttcc gtcgtggtcg agacggcgct 540
   ggtggttggg ctcgtggaca aaatcgactg gaagtcaggc gatttcggtc cgcaagcgac 600
   cgctgacatt gccagcgtgg ctaccgcgac cgcgaccgcc aagggtctca cagaatcttg 660
   cggacagcgt cgaccggcca a 681
<210> 117
   <211> 681
   <212> DNA
   <213> Mycobacterium africanum
<400> 117
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgg  120
   tgcgccggtt gggaagacga tccggttcca ggtgtgcagt cgcctgccgt gcaggtcata  180
   actgccctga atcatcgagg acggaaaccc gttgaagtct gccgtcgagg agtccaattc  240
   ggtgaagttc gtcgacagcc gggcatcggc agtgccatgc ttgagcgctt cggcgatatc  300
   gaagtcccgg tgcagcttga acaccatgag catggccgtt ggatagcttt cgcccttggc  360
   gatcatctcc gtgttcgggg tgatgttcgg atttttcatc ggtgcccagc ccggtggtgt  420
   cggaatcgac acggtcaggt cggtcaggct gctcggtgcc accggctctc cggtgacgcc  480
   gacgctttcc agatacttcc acagcgggac cggcacttcc gtcgtggtcg agacggcgct  540
   ggtggttggg ctcgtggaca aaatcgactg gaagtcaggc gatttcggtc cgcaagcgac  600
   cgctgacatt gccagcgtgg ctaccgcgac cgcgaccgcc aagggtctca cagaatcttg  660
   cggacagcgt cgaccggcca a                                            681
<210> 118
   <211> 681
   <212> DNA
   <213> Mycobacterium canettii
<400> 118
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgg  120
   tgcgccggtt gggaagacga tccggttcca ggtgtgcagt cgcctgccgt gcaggtcata  180
   actgccctga atcatcgagg acggaaaccc gttgaagtct gccgtcgagg agtccaattc  240
   ggtgaagttc gtcgacagcc gggcatcggc agtgccatgc ttgagcgctt cggcgatatc  300
   gaagtcccgg tgcagcttga acaccatgag catggccgtt ggatagcttt cgcccttggc  360
   gatcatctcc gtgttcgggg tgatgttcgg atttttcatc ggtgcccagc ccggtggtgt  420
   cggaatcgac acggtcaggt cggtcaggct gctcggtgcc accggctctc cggtgacgcc  480
   gacgctttcc agatacttcc acagcgggac cggcacttcc gtcgtggtcg agacggcgct  540
   ggtggttggg ctcgtggaca aaatcgactg gaagtcaggc gatttcggtc cgcaagcgac  600
   cgctgacatt gccagcgtgg ctaccgcgac cgcgaccgcc aagggtctca cagaatcttg  660
   cggacagcgt cgaccggcca a                                            681
<210> 119
   <211> 681
   <212> DNA
   <213> Mycobacterium microti
<400> 119
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgg  120
   tgcgccggtt gggaagacga tccggttcca ggtgtgcagt cgcctgccgt gcaggtcata  180
   actgccctga atcatcgagg acggaaaccc gttgaagtct gccgtcgagg agtccaattc  240
   ggtgaagttc gtcgacagcc gggcatcggc agtgccatgc ttgagcgctt cggcgatatc  300
   gaagtcccgg tgcagcttga acaccatgag catggccgtt ggatagcttt cgcccttggc  360
   gatcatctcc gtgttcgggg tgatgttcgg atttttcatc ggtgcccagc ccggtggtgt  420
   cggaatcgac acggtcaggt cggtcaggct gctcggtgcc accggctctc cggtgacgcc  480
   gacgctttcc agatacttcc acagcgggac cggcacttcc gtcgtggtcg agacggcgct  540
   ggtggttggg ctcgtggaca aaatcgactg gaagtcaggc gatttcggtc cgcaagcgac  600
   cgctgacatt gccagcgtgg ctaccgcgac cgcgaccgcc aagggtctca cagaatcttg  660
   cggacagcgt cgaccggcca a                                            681
<210> 120
   <211> 660
   <212> DNA
   <213> Mycobacterium avium
<400> 120
   ctacttcgcc gccaccacga agccgtggat gatcgcctcg atgtcggtcg actccgcggc   60
   ggcctgatcg gccaggctgg tgatcgtgag ctgcaccaga tagcgctgat gcgccggcgg  120
   cgatccggtg gcgatgacga tccggttcca gctgtgcagc cgcatgccgt ccaggtcgta  180
   gctgccctgg atcatcgacg agggaaagcc gttgtacggg gcgccggacg cgtccagctg  240
   cttgaagttc tcgaacagct gcgcgtcgtc gttgccgtgc ttgatgactt gggccgggtc  300
   gaaatcgccg ctcagcttga agaccaccag ccgcgccgtg gggaacttgc cgcccttgga  360
   gatcatcacc gtctgcgggc tgatgttcgg gctgctgaac ggcgcccagc ccggcggggt  420
   cgggatcgac accgtcagat ccggcagcga cgccggggcc acctgctgcc cggtgacacc  480
   gatgctctgc agatactgcg acagcgggac cggcttcgcc gtggcgctgg tggtggtggt  540
   cgtcgtcggc gtcttcgaca ggatcgattg gtagtcgggc ggtttcggtg cgcagccggc  600
   ggtggccacg gccagtgcaa ccgccgcggc gacggccgcg cagccgcgga gtcggttcac  660
<210> 121
   <211> 660
   <212> DNA
   <213> Mycobacterium avium
<400> 121
   ctacttcgcc gccaccacga agccgtggat gatcgcctcg atgtcggtcg actccgcggc   60
   ggcctgatcg gccaggctgg tgatcgtgag ctgcaccaga tagcgctgat gcgccggcgg  120
   cgatccggtg gcgatgacga tccggttcca gctgtgcagc cgcatgccgt ccaggtcgta  180
   gctgccctgg atcatcgacg agggaaagcc gttgtacggg gcgccggacg cgtccagctg  240
   cttgaagttc tcgaacagct gcgcgtcgtc gttgccgtgc ttgatgactt gggccgggtc  300
   gaaatcgccg ctcagcttga agaccaccag ccgcgccgtg gggaacttgc cgcccttgga  360
   gatcatcacc gtctgcgggc tgatgttcgg gctgctgaac ggcgcccagc ccggcggggt  420
   cgggatcgac accgtcagat ccggcagcga cgccggggcc acctgctgcc cggtgacacc  480
   gatgctctgc agatactgcg acagcgggac cggcttcgcc gtggcgctgg tggtggtggt  540
   cgtcgtcggc gtcttcgaca ggatcgattg gtagtcgggc ggtttcggtg cgcagccggc  600
   ggtggccacg gccagtgcaa ccgctgcggc gactgccgcg cagccgcgga gtcggttcac  660
<210> 122
   <211> 657
   <212> DNA
   <213> Mycobacterium leprae
<400> 122
   ttacttgggg gcgacgacaa agccgcggat gatagcctcg atatcgtttg attgtgcgac   60
   cgcctcgttg gccaagctgg taatggtgag ctgaacgaga tactgttgct tagagggtgg  120
   tggaccggtg gggatcacga ttcggttcca ggcatgtagt cgcctgccct cgaggtcata  180
   gctgccttgg atcattgccg acggaaaacc gttgtagttt gccgtcgaaa cgtccagctg  240
   cctgaagttc tcgaagagtt gggcatcgtc gttcccgtgt ttgatgactt gggttgggtc  300
   gaagtctccg cgcagcttga acgctacgag ccttgccgtc gggtacttgc cgcttttggc  360
   gatgatcagc gtctccggtg tgatgttcgg attgctatac ggcgaccagc ccggtggggt  420
   cggtatcgac acggtcagac cgggcaggga gctcggcgcc acctgctgcc cggtgacgcc  480
   gatactttcc agatattgcg gcaaagggat gggcttgtcg ggtgtggtcg tagtggttgt  540
   agaacttttc gacaggatta attggtagtc aggggttttc gtcccgcagg agaccgcgga  600
   tatgctcagc gtgactaccg cggcagcggt gtgcagcccg agacggattg cctgcat     657
<210> 123
   <211> 657
   <212> DNA
   <213> Mycobacterium marinum
<400> 123
   tcacttcacc gcaaccacga atccgttgat gatcgactcg atgtcggatg cgctgccggc   60
   ggcctgactg gccaggctgg tgatggtcag ttggaccaga taccgctgat tgtccggcgg  120
   cggcccggtg ggaatgacga tccgattcca gctgtgcatg cgggcaccct cgaggtcgta  180
   gctgccctgc atcatcgacg agggaaaacc gtgaaagtct gccgacgagg cgtccagctg  240
   tttgaagttc tcgaacagtt gcgcgtcgtc gttgccgcgc ctggcgacgt cggcggggtc  300
   gaagttcccg cgcagcaaga acacgaccag tctggccgtg gggtagtggc  cgcccttgga  360
   gatgatcacc gtttccggat tgatcttcgg gccctcgtag ggggaccagc ccggtggtgt  420
   cgggatcgac acggtaaggc ctttgaggtc gctcggcgcg atctgctttc cgctgacccc  480
   gatgctttcc aggtactgcg acagcggcac gggtggggcg gtggtggcgg tggtgctggt  540
   tgttgcgctc gtggtccaga tggatttgta gtcagggggc tcgggtgtgc tgcaggcaac  600
   cgccggcatc gtgaccgcga gtgaaacgac ggccgctgcg atccggcgag ctgtcac     657
<210> 124
   <211> 657
   <212> DNA
   <213> Mycobacterium ulcerans
<400> 124
   tcacttcacc gcaaccacga atccgttgat gatcgactcg atgtcggatg cgctgccggc   60
   ggcctgactg gccaggctgg tgatggtcag ttggaccaga taccgctggt tgtccggcgg  120
   cggcccggtg ggaatgacga tccgattcca gctgtgcatg cgggcaccct cgaggtcgta  180
   gctgccctgc atcatcgacg agggaaaacc gtgaaagtct gccgatgagg cgtccagctg  240
   tttgaagttc tcgaacagtt gcgcgtcgtc gttgccgcgc ctggcgacgt cggcggggtc  300
   gaagttcccg cgcagcaaga acacgaccag tctggccgtg gggtagtggc  cgcccttgga  360
   gatgatcacc gtttccgggt tgatcttcgg gccctcgtag ggggaccagc ccggtggtgt  420
   cgggatcgat acggtaaggc ctttgaggtc gctcggcgcg atctgctttc cgctgacccc  480
   gatgctttcc aggtactgcg acagcggcac gggcggggcg gtggtggcgg tggtgctggt  540
   tgttgcgctc gtggtccaga tggatttgta gtcagggggc tcgggtgtgc tgcaggcaac  600
   cgccggcatc gtgaccgcga gtgaaacgac ggccgctgcg atccggcgag ctgtcac     657
<210> 125
   <211> 641
   <212> DNA
   <213> Artificial Sequence
<220>
   <223> Mycobacterium africanum DNA fragment
<400> 125
   ttactttgcc gcgacgacga atccggcgat gatcgcctcg atgtcggaag cgtgcttgac 60
   ggcctcgttg gccagactcg tgatggtgag ctgcaccagg tagcgctgct tggccggcgg 120
   tgcgccggtt gggaagacga tccggttcca ggtgtgcagt cgcctgccgt gcaggtcata 180
   actgccctga atcatcgagg acggaaaccc gttgaagtct gccgtcgagg agtccaattc 240
   ggtgaagttc gtcgacagcc gggcatcggc agtgccatgc ttgagcgctt cggcgatatc 300
   gaagtcccgg tgcagcttga acaccatgag catggccgtt ggatagcttt cgcccttggc 360
   gatcatctcc gtgttcgggg tgatgttcgg atttttcatc ggtgcccagc ccggtggtgt 420
   cggaatcgac acggtcaggt cggtcaggct gctcggtgcc accggctctc cggtgacgcc 480
   gacgctttcc agatacttcc acagcgggac cggcacttcc gtcgtggtcg agacggcgct 540
   ggtggttggg ctcgtggaca aaatcgactg gaagtcaggc gatttcggtc cgcaagcgac 600
   cgctgacatt gccagcgtgg ctaccgcgac cgcgaccgcc a 641
<210> 126
   <211> 641
   <212> DNA
   <213> Artificial Sequence
<220>
   <223> Mycobacterium microti DNA fragment
<400> 126
   ttactttgcc gcgacgacga atccggcgat gatcgcctcg atgtcggaag cgtgcttgac 60
   ggcctcgttg gccagactcg tgatggtgag ctgcaccagg tagcgctgct tggccggcgg 120
   tgcgccggtt gggaagacga tccggttcca ggtgtgcagt cgcctgccgt gcaggtcata 180
   actgccctga atcatcgagg acggaaaccc gttgaagtct gccgtcgagg agtccaattc 240
   ggtgaagttc gtcgacagcc gggcatcggc agtgccatgc ttgagcgctt cggcgatatc 300
   gaagtcccgg tgcagcttga acaccatgag catggccgtt ggatagcttt cgcccttggc 360
   gatcatctcc gtgttcgggg tgatgttcgg atttttcatc ggtgcccagc ccggtggtgt 420
   cggaatcgac acggtcaggt cggtcaggct gctcggtgcc accggctctc cggtgacgcc 480
   gacgctttcc agatacttcc acagcgggac cggcacttcc gtcgtggtcg agacggcgct 540
   ggtggttggg ctcgtggaca aaatcgactg gaagtcaggc gatttcggtc cgcaagcgac 600
   cgctgacatt gccagcgtgg ctaccgcgac cgcgaccgcc a 641
<210> 127
   <211> 641
   <212> DNA
   <213> Artificial Sequence
<220>
   <223> Mycobacterium pinnipedii DNA fragment
<400> 127
   ttactttgcc gcgacgacga atccggcgat gatcgcctcg atgtcggaag cgtgcttgac 60
   ggcctcgttg gccagactcg tgatggtgag ctgcaccagg tagcgctgct tggccggcgg 120
   tgcgccggtt gggaagacga tccggttcca ggtgtgcagt cgcctgccgt gcaggtcata 180
   actgccctga atcatcgagg acggaaaccc gttgaagtct gccgtcgagg agtccaattc 240
   ggtgaagttc gtcgacagcc gggcatcggc agtgccatgc ttgagcgctt cggcgatatc 300
   gaagtcccgg tgcagcttga acaccatgag catggccgtt ggatagcttt cgcccttggc 360
   gatcatctcc gtgttcgggg tgatgttcgg atttttcatc ggtgcccagc ccggtggtgt 420
   cggaatcgac acggtcaggt cggtcaggct gctcggtgcc accggctctc cggtgacgcc 480
   gacgctttcc agatacttcc acagcgggac cggcacttcc gtcgtggtcg agacggcgct 540
   ggtggttggg ctcgtggaca aaatcgactg gaagtcaggc gatttcggtc cgcaagcgac 600
   cgctgacatt gccagcgtgg ctaccgcgac cgcgaccgcc a 641
<210> 128
   <211> 636
   <212> DNA
   <213> Artificial Sequence
<220>
   <223> Mycobacterium caprae DNA fragment
<400> 128
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgc  120
   cggttgggaa gacgatccgg ttccaggtgt gcagtcgcct gccgtgcagg tcataactgc  180
   cctgaatcat cgaggacgga aacccgttga agtctgccgt cgaggagtcc aattcggtga  240
   agttcgtcga cagccgggca tcggcagtgc catgcttgag cgcttcggcg atatcgaagt  300
   cccggtgcag cttgaacacc atgagcatgg ccgttggata gctttcgccc ttggcgatca  360
   tctccgtgtt cggggtgatg ttcggatttt tcatcggtgc ccagcccggt ggtgtcggaa  420
   tcgacacggt caggtcggtc aggctgctcg gtgccaccgg ctctccggtg acgccgacgc  480
   tttccagata cttccacagc gggaccggca cttccgtcgt ggtcgagacg gcgctggtgg  540
   ttgggctcgt ggacaaaatc gactggaagt caggcgattt cggtccgcaa gcgaccgctg  600
   acattgccag cgtggctacc gcgaccgcga ccgcca                            636
<210> 129
   <211> 636
   <212> DNA
   <213> Artificial Sequence
<220>
   <223> Mycobacterium caprae DNA fragment
<400> 129
   ttactttgcc gcgacgacga atccggcgat gatcgcctcg atgtcggaag cgtgcttgac   60
   ggcctcgttg gccagactcg tgatggtgag ctgcaccagg tagcgctgct tggccggcgc  120
   cggttgggaa gacgatccgg ttccaggtgt gcagtcgcct gccgtgcagg tcataactgc  180
   cctgaatcat cgaggacgga aacccgttga agtctgccgt cgaggagtcc aattcggtga  240
   agttcgtcga cagccgggca tcggcagtgc catgcttgag cgcttcggcg atatcgaagt  300
   cccggtgcag cttgaacacc atgagcatgg ccgttggata gctttcgccc ttggcgatca  360
   tctccgtgtt cggggtgatg ttcggatttt tcatcggtgc ccagcccggt ggtgtcggaa  420
   tcgacacggt caggtcggtc aggctgctcg gtgccaccgg ctctccggtg acgccgacgc  480
   tttccagata cttccacagc gggaccggca cttccgtcgt ggtcgagacg gcgctggtgg  540
   ttgggctcgt ggacaaaatc gactggaagt caggcgattt cggtccgcaa gcgaccgctg  600
   acattgccag cgtggctacc gcgaccgcga ccgcca                            636
<210> 130
   <211> 2081
   <212> DNA
   <213> Mycobacterium tuberculosis
<400> 130
   ctgtgcaggt ggtcgtttcg aaggctaccc acgccaagct caaggagctg gcgcgcagcc 60
   ggaagatgag cgtatctaag ctgctgcgtc ccgtgctcga cgagttcgta cagcgagaaa 120
   cgggtcggat tctcccacgg cgttagcttg tgctcagccg ccgctcgacg tcgcgaagtc 180
   tggacagtca gctgtcgcag ccgtgaccag cggacatctc gggcagctag cccgacaggg 240
   tgcgcgtgca cctggcccgg gtggtaatcc attgacgcgc acggcaattg gccggctcgg 300
   tctcggtctg cggataccgc actgaagggc gacaattttg gcgaaaaggc cgtgtgcggt 360
   gccgggtcgc gctacgttca gattcaccta acaatgtcgt ccgccaacga gcgtgttcgc 420
   cggtggtggg gcgggcgggt tggggaggtg tgtgatgtcg tttgtcagcg tagccccgga 480
   gattgtggtg gccgcggcaa cagacctggc gggtatcgga tcggcgatca gcgcggccaa 540
   tgccgccgcg gctgcgccga ccaccgccgt gctggccgcg ggtgccgatg aggtgtcggc 600
   ggcgatcgcg gcgctgtttt ccggccacgc tcaggcctat caggcgctca gcgcccaggc 660
   ggcggcgttt catcagcagt tcgtgcagac gcttgccggt ggcgctggag catatgcggc 720
   cgccgaggcc caggtcgagc agcagctgct ggccgcgatc aacgcgccca cccaggcgct 780
   gctggggcgc cccttgatcg gcaacggtgc cgatggggcg ccggggactg ggcaggccgg 840
   cggggctggg gggatcttgt acggcaatgg cggcaatggc ggctccgggg cggctgggca 900
   ggccgggggt gccggcgggc cggccgggct gatcggccat ggcgggtccg gcggggccgg 960
   cggctccggc gcggccggcg gggccggcgg gcacggcgga tggctgtggg gcaacggcgg 1020
   cgtcggcgga tccggcgggg cgggtgtcgg cgcaggcgtg gctggcggtc acggcggtgc 1080
   gggcggtgcc gccgggctgt ggggcgccgg cggcggcggt ggcaatggcg ggaacggcgc 1140
   cgatgccaac atcgtcagcg gtggagacgg tggcctcggc ggtgccggtg gcggtggcgg 1200
   atggctctac ggcgacggcg gggccggcgg acacggcgga caaggcgcaa tcggcctcgg 1260
   cggcggcgcc ggcggcgacg gggccagggg cggcgccggc cgcggactgt ggggtactgg 1320
   cggcgccggc ggacacggcg ggcaaggcgg tggtaccggg ggcccaccgc tgcccggtca 1380
   ggcaggcatg ggcgccgcgg gtggcgccgg tgggctgatc ggcaacggcg gggccggcgg 1440
   cgacggcggt gtcggcgcgt ccggcggggt cgccggagta ggcggtgccg gcgggaacgc 1500
   catgctgatc gggcacggcg gcgccggcgg cgccggcgga gacagcagtt tcgctaatgg 1560
   cgcggccggc ggcgcgggcg gtgccggagg gcacctcttc ggcaatggcg ggtccggcgg 1620
   ccacggcgga gccgtcacgg ccggcaacac cggtatcggt ggcgccggcg gcgtcggtgg 1680
   ggacgccagg ctgatcggcc acggtggcgc cggcggtgcc ggcggggacc gcgccggagc 1740
   cttggttggc cgtgacggcg ggcccggtgg gaacgggggc gctggcggcc agctatacgg 1800
   caacggcggc gacggcgccc ccggcaccgg cggaacactg caggcggcgg tgagcggatt 1860
   ggtgacggct ttgttcggtg cacccggcca acccggcgac accggccaac ccggctagcc 1920
   ccgatcaacg agggtttcgg tgccggtccg gggcatggcc atccgctgag ctggcgatct 1980
   ggactacgtt ggtgtagaaa aatcctgccg cccggaccct taaggctggg acaatttctg 2040
   atagctaccc cgacacagga ggttacggga tgagcaattc g 2081
<210> 131
   <211> 2081
   <212> DNA
   <213> Mycobacterium tuberculosis
<400> 131
   ctgtgcaggt ggtcgtttcg aaggctaccc acgccaagct caaggagctg gcgcgcagcc 60
   ggaagatgag cgtatctaag ctgctgcgtc ccgtgctcga cgagttcgta cagcgagaaa 120
   cgggtcggat tctcccacgg cgttagcttg tgctcagccg ccgctcgacg tcgcgaagtc 180
   tggacagtca gctgtcgcag ccgtgaccag cggacatctc gggcagctag cccgacaggg 240
   tgcgcgtgca cctggcccgg gtggtaatcc attgacgcgc acggcaattg gccggctcgg 300
   tctcggtctg cggataccgc actgaagggc gacaattttg gcgaaaaggc cgtgtgcggt 360
   gccgggtcgc gctacgttca gattcaccta acaatgtcgt ccgccaacga gcgtgttcgc  420
   cggtggtggg gcgggcgggt tggggaggtg tgtgatgtcg tttgtcagcg tagccccgga  480
   gattgtggtg gccgcggcaa cagacctggc gggtatcgga tcggcgatca gcgcggccaa  540
   tgccgccgcg gctgcgccga ccaccgccgt gctggccgcg ggtgccgatg aggtgtcggc  600
   ggcgatcgcg gcgctgtttt ccggccacgc tcaggcctat caggcgctca gcgcccaggc  660
   ggcggcgttt catcagcagt tcgtgcagac gcttgccggt ggcgctggag catatgcggc  720
   cgccgaggcc caggtcgagc agcagctgct ggccgcgatc aacgcgccca cccaggcgct  780
   gctggggcgc cccttgatcg gcaacggtgc cgatggggcg ccggggactg ggcaggccgg  840
   cggggctggg gggatcttgt acggcaatgg cggcaatggc ggctccgggg cggctgggca  900
   ggccgggggt gccggcgggc cggccgggct gatcggccat ggcgggtccg gcggggccgg  960
   cggctccggc gcggccggcg gggccggcgg gcacggcgga tggctgtggg gcaacggcgg  1020
   cgtcggcgga tccggcgggg cgggtgtcgg cgcaggcgtg gctggcggtc acggcggtgc  1080
   gggcggtgcc gccgggctgt ggggcgccgg cggcggcggt ggcaatggcg ggaacggcgc  1140
   cgatgccaac atcgtcagcg gtggagacgg tggcctcggc ggtgccggtg gcggtggcgg  1200
   atggctctac ggcgacggcg gggccggcgg acacggcgga caaggcgcaa tcggcctcgg  1260
   cggcggcgcc ggcggcgacg gggccagggg cggcgccggc cgcggactgt ggggtactgg  1320
   cggcgccggc ggacacggcg ggcaaggcgg tggtaccggg ggcccaccgc tgcccggtca  1380
   ggcaggcatg ggcgccgcgg gtggcgccgg tgggctgatc ggcaacggcg gggccggcgg  1440
   cgacggcggt gtcggcgcgt ccggcggggt cgccggagta ggcggtgccg gcgggaacgc  1500
   catgctgatc gggcacggcg gcgccggcgg cgccggcgga gacagcagtt tcgttaatgg  1560
   cgcggccggc ggcgcgggcg gtgccggagg gcacctcttc ggcaatggcg ggtccggcgg  1620
   ccacggcgga gccgtcacgg ccggcaacac cggtatcggt ggcgccggcg gcgtcggtgg  1680
   ggacgccagg ctgatcggcc acggtggcgc cggcggtgcc ggcggggacc gcgccggagc  1740
   cttggttggc cgtgacggcg ggcccggtgg gaacgggggc gctggcggcc agctatacgg  1800
   caacggcggc gacggcgccc ccggcaccgg cggaacactg caggcggcgg tgagcggatt  1860
   ggtgacggct ttgttcggtg cacccggcca acccggcgac accggccaac ccggctagcc  1920
   ccgatcaacg agggtttcgg tgccggtccg gggcatggcc atccgctgag ctggcgatct  1980
   ggactacgtt ggtgtagaaa aatcctgccg cccggaccct taaggctggg acaatttctg  2040
   atagctaccc cgacacagga ggttacggga tgagcaattc g                      2081
<210> 132
   <211> 340
   <212> DNA
   <213> Mycobacterium africanum
<400> 132
   ctgtgcaggt ggtcgtttcg aaggctaccc acgccaagct caaggagctg gcgcgcagcc  60
   ggaagatgag cgtatctaag ctgctgcgtc ccgtgctcga cgagttcgta cagcgagaaa  120
   cgggtcggat tctcccacgg cgttagcttg tgctcagccg ccggccaacc cggctagccc  180
   cgatcaacga gggtttcggt gccggtccgg ggcatggcca tccgctgagc tggcgatctg  240
   gactacgttg gtgtagaaaa atcctgccgc ccggaccctt aaggctggga caatttctga  300
   tagctacccc gacacaggag gttacgggat gagcaattcg                        340
<210> 133
   <211> 340
   <212> DNA
   <213> Artificial Sequence
<220>
   <223> Mycobacterium africanum DNA fragment
<400> 133
   ctgtgcaggt ggtcgtttcg aaggctaccc acgccaagct caaggagctg gcgcgcagcc 60
   ggaagatgag cgtatctaag ctgctgcgtc ccgtgctcga cgagttcgta cagcgagaaa 120
   cgggtcggat tctcccacgg cgttagcttg tgctcagccg ccggccaacc cggctagccc 180
   cgatcaacga gggtttcggt gccggtccgg ggcatggcca tccgctgagc tggcgatctg 240
   gactacgttg gtgtagaaaa atcctgccgc ccggaccctt aaggctggga caatttctga 300
   tagctacccc gacacaggag gttacgggat gagcaattcg 340
<210> 134
   <211> 340
   <212> DNA
   <213> Artificial Sequence
<220>
   <223> Mycobacterium africanum DNA fragment
<400> 134
   ctgtgcaggt ggtcgtttcg aaggctaccc acgccaagct caaggagctg gcgcgcagcc 60
   ggaagatgag cgtatctaag ctgctgcgtc ccgtgctcga cgagttcgta cagcgagaaa 120
   cgggtcggat tctcccacgg cgttagcttg tgctcagccg ccggccaacc cggctagccc 180
   cgatcaacga gggtttcggt gccggtccgg ggcatggcca tccgctgagc tggcgatctg 240
   gactacgttg gtgtagaaaa atcctgccgc ccggaccctt aaggctggga caatttctga 300
   tagctacccc gacacaggag gttacgggat gagcaattcg 340
<210> 135
   <211> 1275
   <212> DNA
   <213> Mycobacterium smegmatis
<400> 135
   tcaggatcct ccggccttga agccacgcca gacgttgtgc cacgcggcgt cgttctcggc 60
   gtcgagttcc agcaggcggt atccggcgtc gaagtactcg ggtttgacga tcgccgacgt 120
   caggttctcg gggatggttc cgtccgcgac cagggtgtcg ggattgatcg acttctgcgg 180
   cggctggtag ccgatctgcg cgaagttctg ttgtgccgac accggatcca gcatgtggtt 240
   gaggaacagg tgcgccagca ccgggttctt gccgctcttg aggatgacca tgaggtcgtt 300
   gtccaccagg cccttgccat cggccgggaa ccagtactgc aggatctccg gcggggtgtt 360
   ctcgggcagg tagccgagcg cctggatgat gtcgcccgac cacatctggg cgaggccgat 420
   ctggcctgcg ggcacatcgt tgtacatcgt gatggtgacc ttgggggagg tcgcggccac 480
   catctgccgg agttgttcac cgaccatgtc cagatcggtc tgcgacgacg tgttgacatc 540
   ggtgataccg ttgcgcagca gtaccatcgc catcgcggtg tgccagtcgt cgatgatcgc 600
   ggtcttgttc ttgtatctcg gatcccacag cgcgtcatac ggattgggca acgcgccgat 660
   gtcctcgggg acctggtcgg tgcgccaccc gattccggtg gtgtagacgg tgtacggcgt 720
   ggtgtagcgc cactccttgt cgtaccacgg gttggtgaac accggccaca cgttctcgat 780
   gttcggaatg tagctgtggt tcaacggttt gagcaggcca ccgttgacca gccggctgat 840
   ctggtcgtag ctggggaagt agatgtcgta atcgacgttg ccgccgcgga tcttggtgat 900
   ggcttcgtcg gtgtcgttga acgtcgagac ctggaccttg gtctggtact tgtcctcgaa 960
   cgaggacacc gcgtcgggcg agatgtaatc ggcatagctg tacagctgca gggtggcgcc 1020
   cttctcgggt gccagcccgt cggcgatcgg ctcgttgtcg gaggcgatgt cccatgtcac 1080
   cgggctgctc ggtgacgcga tggtcaggct gggggccgac gacgacggcg gaccgccctt 1140
   ggagcacgcg tcgagcagca cgcccagcgc cggggcggtc gcggcgatca acgcggcacg 1200
   cgtcaggaac tgtctacggg tgggcccgga ccggggcatc ccgtgcggca gtgcgtttcc 1260
   ggtgggtcca ggcat 1275
<210> 136
   <211> 156
   <212> DNA
   <213> Artificial Sequence
<220>
   <223> Mycobacterium smegmatis DNA fragment
<400> 136
   caccgaccat gtccagatcg gtctgcgacg acgtgttgac atcggtgata ccgttgcgca  60
   gcagtaccat cgccatcgcg gtgtgccagt cgtcgatgat cgcggtcttg ttcttgtatc  120
   tcggatccca cagcgcgtca tacggattgg gcaacg                             156
<210> 137
   <211> 2799
   <212> DNA
   <213> Mycobacterium africanum
<400> 137
   ccatctgcgc tttcggtgct tcttcagctc ttgctggaac ttctggtaat gctccagcgc   60
   gaatcgctct tccaaagccc caagggcgtt aatgacctcc aggtgcgaaa ggaggagcag  120
   cccattcaac tgaaggactt gttcgggacc agttggcacg ttccgtccgt cctcggtgtt  180
   ggccagcaag aagtcgcgga gcgcctggct gctggtcata cgctcgggga tcttcatctg  240
   ccgcaagacg gtggcgatgt cggggtcggt caccacacct ctccttcgct aacgagtagt  300
   agcgcaagcg taagagaccg ctcccaggcc tacggatggg tctggggcta cggccgtgac  360
   agcgaaagca acgaaaagta acgagttgaa cgtcgcgggc ggctacgcca agcgctcacc  420
   actgggctgg tcgcgccggt cttccgggtc cttgtcatcc tcgtccgccg gcccggtggc  480
   cgagaccagc cctgctttgg agctgccgcc ggctggcgtt ccggccccca ttccgctgcc  540
   caccggggca gctccactca tcgccgacga tccggcgtta gcggcggtcg atgccatcgg  600
   cgaggatgcc ccctccaaca actgagccat cagcggggtg cgcgccgccg atccggcttc  660
   gccgggcagc gatcccgcgc gcatcagtcc cgcacctgcg cttgcgcccg acccacccgc  720
   gagcgggtgg cgcgatgtca ggccggctcc gatcgtcaac gcacccgccg cattgtcatc  780
   ggtatcccca tacatccgag cgatgtccgt caacgctgtc cccgctcgca tcagctcctc  840
   ttgagctgcc gtgttcgaag cgatcattga agcggcctcc gtcgcgaacg ccatcacagc  900
   ctgcgcggac acctcttcgc cccccgcggg taccagcccg gtcatggtcg gcatcgtcgc  960
   cgcgttgccg gccgccaggc cacggctacc gatctcgacc agttgcgatc cgatgtcgcc 1020
   agcggccgga tcgtgtgaca aggaatccat ctggttattg ctcctgtgtg tttgtgcgcg 1080
   gactcgaacg cttgtgacgc ccccgtagca atccccgcgg aaagccggcg cgactaccgc 1140
   cgcaaagccc ggtccggctg cgccggacaa taagacaatt ctagacccgc tgcgggttag 1200
   cagacccgcg aagccgcaga aatacgtttg cagccacctg accttgcgcc ggatcgccct 1260
   gtgcgaaggt cggaaccagc gttgctcgaa ggtgatgcac ccagccgcaa gtgtcgacct 1320
   attgcgcaaa tcacactgcg gcacgcggtc tgcctgcccg tgggaccgaa cacaacgaac 1380
   gaaacggtca gtcgcacccc tgagttcggt ctggcaaaca ccgaaacaat catgcgatct 1440
   gccggaataa atagctattt gcaacacttt cacatgcgta atgaaagttg ggcgtcaaac 1500
   aaaagctaag gcgtacgcaa attccatgcc ggggctcggc cgactgtgtc acacctgcca 1560
   tcgcgggcgg ggaagccgcc gttgtgtctt cggccgcaat gccgcgctga acgctaatgt 1620
   gtacggcgac accccggtgg cgatgcggac gccgcgcaga ccggcccgcg gggaggagca 1680
   cgaattgcgg ttcaatcggt tcagcgcgtc cacagctcgg ccgtgctgat ggataacctc 1740
   gagcggcttc gtggtcacct tttcgatcgg tgatgcgttg gccagctagt acaccgtcac 1800
   cgagagcgat aggtgctatt tcccttgccg tgctgggcgc ctgcggtgcg gccttggtgc 1860
   tgaccgcgcc gccggccaac caggcccgag ccgcggcgag cctgctgtca cgatcgaact 1920
   gggtgctcgc cactgcgttg cccgccagtc aggacgtccc ggccgattgg ggctactcgt 1980
   tgaccgggcg gttgcgacga gcggtctcgc caagcaccgt gccgccggcc gcgctgccta 2040
   acacgagccg agcagccgtc tattcgccgg ctggatgcgg aaacattccg aaaatcctgg 2100
   accactccag cgccgacttg gccgcctatg tccagataga ccgcgacgtg caggtgttcg 2160
   ggcaagatgc gcccctggat gctgccgcga ccggggaaag cgatgagcgc ggacccaacg 2220
   cccgcttcgc actctgggcc gttgccgacg gcccggcgcg gatcgccaac tacctggact 2280
   ggctaaaccg gtgcggttct taccaggtca ccaaccactt tttggacgga acggtcaaga 2340
   acgaacgaac cgtcaccacc gaggtggaag cgctttcggc cggcggtgcc gacgccgccg 2400
   tcgcggtcac aaggacgtta atcctctaac gcaccataga ttctctagcg acgattcttg 2460
   agctcccggc ctgtcgatgc cggcgctgca ggtgagtcac cgcagtgggc gcaccgaaca 2520
   ctcacttccg ccgccccaaa tccgcgcagt gaccaccgcg cggtcctcgc gagtctaggc 2580
   cagcatcgag tcgatcgcgg aacgtgggac caatacctgg gttgggccgg ctgcttcggg 2640
   cagcaactcc cccgggttga agaagaaaat caccccgtcg ttcgtgactg cgaagttctg 2700
   ataattcacc gggtccaagc cggcattcgg cgctatcgat acctgttgtc cggtctgctt 2760
   gctcagttca ccttgcacaa tggggaagac gactggcag                        2799
<210> 138
   <211> 4248
   <212> DNA
   <213> Mycobacterium tuberculosis
<400> 138
   ccatctgcgc tttcggtgct tcttcagctc ttgctggaac ttctggtaat gctccagcgc  60
   gaatcgctct tccaaagccc caagggcgtt aatgacctcg ggatctttga ccccaggggt  120
   cgatggccaa tctcaggttg gtaaatcggg tgctcagatc ggccctccgg accaggttgt  180
   cgcctgggca gatgtgcgct cgctaaccgc caactcactt tcaaactacg ctgcgagttg  240
   tgagcgtaat gtcagtgatc tgacggcaaa ggtcacggat ttcgtcgagc agatggacgg  300
   tatttcgcga aaagcggttc gacctactgg ctcctggtgt gtggcctccc agggtgctgg  360
   gctgcggttt cgccaaccaa cctgctggtc ggcgcgccgt attctgaaga ccggaccaac  420
   gaggggaccg agccatgtct cagacacccg ctacaacccg caaaacgttt cccgagatca  480
   gctcaagagc gtgggagcac cccgccgacc ggaccgccct ttccgcgctg cgccggctca  540
   aaggcttcga ccagatcttg aagctgatgt cggggatgtt gcgggaacgg cagcaccggc  600
   tgctgtacct ggccagcgcg gcacgggtcg ggccgcggca gttcgccgac ctcgacgcgc  660
   tgctggacga atgcgtggat gtgctggacg cgtcggcgaa acccgaactc tacgtgatgc  720
   agtcaccaat cgcggatgcc ttcaccatcg gcatgggcaa gccattcacc gtgatcacct  780
   cggggctgta cgacctggtg acacacgacg agatgcggtt cgtgatgggc cacgagctcg  840
   gccacgcact gtccggccac gcggtgtacc gcacgatgat gatgcatctg ctgcggttgg  900
   cccggtcatt cggcgtcttg ccggttggcg gctgggcgct gcgcgcaatc gtggctgcgc  960
   tgctggaatg gcagcgcaaa tcggagctgt ccggcgatcg cgctgggttg ctgtgcgcgc  1020
   aggatttgga caccgcgctc agggtggaga tgaagctcgc tggcggctgc cggctggaca  1080
   agctggactc ggaggccttc ttggctcagg cccgggaata cgagacatcc ggcgatatgc  1140
   gcgacggggt gctcaagctg ctcaacctgg agctgcagac ccatccgttc tctgtgctgc  1200
   gggctgccgc cttgactcac tgggtggaca ccggcggcta tgccaaggtg atagccggcg  1260
   agtacccgcg tcgggccgac gacggcaacg ccaaatttgc agacgacctt ggcgcggccg  1320
   cccggtacta ccgggacggc ttcgaccagt ccaacgaccc gctgatcaaa ggtatccgcg  1380
   acggattcgg tggcatcgtc gagggcgtgg gacgggcagc ctcgaacgcg gccgattcat  1440
   tgggccgcaa gatcaccgag tggcggcagc cctcgaagtg acggcccctc tgctacgtag  1500
   ctaagcacgc gcgaccggcg ggctggggag cccggtcagc ggtctcatag cattgcgaac  1560
   acgggacgtc gagaggggaa gagctgccat gggtgaggcg aacatccgcg agcaggcgat  1620
   cgccacgatg ccacggggtg gccccgacgc gtcttggctg gatcgtcgat tccagaccga  1680
   cgcactggag tacctcgacc gcgacgatgt gcccgatgag gtcaaacaga agatcatcgg  1740
   ggtgctcgac cgggtgggca ccctgaccaa cctgcacgag aagtacgccc ggatagccct  1800
   gaaacttgtt tctgacattc ccaacccgcg aatcctggaa cttggtgcgg gccatggcaa  1860
   gctctcagcg aaaatcctcg agctacaccc gacagcgacg gtgacgatca gcgatctaga  1920
   tcccacctcg gtggccaaca tcgccgcggg agagctggga acacatccgc gagcacgcac  1980
   ccaagtgatc gacgccaccg caatcgacgg ccacgaccac agctatgacc tggcggtctt  2040
   cgcgctggca tttcaccacc tgccgcctac ggtcgcctgc aaagcgatcg ccgaggccac  2100
   ccgggtgggg aagcgctttc tgatcatcga cctcaaacgg cagaaaccgc tgtcgttcac  2160
   gctctcttcg gtgctgctac tgccgctcca cctactgctg ctgccatggt cgtcgatgcg  2220
   ctcgagcatg cacgacggct ttatcagcgc actacgtgcc tacagtccct cggcgttgca  2280
   gacgcttgcc cgcgccgccg atccgggaat gcaggttgaa atcttgcccg caccgaccag  2340
   gctattcccg ccatcgctcg ccgttgtgtt ctcccgttcg agctcagcgc caacggaatc  2400
   tagcgagtgc tcggccgatc gccaacccgg cgaatgattc ggtagtagtg cagataagcc  2460
   atcgccggta ccacgacgaa cgtgatcacg atcaaagcaa tcgagaagta gttcggacca  2520
   ccccgcacta gaaagatgca gcggtagtcg taggacactg ccagcccaac cgagaccacg  2580
   atcgcaacaa gcggtaacac cttgtcggtg aacgcatttc gccgcacagc agcatgttct  2640
   actgcctgag acctcgccaa tgcgatgaga gcgatcggca cgatgatgaa ctggacgaat  2700
   cgggcgatca ccgccaggcc ggtcaggtgc aggttgtcga accgcagcgc caacgggaat  2760
   gcgagcgcca acgacgccgt aattgcgaag gagaccatcg gcacgtcgta ttggttcttg  2820
   cgtgacaagc gtgtcggcag aaccccgctg tccgctaacg cggtccaaag ccgcggtgca  2880
   ccgaacgagg ccgcgacatt gatgccgaac atcgatatca gggctccgac gacgatgatc  2940
   gttcggaagg tagcgtttcc gatggccgcg gccagtttca cggtgtcgtc cgacgcggcg  3000
   atcttgttcg atccgagcag catcgctacc gttagggtga gcaagtagat cgcgccaacc  3060
   gagaagatcg cgatcggtat agctctcggc aggttccggt ccggcgcgtc catttcttcg  3120
   gcggcgttcg cgatcgattc gaaaccggtg aatgcgtaca acgcgacaat cgtggccagc  3180
   gccatactcg agaacgtgcc cttgccaatt tcggcgacgc caagcaacga gtacggggtc  3240
   gcgctgtatg ccgaccacgc cgttgcgtag ttgttcacgt gctgggtggt gatgatccac  3300
   agcccgccga caatgaatgc cgagagcgcg aatgccttgc ctaccgttga cgttccgttg  3360
   gcccacttga tcgcccggtt gccgaagagg ttgatggcca acagcacgcc gataaagccg  3420
   agaaacgtca gcgtcttcac actgaacagt tgctcggcgt cggcccaggc cttgtcgggg   3480
   aaggccactc gcaacagcgt cgagacgaaa aaagaagcca acacccccca agcgatggac   3540
   gcggtaatgg cgtgggtgac accgacatag atgccgatcc ggcgcccaaa tgcggccgtt  3600
   gtgtaggcgt aggaggcacc gtttgttctg acgtaccttg ccgccgtcgc gaagacgatc  3660
   gccacgacac ccgcgaaaat gccagctaaa acataggcca tcggcgcgaa gggtcctgcg  3720
   agcccgatca cctcacctgg agttaggaag ataccggcgc cgattatcga gttgatcccg  3780
   agcatgacga cgctgcagaa acccagcttg tggatcgcat atcctctcgt ccgcgggccg  3840
   accaccgcac caaggctgtc tagcagggaa tcctctaacg caccatagat tctctagcga  3900
   cgattcttga gctcccggcc tgtcgatgcc ggcgctgcag gtgagtcacc gcagtgggcg  3960
   caccgaacac tcatttccgc cgccccaaat ccgcgcagtg accaccgcgc ggtcctcgcg  4020
   agtctaggcc agcatcgagt cgatcgcgga acgtgggacc aatacctggg ttgggccggc  4080
   tgcttcgggc agcaactccc ccgggttgaa gaagaaaatc accccgtcgt tcgtgactgc  4140
   gaagttctga taattcaccg ggtccaagcc ggcattcggc gctatcgata cctgttgtcc  4200
   ggtctgcttg ctcagttcac cttgcacaat ggggaagacg actggcag                4248
<210> 139
   <211> 923
   <212> DNA
   <213> Artificial Sequence
<220>
   <223> Mycobacterium africanum DNA fragment
<400> 139
   gccgtgctgg gcgcctgcgg tgcggccttg gtgctgaccg cgccgccggc caaccaggcc  60
   cgagccgcgg cgagcctgct gtcacgatcg aactgggtgc tcgccactgc gttgcccgcc  120
   agtcaggacg tcccggccga ttggggctac tcgttgaccg ggcggttgcg acgagcggtc  180
   tcgccaagca ccgtgccgcc ggccgcgctg cctaacacga gccgagcagc cgtctattcg  240
   ccggctggat gcggaaacat tccgaaaatc ctggaccact ccagcgccga cttggccgcc  300
   tatgtccaga tagaccgcga cgtgcaggtg ttcgggcaag atgcgcccct ggatgctgcc  360
   gcgaccgggg aaagcgatga gcgcggaccc aacgcccgct tcgcactctg ggccgttgcc  420
   gacggcccgg cgcggatcgc caactacctg gactggctaa accggtgcgg ttcttaccag  480
   gtcaccaacc actttttgga cggaacggtc aagaacgaac gaaccgtcac caccgaggtg  540
   gaagcgcttt cggccggcgg tgccgacgcc gccgtcgcgg tcacaaggac gttaatcctc  600
   taacgcacca tagattctct agcgacgatt cttgagctcc cggcctgtcg atgccggcgc  660
   tgcaggtgag tcaccgcagt gggcgcaccg aacactcact tccgccgccc caaatccgcg  720
   cagtgaccac cgcgcggtcc tcgcgagtct aggccagcat cgagtcgatc gcggaacgtg  780
   ggaccaatac ctgggttggg ccggctgctt cgggcagcaa ctcccccggg ttgaagaaga  840
   aaatcacccc gtcgttcgtg actgcgaagt tctgataatt caccgggtcc aagccggcat  900
   tcggcgctat cgatacctgt tgt                                           923
<210> 140
   <211> 923
   <212> DNA
   <213> Artificial Sequence
<220>
   <223> Mycobacterium africanum DNA fragment
<400> 140
   gccgtgctgg gcgcctgcgg tgcggccttg gtgctgaccg cgccgccggc caaccaggcc   60
   cgagccgcgg cgagcctgct gtcacgatcg aactgggtgc tcgccactgc gttgcccgcc  120
   agtcaggacg tcccggccga ttggggctac tcgttgaccg ggcggttgcg acgagcggtc  180
   tcgccaagca ccgtgccgcc ggccgcgctg cctaacacga gccgagcagc cgtctattcg  240
   ccggctggat gcggaaacat tccgaaaatc ctggaccact ccagcgccga cttggccgcc  300
   tatgtccaga tagaccgcga cgtgcaggtg ttcgggcaag atgcgcccct ggatgctgcc  360
   gcgaccgggg aaagcgatga gcgcggaccc aacgcccgct tcgcactctg ggccgttgcc  420
   gacggcccgg cgcggatcgc caactacctg gactggctaa accggtgcgg ttcttaccag  480
   gtcaccaacc actttttgga cggaacggtc aagaacgaac gaaccgtcac caccgaggtg  540
   gaagcgcttt cggccggcgg tgccgacgcc gccgtcgcgg tcacaaggac gttaatcctc  600
   taacgcacca tagattctct agcgacgatt cttgagctcc cggcctgtcg atgccggcgc  660
   tgcaggtgag tcaccgcagt gggcgcaccg aacactcact tccgccgccc caaatccgcg  720
   cagtgaccac cgcgcggtcc tcgcgagtct aggccagcat cgagtcgatc gcggaacgtg  780
   ggaccaatac ctgggttggg ccggctgctt cgggcagcaa ctcccccggg ttgaagaaga  840
   aaatcacccc gtcgttcgtg actgcgaagt tctgataatt caccgggtcc aagccggcat  900
   tcggcgctat cgatacctgt tgt                                          923
<210> 141
   <211> 2001
   <212> DNA
   <213> Mycobacterium tuberculosis
<400> 141
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ccggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                           2001
<210> 142
   <211> 2001
   <212> DNA
   <213> Mycobacterium tuberculosis
<400> 142
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ccggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                            2001
<210> 143
   <211> 2001
   <212> DNA
   <213> Mycobacterium tuberculosis
<400> 143
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ccggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                          2001
<210> 144
   <211> 2001
   <212> DNA
   <213> Mycobacterium tuberculosis
<400> 144
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ccggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                            2001
<210> 145
   <211> 2001
   <212> DNA
   <213> Mycobacterium tuberculosis
<400> 145
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ccggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                           2001
<210> 146
   <211> 2001
   <212> DNA
   <213> Mycobacterium tuberculosis
<400> 146
   atggcggccg actacgacaa gctcttccgg ccgcacgaag gtatggaagc tccggacgat 60
   atggcagcgc agccgttctt cgaccccagt gcttcgtttc cgccggcgcc cgcatcggca 120
   aacctaccga agcccaacgg ccagactccg cccccgacgt ccgacgacct gtcggagcgg 180
   ttcgtgtcgg ccccgccgcc gccaccccca cccccacctc cgcctccgcc aactccgatg 240
   ccgatcgccg caggagagcc gccctcgccg gaaccggccg catctaaacc acccacaccc 300
   cccatgccca tcgccggacc cgaaccggcc ccacccaaac cacccacacc ccccatgccc 360
   atcgccggac ccgaaccggc cccacccaaa ccacccacac ctccgatgcc catcgccgga 420
   cctgcaccca ccccaaccga atcccagttg gcgcccccca gaccaccgac accacaaacg 480
   ccaaccggag cgccgcagca accggaatca ccggcgcccc acgtaccctc gcacgggcca 540
   catcaacccc ggcgcaccgc accagcaccg ccctgggcaa agatgccaat cggcgaaccc 600
   ccgcccgctc cgtccagacc gtctgcgtcc ccggccgaac caccgacccg gcctgccccc 660
   caacactccc gacgtgcgcg ccggggtcac cgctatcgca cagacaccga acgaaacgtc 720
   gggaaggtag caactggtcc atccatccag gcgcggctgc gggcagagga agcatccggc 780
   gcgcagctcg cccccggaac ggagccctcg ccagcgccgt tgggccaacc gagatcgtat 840
   ctggctccgc ccacccgccc cgcgccgaca gaacctcccc ccagcccctc gccgcagcgc 900
   aactccggtc ggcgtgccga gcgacgcgtc caccccgatt tagccgccca acatgccgcg 960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ctggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                           2001
<210> 147
   <211> 2001
   <212> DNA
   <213> Mycobacterium tuberculosis
<400> 147
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg  1020
   ccggatctcg acgcgacaca gaaatcctta aggccggcgg ccaaggggcc gaaggtgaag  1080
   aaggtgaagc cccagaaacc gaaggccacg aagccgccca aagtggtgtc gcagcgcggc  1140
   tggcgacatt gggtgcatgc gttgacgcga atcaacctgg gcctgtcacc cgacgagaag  1200
   tacgagctgg acctgcacgc tcgagtccgc cgcaatcccc gcgggtcgta tcagatcgcc  1260
   gtcgtcggtc tcaaaggtgg ggctggcaaa accacgctga cagcagcgtt ggggtcgacg  1320
   ttggctcagg tgcgggccga ccggatcctg gctctagacg cggatccagg cgccggaaac  1380
   ctcgccgatc gggtagggcg acaatcgggc gcgaccatcg ctgatgtgct tgcagaaaaa  1440
   gagctgtcgc actacaacga catccgcgca cacactagcg tcaatgcggt caatctggaa  1500
   gtgctgccgg caccggaata cagctcggcg cagcgcgcgc tcagcgacgc cgactggcat  1560
   ttcatcgccg atcctgcgtc gaggttttac aacctcgtct tggctgattg tggggccggc  1620
   ttcttcgacc cgctgacccg cggcgtgctg tccacggtgt ctggtgtcgt ggtcgtggca  1680
   agtgtctcaa tcgacggcgc acaacaggcg tcggtcgcgt tggactggtt gcgcaacaac  1740
   ggttaccaag atttggcgag ccgcgcatgc gtggtcatca atcacatcat gccgggagaa  1800
   cccaatgtcg cagttaaaga cctggtgcgg catttcgaac agcaagttca acccggccgg  1860
   gtcgtggtca tgccgtggga caggcacatt gcggccggaa ccgagatttc actcgacttg  1920
   ctcgacccta tctacaagcg caaggtcctc gaattggccg cagcgctatc cgacgatttc  1980
   gagagggctg gacgtcgttg a                                           2001
<210> 148
   <211> 2001
   <212> DNA
   <213> Mycobacterium bovis
<400> 148
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg  1020
   ccggatctcg acgcgacaca gaaatcctta aggccggcgg ccaaggggcc gaaggtgaag  1080
   aaggtgaagc cccagaaacc gaaggccacg aagccgccca aagtggtgtc gcagcgcggc  1140
   tggcgacatt gggtgcatgc gttgacgcga atcaacctgg gcctgtcacc cgacgagaag  1200
   tacgagctgg acctgcacgc tcgagtccgc cgcaatcccc gcgggtcgta tcagatcgcc  1260
   gtcgtcggtc tcaaaggtgg ggctggcaaa accacgctga cagcagcgtt ggggtcgacg  1320
   ttggctcagg tgcgggccga ccggatcctg gctctagacg cggatccagg cgccggaaac  1380
   ctcgccgatc gggtagggcg acaatcgggc gcgaccatcg ctgatgtgct tgcagaaaaa  1440
   gagctgtcgc actacaacga catccgcgca cacactagcg tcaatgcggt caatctggaa  1500
   gtgctgccgg caccggaata cagctcggcg cagcgcgcgc tcagcgacgc cgactggcat  1560
   ttcatcgccg atcctgcgtc gaggttttac aacctcgtct tggctgattg tggggccggc  1620
   ttcttcgacc cgctgacccg cggcgtgctg tccacggtgt ccggtgtcgt ggtcgtggca  1680
   agtgtctcaa tcgacggcgc acaacaggcg tcggtcgcgt tggactggtt gcgcaacaac  1740
   ggttaccaag atttggcgag ccgcgcatgc gtggtcatca atcacatcat gccgggagaa  1800
   cccaatgtcg cagttaaaga cctggtgcgg catttcgaac agcaagttca acccggccgg  1860
   gtcgtggtca tgccgtggga caggcacatt gcggccggaa ccgagatttc actcgacttg  1920
   ctcgacccta tctacaagcg caaggtcctc gaattggccg cagcgctatc cgacgatttc  1980
   gagagggctg gacgtcgttg a                                            2001
<210> 149
   <211> 2001
   <212> DNA
   <213> Mycobacterium africanum
<400> 149
   atggcggccg actacgacaa gctcttccgg ccgcacgaag gtatggaagc tccggacgat   60
   atggcagcgc agccgttctt cgaccccagt gcttcgtttc cgccggcgcc cgcatcggca  120
   aacctaccga agcccaacgg ccagactccg cccccgacgt ccgacgacct gtcggagcgg  180
   ttcgtgtcgg ccccgccgcc gccaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccacacc ccccatgccc  360
   atcgccggac ccgaaccggc cccacccaaa ccacccacac ctccgatgcc catcgccgga  420
   cctgcaccca ccccaaccga atcccagttg gcgcccccca gaccaccgac accacaaacg  480
   ccaaccggag cgccgcagca accggaatca ccggcgcccc acgtaccctc gcacgggcca  540
   catcaacccc ggcgcaccgc accagcaccg ccctgggcaa agatgccaat cggcgaaccc  600
   ccgcccgctc cgtccagacc gtctgcgtcc ccggccgaac caccgacccg gcctgccccc  660
   caacactccc gacgtgcgcg ccggggtcac cgctatcgca cagacaccga acgaaacgtc  720
   gggaaggtag caactggtcc atccatccag gcgcggctgc gggcagagga agcatccggc  780
   gcgcagctcg cccccggaac ggagccctcg ccagcgccgt tgggccaacc gagatcgtat  840
   ctggctccgc ccacccgccc cgcgccgaca gaacctcccc ccagcccctc gccgcagcgc  900
   aactccggtc ggcgtgccga gcgacgcgtc caccccgatt tagccgccca acatgccgcg  960
   gcgcaacctg attcaattac ggccgcaacc actggcggtc gtcgccgcaa gcgtgcagcg 1020
   ccggatctcg acgcgacaca gaaatcctta aggccggcgg ccaaggggcc gaaggtgaag 1080
   aaggtgaagc cccagaaacc gaaggccacg aagccgccca aagtggtgtc gcagcgcggc 1140
   tggcgacatt gggtgcatgc gttgacgcga atcaacctgg gcctgtcacc cgacgagaag 1200
   tacgagctgg acctgcacgc tcgagtccgc cgcaatcccc gcgggtcgta tcagatcgcc 1260
   gtcgtcggtc tcaaaggtgg ggctggcaaa accacgctga cagcagcgtt ggggtcgacg 1320
   ttggctcagg tgcgggccga ccggatcctg gctctagacg cggatccagg cgccggaaac 1380
   ctcgccgatc gggtagggcg acaatcgggc gcgaccatcg ctgatgtgct tgcagaaaaa 1440
   gagctgtcgc actacaacga catccgcgca cacactagcg tcaatgcggt caatctggaa 1500
   gtgctgccgg caccggaata cagctcggcg cagcgcgcgc tcagcgacgc cgactggcat 1560
   ttcatcgccg atcctgcgtc gaggttttac aacctcgtct tggctgattg tggggccggc 1620
   ttcttcgacc cgctgacccg cggcgtgctg tccacggtgt ccggtgtcgt ggtcgtggca 1680
   agtgtctcaa tcgacggcgc acaacaggcg tcggtcgcgt tggactggtt gcgcaacaac 1740
   ggttaccaag atttggcgag ccgcgcatgc gtggtcatca atcacatcat gccgggagaa 1800
   cccaatgtcg cagttaaaga cctggtgcgg catttcgaac agcaagttca acccggccgg 1860
   gtcgtggtca tgccgtggga caggcacatt gcggccggaa ccgagatttc actcgacttg 1920
   ctcgacccta tctacaagcg caaggtcctc gaattggccg cagcgctatc cgacgatttc 1980
   gagagggctg gacgtcgttg a                                            2001
<210> 150
   <211> 1950
   <212> DNA
   <213> Mycobacterium canettii
<400> 150
   atggcggccg actacgacaa gctcttccgg ccgcacgaag gtatggaagc tccggacgat   60
   acggcagcgc agccgttctt cgaccccagt gcttcgtttc cgccggcgcc cgcatcggca  120
   aacctaccga agcccaacgg ccagactccg cccccgacgt ccgacggcct gtcggagcgg  180
   ttcgtgtcgg ccccgccgcc accaccccca cccccacctc cgcctccgcc aactccgatg  240
   ccgatcgccg caggagagcc gccctcgccg gaaccggccg catctaaacc acccacaccc  300
   cccatgccca tcgccggacc cgaaccggcc ccacccaaac cacccgcacc tccgatgccc  360
   atcgccggac ctgcacccac cccaaccgaa tcccagttgg cgccccccag accaccgaca  420
   ccacaaacgc caaccggagc gccgcagcaa ccggaatcac cggcgcccca cgtaccctcg  480
   cacgggccac aacaaccccg gcgcaccgca cccgcaccgc cctgggcaaa gatgcctatc  540
   ggcgaacccc cgcccgctcc gtccagaccg tttgcgtccc cggccgaacc accgacccgg  600
   cctgcccccc aacactcccg acgtgcgcgc cggggtcacc gctatcgcac agacaccgaa  660
   cgaaacgtcg ggaaggtagc aactggtcca tccatccaag cgcggctgcg ggcagaggaa  720
   gcatccggcg cgcagctcgc ccccggaacg gagccctcgc cggcgccgtt gggccaaccg   780
   agatcgtatc tggctccgcc cacccgtccc gcctcgacag aacctccccc cagccccgcg  840
   ccgcagcgcg actccggtcg gcgtgccgag cgacgcgtcc accccgactt agccgctcaa  900
   catgctgcgg ctcaacctga ttcgattacg gccgcaacca ctggcggtcg tcgccgcaag  960
   cgcgcagcgc ccgatctcga cgcgacacag aaatccttaa ggccggcggc caaggggccg 1020
   aaggttaaga aggtgaagcc ccagaaaccg aaggccacga agccgcccaa agtggtgtcg 1080
   cagcgcggct ggcgacattg ggtgcatgcg ttgacgcgaa tcaacctggg cctgtcaccc 1140
   gacgagaagt acgagctgga cctgcacgct cgagtccgcc gcaatccccg cgggtcgtat 1200
   cagatcgccg tcgtcggtct caaaggtggg gctggcaaaa ccacgctgac agcagcgttg 1260
   gggtcgacgt tggctcaggt gcgggccgac cggatcctgg ctctagacgc ggatccaggc 1320
   gccggaaacc tcgccgatcg ggtagggcga caatcgggcg cgaccatcgc tgatgtgctt 1380
   gcagaaaaag agctgtcgca ctacaacgac atccgcgcac acaccagtgt caatgcggtc 1440
   aatctggaag tgctgccggc accggaatac agctcggcgc agcgcgcgct cagcgacgcc 1500
   gactggcatt tcatcgccga tccggcgtcg aggttttaca acctcgtctt ggctgattgt 1560
   ggggccggct tcttcgaccc gctgacccgc ggcgtgctgt ccacggtgtc cggtgtcgtg 1620
   gtcgtggcaa gtgtctcaat cgacggcgca cagcaagcct cggtcgcgtt ggactggttg 1680
   cgcaacaacg gttaccaaga tttggcgagc cgcgcatgcg tggtcatcaa tcacatcatg 1740
   ccgggagaac ccaatgtcgc agttaaagac ctggtgcggc atttcgaaca gcaagttcaa 1800
   cccggccggg tcgtggtcat gccgtgggac aggcacattg cggccggaac cgagatttca 1860
   ctcgacttgc tcgaccctat ctacaagcgc aaggtcctcg aattggccgc agcgctatcc 1920
   gacgatttcg agagggctgg acgtcgttga                                   1950
<210> 151
   <211> 98
   <212> DNA
   <213> Artificial Sequence
<220>
   <223> Mycobacterium africanum DNA fragment
<400> 151
   agaaaaagag ctgtcgcact acaacgacat ccgcgcacac actagcgtca atgcggtcaa   60
   tctggaagtg ctgccggcac cggaatacag ctcggcgc                           98
<210> 152
   <211> 98
   <212> DNA
   <213> Artificial Sequence
<220>
   <223> Mycobacterium caprae DNA fragment
<400> 152
   agaaaaagag ctgtcgcact acaacgacat ccgcgcacac actagcgtca atgcggtcaa 60
   tctggaagtg ctgccggcac cggaatacag ctcggcgc 98
<210> 153
   <211> 98
   <212> DNA
   <213> Artificial Sequence
<220>
   <223> Mycobacterium canettii DNA fragment
<400> 153
   agaaaaagag ctgtcgcact acaacgacat ccgcgcacac actagcgtca atgcggtcaa 60
   tctggaagtg ctgccggcac cggaatacag ctcggcgc 98
<210> 154
   <211> 98
   <212> DNA
   <213> Artificial Sequence
<220>
   <223> Mycobacterium pinnipedii DNA fragment
<400> 154
   agaaaaagag ctgtcgcact acaacgacat ccgcgcacac actagcgtca atgcggtcaa 60
   tctggaagtg ctgccggcac cggaatacag ctcggcgc 98
<210> 155
   <211> 17
   <212> DNA
   <213> Artificial Sequence
<220>
   <223> Primer
<400> 155
   tcaccgacca tgtccag 17
<210> 156
   <211> 18
   <212> DNA
   <213> Artificial Sequence
<220>
   <223> Primer
<400> 156
   cgttgcccaa tccgtatg 18
<210> 157
   <211> 20
   <212> DNA
   <213> Artificial Sequence
<220>
   <223> Probe
<400> 157
   cagcagtacc atcgccatcg 20
<210> 158
   <211> 18
   <212> DNA
   <213> Artificial Sequence
<220>
   <223> Primer
<400> 158
   acgaatccgg cgatgatc 18
<210> 159
   <211> 17
   <212> DNA
   <213> Artificial Sequence
<220>
   <223> Primer
<400> 159
   cgactgcaca cctggaa 17
<210> 160
   <211> 16
   <212> DNA
   <213> Artificial Sequence
<220>
   <223> Probe
<400> 160
   ttggccggcg ccggtt 16
<210> 161
   <211> 18
   <212> DNA
   <213> Artificial Sequence
<220>
   <223> Primer
<400> 161
   catcgctgat gtgcttgc 18
<210> 162
   <211> 17
   <212> DNA
   <213> Artificial Sequence
<220>
   <223> Primer
<400> 162
   tgcgccgagc tgtattc 17
<210> 163
   <211> 21
   <212> DNA
   <213> Artificial Sequence
<220>
   <223> Probe
<400> 163
   acactagcgt caatgcggtc a 21
<210> 164
   <211> 17
   <212> DNA
   <213> Artificial Sequence
<220>
   <223> Primer
<400> 164
   agaccgtgcg gatcttg 17
<210> 165
   <211> 18
   <212> DNA
   <213> Artificial Sequence
<220>
   <223> Primer
<400> 165
   catggagatc acccgtga 18
<210> 166
   <211> 20
   <212> DNA
   <213> Artificial Sequence
<220>
   <223> Probe
<400> 166
   tatcgggtac acaaagacga 20
<210> 167
   <211> 17
   <212> DNA
   <213> Artificial Sequence
<220>
   <223> Primer
<400> 167
   acggaacggt caagaac 17
<210> 168
   <211> 18
   <212> DNA
   <213> Artificial Sequence
<220>
   <223> Primer
<400> 168
   gctcaagaat cgtcgcta 18
<210> 169
   <211> 19
   <212> DNA
   <213> Artificial Sequence
<220>
   <223> Probe
<400> 169
   acgtccttgt gaccgcgac 19
<210> 170
   <211> 17
   <212> DNA
   <213> Artificial Sequence
<220>
   <223> Primer
<400> 170
   aacgggtcgg attctcc 17
<210> 171
   <211> 18
   <212> DNA
   <213> Artificial Sequence
<220>
   <223> Primer
<400> 171
   ccgaaaccct cgttgatc 18
<210> 172
   <211> 17
   <212> DNA
   <213> Artificial Sequence
<220>
   <223> Probe
<400> 172
   tcagccgccg gccaacc 17
<210> 173
   <211> 21
   <212> DNA
   <213> Artificial Sequence
<220>
   <223> Probe
<400> 173
   attggtcacc cggatttcgg t 21

## Description

### Background to the invention

Tuberculosis (TB) is the leading cause of death worldwide from an infectious agent (Flint *et al.*, 2004), with the WHO estimating that one third of the global population are infected with TB. In a global report from the WHO (2009), it was estimated that there were 9.27 million cases of TB in 2007, with 2 million associated deaths. TB in mammals is caused by members of the *Mycobacterium tuberculosis* Complex (MTC). The eight closely related species in the complex have a wide range of natural hosts including humans hosts (*M. tuberculosis M. africanum M. canetti*), bovine hosts (*M. bovis*), caprine hosts (*M. caprae*)*,* rodent hosts (*M. microti*) and pinniped hosts (*M. pinnipedii*) along with the attenuated *M. bovis* strain BCG (*Bacillus Calmette-Guérin),* the commonly used vaccine strain. While there are a number of natural hosts, each member of the MTC has been implicated in human infection (Brosch *et al.,* 2002; Kiers *et al.,* 2008a).

Traditionally, diagnosis of TB relies on culture techniques and a battery of biochemical tests which are time consuming, labour intensive and often yield insensitive results (Huard *et al.,* 2003). Nucleic Acid Diagnostics (NAD), in particular real-time PCR, offer a rapid, reliable and highly sensitive alternative diagnostic tool for many infectious agents (Malhotra*-*Kumar *et al.,* 2008; Yang & Rothman, 2004). Advances in real-time PCR such as the availability of multiple fluorophores, along with the development of non-fluorescent quenchers has facilitated multiplexing, allowing for the simultaneous detection and discrimination of multiple targets, along with internal controls, in one reaction (Arya *et al.,* 2005).

While significant advances have been made in the diagnosis of TB using NAD (Huard *et al.,* 2006), the differentiation of members of the MTC to the species level is not routinely performed. Conventional PCR and real-time PCR assays for the rapid diagnosis of the MTC have been described (Huard *et al.,* 2003; Parsons *et al.,* 2002). Also, commercially available real-time PCR kits for the diagnosis of TB are available, such as AMPLIFIED MTD (Gen-Probe, San Diego, CA), Xpert MTB/RIF (Cepheid, Sunnyvale, CA) and AMPLICOR MTB (Roche, Branchburg, N.J.). These kits identify the MTC, but not individual species.
Warren *et al* describe a two -step multiplex polymerase chain reaction method based on genomic regions of difference (RD1, RD1^{mic}, RD2^{seal}, RD4, RD9 and RD12) for the differentiation of *M. canettii, M. tuberculosis, M. africanum, M. microti, M. pinnipedii, M. caprae, M. bovis* and *M. bovis BCG.*

The high degree of nucleotide sequence homology between members of the complex makes discrimination of species challenging, which may explain why it is not routinely carried out (Pinsky & Banaei, 2008). Comparative genomics revealed that *M. tuberculosis* and *M. bovis* genomes are 99.95 % similar (Gamier *et al.,* 2003), with whole genome DNA microarrays identifying 16 regions of difference (RD 1-16). (Behr *et al.,* 1999). These RDs represent regions of the genome deleted in *M. bovis BCG* which are present in *M. tuberculosis* and have been used for the differentiation of members of the MTC. One RD commonly targeted for the specific detection of *M. tuberculosis* is RD9 (Pinsky & Banaei, 2008), however this RD is also present in *M. canettii* (Brosch *et al.,* 2000). There is currently no real-time PCR test which can diagnose TB, whilst identifying the exact causative agent of infection.

Differentiation of the MTC allows health care professionals to determine the most appropriate course of treatment for infected patients and also provides valuable epidemiological information with relation to prevalence, transmission and geographical distribution of the neglected members of the MTC including members associated with zoonotic TB infection in humans. There is currently one molecular based kit commercially available for differentiation of the MTC, the GenoType MTBC (Hain Lifesciences GmbH, Nehren, Germany). However this kit is unable to differentiate between *M. tuberculosis* and *M. canettii* or between the two clades of *M*. *africanum*, and the target used in this kit for the detection of *M. africanum* also crossreacts with *M. pinnipedii.*

*M. Tuberculosis* is the most important human pathogen in the MTC and is thought to be responsible for 95% of human cases of TB, yet rarely causes disease in other mammals (Brosch *et al.,* 2000; Das *et al.,* 2007). While drug resistant strains of *M. tuberculosis* are emerging, it is considered sensitive to anti-tuberculosis drugs such as Pyrazinamide (PZA), a first line antibiotic that reduces patient treatment time from 9 months to 6 months (Niemann *et al.*, 2000; Somoskovi *et al.,* 2006).

*M. canettii* is thought to be the most phylogenetically distant member of the MTC and is considered the species from which other members of the complex may have evolved (Brosch *et al.,* 2002). *M. canettii* is phenotypically characterised by its smooth glossy white colonies, however a small number of these colonies have been shown to revert to rough colony variants when individual colonies are replated (van Soolingen *et al.,* 1997). Smooth colonies are uncharacteristic of the MTC and are due to the presence of large amounts of lipooligosaccharides in the *M. canettii* cell wall (Pfyffer *et al.,* 1998). Like *M. tuberculosis, M. canettii* contains all the RDs with the exception of RD12 *canettii* (RD12^{can}) which has been targeted for the specific detection of *M. canettii* in a complicated conventional PCR approach (Huard *et al.,* 2003). The method provided by Huard *et al.* requires time-consuming multiple reactions and produces results that require detailed interpretation. To achieve the limited distinction that the methods of Huard *et al.* and other methods of the prior art offer, detailed analysis of gels must be undertaken. This requires that polyacrylamide gels, for example, are prepared and run and then analysed by eye.

While infection with *M. canettii* is thought to be rare, there is a lack of rapid diagnostic tests available to differentiate between *M. tuberculosis* and *M. canettii.* Also recent reports have suggested that the true cases of TB caused by *M. canettii* may in fact be underrepresented (Goh *et al.,* 2001; Somoskovi *et al.,* 2009). While differentiation between *M. tuberculosis* and *M. canettii* is useful from an epidemiological point of view, it is also important for indicating the therapeutic approach to treatment as *M. tuberculosis* is sensitive to PZA, whereas *M. canettii* is resistant (Somoskovi *et al.,* 2009).

The major ethologic agents of zoonotic TB in humans are the phylogenetically related species *M. bovis* and *M. caprae.* These species occur worldwide and there are indications which suggest the true prevalence of zoonotic human TB infection may be underrepresented (Ojo *et al.,* 2008; Cicero *et al.,* 2009; Allix-Beguec *et al.,* 2010). In developed countries it has been suggested that the burden of bovine TB in humans ranges from 0.5 to 7.2% of TB cases; while in developing countries, where very little data are available, this figure may be up to 15% (de la Rua-Domenech, 2006; Kubica *et al.,* 2003). Recent reports have identified TB in humans caused by *M. bovis* in countries officially free from bovine TB and suggest that the true prevalence of zoonotic TB may be underestimated clinically (Cicero *et al.,* 2009; Allix-Beguec *et al.,* 2010). Moreover, zoonotic TB remains a significant threat to human health in developing countries where its prevalence is currently unknown, as speciation of the MTC is not routinely performed (de la Rua-Domenech, 2006). *M. bovis* and *M. bovis BCG* are intrinsically resistant to pyrazinamide (PZA), and this important first line drug for treating disease caused by *M. tuberculosis* and *M. caprae* infection should not be used for treating *M. bovis,* or *M. bovis BCG* infection. It is therefore important to distinguish between these members of the MTC in order to provide a useful treatment regimen.

This invention provides a multiplex *in vitro* nucleic acid amplification assay using novel nucleic acid targets which can diagnose *Mycobacterium canetti* from clinical isolates. Also disclosed is a *multiplex in vitro* nucleic acid amplification assay using novel nucleic acid targets which can diagnose TB from clinical isolates by detecting the MTC while simultaneously differentiating between the different species that are members of the MTC.

### Description of the invention

Disclosed herein is a multiplex *in vitro* nucleic acid amplification method for identifying a species of the *Mycobacterium tuberculosis* complex (MTC) present in a sample, wherein the method comprises detecting the presence or absence of a plurality of nucleic acid molecule targets in the sample in one reaction, wherein at least one of the nucleic acid molecule targets is present in the genome of one or more, but not all, of the species of the *Mycobacterium tuberculosis* complex.

The invention provides a multiplex *in vitro* nucleic acid amplification method for identifying *Mycobacterium canettii* present in a sample, wherein the method comprises detecting the presence or absence of a plurality of nucleic acid molecule targets in the sample in one reaction, which, in combination, are unique in their presence or absence to *Mycobacterium canettii,* wherein the method comprises the use of primers or probes specific for a nucleic acid sequence that is present in *M. canettii,* but is not present in *M. tuberculosis* and comprises a region of RD^{canetti1}, SEQ ID NO: 78.

Previous methods for the detection of the MTC have not been capable of identifying the specific members of the MTC that are present in a manner that is practically useful. This means that diagnosis and treatment provision is not tailored to the specific species present unless extensive experimentation is carried out. This requires significant time and effort that is incompatible with rapid and effective diagnosis and treatment. This invention, for the first time, provides a method that is able to identify different members of the MTC in a rapid and easily-interpretable manner. The inventors have surprisingly found that there is sufficient variation between species yet sufficient conservation between isolates of the same species to identify specific species of the MTC in a single reaction multiplex nucleic acid amplification assay. By identifying and characterising a series of sequences that are either shared or not shared between the different members of the MTC, the present invention thus allows the use of multiplex nucleic acid amplification methods to detect specific individual members of the MTC.

The species that make up the MTC are closely related but differ significantly in their pathology and susceptibility to certain treatments. Therefore, although it is important to be able to distinguish between the different species, it has previously not been possible to do this in any straightforward way that is amenable to use in rapid diagnosis. The different members of the MTC share a significant proportion of their genetic material. The present invention has successfully identified sufficient differences between the genomes of the members of the MTC to be able to distinguish between them in multiplex *in vitro* nucleic acid amplification assays. In contrast to the methods of the prior art, the methods of the present invention allow a single multiplex reaction to be performed that gives clear signals to identify which species is present in the tested sample. It is not necessary to run gels or to undertake complex interpretation of the results.

In addition to providing the first methods for discriminating between the different members of the MTC in a rapid and effective manner and the first methods for specifically identifying *M*. *tuberculosis,* the present invention additionally provides the first methods for specifically identifying *M. canettii, M. africanum* clade 1 and *M. africanum* clade 2. These members may be identified in a second multiplex reaction. Further, the present invention provides the first method for specifically identifying *M. pinnipedii.* This member of the MTC may be identified by combining the results of a first and second multiplex reaction.

There is a clinical need to differentiate between *M tuberculosis and M. canettii,* and between *M. caprae* and *M. bovis* and *M. bovis BCG* as they require different therapeutic treatment regimes (Somoskovi *et al.,* 2009).

Infection by *M. canettii* is considered to be rare and confined to Africa and it is not considered to be a significant concern for healthcare professionals. However, in the absence of methods for specifically identifying *M. canettii,* it is possible that the number of cases of *M. canettii* has been underestimated (Goh *et al.,* 2001). Therefore, the present invention identifies that there is a need to be able to identify *M. canettii* specifically, and in particular to distinguish it from *M. tuberculosis*. The present invention also provides methods that are able to achieve such differentiation as well as to distinguish between other members of the MTC in order to provide a suitable treatment profile.

For monitoring of zoonotic TB in humans it is also important to accurately identify *M. pinnipedii* and *M. microti* as causes of infection. While these members of the MTC are rare, outbreaks of human TB cuased by these members of the MTC have been observed (Kiers *et al.,* 2008b; Panteix *et al.*, 2010). Accurate identification of these members of the MTC is important for tracing source exposure (Djelouadji *et al.,* 2008).

*M. africanum* has been shown to cause upto 50% of human TB cases in certain regions in Africa, yet is rarely observed elsewhere (de Jong *et al.,* 2010). Since the reclassification of *M. africanum* into two distinct lineages *M. africanum* clade 1 and *M. africanum* clade 2, little is known as to the prevalence of TB caused by each lineage (Vasconcellos *et al.*, 2010) as there is currently no commercially available diagnostic kit with the capability to differentiate between these clades. The capability to accurately differentiate between these clades of *M. africanum* will be important for epidemiological studies.

The present inventors have surprisingly found that it is possible to identify species of the MTC, despite the high sequence homology that exists between the members of the MTC. In particular, the present invention provides a multiplex *in vitro* nucleic acid amplification assay that is capable of identifying *Mycobacterium canettii* in a single reaction. The inventors have also discovered that *Mycobacterium africanum* clade 1 can also be identified in the same single reaction. Furthermore, the inventors have devised a method for discriminating other members of the MTC, including *Mycobacterium bovis, Mycobacterium bovis* BCG, *Mycobacterium caprae* and *Mycobacterium africanum* clade 2. Preferably, these members are identified in a second multiplex reaction. In one embodiment the method of the invention may be performed in a stepwise manner, for example, with two separate multiplex steps, with *Mycobacterium tuberculosis, Mycobacterium canettii* and *Mycobacterium africanum* clade 1 distinguished in a first multiplex reaction (Multiplex 1) and *Mycobacterium bovis, Mycobacterium bovis* BCG, *Mycobacterium caprae,* and *Mycobacterium africanum* clade 2 distinguished in a second multiplex reaction (Multiplex 2). The inventors have also discovered that combining the results of Multiplex 1 with the results of Multiplex 2 allows the identification of *Mycobacterium pinnipedii* and *Mycobacterium microti.*

Prior to this invention, it was not expected that such assays could be developed or that any nucleic acid sequences necessary for such assays existed or could be identified.

### Assay components

As indicated above, the multiplex *in vitro* nucleic acid amplification assay used in the methods of the invention utilises genomic differences between members of the MTC to determine which member is present in a sample. Generally, this is achieved by incorporating one or more pairs of primers specific for target nucleic acid sequences which are uniquely present or absent in a particular member of the MTC into a sample and using a probe to determine which target nucleic acid sequences are present in the sample. The target nucleic acid sequences used in the methods of the present invention are described below.

### Identification of the MTC

In certain embodiments, the method of the present invention comprises detecting the presence or absence of the MTC in a sample. This is preferably performed by detecting the presence or absence of MTC *lepA* in a sample. In certain embodiments, the method comprises the use of primers or probes specific for MTC *lepA*, SEQ ID NO: 47.

In one embodiment *lepA* may be amplified using primers comprising or consisting of SEQ ID NOs: 164 and 165.

The presence or absence of the MTC may be determined using a probe comprising or consisting of SEQ ID NO: 173. The probe is preferably labelled and the label may be a fluorescent label. In one embodiment the label may be HEX.

In certain preferred embodiments, the method comprises the use of more than one probe specific for MTC *lepA.* In such embodiments, the *lepA* probes are labelled with different fluorescent labels.

In certain embodiments, at least one of the probes specific for MTC *lepA* does not significantly bind to nucleic acid amplified from a member of the *Mycobacterium tuberculosis* complex.

This is the first description of a real-time PCR diagnostics assay using the MTC *lepA* gene as the diagnostics target. This gene has demonstrated promising potential as a candidate for nucleic acid diagnostics as it exhibits 5' and 3' flanking sequence conservation, but also internal sequence variability which enables the design of genus and species-specific NAD assays (O' Grady *et al.,* 2008).

The MTC *lepA* region is defined by SED ID NO: 47. Any part of the MTC *lepA* region could be used to identify the MTC. Therefore, this invention contemplates the use of primers, probes and arrays directed to any part of the MTC *lepA* region. Preferably, the primers, probes or arrays are suitably designed according to methods known in the art so that they bind specifically to the MTC *lepA* region, and not to regions outside this sequence.

### Internal Amplification Control (IAC)

In one embodiment the multiplex *in vitro* nucleic acid amplification method of the invention includes the use of an Internal Amplification Control (IAC).

The use of an IAC gives the assay greater reliability as false negative results are reduced. Without an IAC, if amplification fails in an individual reaction, no signals will be produced. There is a danger that this will be incorrectly interpreted as a negative result indicating the absence of a species of the MTC in a sample. However, by adding a control sample to each aliquot and detecting its amplification using an IAC probe, it can be checked that amplification is successful. If only the IAC signal is detected, no MTC species are present in a sample. However, if no signals at all are detected, amplification failed and the test must be repeated. The present invention has developed an IAC target that is also used for the simultaneous detection of the MTC, reducing the complexity of the multiplex PCR assay. One set of primers and two different probes are used to detect species of the MTC and to check that amplification is successful.

In one embodiment the IAC may be *lepA*, SEQ ID NO: 84. This gene has demonstrated promising potential as a candidate for nucleic acid diagnostics as it exhibits 5' and 3' flanking sequence conservation, but also internal sequence variability which enables the design of genus and species-specific NAD assays (O' Grady *et al.,* 2008). This surprising feature of the *lepA* sequence has allowed an internal amplification control (IAC) to be incorporated into the multiplex assay, according to the guidelines set out in a review by Hoorfar *et al.,* (2004). The *lepA* gene is present in all bacteria sequenced to date and codes for one of the most conserved proteins in bacteria. Surprisingly, however, there is enough sequence heterogeneity between the *M. smegmatis* (or any other species to be used as a control) and the MTC *lepA* sequences for the design of independent, specific probes to detect either members of the MTC or the internal amplification control, here *M. smegmatis.* The 5' and 3' homology allows the design of a single pair of primers for the amplification of both the IAC and the target, therefore reducing the complexity of the multiplex assay and aiding its effectiveness.

In certain embodiments, the IAC *lepA* may be amplified using primers which comprise or consist of SEQ ID NOs 164 and 165.

The presence or absence of the IAC *lepA* may be determined using a probe comprising or consisting of SEQ ID NO: 107. The probe is preferably labelled and may be a fluorescent label. In one embodiment the label may be Cy5.

The present invention additionally contemplates assays which do not use *lepA* or which do not use an IAC.

In another embodiment the IAC may be MSMEG_0660. In certain embodiments, the method comprises the use of primers or probes specific for MSMEG_600, SEQ ID NO: 135.

The MSMEG_0660 gene was chosen as the target for the IAC because this gene is present only in *M. smegmatis.* This gene is thought to code for an extracellular solute-binding protein.

In one embodiment the IAC MSMEG_0660 may be amplified using primers comprising or consisting of SEQ ID NOs: 155 and 156.

The presence or absence of the IAC MSMEG_0660 may be determined using a probe comprising or consisting of SEQ ID NO: 157. The probe is preferably labelled and may be a fluorescent label. In one embodiment the label may be Cy5.

If the internal control is not detected, the result is considered invalid and must be repeated (Hoorfar *et al.,* 2004; O' Grady *et al.,* 2008).

### Identification of M. canettii

The *multiplex in vitro* nucleic acid amplification method of the invention is for identifying *M. canettii.* The method of the present invention comprises detecting a plurality of nucleic acid molecules which, in combination, are unique in their presence or absence to *Mycobacterium canettii.*

*M. canettii* and *M. Tuberculosis* are considered to be the most closely related members of the MTC (Brosch *et al.,* 2002). Of the 16 RDs identified between members of the MTC, none have been capable of differentiating between *M. canettii* and *M. tuberculosis,* highlighting the high degree of evolutionary constraint between the two species. Therefore, it is surprising that the present inventors have been able to develop an assay that can distinguish between them.

It has been proposed that *M. canettii* is the most phylogenetically distinct member of the MTC, with other MTC members evolving from an *M. canettii-like* organism (Brosch *et al.,* 2002; Huard *et al.,* 2006). An *M. canettii* RD has previously been described by Huard *et al.* (2003) which represents a region of the genome flanking RD12 which is deleted in *M. canettii* but present in *M. tuberculosis.* A conventional PCR was performed for differentiation between *M. tuberculosis* and *M. canettii* based on the PCR product size. If a particular PCR product size was observed for both RD 9 and a region of RD 12, *M. tuberculosis* was present. If the same PCR product was observed for RD 9 but not RD 12, *M. canettii* was present. Interpretation of these results is complex as the particular region which was not amplified for *M. canettii* was also not amplified in *M. bovis* or *M. bovis BCG.* This invention, in contrast, provides a new RD which is present in *M. canettii* but deleted in *M. tuberculosis* and all other members of the MTC. As this region is only present in *M. canettii,* the interpretation of results becomes less complex, thus avoiding false reporting of the organism present. Therefore, the present invention has surprisingly found that there are sequences that allow the specific identification of *M. canettii.*

The method of the present invention includes the use of primers and probes specific for a plurality of nucleic acid molecules which are unique in their presence or absence to *Mycobacterium canettii.*

The method of the invention includes the use of primers or probes specific for a nucleic acid that is present in *M. canettii* but is not present in *M. tuberculosis,* and optionally is also not present in *M. africanum* clade 2, *M. bovis, M. bovis BCG, M. caprae, M. pinnipedii,* and *M. microti.*

The method of the present invention comprises detecting the presence or absence of RD^{canetti1} in a sample. Primers or probes that are specific for a region of RD^{canetti1}, SEQ ID NO: 78 are used.

In certain embodiments RD^{canetti1} may be amplified using primers which comprise or consist of SEQ ID NOs 103 and 105.

In certain embodiments, the presence or absence of RD^{canetti1} may be detected using a probe which comprises or consists of SEQ ID NO: 104. The probe is preferably labelled and the label may be a fluorescent label. In one embodiment the label may be ROX.

*M. canettii* is present in a sample if *lepA, wbbl1*, RD^{canettii1} and the IAC (*lepA* or MSMEG_0660) diagnostics assays generate a positive signal, but the RD713 diagnostics assay (described below) does not generate a positive signal.

In a specific embodiment *M. canettii* is present if the HEX labelled MTC *lepA,* the FAM labelled *wbbl1*, the ROX labelled RD^{canettii1} and the Cy5 labelled IAC MSMEG_0660 diagnostics assays generate a positive signal, but the Cyan 500 labelled RD713 diagnostics assay (described below) does not generate a positive signal.

The present invention provides the use of this RD^{canetti1} region in identifying members of the MTC, either in the methods of the invention described above or in any other methods. The RD^{canetti1} region is the first to be identified that is unique to M. *canettii.* By allowing the discrimination of *M. canettii* from *M. tuberculosis* and all the other members of the MTC, the RD^{canetti1} region allows both *M. canettii* and *M. tuberculosis* to be identified in a simple multiplex nucleic acid amplification assay. In one embodiment the RD^{canetti1} region allows both *M. canettii* and *M. tuberculosis* to be identified in a single multiplex nucleic acid amplification assay.

Vitally, the RD^{canetti1} region is not only absent in all of the other members of the MTC, it is present in all of the *M. canettii* isolates tested. Therefore, it allows the specific and unambiguous identification of *M. canettii.* Due to the high similarity between *M. canettii* and *M. tuberculosis,* it is surprising that such a sequence exists. Furthermore, due to the variability between isolates of *M. canettii*, it is surprising that the region is conserved between isolates. Other *M. canettii* sequences that have been characterised have shown significant variability and polymorphisms, for example *gyrB* (Goh et al. 2003), *pncA* (Somoskovi et al. 2007) and *hsp65* (Fabre et al. 2004).

The RD^{canetti1} region is defined by SED ID NO: 78. Any part of the RD^{canetti1} region could be used to identify *M. canettii* or to identify *M. tuberculosis* by distinguishing it from *M. canettii.* Therefore, this invention contemplates the use of primers, probes and arrays directed to any part of the RD^{canetti1} region. Preferably, the primers, probes or arrays are suitably designed according to methods known in the art so that they bind specifically to the RD^{canetti1} region, and not to regions outside this sequence.

### Identification of M. tuberculosis

In certain embodiments of the invention, the multiplex *in vitro* nucleic acid amplification method is for identifying *M. Tuberculosis.* In certain embodiments, the method comprises detecting a plurality of nucleic acid molecules which, in combination, are unique in their presence or absence to *Mycobacterium tuberculoses.*

The majority of human cases of tuberculosis are caused by *Mycobacterium tuberculosis.* However, significant numbers of cases are caused by other pathogens such as *M. canettii* (Goh *et al* 2001.). Therefore, assays are required that are able to distinguish *Mycobacterium tuberculosis* from other members of the MTC. The methods of the present invention are the first to achieve this. The present invention has identified and characterised types of sequences that, by virtue of their presence or absence in the genome of *M. tuberculosis* and the other members of the MTC, allow *M. tuberculosis* to be identified in a simple *multiplex in vitro* nucleic acid amplification assay. This may be achieved in a single multiplex *in vitro* nucleic acid amplification assay.

In certain embodiments, the method of the invention detects the presence or absence of a gene region that is present in *M*. *tuberculosis, M. canettii,* and *M. africanum* clade 1, but optionally is also not present in *M. africanum* clade 2, *M. bovis, M. bovis BCG, M caprae, M. pinnipedii,* and *M. microti.*

In certain embodiments, the method of the present invention detects the presence or absence of *wbbl1* in a sample. In certain embodiments, the method comprises the use of primers or probes specific for a region of *wbbl1*, SEQ ID NO: 1.

This novel molecular target, identified and evaluated in this study, is based on the *wbbl1* gene which enables the simultaneous detection of *M. tuberculosis* and *M. canettii,* a target with the same properties as the widely used RD9 region for *M*. *tuberculosis* identification. As the aim of this study was to identify novel nucleic acid diagnostic targets for the detection of tuberculosis, this *wbbl1* target may be used in the multiplex assay described. In certain embodiments, RD9 could be used in conjunction with any of the assays outlined below.

Therefore, this invention contemplates the use of primers, probes and arrays directed to any part of the region of *wbbl1* represented by SEQ ID NO: 1. Preferably, the primers, probes or arrays are suitably designed according to methods known in the art so that they bind specifically to this region, and not to regions outside this sequence.

In certain embodiments *wbb1* may be amplified using primers which comprise or consist of SEQ ID NOs 97 and 99.

In certain embodiments the presence or absence of *wbbl1* may be determined using a probe which comprises or consists of SEQ ID NO: 98. The probe is preferably labelled and the label may be a fluorescent label. In one embodiment the label may be FAM.

*M. tuberculosis* is present in a sample if MTC *lepA, wbbl1* and the IAC (*lepA* or MSMEG_0660) diagnostics assays generate a positive signal, but RD713 (discussed below) and RD^{canettii1} diagnostics assays do not generate positive signals in these channels.

In a specific embodiment *M. tuberculosis* is present if the HEX labelled MTC *lepA,* the FAM labelled *wbbll* and the Cy5 labelled IAC MSMEG_0660 diagnostics assays generate a positive signal but the Cyan 500 labelled RD713 and the ROX labelled RD^{canettii1} diagnostics assays do not generate positive signals.

### Identification of M. africanum clade 1

In certain embodiments of the invention, the multiplex *in vitro* nucleic acid amplification method is for identifying *M. africanum* clade 1. In certain embodiments, the method of the present invention comprises detecting a plurality of nucleic acid molecules which, in combination, are unique in their presence or absence to *Mycobacterium africanum* clade 1.

*M. africanum* is a member of the MTC originally thought to have a natural host in humans. In certain geographical regions, *M. africanum* is thought to cause up to half the cases of human TB infection (de Jong *et al.,* 2010). Prior to 2004, *M*. *africanum* was divided into two subgroups, namely *M. africanum* subtype 1 and *M africanum* subtype 2. Differentiation between these species was difficult owing to the variable biochemical test results observed. Based on genomic analysis studies M. *africanum* subtype 2 was reclassified as *M. tuberculoses* (Mostowy *et al.,* 2004).

Recent studies have subsequently further classified *M. africanum* subtype 1 into two distinct lineages namely *M. africanum* West African-1 (clade 1) and *M. africanum* West African-2 (clade 2) (de Jong *et al.,* 2010 & Vasconcellos *et al.,* 2010). *M. africanum* clade 1 appears to be closely related to *M. tuberculosis* whereas *M. africanum* clade 2 is phylogenetically more closely related to animal isolates of the MTC (de Jong *et al.,* 2010). These recent studies have discovered robust molecular markers for each lineage of *M. africanum* based on deletions and single nucleotide polymorphisms (SNP). However, there is currently no commercially available method or diagnostics kit with the capability of differentiating between these described clades of *M. africanum.* The present invention provides such methods and kits.

In certain embodiments, the invention identities *M. africanum* using a set of primers and probes specific for a plurality of nucleic acid molecules which are unique in their presence or absence to *Mycobacterium africanum* clade 1.

In certain embodiments, the invention provides the use of primers or probes specific for a nucleic acid that is present in *M. africanum* clade 1 but is not present in *M. canettii or M. tuberculosis,* and optionally not present in *M. bovis, M. bovis BCG, M. caprae, M. africanum* clade 2, *M pinnipedii* and *M. microti.*

In certain embodiments, *M. africanum* clade 1 is identified by detecting the presence or absence of RD713.

The presence or absence of RD713 may be determined using primers or probes specific for a region of RD713, SEQ ID NO: 137.

In certain embodiments, RD713 may be amplified using primers which comprise or consist of SEQ ID NOs 167 and 168.

The presence or absence of RD713 may be detecting using a probe which comprises or consists of SEQ ID NO: 169. The probe is preferably labelled and the label may be a fluorescent label. In one embodiment the label is Cyan 500.

*M. africanum* clade 1 is present if the MTC *lepA, wbbl1*, RD713 and IAC *(lepA* or MSMEG_0660) diagnostics assays generate a positive signal, but the RD^{canettii1} diagnostics assay does not generate a positive signal.

In a specific embodiment, *M. africanum* is present if the HEX labelled MTC *lepA,* the FAM labelled *wbbl1*, the Cyan 500 labelled RD713 and the Cy5 labelled IAC MSMEG_0660 diagnostics assay generate a positive signal, but the ROX labelled RD^{canettii1} diagnostics assay does not generate a positive signal.

### Identification of M. bovis

In certain embodiments of the invention, the multiplex *in vitro* nucleic acid amplification method is for identifying *M. bovis.* In certain embodiments, the method of the present invention comprises detecting a plurality of nucleic acid molecules which, in combination, are unique in their presence or absence to *Mycobacterium bovis.*

In certain embodiments, the method uses primers or probes specific for a nucleic acid that is present in *M. bovis, M. bovis BCG* and *M. caprae* but is not present in *M. africanum* clade 2, *M. pinnipedii* and *M. microti,* and optionally is not present in *M. tuberculosis* and *M. canettii.*

In certain embodiments, the identification of *M. bovis* is determined using primers or probes specific for a region of *lpqT,* SEQ ID NO: 109.

In certain embodiments, *lpqT* is amplified using primers which comprise or consist of SEQ ID NOs 158 and 159.

In certain embodiments, the the presence or absence of *lpqT is* determined using a probe which comprises or consists of SEQ ID NO: 160. The probe is preferably labelled and the label may be fluorescent. In one embodiment the label may be FAM.

In one embodiment, *M. bovis* is present if the MTC *lepA and* IAC (*lepA* or MSMEG_0660) diagnostics assays generate a positive signal, but the *wbbl1*, the RD^{canettii1} and the RD713 diagnostics assays do not generate positive signals, and the *lpqT,* RD1 and the IAC (*lepA* or MSMEG_0660) diagnostics assay generate positive signals, but the *M. caprae lepA* (described below) and the RD701 (described below) diagnostics assays do not generate positive signals.

In a specific embodiment, *M. bovis* is present if in the first multiplex the HEX labelled MTC *lepA* and Cy5 labelled IAC MSMEG_0660 diagnostics assays generate a positive signal, but the FAM labelled *wbbl1*, the ROX labelled RD^{canettii1} and the Cyan 500 labelled RD713 diagnostics assays do not generate positive signals. This indicates that a member of the MTC other than *M. tuberculosis, M. canettii* and *M. africanum* clade 1 is present in the sample and the user should proceed to the second multiplex real-time PCR disclosed in this invention. The second multiplex indicates that *M. bovis* is present if a positive signal is observed in the FAM labelled *lpqT,* the HEX labelled RD1 and the Cy5 labelled IAC MSMEG_0660 diagnostics assay, but the Cyan500 labelled *M. caprae lepA* and the ROX labelled RD701 diagnostics assays do not generate positive signals.

### Identification of M. bovis BCG

In certain embodiments of the invention, the multiplex *in vitro* nucleic acid amplification method is for identifying *M. bovis BCG.* In certain embodiments, the method of the present invention comprises detecting a plurality of nucleic acid molecules which, in combination, are unique in their presence or absence to *Mycobacterium bovis BCG.*

In certain embodiments, the method uses primers or probes specific for a nucleic acid that is deleted in *M. bovis BCG* and *M. microti* but is present in *M. bovis, M. caprae, M. africanum* clade 2, and *M. pinnipedii,* and optionally is present in *M. tuberculosis* and *M. canettii.*

In certain embodiments, the presence or absence of *M. bovis BCG* is determined using primers or probes specific for a region of RD1, SEQ ID NO: 141.

In certain embodiments, RD1 is amplified using primers which comprise or consist of SEQ ID NOs 161 and 162.

In certain embodiments, the presence or absence of RD1 is determined using a probe which comprises or consists of SEQ ID NO: 163.

In one embodiment, *M. bovis BCG* is present if the MTC *lepA* and Cy5 labelled IAC (*lepA* or MSMEG_0660) diagnostics assays generate a positive signal, but the *wbbl1,* the RD^{canettii1} and the RD713 diagnostics assays do not generate positive signals, and the *lpqT* and the IAC (*lepA* or MSMEG_0660) diagnostics assays generate positive signals but the *M. caprae lepA* (discussed below), the RD1 and RD701 (discussed below) diagnostics assays do not generate positive signals.

In a specific embodiment, *M. bovis BCG* is present if in the first multiplex the HEX labelled MTC *lepA* and Cy5 labelled IAC MSMEG_0660 diagnostics assays generate a positive signal, but the FAM labelled *wbbl1*, the ROX labelled RD^{canettii1} and the Cyan 500 labelled RD713 diagnostics assays do not generate positive signals. This indicates that a member of the MTC other than *M. tuberculosis, M. canettii* and *M. africanum* clade 1 is present in the sample and the user should now proceed to the second multiplex real-time PCR disclosed in this invention.

Using multiplex 2, *M. bovis BCG* is present if a positive signal is observed in the FAM labelled *lpqT* and the Cy5 labelled IAC MSMEG_0660 diagnostics assays, but the Cyan 500 labelled *M. caprae lepA* (discussed below), the HEX labelled RD1 and the ROX labelled RD701 (discussed below) diagnostics assays do not generate positive signals.

### Identification of M. caprae

In certain embodiments of the invention, the multiplex *in vitro* nucleic acid amplification method is for identifying *M. caprae.* In certain embodiments, the method of the present invention comprises detecting a plurality of nucleic acid molecules which, in combination, are unique in their presence or absence to *Mycobacterium caprae.*

In certain embodiments, the method of the invention uses primers or probes specific for a nucleic acid that is present in *M. caprae* but is not present in *M. bovis, M. bovis BCG. M. africanum* clade 2, *M. pinnipedii* or *M. microtii,* and optionally is not present in *M. Tuberculosis* and *M. canettii.*

In certain embodiments, *M. caprae* may be identified using primers or probes specific for a region of *M. caprae lepA,* SEQ ID NO: 76.

As discussed above, MTC *lepA* (encoded by SEQ ID NO: 47) is used to identify the presence of the MTC and *lepA* (encded by SEQ ID NO: 84) may be used as an IAC. However, the inventors have surprisingly identified an SNP in the *lepA* gene of *M. caprae* which allows this gene to simultaneously be used to detect the presence or absence of *M. caprae.* The use of the same gene for these two or three identification purposes will simplify the diagnostic assay by reducing the number of primers required. It is surprising that this SNP is conserved between all isolates of *M*. *caprae* tested, but is not present in any of the other members of the MTC, despite the high level of sequence homology between the genomes of all members of the MTC.

In certain embodiments, *M. caprae lepA* may be amplified using primers which comprise or consist of SEQ ID NOs 164 and 165. These are the same primers which are used to amplify the *lepA* sequence which identified the presence of the MTC, and which may be used as an IAC. This reduces the numbers of primers required to perform the diagnostic assay and therefore reduces the complexity of the assay.

In certain embodiments, the presence or absence of *M. caprae lepA* may be determined using a probe which comprises or consists of SEQ ID NO: 166. This probe differs from the probe which is used to determine the presence of the MTC or as an IAC as it binds to the region of the *lepA* gene which includes the *M. caprae* specific SNP. The probe is preferably labelled and the label may be fluorescent. In one embodiment the label may be Cyan 500.

If the MTC *lepA* and IAC (*lepA* or MSMEG_0660) diagnostics assays generate a positive signal, but the *wbbl1*, RD^{canettii1} and RD713 diagnostics assays do not generate positive signals, and a positive signal is observed for the *M. caprae lepA, lpqT,* RD1 and IAC *(lepA* or MSMEG_0660) diagnostics assay, but the RD701 diagnostics assay does not generate a positive signal, *M. caprae* is present in the sample.

In one specific embodiment, *M. caprae* is present if in the first multiplex the HEX labelled MTC *lepA* and Cy5 labelled IAC MSMEG_0660 diagnostics assays generate a positive signal, but the FAM labelled *wbbl1*, the ROX labelled RD^{canettii1} and the Cyan 500 labelled RD713 diagnostics assays do not generate positive signals. This indicates that a member of the MTC other than *M. tuberculosis, M canettii* and *M. africanum* clade 1 is present in the sample and the user should now proceed to the second multiplex real-time PCR disclosed in this invention.

Using multiplex 2, *M. caprae* is present if a positive signal is observed for the Cyan 500 labelled *M. caprae lepA,* the FAM labelled *lpqT,* the HEX labelled RD1 and the Cy5 labelled IAC MSMEG_0660 diagnostics assay, but the ROX labelled RD701 diagnostics assay does not generate a positive signal.

### Identification of M. africanum clade 2

In certain embodiments of the invention, the multiplex *in vitro* nucleic acid amplification method is for identifying *M. africanum clade 2.* In certain embodiments, the method of the present invention comprises detecting a plurality of nucleic acid molecules which, in combination, are unique in their presence or absence to *Mycobacterium africanum* clade 2.

In certain embodiments, the invention uses primers or probes specific for a nucleic acid that is present in *M*. *africanum* clade 2 but is not present in *M. bovis, M. bovis BCG. M. caprae, M. pinnipedii,* and *M. microti,* and optionally is not present in *M. tuberculosis* and *M. canettii.*

In certain embodiments, the presence or absence of *M africanum* clade 2 is identified using primers or probes specific for a region of RD701, SEQ ID NO: 132.

In certain embodiments, RD701 is amplified using primers which comprise or consist of SEQ ID NOs 170 and 171.

In certain embodiments, presence or absence of RD701 is determined using a probe which comprises or consists of SEQ ID NO: 172. The probe is preferably labelled and the label may be fluorescent. In one embodiment the label is ROX.

In certain embodiments, *M. africanum* clade 2 is present if the MTC *lepA* and IAC (*lepA* or MSMEG_0660) diagnostics assays generate a positive signal, but the *wbbl1*, the RD^{canettii1} and the RD713 diagnostics assays do not generate positive signals, and the RD701, the RD1 and the IAC (*lepA* or MSMEG_0660) diagnostics assays generate positive signals, but the *M. caprae lepA* and the *lpqT* diagnostics assays do not generate positive signals.

In one specific embodiment, *M. africanum* clade 2 is present if in multiplex 1, the HEX labelled MTC *lepA* and Cy5 labelled IAC MSMEG_0660 diagnostics assays generate a positive signal, but the FAM labelled *wbbl1,* the ROX labelled RD^{canettii1} and the Cyan 500 labelled RD713 diagnostics assays do not generate positive signals. This indicates that a member of the MTC other than *M. tuberculosis, M. canettii* and *M. africanum* clade 1 is present in the sample and the user should proceed to the second multiplex real-time PCR disclosed in this invention.

Using multiplex 2, *M. africanum* clade 2 is present if a positive signal is observed in the ROX labelled RD701, the HEX labelled RD1 and the Cy5 labelled IAC MSMEG_0660 diagnostics assays, but the Cyan500 labelled *M. caprae lepA* and the FAM labelled *lpqT* diagnostics assays do not generate positive signals.

### Identification of M. pinnipedii

In certain embodiments of the invention, the multiplex *in vitro* nucleic acid amplification method is for identifying *M. pinnipedii.* In certain embodiments, the method of the present invention comprises detecting a plurality of nucleic acid molecules which, in combination, are unique in their presence or absence to *Mycobacterium pinnipedii.*

In certain embodiments, *M. pinnipedii* is present if a positive signal is observed for the MTC *lepA,* IAC (*lepA* or MSMEG_0660) and RD1 diagnostics assays and no further positive signals are identified.

In one specific embodiment, *M. pinnipedii* is present if a positive signal is observed in the HEX labelled MTC *lepA* and the Cy5 labelled IAC MSMEG_0660 diagnostics assay in multiplex 1 and no positive signal is observed for all other assays in multiplex 1 and multiplex 2, with the exception of a positive signal in the HEX labelled RD1 and the Cy5 labelled MSMEG_0660 diagnostics assays in multiplex 2.

### Identification of M. microtii

In certain preferred embodiments of the invention, the *multiplex in vitro* nucleic acid amplification method is for identifying *M. microtii.* In certain embodiments, the method of the present invention comprises detecting a plurality of nucleic acid molecules which, in combination, are unique in their presence or absence to *"Mycobacterium microti.*

In certain embodiments *M. microti* is present if a positive signal is observed in the MTC *lepA* and the IAC (*lepA* or MSMEG_0660) diagnostics assays and no other positive signal is observed.

In one specific example, *M. microti* is present if a positive signal is observed in the HEX labelled MTC *lepA* and the Cy5 labelled IAC MSMEG_0660 diagnostics assay in Multiplex 1 and a positive signal is observed in the Cy5 labelled IAC MSMEG_0660 diagnostics assay in Multiplex 2.

### Assay methods

The method of the present invention preferably comprises the steps of DNA isolation, amplification and detection. DNA isolation can be performed using any technique known in the art from whichever sample is to be tested. DNA amplification is preferably performed with the use of primers and the polymerase chain reaction (PCR). Other methods of amplification will be apparent to those skilled in the art. One or more pairs of primers is designed to anneal to each nucleic acid molecule target and DNA polymerases are used to amplify the nucleic acid sequence between the primer annealing sites during thermal cycling. The presence of the amplified nucleic acid molecule targets is then detected. This can be done through the use of gel electrophoresis but in preferred embodiments of the invention, detection is performed with the use of labelled probes specific for the amplified nucleic acid molecule targets. Preferably, fluorescent probes are used in detection. A wide range of fluorescent probes are available and include FAM, HEX, ROX, CY5, JOE, VIC and Texas Red and many more will be known to those skilled in the art. Quencher dyes such as Black Hole Quenchers are preferably used in conjunction with the fluorescent probes. As detailed below, in the multiplex assays of the present invention, each probe preferably uses a different fluorescent marker with a different output wavelength so that amplification of all the different nucleic acid molecule targets can be detected at the same time, in the single reaction.

The methods of the invention utilise multiplex PCR assays wherein more than one nucleic acid molecule target is amplified and detected in a single PCR reaction with the use of a plurality of probes and sets of primers. Multiple sets of primers are used; each specific for a different nucleic acid molecule target. Multiple different probes are used; each specific for an amplified nucleic acid molecule targets. Preferably, each probe is labelled differently, for example with different fluorophores, so that amplification of each target can be detected independently but at the same time in the single multiplex reactions, for example through the use of different colour channels. In the detection phase, the presence or lack of a signal in the different channels, indicating the presence or absence of amplification of the different nucleic acid molecule targets, is used to determine the identity of the species in a sample.

The present invention contemplates the use of any appropriate method for amplification of target molecules. Preferably, the method of amplification is multiplex PCR. However, the teaching of the present invention and the sequences identified herein as allowing the identification of specific members of the MTC can be used with any appropriate method of amplification.

Also contemplated for use in the present invention is Nucleic Acid Sequence Based Amplification (NASBA). Nucleic acid sequence-based amplification (NASBA) is an isothermal amplification technique which uses three enzymes - RNase H, AMV reverse transcriptase and T7 RNA polymerase - working in concert at a low isothermal temperature (generally 41°C). The product of a NASBA reaction is mainly single-stranded RNA, which can be detected by gel electrophoresis, enzyme-linked gel assay (ELGA) or electrochemiluminescent detection (ECL). Alternatively, NASBA products can be detected in real time using molecular beacons included in the reaction (Rodriguez-Làzaro *et al.,* 2004). In microbial diagnostics, NASBA has been successfully combined with electrochemiluminescent (ECL), ELISA labelled dendrimer and molecular beacon-based methods to detect and identify viral and bacterial pathogens. (Scheler et al., 2009).

Also contemplated for use in the present invention is Rolling Circle Amplification (RCA). RCA describes a process of unidirectional nucleic acid replication that can rapidly synthesize multiple copies of circular molecules of DNA or RNA. RCA is a technology that is adaptable to an on-chip signal amplification format. RCA is well suited to solid phase formats such as microarrays for generating localized signals at specific microarray locations. This distinctive property of RCA should allow many assays to be performed simultaneously (multiplexing) without interference (Nallur *et al.,* 2001).

Also contemplated for use in the present invention is the Ligase Chain Reaction (LCR). LCR uses two complementary pairs of probes which, when the correct template is available, hybridize next to each other and then are ligated together. These ligated probes plus the original template serve as the template for the next cycle of hybridization and ligation. As subsequent cycles are performed, the amplification proceeds exponentially (Dille *et al.,* 1993). A commercially available kit using this technology is the LCx *M. tuberculosis* complex specific kit available from Abbott Diagnostics (Tortoli *et al.,* 1997).

Further isothermal amplification technologies that are contemplated for use with the present invention are provided in Gill and Ghaemi, 2007 and include signal mediated amplification of RNA technology (SMART), strand displacement amplification (SDA), loop mediated isothermal amplification (LAMP), isothermal multiple displacement amplification (IMDA), helicase-dependent amplification (HDA), single primer isothermal amplification (SIPA) and circular helicase dependent amplification (cHDA). As exemplified by SMART, the amplification method used with the invention may comprise signal amplification rather than target amplification.

Also contemplated for use in the present invention is Next Generation Sequencing (NGS). Next generation sequencing is a relatively new field of sequencing which allows for the rapid high throughput process. NGS has the capacity to generate gigabases of nucleotide sequence, depending on the instrument used, in a single run (Voelkerding *et al.,* 2009). A recently described assay combines the use of real-time PCR in combination with pyrosequencing which allows for the rapid detection of MTC DNA in addition to sequencing of an 81-bp core region of the rpoB gene associated with rifampin resistance (Halse *et al.*)

### Multiplex assay formats

As discussed above, the methods of the present invention utilise different nucleic acid molecule targets to identify species of the MTC in a multiplex *in vitro* nucleic acid amplification assay.

In certain embodiments one or more multiplex *in vitro* nucleic acid amplification assays each utilising one or more nucleic acid molecule targets are performed sequentially in order to fully characterise the MTC member present in a sample.

In preferred embodiments of the invention, the initial multiplex *in vitro* nucleic acid amplification assay (Multiplex 1) utilises a target that is present in all members of the MTC, a target that is present in only two members of the MTC and a target that is present in only one of these two members. In preferred embodiments, *lepA* is used to identify the MTC, *wbbl11* is used to identify *M. tuberculosis* and *M. canettii* and RD*^{canettii1}* is used to identify *M. canettii.* This allows the identification of the MTC in general, and also specific evaluation of the presence or absence of *M. tuberculosis* and *M. canettii.* RD^{canettii1} is used to identify *M. canettii* in embodiments of the invention.

In alternative preferred embodiments of the invention, the initial multiplex *in vitro* nucleic acid amplification assay (Multiplex 1) utilises a target that is present in all members of the MTC, a target that is present in only three members of the MTC and two targets that are present in only one of these three members. *lepA* may be used to identify the MTC, *wbbl11* may be used to identify *M. tuberculosis, M. canettii* and *M. africanum* clade 1. *RD*^{*canettii*1} is used to identify *M. canettii* and RD713 may be used to identify *M. africanum* clade 1. This allows the identification of the MTC in general, and also specific evaluation of the presence or absence of *M. tuberculosis, M. canettii* and *M. africanum clade 1.* It will be appreciated that when performing the Multiplex 1 assay that any of the nucleic acid molecule targets, primers and probes described in the various embodiments of the invention presented above may be utilised.

A second multiplex *in vitro* nucleic acid amplification assay may be performed if additional characterisation is required. In particular, a second multiplex *in vitro* nucleic acid amplification assay may be performed if Multiplex 1 indicates that a member of the MTC other than *M. tuberculosis* or *M. canettii* or other than *M. tuberculosis, M. canettii* or *M. africanum* clade 1 is present.

In further preferred embodiments on the invention, a second multiplex *in vitro* nucleic acid amplification assay (Multiplex 2) utilises a target that is present in only three members of the MTC, a target that is present in only one of these three members, a target which is deleted in only one of these three members and one additional member, and a target which is only present in one additional member.

*lpqT* may be used to identify *M. bovis, M. bovis, BCG* and *M. caprae., M. caprae lepA* may be used to identify *M. caprae.* Deletion of RD1 may be used to identify *M. bovis BCG* and *M. microti.* RD701 may be used to identify *M. africanum* clade 2. *M. pinnipedii* can subsequently be identified by a positive result for RD1 only. It will be appreciated that when performing Multiplex 2, any of the nucleic acid molecule targets, primers and probes described in any of the embodiments of the invention presented above, may be utilised.

Those skilled in the art will appreciate how using different selections of sequences will allow different species to be identified. For example, it is not essential to use a target to identify the MTC in Multiplex 1 if it is desired only to confirm the presence or absence of, for example, *M. tuberculosis* and *M. canettii.* Therefore, in certain embodiments of the invention, only one or two nucleic acid molecule targets are used. It will also be appreciated that either Multiplex 1 or Multiplex 2 can be performed independently, or that Multiplex 1 and Multiplex 2 can be performed sequentially in any order. It will also be appreciated that if Multiplex 1 identifies the member of the MTC, performance of Multiplex 2 may not be required.

In addition to the nucleic acid molecule targets discussed for each multiplex above, an IAC may be included in the multiplex reaction. In one embodiment the IAC may be *lepA* or MSMEG_0660, and the primers and probes discussed above for this nucleic acid molecule target may be used.

It will be apparent to the skilled person that the invention is not restricted to the detection of members of the MTC in a two step multiplex assay format, and the invention therefore encompasses the use of any number of the nucleic acid molecule targets and methods described above in a single multiplex assay. In one embodiment all of the nucleic acid molecule targets described above may be used in a single multiplex assay. In alternative embodiments 3, 4, 5, 6, 7, 8, 9 or 10 targets are used, to allow identification of more species, and potentially with more confidence. Embodiments of the invention use primers or probes specific for a nucleic acid sequence that is present in *M. canettii* but not present in *M.tuberculosis* and comprises a region of RD^{canettii1} SEQ ID NO: 78.

The use of a number of different multiplex *in vitro* nucleic acid amplification assays of the invention allows identification of a greater number of species of the MTC. Different multiplex assays, each using a certain combination of primers and probes to identify different species of the MTC, may be performed on a series of aliquots of a sample or a group of samples, either in parallel or sequentially.

### Uses of the invention

The methods and kits of the present invention and as disclosed herein can be used to perform various analyses. In general, the use of a method or kit of the invention or as disclosed herein to analyse the nucleic acids present in a sample is disclosed. Some of the types of analyses envisaged by the inventors are described below.

The use of a method or kit of the invention or as disclosed herein to identify the type of cell(s) present in a biological sample (such as a sample taken from a patient) is disclosed. For example, a method for identifying the type of cell(s) present in a biological sample, the method comprising analysing a sample nucleic acid obtained from the biological sample using an analysis method as described herein, and using the results of the analysis to identify the type of cell(s) present in the biological sample is disclosed.

Methods or kits as disclosed herein or of the invention can be used to analyse a sample taken from a human patient, such as a sputum sample, a pus sample, a lung fluid sample, a lymph node sample, a pleural fluid sample, a pleural tissue sample, a blood sample, a plasma sample, a serum sample, a urine sample, a tissue sample, or a saliva sample.

A method or kit as disclosed herein or of the invention can be used to diagnose a disease or condition in a patient (e.g. a human patient). Preferably, the disease is tuberculosis or a related condition. For example, a method for diagnosing a disease or condition in a patient, comprising analysing the nucleic acid in a sample obtained from the patient using an analysis method as described herein, and using the results of the analysis to diagnose a disease or condition in the patient is disclosed. In some embodiments, methods of diagnosis as described herein are performed *in vitro* on a sample taken from a patient.

A method or kit as disclosed herein or of the invention may be used to select a therapeutic strategy or treatment regimen for treating a disease or condition in a patient. For example, a method for selecting a therapeutic strategy or treatment regimen for treating a disease or condition in a patient (e.g. a human patient), comprising analysing a sample nucleic acid obtained from the patient using an analysis method as described herein, identifying the presence of a pathogenic species and using the results of the analysis to select a therapeutic strategy or treatment regimen for treating the disease or condition is disclosed. These methods may be performed *in vitro* on a sample taken from a patient.

In one embodiment, the invention provides the method or kit or diagnostic technique of the invention for use in monitoring disease progression or status of a disease or condition in a patient, e.g. to monitor a patient's response to treatment.

The use of a method or kit as disclosed herein or of the invention for biosurveillance, e.g. to detect pathogens in samples, such as water, food or soil samples is disclosed.

As discussed above, the assays provided in the present invention and the sequences disclosed and characterised herein are useful for the identification of species of the MTC and allow a rapid and specific identification that is not achieved with the methods of the prior art and which would not be possible with the sequences that have been previously identified and characterised. The methods, assays and sequences of the present invention will be useful in diagnosis of disease. As discussed above, the different members of the MTC respond differently to treatment and differ in their pathology. Therefore, it is essential that medical professionals are able to identify which species are present to make an accurate diagnosis and provide suitable treatment.

The methods, assays and sequences of the present invention and as disclosed herein will also be useful in a range of other applications. The simple and effective nature of the multiplex assays that are made possible with the types of sequences identified and characterised herein mean that the assays are suitable for routine screening and diagnosis of not only patients with tuberculosis symptoms but also patients potentially at risk, such as HIV patients and patients who have spent time in certain risk areas, for example.

The methods, assays and sequences of the present invention and as disclosed herein will also be useful in maintenance of research stocks of MTC species. Due to the high similarity between different species, it was not easy, prior to the present invention, to identify or confirm the identity of MTC species kept as stocks in, for example, research laboratory situations.

The present invention will also be useful in other research situations, including monitoring the growth and survival of different MTC species and, for example, the effectiveness of drug treatments and development of drug resistance.

The individual sequences identified and characterised herein and primers and probes directed to these sequences will also be useful a range of other applications, including, as discussed below, the development and use of microarray platforms. Also, the RD^{canetti1} region in particular, which is herein identified and characterised as unique to *M. canettii,* is currently not annotated. Therefore, primers and probes directed to the region will be useful in further characterising the region, identifying genes present in the region and in analysis of expression of the region.

### Alternative methods of MTC member identification

In addition to the methods described above, this invention contemplates the use of some of or all of the sequences provided in alternative methods for the identification of species of the MTC.

The present invention provides the use of hybridisation techniques using probes specific for RD^{canetti1} and optionally, *wbbl1,* MTC *lepA,* RD713, *M. caprae lepA, lpqT,* RD1 and RD701 either individually or in combination and preferably as part of an array comprising a plurality of probes which can specifically detect a number of different members of the MTC.

Preferably the present invention provides the use of hybridisation techniques using probes specific for one or more of RD^{canetti1}, *wbbl1,* MTC *lepA* and RD713 in combination and preferably as part of an array comprising a plurality of probes which can specifically detect *M. canettii, M. tuberculosis,* any member of the MTC and *M. africanum* clade 1, respectively. More preferably the invention provides the use of hybridisation techniques using probes specific for all of RD^{canetti1}, *wbbl1, lepA* and RD713 in combination and preferably as part of an array. Probes specific for RD*^{canettii}* are used in embodiments of the invention.

Probes specific for *M. caprae lepA, lpqT,* RD1 and RD701 may additionally be used, either individually or in combination and preferably as part of an array comprising a plurality of probes which can specifically detect *M. caprae, M. bovis, M. bovis BCG,* and *M. africanum* clade 2, respectively. More preferably the invention provides the use of hybridisation techniques using probes specific for all of *M. caprae lepA, lpqT,* RD 1 and RD701 in combination and preferably as part of an array.

In one embodiment hybridisation techniques may be used with probes specific for the two groups of nucleic acid molecule targets described below in combination and preferably as part of an array. The two groups of nucleic acid molecule targets may be arrange as a single array with the two groups positioned apart from one another at spaced locations, or as two separate arrays.

Such arrays will allow the high-throughput screening of samples and rapid diagnosis of specific pathogens. The properties of the sequences provided herein, as described above, make them suitable for use in such microarray platforms and screening methods. Due to their specific presence or absence in the different species of the MTC, the sequences provided herein are suitable for use in a range of different hybridisation techniques and microarray applications.

The multiplex real-time PCR developed in this study is the first description of a hydrolysis probe based diagnostic tool capable of rapid detection of the MTC, combined with the detection and differentiation of members of the MTC using novel targets. As exemplified herein, this rapid, specific and sensitive multiplex real-time PCR assay takes approximately 50 minutes after DNA extraction. Depending on the number of samples and the extraction methods used the total assay time may be approximately 2-3 hours. This assay has been tested on *Mycobacteria* DNA from clinical isolates. Testing of TB positive and negative patient samples, for example sputum and bronchial lavage, will further validate the assay in due course. The multiplex real-time PCR assay presented here may be used in the hospital laboratory for the routine detection of the MTC and detection of the member of the MTC present, including simultaneous differentiation of *M. tuberculosis* and *M. canettii.*

### Kits

The present invention additionally provides kits suitable for use in the methods provided herein. A kit comprising sets of primers and probes which are specific for a plurality of nucleic acid molecule targets, wherein at least one of the nucleic acid molecule targets is present in the genome of one or more, but not all, of the species of the *Mycobacterium tuberculosis* complex is disclosed.

A kit comprising sets of primers and probes specific for a plurality of nucleic acid molecules which are unique in their presence or absence to *Mycobacterium tuberculosis* is disclosed.

A kit comprising sets of primers and probes specific for a plurality of nucleic acid molecules which are unique in their presence or absence to *Mycobacterium canettii* is disclosed.

A kit comprising primers or probes specific for a nucleic acid that is not present in both *M. tuberculosis* and *M. canettii* is disclosed.

A kit comprising primers or probes specific for a nucleic acid that is present in *M. canettii* but is not present in *M. tuberculosis,* and optionally is also not present in *M. africanum* clade 2, *M. bovis, M. bovis BCG, M. caprae, M. pinnipedii,* and *M*. *microti* is disclosed.

The invention provides a kit comprising a set of primers and probes specific for a plurality of nucleic acid molecule targets, which in combination are unique in their presence or absence to *Mycobacterium canettii* and wherein the kit comprises primers and probes specific for a nucleic acid that is present in *M. canettii* but is not present in *M. tuberculosis* and specific for a region of RD canettii, SEQ ID NO: 78.

In certain embodiments, the invention provides a kit wherein the primers comprise or consist of SEQ ID NOs 103 and 105.

In certain embodiments, the invention provides a kit wherein the probe comprises or consists of SEQ ID NO: 104.

A kit comprising primers or probes specific for a nucleic acid that is present in both *M. tuberculosis* and *M. canettii,* but is not present in *M. africanum* clade 1 and optionally is not present in *M. africanum* clade 2, *M. bovis, M. bovis BCG, M. caprae, M. pinnipedii,* and *M. microti* is disclosed.

A kit comprising primers or probes specific for *wbbl1,* SEQ ID NO: 1 is disclosed.

A kit wherein the primers comprise or consist of SEQ ID NOs 97 and 99 is disclosed.

A kit wherein the probe comprises or consists of SEQ ID NO: 98 is disclosed.

A kit comprising sets of primers and probes specific for a plurality of nucleic acid molecules which are unique in their presence or absence to *Mycobacterium africanum* clade 1 is disclosed.

A kit comprising primers or probes specific for a nucleic acid that is present in *M. africanum* clade 1 but is not present in *M. canettii or M. tuberculosis,* and optionally is also not present in *M. africanum* clade 2, *M. bovis, M. bovis BCG, M. caprae, M. pinnipedii,* and *M. microti* is disclosed.

A kit comprising primers or probes specific for a region of RD713, SEQ ID NO: 137 is disclosed.

A kit wherein the primers comprise or consist of SEQ ID NOs 167 and 168 is disclosed.

A kit wherein the probe comprises or consists of SEQ ID NO: 169 is disclosed.

A kit comprising primers or probes specific for a nucleic acid that is present in *M. bovis, M. bovis BCG* and *M. caprae* but is not present in *M. africanum* clade 2, *M. pinnipedii* and *M. microti,* and optionally is not present in *M. tuberculosis* and *M. canettii* or *M. africanum* clade 1 is disclosed, as is a kit comprising primers or probes specific for a region of *lpqT*, SEQ ID NO: 109, a kit wherein the primers comprise or consist of SEQ ID NOs 158 and 159, and a kit wherein the probe comprises or consists of SEQ ID NO: 160.A kit comprising sets of primers and probes specific for a plurality of nucleic acid molecules which are unique in their presence or absence to *Mycobacterium bovis* is disclosed, as is a kit comprising sets of primers and probes specific for a plurality of nucleic acid molecules which are unique in their presence or absence to *Mycobacterium caprae.*

A kit comprising primers or probes specific for a nucleic acid that is present in *M. caprae* but is not present in *M. bovis, M. bovis BCG, M. africanum* clade 2, *M. pinnipedii* or *M. microtti,* and optionally is not present in *M. tuberculosis* and *M. canettii* is disclosed, as is a kit comprising primers or probes specific for a region of *M. caprae lepA,* SEQ ID NO: 76, a kit wherein the primers comprise or consist of SEQ ID NOs 164 and 165 and a kit wherein the probe comprises or consists of SEQ ID NO: 166.

A kit comprising sets of primers and probes specific for a plurality of nucleic acid molecules which are unique in their presence or absence to *Mycobacterium bovis BCG* is disclosed, as is a kit comprising sets of primers and probes specific for a plurality of nucleic acid molecules which are unique in their presence or absence to *Mycobacterium microti.*

A kit comprising primers or probes specific for a nucleic acid that is deleted in *M. bovis BCG* and *M. microti* but is present in *M. bovis, M. caprae, M. africanum* clade 2, and *M. pinnipedii,* and optionally is present in *M. tuberculosis* and *M. canettii* and *M. africanum* clade 1 is disclosed, as is a kit comprising primers or probes specific for a region of RD1, SEQ ID NO: 141, a kit wherein the primers comprise or consist of SEQ ID NOs 161 and 162and a kit wherein the probe comprises or consists of SEQ ID NO: 163.

A kit comprising sets of primers and probes specific for a plurality of nucleic acid molecules which are unique in their presence or absence to *Mycobacterium africanum* clade 2 is disclosed.

A kit comprising primers or probes specific for a nucleic acid that is present in *M. africanum* clade 2 but is not present in *M. bovis, M. bovis BCG, M. caprae, M. pinnipedii,* and *M. microti,* and optionally is not present in *M. tuberculosis, M. canettii* and *M. africanum* clade 1 is disclosed, as is a kit comprising primers or probes specific for a region of RD701, SEQ ID NO: 132, a kit wherein the primers comprise or consist of SEQ ID NOs 170 and 171 and a kit wherein the probe comprises or consists of SEQ ID NO: 172.

A kit comprising sets of primers and probes specific for a plurality of nucleic acid molecules which are unique in their presence or absence to *Mycobacterium pinnipedii* is disclosed.

In one embodiment, the invention comprises a kit comprising primers or probes which allow differentiation between *M. tuberculosis* and *M. canettii.* Such a kit may comprise primers comprising or consisting of SEQ ID NOs: 103 and 105 and 97 and 99. Such a kit may also comprise probes comprising or consisting of SEQ ID NOs: 104 and 98.

In one embodiment, the invention comprises a kit comprising primers or probes which allow differentiation between *M. tuberculosis, M. canettii,* and *M. africanum* clade 1. Such a kit may comprise primers comprising or consisting of SEQ ID NOs: 103 and 105, 97 and 99, and 167 and 168. Such a kit may also comprise probes comprising or consisting of SEQ ID NOs: 104, 98 and 169.

Also disclosed is a kit comprising primers or probes which allow differentiation between *M. caprae, M. bovis, M. bovis BCG, M. africanum* clade 2, *M. pinnipedii* and *M. micoti.* Such a kit may comprise primers comprising or consisting of SEQ ID NOs: 164 and 165, 158 and 159, 161 and 162, and 170 and 171. Such a kit may also comprise probes comprising or consisting of SEQ ID NOs: 166, 160, 163 and 172.

In one embodiment any of the kits of the invention or disclosed above may additionally comprise primers and probes specific for the identification of a member of the MTC. Also disclosed is a kit comprising primers or probes specific for a region of MTC *lepA,* SEQ ID NO: 47, a kit wherein the primers comprise or consist of SEQ ID NOs 164 and 165 and a kit wherein the probe comprises or consists of SEQ ID NO: 173.

In another embodiment any of the kits of the invention or disclosed above may additionally comprise primers and probes specific for an IAC.

Also disclosed is a kit comprising primers or probes specific for a region of *lepA,* SEQ ID NO:84 a kit wherein the primers comprise or consist of SEQ ID NOs 164 and 165 and a kit wherein the probe comprises or consists of SEQ ID NO: 107.

A kit comprising primers or probes specific for a region of MSMEG_0660, SEQ ID NO: 135 is disclosed, as is
a kit wherein the primers comprise or consist of SEQ ID NOs 155 and 156 and a kit wherein the probe comprises or consists of SEQ ID NO: 157.

Kits of the invention are as defined in claims 12 and 13.

One or more nucleic acid molecules comprising a sequence selected from the group consisting of SEQ ID NOs: 1-78 and 109-154 and sequences complementary to SEQ ID NOs: 1-78 and 109-154, for use in identifying a species of the *Mycobacterium tuberculosis* complex is disclosed.

Preferably, the one or more nucleic acid molecules correspond to the species of the MTC that is to be identified (for example, *Mycobacterium tuberculosis -* SEQ ID NOs:1-5, 15-39 and 47-53, *M. canettii -* SEQ ID NOs: 40-44, 58, 71, 72 and 78-83, *M. bovis -* 115 and 148, *M. bovis BCG -* 113, 114, *M. caprae* 128, 129, 151 and 152, *M. africanum* clade 1 - 137, 139, 140, *M. africanum* clade 2 - 132-134).

### Definitions

As used herein, the following terms and phrases shall have the meanings set forth below. Unless defined otherwise, all technical and scientific terms used herein have the same meaning as commonly understood by one of ordinary skill in the art to which this invention belongs.

The articles "a" and "an" refer to one or to more than one (i.e., to at least one) of the grammatical object of the article. By way of example, "an element" means one element or more than one element.

The terms "comprise" and "comprising" are used in the inclusive, open sense, meaning that additional elements may be included.

The terms "consists" and "consisting of" are used in the exclusive, closed sense, meaning that no additional elements may be included.

The term "including" is used herein to mean "including but not limited to". "Including" and "including but not limited to" are used interchangeably.

A "patient", "subject" or "host" to be tested by the method of the invention may mean either a human or non-human animal and is preferably a mammal, more preferably a human. The human may be a child or an adult.

The *Mycobacterium tuberculosis* complex (MTC) includes species that are known to cause tuberculosis in humans or animals.

The term "nucleic acid molecule target" is used herein to mean a nucleic acid molecule that is specifically amplified and detected to identify species of the MTC. Preferably, the target is a genomic DNA sequence.

By the term "specific for" is meant that primers or probes hybridize to a particular target nucleic acid sequence in preference to other nucleic acid sequences, particularly in preference to another gene implicated in the MTC. A probe or primer which is "specific for" a target sequence preferentially hybridises to that sequence and a primer or probe is considered to be "specific for" a target sequence if it binds to that sequence with greater affinity than a sequence from another gene implicated in the MTC. For example, the probe or primer may bind with 2-fold, 3-fold, 4-fold, 5-fold, six-fold, seven-fold, eight-fold, nine-fold, 10-fold, 15-fold, 20-fold, 30-fold, 30-fold, 40-fold, 50-fold, 60-fold, 70-fold, 80-fold, 90-fold, 100-fold, 200-fold, 300-fold, 400-fold, 500-fold, or 1000-fold greater affinity to the target sequence which it is "specific for" than to a sequence from another gene implicated in the MTC.

The term "primer" is used herein to mean a pair of nucleic acid molecules for use in assisting amplification of specific nucleic acid molecules. Primers hybridize specifically to their target sequence and are "specific for" that sequence. Primers are preferably 100% complementary to the sequence to which they are targeted. However, primers may be less than completely complementary in sequence, and may be, for example, 99%, 98%, 97%, 96% 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% or less complementary to the sequence to which they are targeted. Primers are preferably between 1 and 100 base pairs long, more preferably 5-50 base pairs long and even more preferably, between 10 and 30 base pairs long such as 10, 11, 12, 13, 14, 15, 16, 17, 18, 19, 20, 21, 22, 23, 24, 25, 26, 27, 28, 29 or 30 base pairs long.

The term "probe" is used herein to mean nucleic acid molecules for the detection of specific nucleic acid molecules. Probes hybridize specifically to their target sequence and are "specific for" that sequence. Probes are preferably 100% complementary to the sequence to which they are targeted. However, probes may be less than completely complementary in sequence, and may be, for example, 99%, 98%, 97%, 96% 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% or less complementary to the sequence to which they are targeted. Probes are preferably between 1 and 100 base pairs long, more preferably 5-50 base pairs long and even more preferably, between 10 and 30 base pairs long, such as 10, 11, 12, 13, 14, 15, 16, 17, 18, 19, 20, 21, 22, 23, 24, 25, 26, 27, 28, 29 or 30 base pairs long.

Probes are preferably labelled to assist detection. Preferably, they are labelled with a fluorescent dye. Preferably they also comprise a quencher.

The term "sample" is used herein to mean any substance in which a member of the MTC may be found. Preferably the sample is taken from a patient or subject. For example, the sample may be a sputum sample, a pus sample, a lung fluid sample, a lymph node sample, a pleural fluid sample, a pleural tissue sample, a blood sample, a plasma sample, a serum sample, a urine sample, a tissue sample, or a saliva sample.

The term "multiplex *in vitro* nucleic acid amplification assay" is used herein to mean a single reaction wherein two or more different nucleic acid molecules are amplified and preferably detected. In a multiplex PCR assay, this is a polymerase chain reaction and this is achieved with the use of more than one set of primers. The multiplex assays of the invention may amplify and detect a plurality of nucleic acid molecule targets. A "plurality" means more than 1 and includes, for example, 2, 3, 4, 5, 6, 7, 8, 9, 10, 11, 12, 13, 14, 15 or more targets. In certain embodiments the invention may require the performance of one or more separate *"multiplex in vitro* nucleic acid amplification assays".

When referring to nucleic acid molecule targets, the term "present in" is used herein to mean found in the genome of the organism in question. Preferably the genomic sequence is identical to the target but it may be 100-95%, 100-90%, 100-80%, 100-70%, 100-60% or 100-50% identical to the target.

"Microarrays" are a collection of small microscopic features (DNA/ RNA/ Proteins) which are usually probed with target molecules to produce data, Some examples of Microarrays are printed microarrays, in situ-synthesized oligonucleotide microarrays, high density bead arrays and electronic microarrays (Miller & Tang, 2009). Whole genome microarrays have been carried out in relation to *M. tuberculosis* and *M. bovis* BCG which identified the initial Regions of Difference (RD's) (Behr et al., 1999), which are now used for identifying particular members of the *Mycobacterium Tuberculosis* Complex (Pinsky & Banaei, 2008).

### Brief description of the figures

Figure 1 - (A) an alignment of publicly available *wbbl1* sequences identifying the region of *wbbl1* unique to *M. tuberculosis* and *M. canettii.* (B) Sequencing analysis and alignment of the *wbbl1* region unique to *M. Tuberculosis* and *M. canettii.*
Figure 2 - an alignment of publicly available *lepA* sequences with the forward and reverse primers and the MTC, IAC and *M. caprae* /*epA* probes annotated. Bases that differ from the primers or the MTC probe are highlighted in black. Bases that differ from the IAC probe are highlighted in black and italicised.
Figure 3 - (A) An alignment of publicly available *lpqT* sequences identifying the region of *lpqT* which is deleted in *M. bovis, M. bovis BCG and M caprae.* (B) Sequencing analysis and alignment of the *lpgT* deletion in *M. bovis, M. bovis BCG and M. caprae.*
Figure 4 - An alignment of publicly available RD701 sequences identifying the region uniquely deleted from *M. africanum* clade 2. Where intact this is a 2081 bp region of sequence. In *M. africanum* clade 2 where the deletion is present this is 320bp region. Alignment is complicated because, while there are 100% homology to members of the MTC, this region inserts in different areas of the genome and PE proteins effect alignments.
Figure 5 - (A) An alignment of publicly available RD713 sequences identifying the RD713 region unique to *M. africanum* clade 1. (B) Sequencing analysis and alignment of the RD713 region unique to *M. africanum* clade 1.
Figure 6 - (A) An alignment of publicly available RD1 sequences identifying the region present in *M. caprae* and *M. bovis* but not in *M. bovis* BCG. (B) Sequencing and alignment of the RD1 region present in *M caprae* and *M. bovis* but not in *M. bovis* BCG.

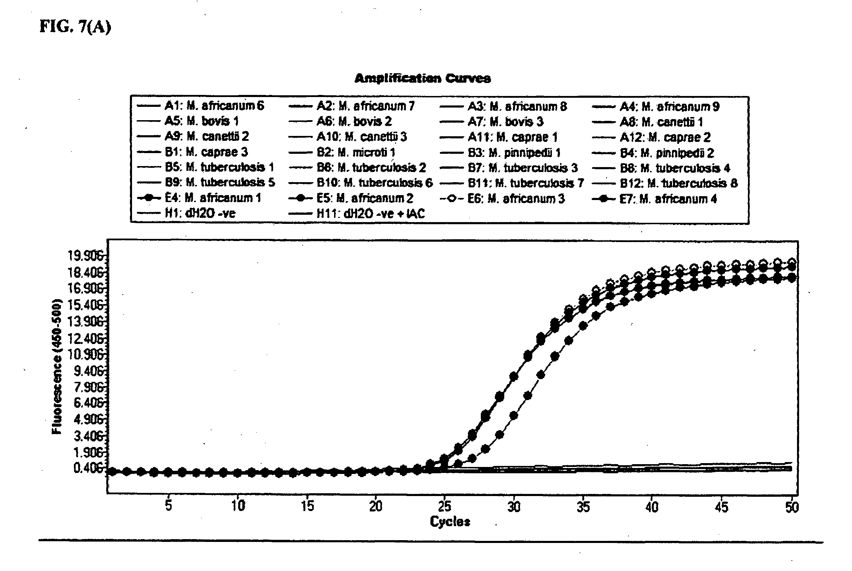

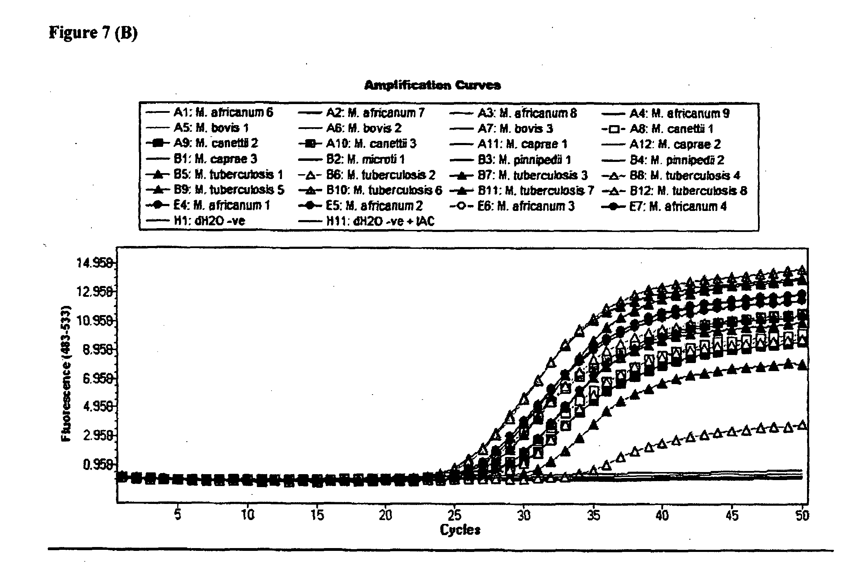

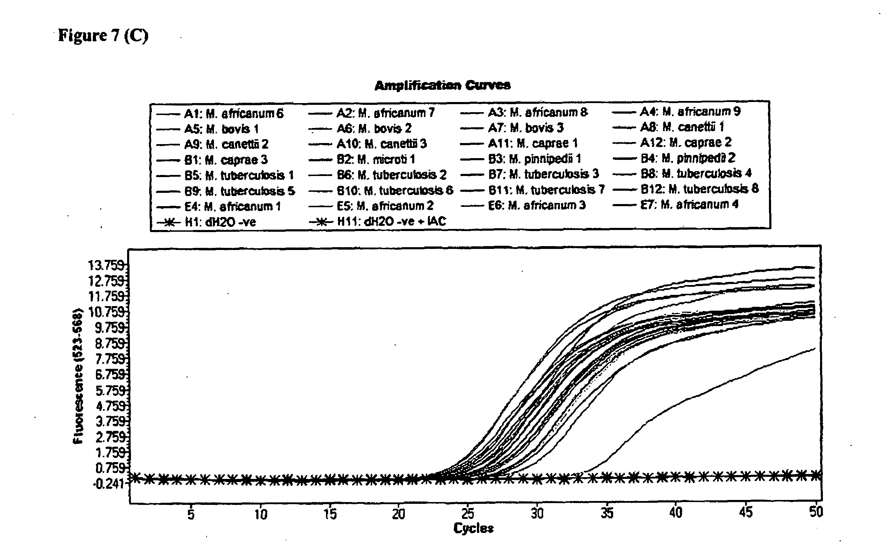

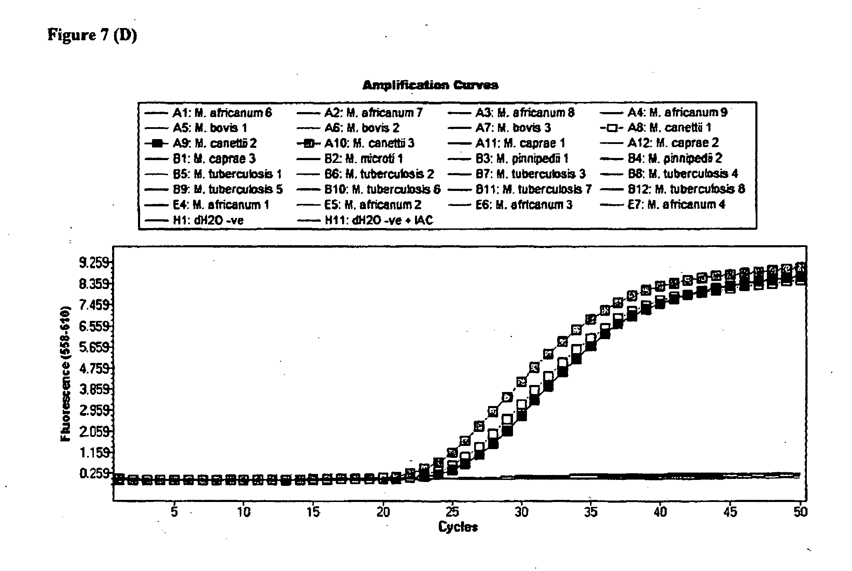

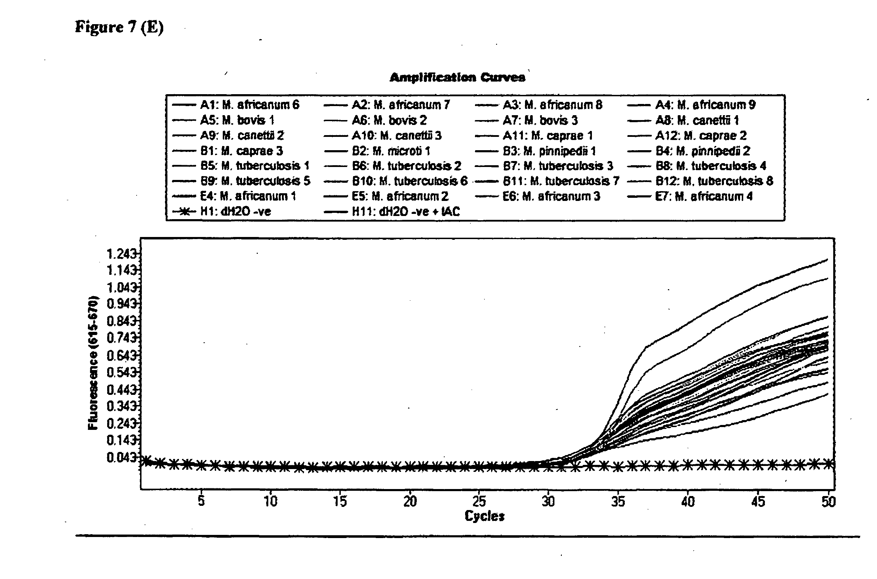

Figure. 7 - (A) Amplification curves for *M. africanum* clade 1 (circle) using a region of RD 713 in Cyan 500 channel (450-500); (B) Amplification curves for *M*. *africanum* clade1 (circle), *M. tuberculosis* (triangle) and *M*. *canettii* (rectangle) using the *wbbl1* gene in FAM channel (483-533); (C) Amplification curves for all MTC using the *lepA* gene in HEX channel (523-568), with the non-template control highlighted with stars through line; (D) Amplification curves for *M. canettii* specific assay in ROX channel (558-610), with *M. canettii,* strains depicted with rectangles; (E) Amplification curves for IAC in Cy5 channel (615-670) with the non-template control highlighted with stars through line.

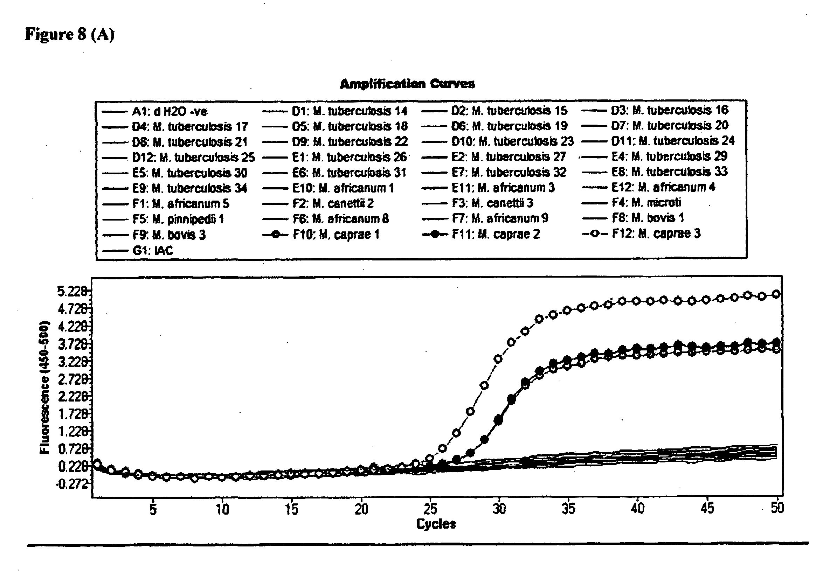

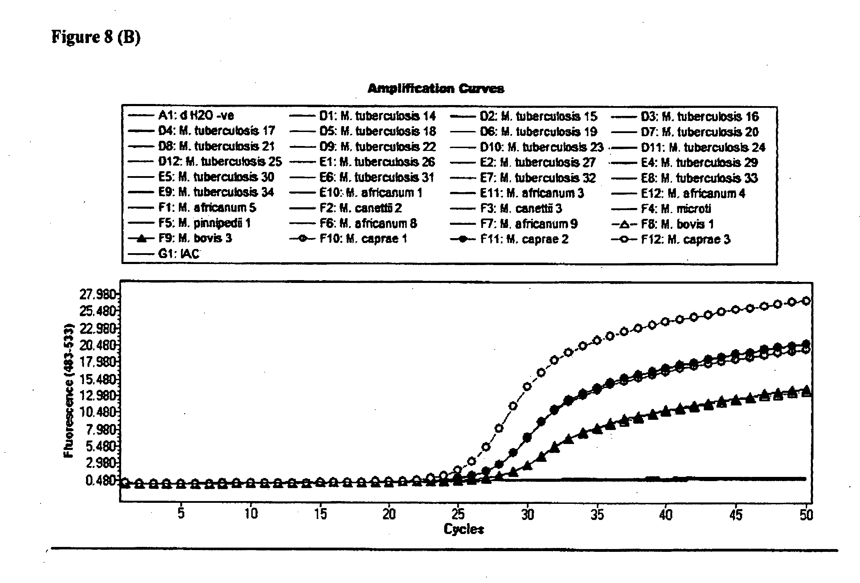

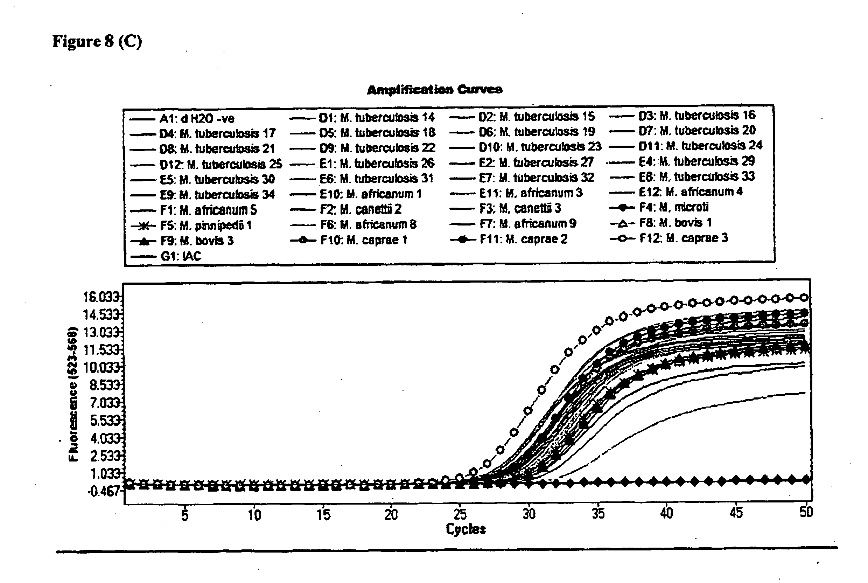

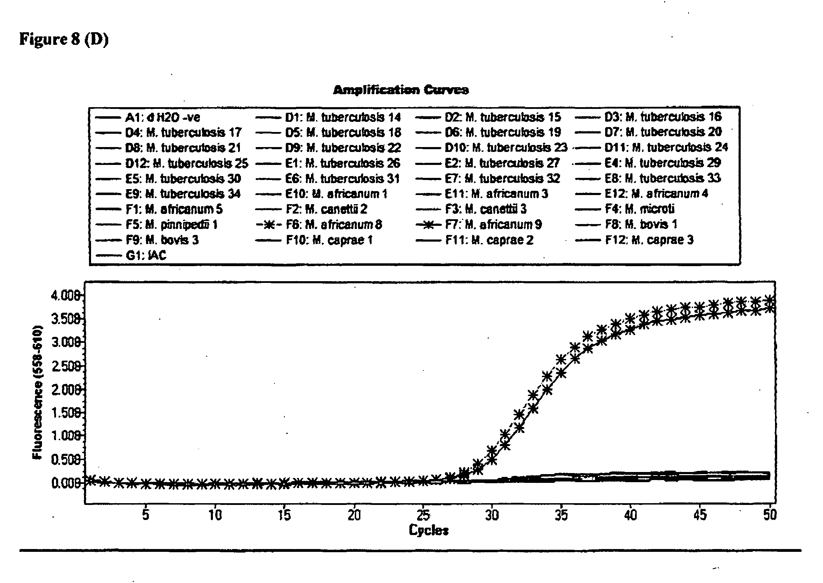

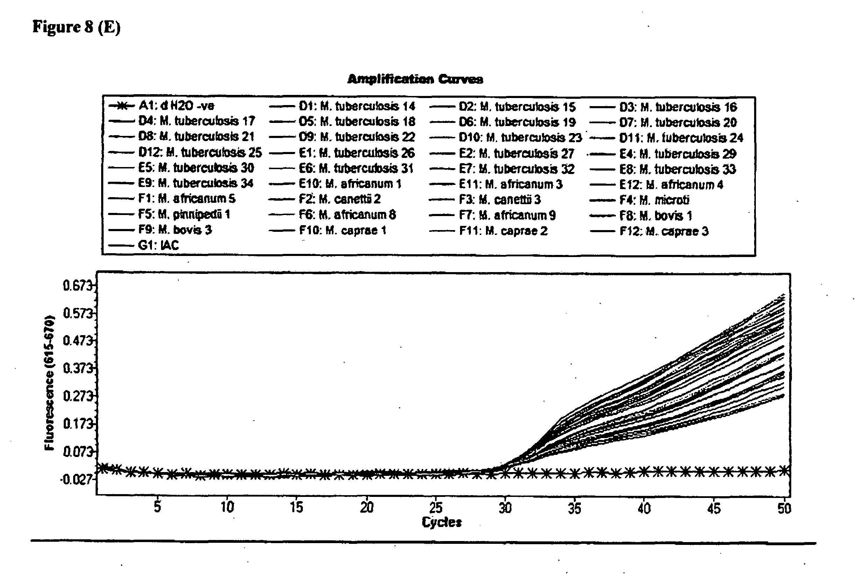

Figure. 8 - (A) Amplification curves for *M. caprae* (circle) using *lepA* in Cyan 500 channel (450-500); (B) Amplification curves for *M. caprae* (circle) and *M. bovis* (triangle) using the *lpqT* gene in FAM channel (483-533); (C) Amplification curves for *M. caprae* (circle), *M. bovis* (triangle), *M. pinnipedii* (star) and *M. microti* (diamond) using a region of RD1 in HEX channel (523-568); (D) Amplification curves for *M. africanum* clade 2 (star) specific assay using a region of RD 701 in ROX channel (558-610); (E) Amplification curves for IAC in Cy5 channel (615-670) with the non-template control highlighted with stars through line.

### Examples

### General techniques

### Bacterial strains, culture media and growth conditions

One hundred and nineteen MTC isolates (60 *M. tuberculosis,* 14 *M. bovis, 7 M. bovis* BCG (of which 2 were from DSMZ and cultured in this study), 8 *M. canettii,* 5 *M. caprae,* 14 *M. africanum,* 6 *M. microti* and 5 *M. pinnipedii*)*,* 44 NTM and 17 other bacteria were used in this study (Tables 2 and 3). Of the 119 MTC, 36 strains previously characterised by spoligotyping, were provided by the National Institute for Health and the Environment, RIVM the Netherlands and 56 strains were provided from the National Reference centre for Mycobacteria, Borstel, Germany. All other MTC strains, provided by Mario Vaneechoutte, were clinical isolates which had been previously characterised to species level, with the exception of the 2 *M. bovis* BCG which were purchased from DSMZ. All NTM were purchased from culture collections (DSMZ) and grown on middle book agar/broth at either 30 °C or 37 °C or DNA was supplied by Mario Vaneechoutte. Mycobacteria considered fast growers were cultured for 3-6 days, whereas slow growers were incubated for six weeks, or until sufficient growth was visible. All media were purchased from BD Biosciences (Oxford, United Kingdom). For all other bacterial species tested, DNA was provided from stocks held within this laboratory.

### DNA isolation and quantification

Genomic DNA from NTM and 2 *M. bovis BCG* cultures was isolated from 1 ml of culture (Middlebrook 7H9 broth, Becton Dickenson), using a modified procedure combining mechanical lysis (IDI lysiskit, GeneOhm, Quebec, Canada) and purification using a DNeasy Blood and Tissue kit (Qiagen, Hilden, Germany). Briefly 1 ml culture was centrifuged in a benchtop centrifuge (Microcentrifuge 5415, Eppendorf) at 13,000 rpm for 3 min. The supernatant was discarded and the pellet resuspended in 250 µl GeneOhm sample buffer. The suspension was transferred to a GeneOhm lysis tube and bead beaten (Mini-Bead-Beater-16™, Stratech, UK) for 3 min. After bead-beating 200 µl was transferred to a sterile microcentrifuge tube and steps 3-8 (add 200 µl of buffer AL and 200 µl ethanol and mix gently by vortexing) for purification of total DNA from animal tissue using the DNeasy Blood and Tissue kit were followed according to the manufacturer's instructions. DNA concentrations were determined using the PicoGreen dsDNA Quantitation Kit (Molecular Probes, Eugene, Oregon, USA) and the TBS-380 mini-fluorometer (Invitrogen Corporation, California, USA). All DNA samples were stored at -20 °C.

### Conventional and real-time PCR primers and hydrolysis probes

Oligonucleotide primers and hydrolysis probes were designed in accordance with the general recommendations and guidelines outlined (Dorak, 2006; Robertson & Walsh-Weller, 1998). All primers and probes (Table 4) used in this study were supplied by MWG-BIOTECH AG (Essenberg, Germany). Sequence data for real-time PCR assay design was generated in-house or was obtained from the National Centre for Biotechnology Information (NCBI) along with BLAST searches carried out on the Sanger website, where partial sequences for *M. canettii, M. africanum* and *M. microti* were available. The primers used for the real-time PCR assays were also used in conventional PCR for generating sequence information for each of the assays used in this study. In addition, sequencing primers were designed to span the full 2869bp *M*. *canettii* specific sequence identified in this study to evaluate if this region is conserved in all *M. canettii* strains in addition to identifying regions which are 100% homologous in each strain.

PCR products were generated as discussed below, followed by purification using high pure PCR product purification kit (Roche Diagnostics Ltd., West Sussex, United Kingdom). The purified products were sent for sequencing externally (Sequiserve, Vaterstetten, Germany).

### Conventional PCR

Conventional PCR was performed using the sequencing primers outlined in Table 4 on the iCycler iQ thermal cycler (Bio-Rad Laboratories Inc., California, USA). All reactions were carried out in a final volume of 50 µl, containing 5 µl 10X buffer (15mM MgCl₂), 1 µl forward and reverse primers (10 µM), 2 µl Taq DNA polymerase (1 U/ µl, Roche Diagnostics, Mannheim, Germany), 1 µl dNTP mix (10 mM: deoxynucleoside triphosphate set, Roche), 2 µl of template DNA, 38 µl Nuclease free water (Applied Biosystems/Ambion, Texas, USA). The cycling parameters consisted of initial denaturation 95 °C for 5 mins, followed by 35 cycles of denaturation 94 °C (1 min), amplification 55 °C (1 min), and extension 72 °C (1 min), followed by a final elongation at 72 °C for 10 min.

### Development of an Internal Amplification Control (IAC) for real-time PCR

An internal control was designed and incorporated in the multiplex assay designed to monitor for PCR inhibition and PCR efficiency. The MSMEG_0660 gene was chosen as the target for the IAC because this gene is present only in *M.* smegmatis. This gene is thought to code for an extracellular solute-binding protein. Titrations of MTC and IAC DNA were performed to determine the optimum concentration of IAC target per reaction such that the IAC is always detected without impacting on detection of the primary MTC target. An internal control concentration of 100 genome equivalents per reaction allowed for the detection of the IAC all real-time PCR experiments performed.

As this is a non-competitive IAC, in order for a result to be considered valid using this assay, a positive signal must be obtained in the Cy5 detection channels on the LightCycler 480. If the internal control is not detected, the result is considered invalid and must be repeated (Hoorfar *et al.,* 2004; O' Grady *et al.,* 2008). *M*. *smegmatis* could also be used as a process control to monitor for DNA extraction efficiency from biological samples.

### Real-time PCR

Monoplex real-time PCR was performed on the LightCycler 2.0 Instrument (Roche Diagnostics) using the LightCycler^{®} TaqMan^{®} Master kit (Roche Diagnostics). A final volume of 20 µl was used in each reaction, containing Master mix 5X, forward and reverse primers (0.5 mM final cone.), FAM labelled probe (0.2 mM final conc.), template DNA (2 µl) and the volume adjusted to 20 µl with the addition of nuclease free dH₂O. The cycling parameters consisted of incubation for 10 min at 95 °C to activate enzymes and DNA denaturation followed by 50 cycles of 95 °C for 10 s and 60 °C for 30 s, followed by a cooling step at 40 °C for 10 s. The temperature transition rate for all cycling steps was 20 °C/s.

Multiplex real-time PCR reactions were carried out on the LightCycler 480 using LightCycler^{®} 480 Probes Master kit (Roche Diagnostics). A final volume of 40 µl was used for each multiplex experiment. The optimised master mix contained 2X LightCycler 480 Probes Master (6.4mM MgCl₂), forward and reverse primer (0.5mM final conc.), FAM labelled probe (0.4 µM final conc.), HEX, ROX and CY5 labelled dyes (0.2 µM final conc.), template DNA (MTC 2 µl, IAC DNA 2 µl, NTM 10 µl) and the volume adjusted to 40 µl with the addition of nuclease free dH₂O. The internal control DNA was diluted to contain 500 genome equivalents per 2 µl and the NTM contained 10,000 genome equivalents per 10 µl.

The cycling parameters used were the same as those used in the LightCycler 2.0, however the temperature transition rate, referred to as the ramp rate on the LightCycler 480 were variable, for the initial incubation the ramp rate was 4.8 °C/s, during the 50 cycles at 95 °C, the ramp rate was 4.8 °C/s and at 60 °C 2.5 °C/s and the final cooling step had a ramp rate of 2.0 °C/s. Prior to experimental analysis on the LightCycler 480, a colour compensation file was generated using the technical note outlined in the Advanced Software Functionalities of the operator manual.

### Example 1 - Diagnostics target identification

A number of approaches were used to search for and identify sequences that would allow the differentiation of the members of the MTC. To identify targets suitable for the detection of *M. tuberculosis* and other species of the MTC and that are suitable for use in a multiplex *in vitro* nucleic acid amplification assay, approximately 3000 genes were evaluated in *in silico* analysis.

It was necessary to identify genomic regions that are deleted in certain species of the MTC but present in others. Potential target regions were identified using the Mycobacterial Genome Divergence Database (MGDD) (http://mirna.jnu.ac.in/mgdd/), which allowed for identification of insertions, deletions and single nucleotide polymorphisms between *M. tuberculosis, M. bovis* and *M. bovis BCG.* Potential target regions were also identified using the web based version of the Artemis comparison tool, WebACT (http://www.webact.org/WebACT/home). Sequence information was retrieved from the *M africanum* and *M. microti* genomes currently being sequenced by the Welcome Trust Sanger Institute (using the Basic Local Alignment Search Tool (BLAST) tool available on the Sanger website) to determine if the candidate regions identified for *M. tuberculosis* detection were specific, based on *in silico* analysis. Sequence information for the remaining members of the complex, namely, *M. canettii, M. caprae* and *M. pinnipedii,* is not available, so specificity of potential targets was determined empirically and further validated through sequencing.

Specific parameters were employed for the *in silico* analysis. Insertions or deletions were preferred as such genomic events are considered to be unidirectional events. Therefore, insertions and deletions are more likely to be unique to an individual species of the MTC. Single nucleotide polymorphisms (SNPs) were only accepted when insertions or deletions could not be identified. This allows for the potential geographical nucleotide sequence variation which is often observed. Nucleic acid diagnostics targets had to be specific for the species of the MTC to be identified. As described above, empirical sequencing analysis then had to be carried out for *M. canettii, M. caprae* and *M. pinnipedii.*

For each putative target identified, alignments were carried out using clustalW multiple sequence alignment programme (http://www.ebi.ac.uk/Tools/clustalw2/index.html) and PCR primers and probes were designed (Table 4).

Of the approximately 3000 genes analysed in this study, very few met the criteria imposed and were suitable for use in *multiplex in vitro* nucleic acid amplification assays capable of identifying species of the MTC. The vast majority of the sequences studied with the *in silico* and empirical sequencing analyses were not found to be unique to a species of the MTC. Of the few that were initially found to be unique, a number were later found to not be conserved between clinical isolates when PCR assays were developed and so the assays did not successfully detect all the isolates.

### Diagnostic target identification - wbbl1

For the specific detection of *M. tuberculosis* and *M. canettii,* the *wbbl1* gene (RV 3265c) was selected. Although present in all members of the MTC, it has surprisingly been identified that there is a 12 base pair region present in *M. tuberculosis* and *M. canettii* which has been deleted in other members of the MTC. Therefore, this gene allows for the specific detection of *M. tuberculosis* and *M. canettii.* The *wbbl1* gene encodes rhamnosyl transferase, which inserts rhamnose into the cell wall and is thought to be essential for Mycobacterial viability (Ma *et al.,* 2002). Nucleotide sequence analysis of members of the MTC surprisingly revealed a 12 base pair region of the *wbbl1* gene present only in *M. tuberculosis* and *M. canettii,* which has been deleted in all other members of the MTC.

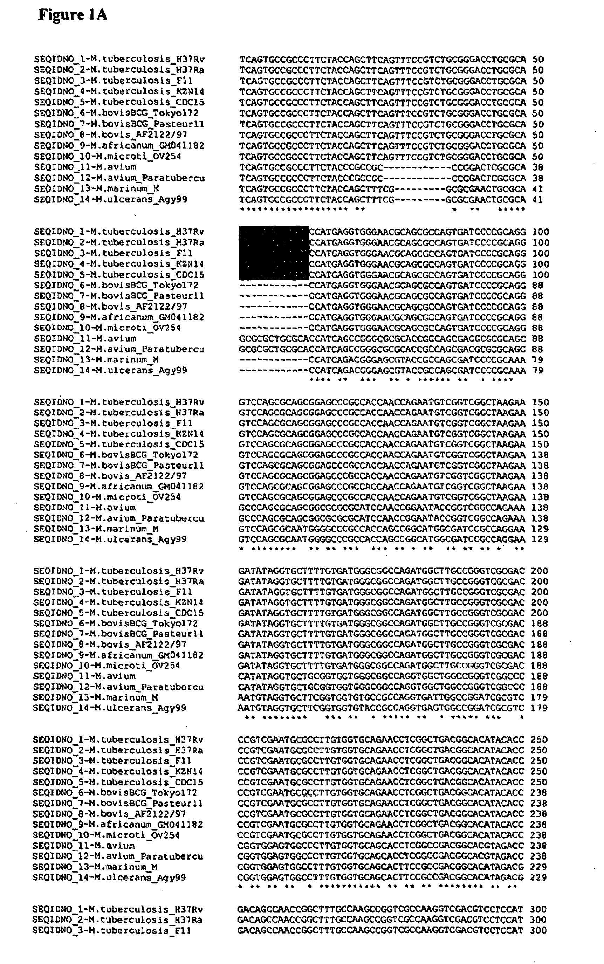

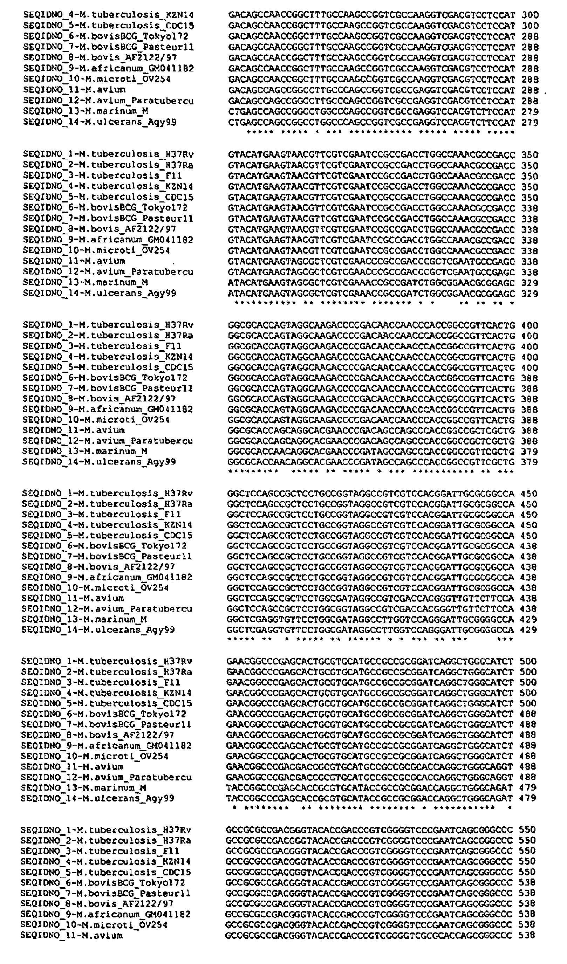

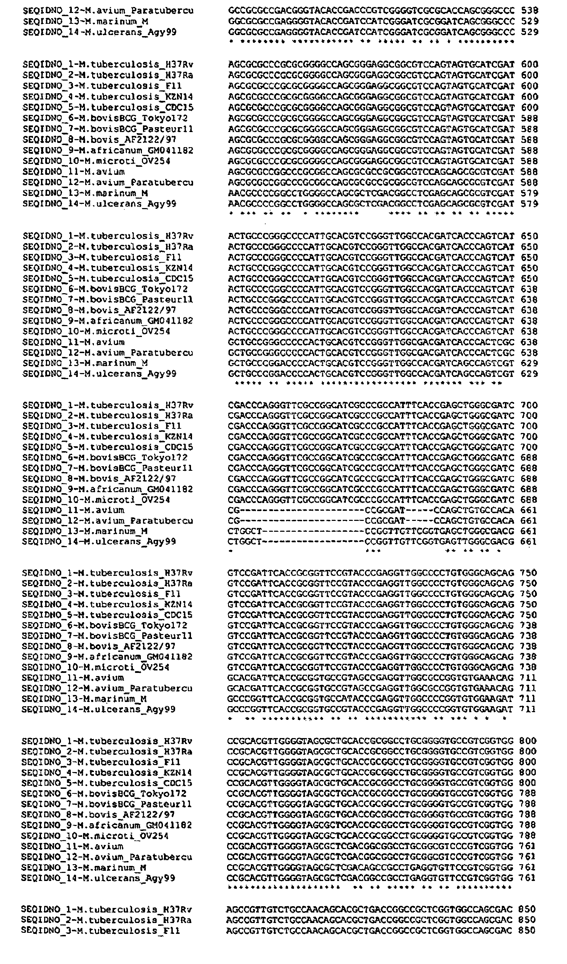

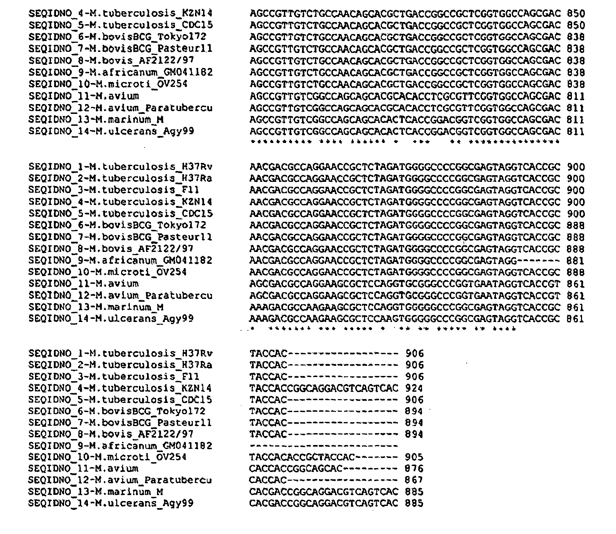

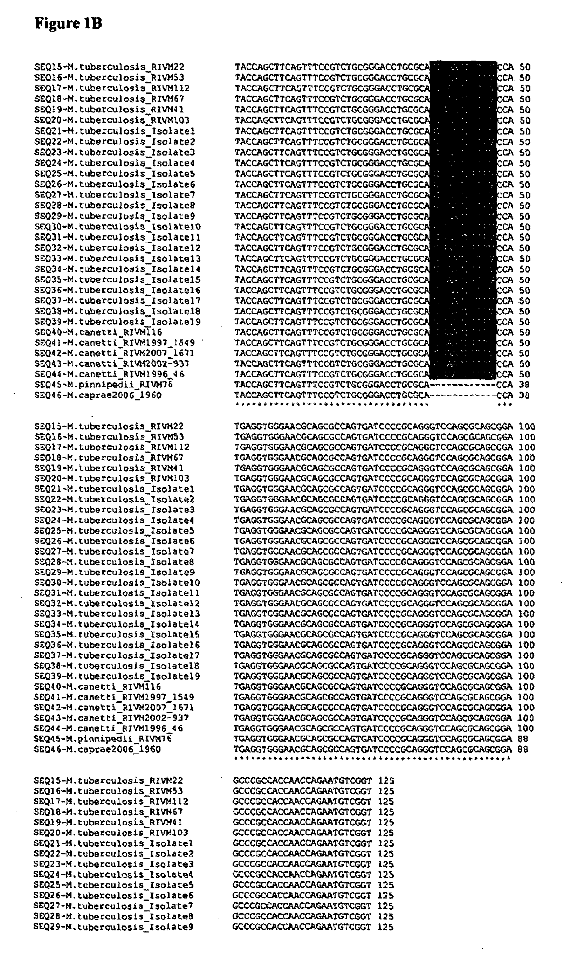

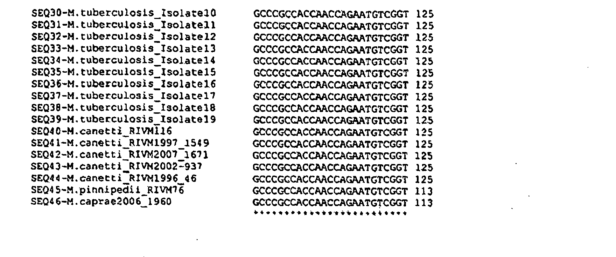

As demonstrated in Figure 1A, *in silico* analysis revealed a region deleted in all the species tested other than *M. tuberculosis.* Sequencing analysis, as detailed in Figure 1B, confirmed that this region is specific to *M. tuberculosis* and *M. canettii* and is deleted in *M. caprae* and *M. pinnipedii.*

### Diagnostic target identification - RD^{canetti1}

To identify *M. tuberculosis* and *M. canettii* specifically and to distinguish between them, a novel target specific for *M. canettii* was also required as the target *wbbl1* was empirically determined to detect both *M. tuberculosis* and *M canettii,.* When sequence information became publicly available on the Sanger website for *M. canettii,* a number of block sequence regions available were evaluated. These blocks of sequence information were BLAST (NCBI http://www.ncbi.nlm.nih.gov/) analysed to identify a potential specific target for *M. canettii.*

The *M. canettii* specific diagnostic target identified herein is a region of the genome that appears to be deleted in all other members of MTC. This 2869bp region was discovered while mapping RD12^{can} to the unfinished genome sequence of *M. canettii* available on the Sanger website. This region appears to be a novel RD specific for *M. canettii.* The *M. canettii* genome is not annotated to date; therefore the function of the gene/s in this region are unknown.

To be able to distinguish *M. canettii* from *M. tuberculosis* and the other members of the MTC, and therefore allow the specific diagnosis of *M. canettii* and *M.* tuberculosis, the selected region should preferably be shared by all *M. canettii* isolates. To identify whether this was true for RD^{canetti1}, the region was sequenced using the primers listed in Table 4 for the 5 *M. canettii* strains used in this study. Sequence analysis revealed 100% homology between the 5 strains and the sequence available on the Sanger website (compare SEQ ID NOs 78-83). As this sequence is present in the 5 M *canettii* strains and is 100 % homologous, we propose this to be a novel RD (RD^{canetti1}) that allows the discrimination of *M. canettii* from *M. tuberculosis* and the other members of the MTC and therefore allows the specific diagnosis of *M. canettii* and *M. tuberculosis.*

### Diagnostic target identification - MTC lepA

In order to identify a target for collective detection of the MTC, a number of housekeeping genes which are highly conserved throughout the *Mycobacterium* genus were evaluated

The diagnostic target gene used in this study for detection of the MTC along with detection of the internal amplification control was *lepA* (Rv2404c). *LepA* is an elongation factor required for accurate and efficient protein synthesis capable of inducing back-translocation of mistranslocated tRNA's. The *LepA* gene is present in all bacteria sequenced to date and codes for one of the most conserved proteins in bacteria (55-68 % amino acid sequence homology between bacteria), with a homologue (Guf1) found in higher organisms (Qin *et al.,* 2006).

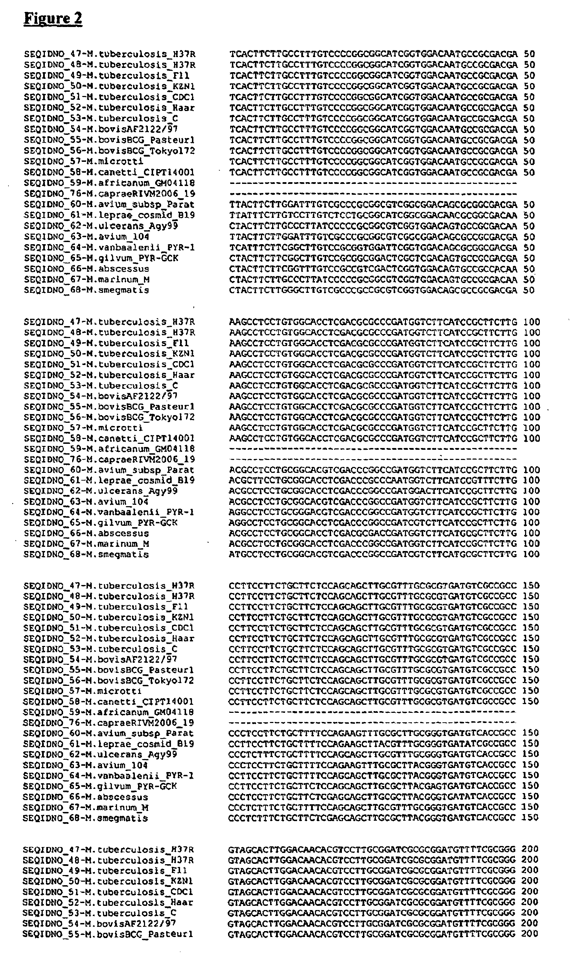

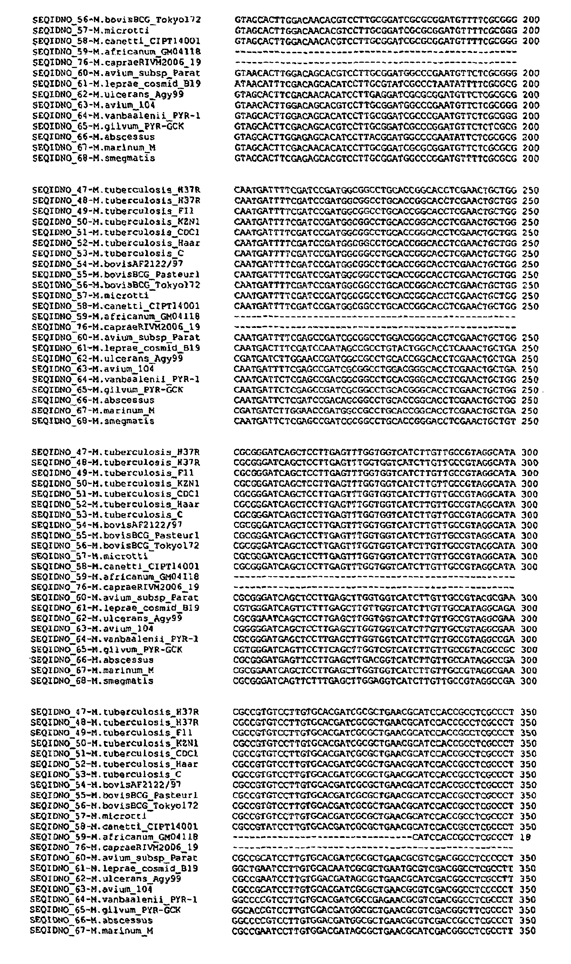

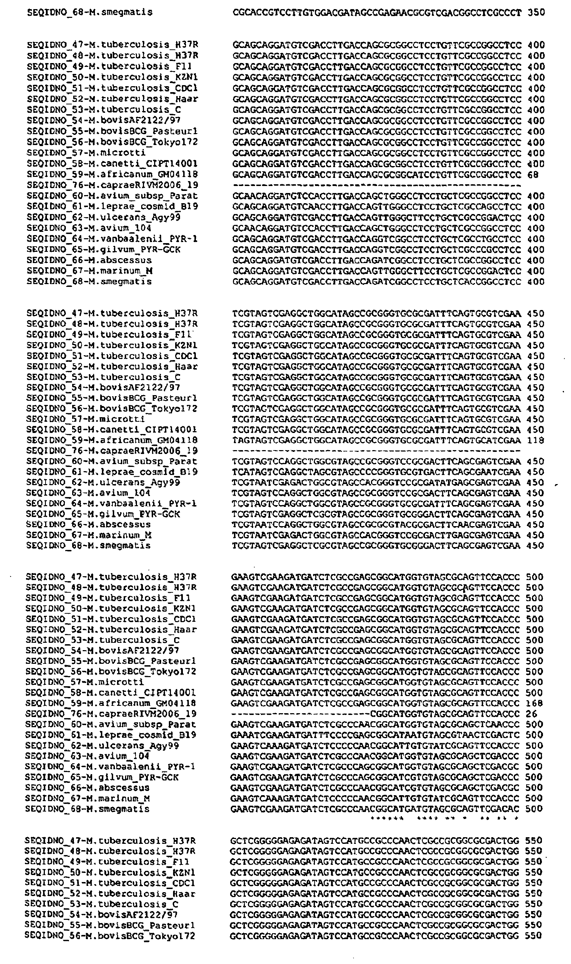

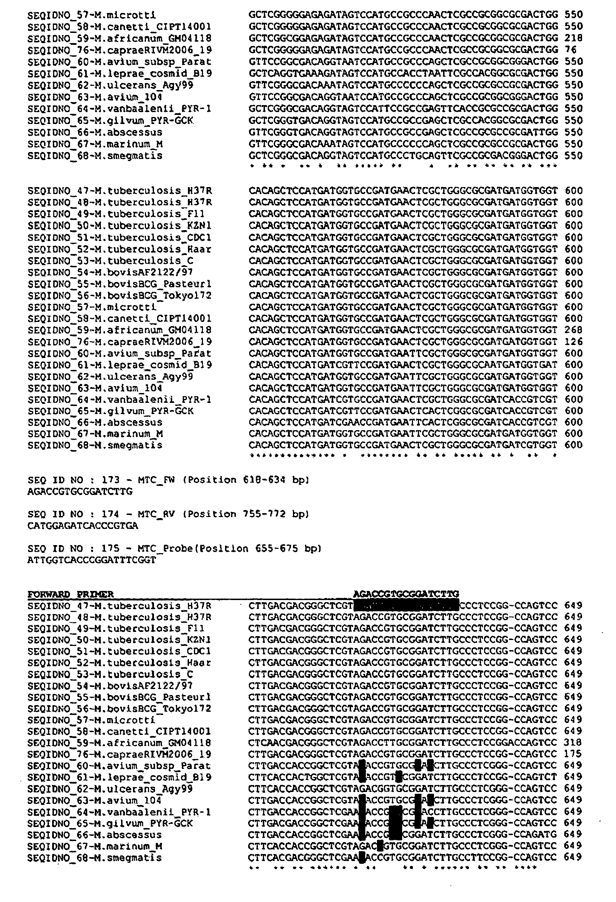

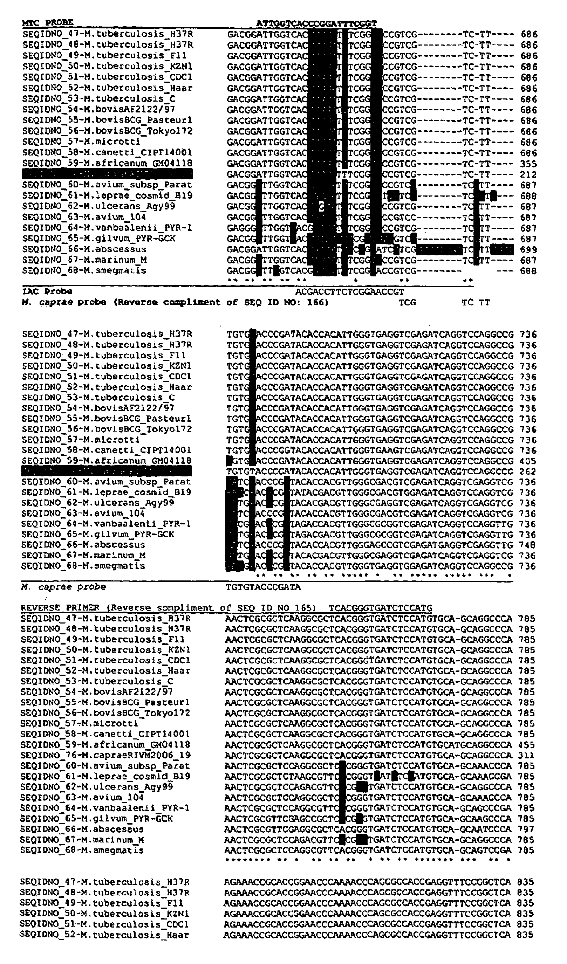

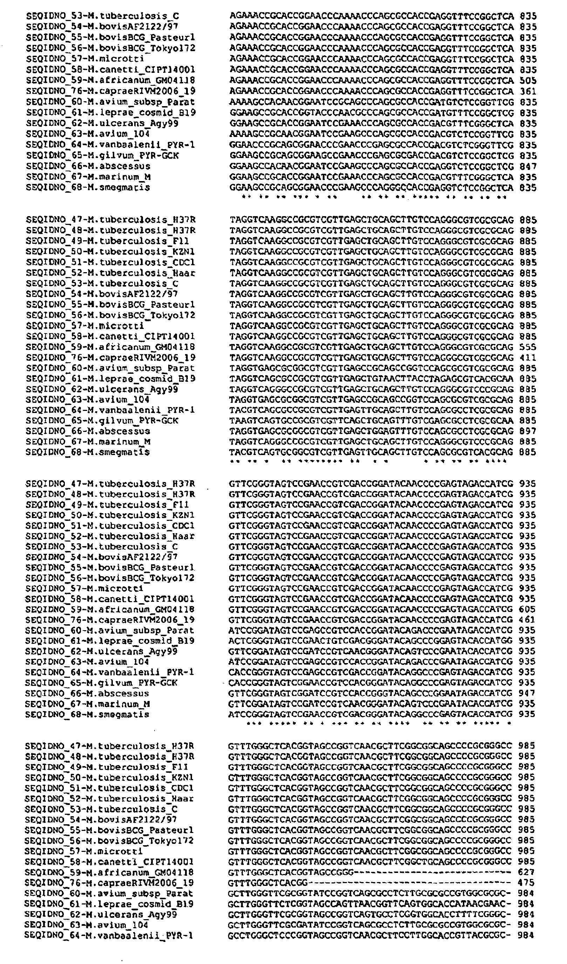

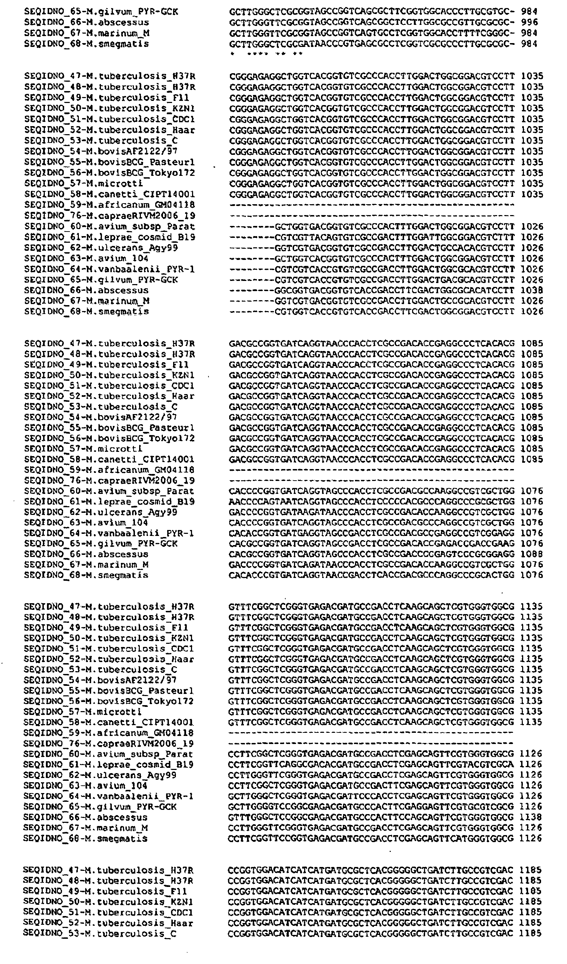

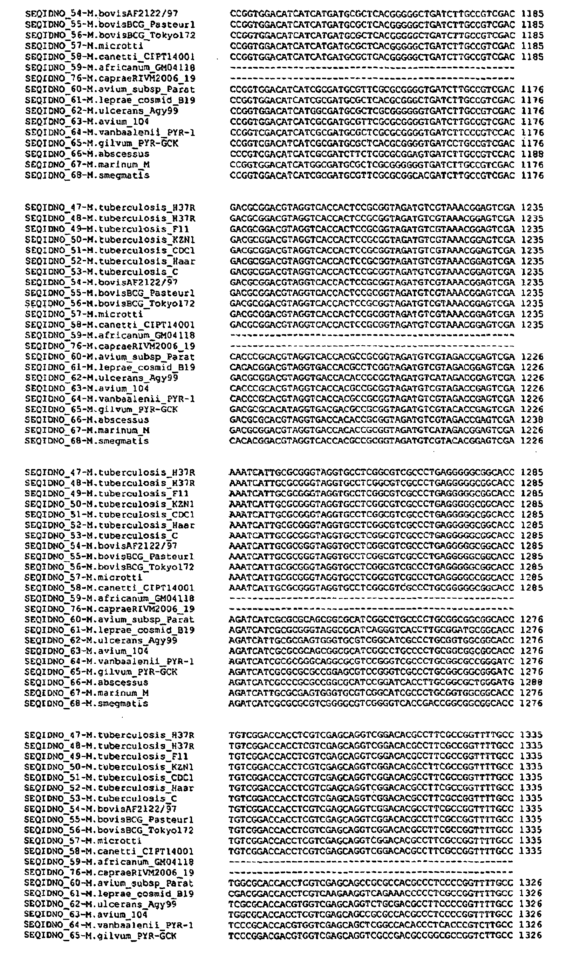

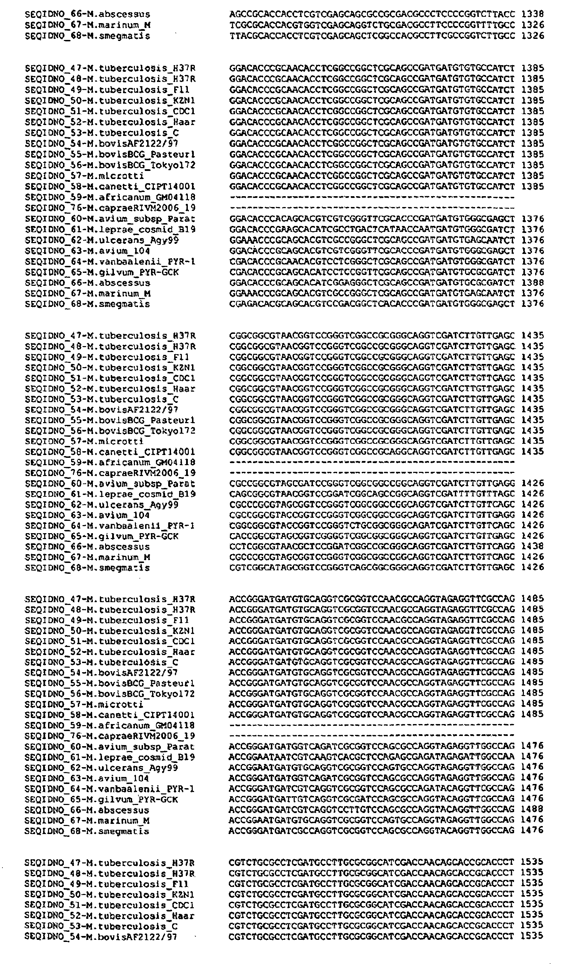

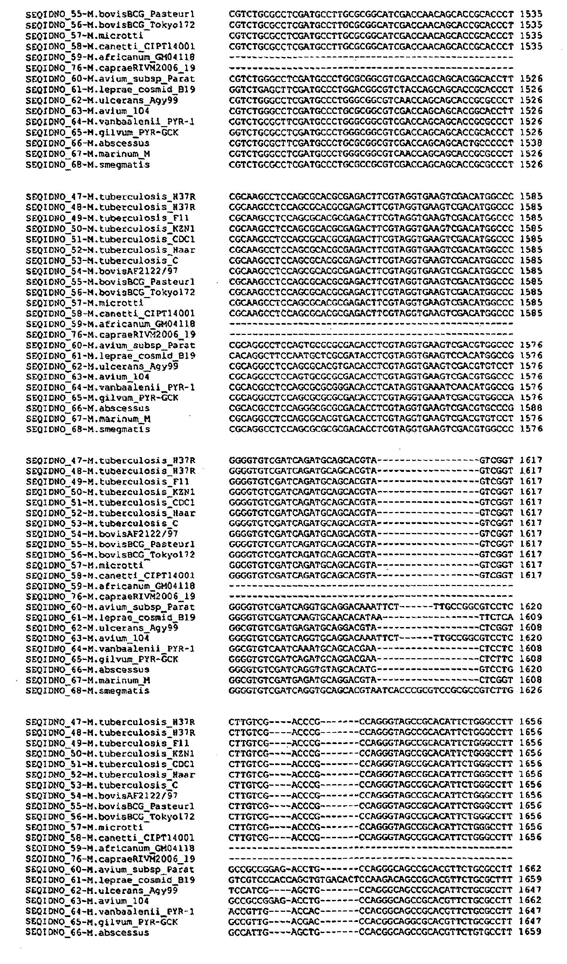

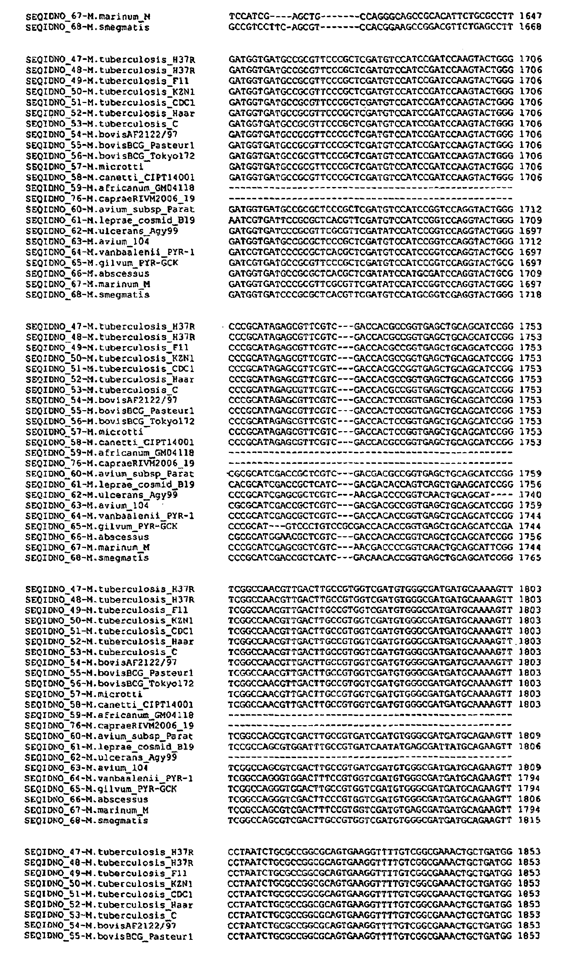

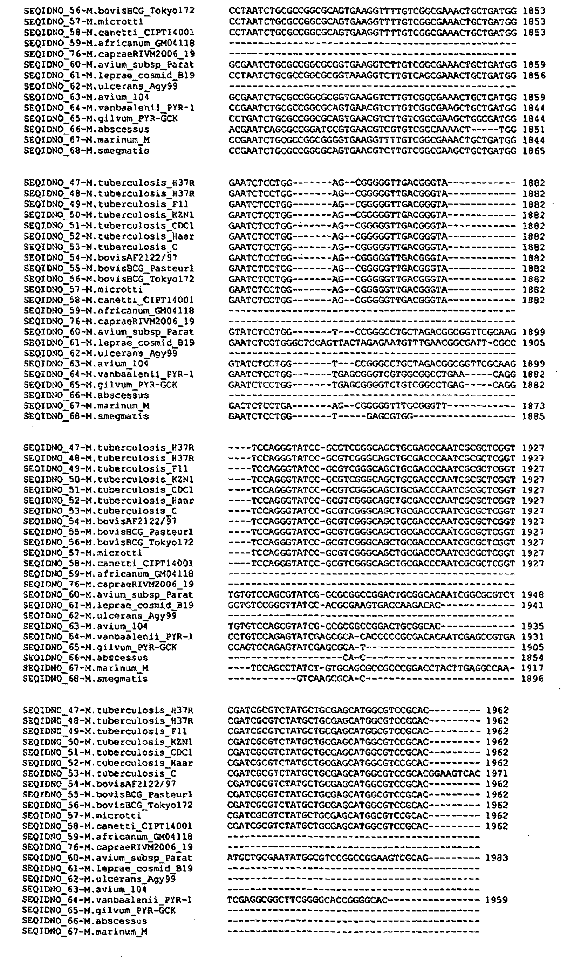

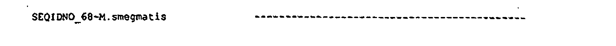

As shown in the alignments of Figure 2, analysis of the publicly available *lepA* sequences and the sequences generated by the inventors revealed that enough sequence heterogeneity exists between the species of the MTC and other species to be used as a internal amplification control (here *M. smegmatis*) for the design of independent, specific probes. There is also enough homology present, flanking these probe regions, to allow the design a single set of primers to amplify both the MTC and internal amplification control targets. This allows a reduction in the complexity of the multiplex PCR assay with a reduction in the number of primer pairs required.

The techniques and methods described in the Example above surprisingly demonstrate that sufficient genetic variation exists between the members of the MTC to identify specific species of the MTC in a multiplex *in vitro* nucleic acid amplification assay. The teaching provided herein above could be used to identify other similar sequences that also allow the discrimination of the different members of the MTC.

### Diagnostics target identification - RD713

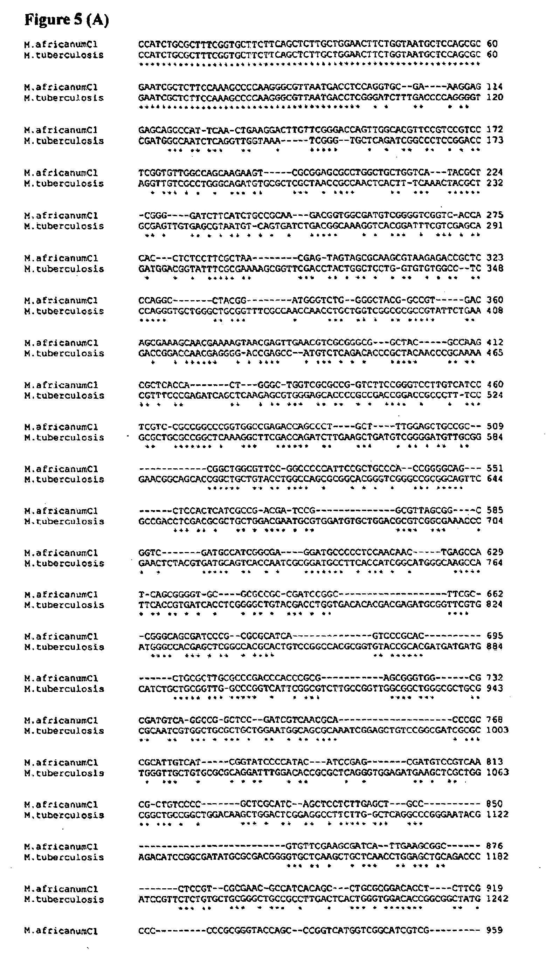

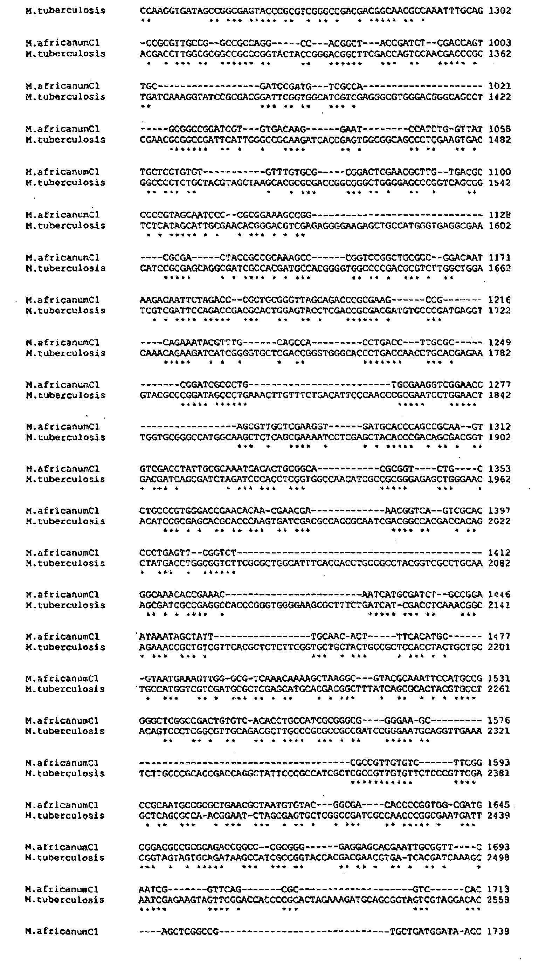

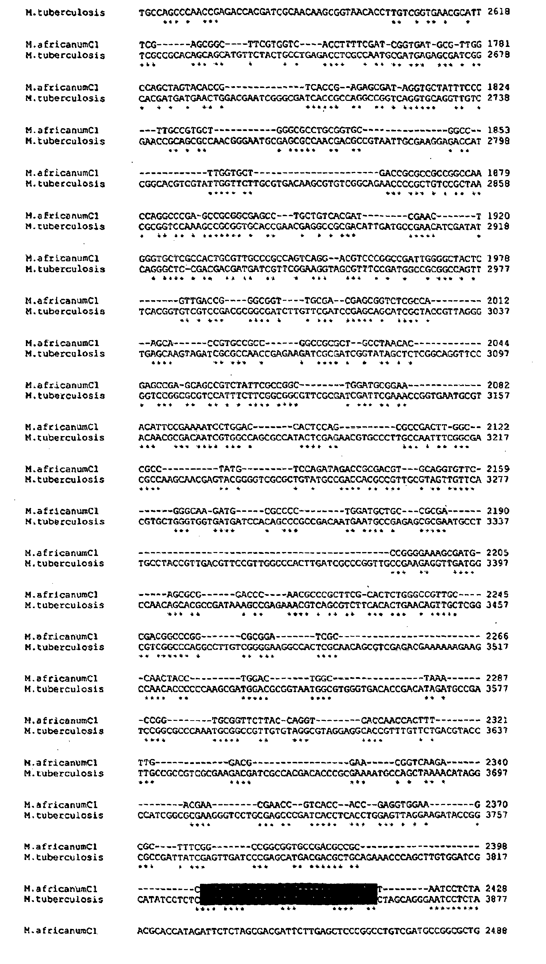

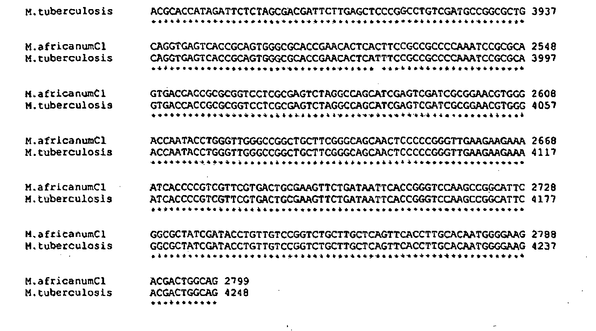

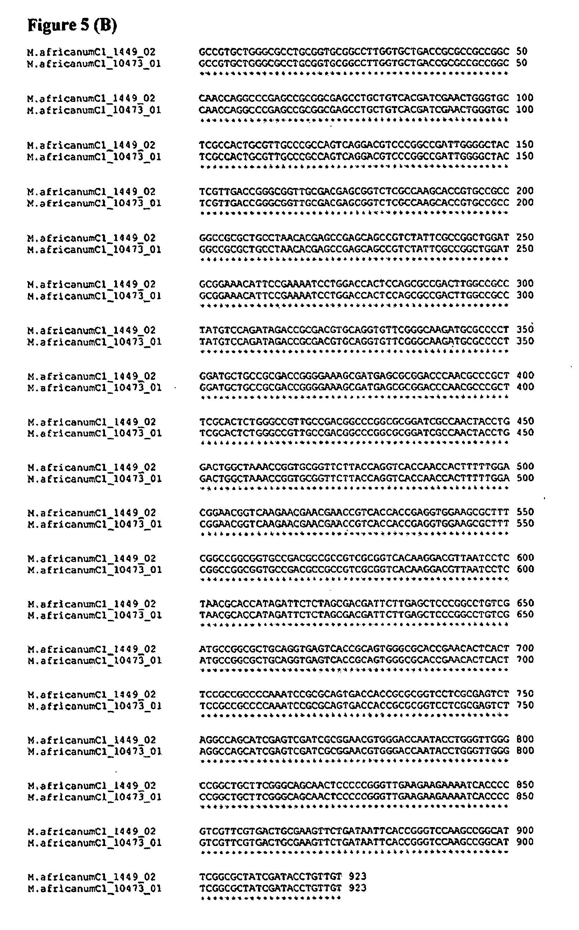

For identification of the newly defined *M. africanum* clade 1 isolates RD713 was selected. *In-silico* analysis, in addition to literature describing this RD, demonstrated its potential for the specific identification of *M. africanum* clade 1 isolates (de Jong *et al.,* 2010; Vasconcellos *et al.,* 2010). While another RD, RD711, has been described for specific detection of *M. africanum* clade 1, this RD is not present in all *M. africanum* clade 1 strains. RD713 relates to a 2799bp region of *M. africanum* clade 1. In other members of the MTC such as *M. tuberculosis* H37Rv, where this deletion is not present, this region relates to a 4248 bp region of nucleotide sequence. In other members of the MTC, owing to the complex region incorporated in this RD, no amplification will be observed for this region (Vasconcellos *et al.,* 2010). As shown in Figure 5 (A) RD713 contains a region unique to *M. africanum* clade 1, which is also conserved between *M. africanum* clade 1 isolates.

### Diagnostic target identification - M. caprae lepA

It was discovered that *M. caprae* contains an SNP which allows its differentiation from all other members of the MTC. As seen in Figure 2, there is a C to T substitution at position 691 of the *lepA* gene nucleotide sequence specific for *M. caprae.* The probe for identifying *M. caprae lepA* targets this SNP.

### Diagnostic target identification - lpqT

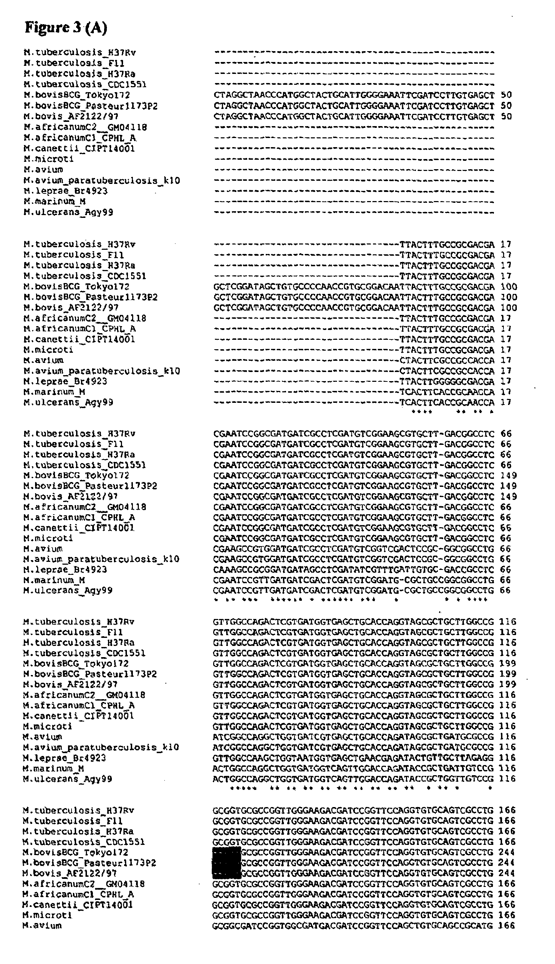

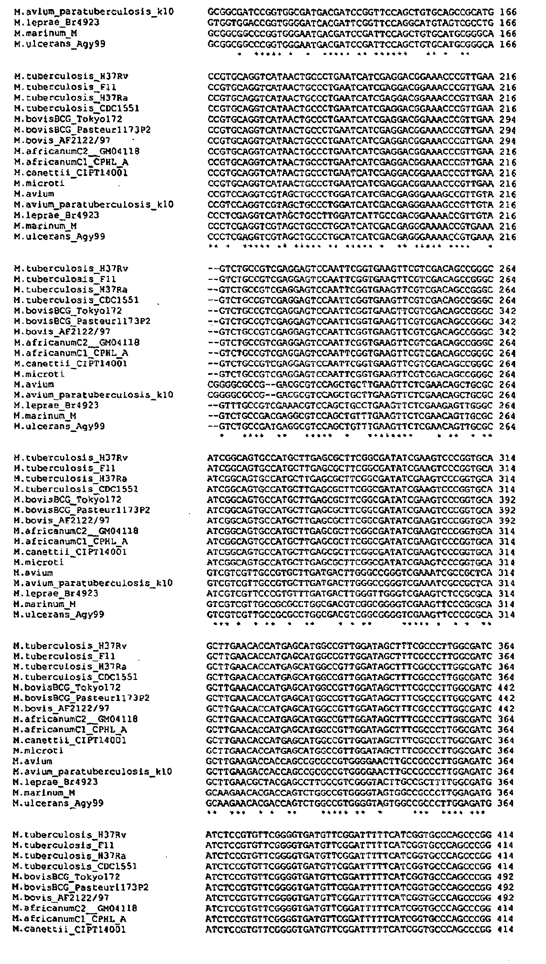

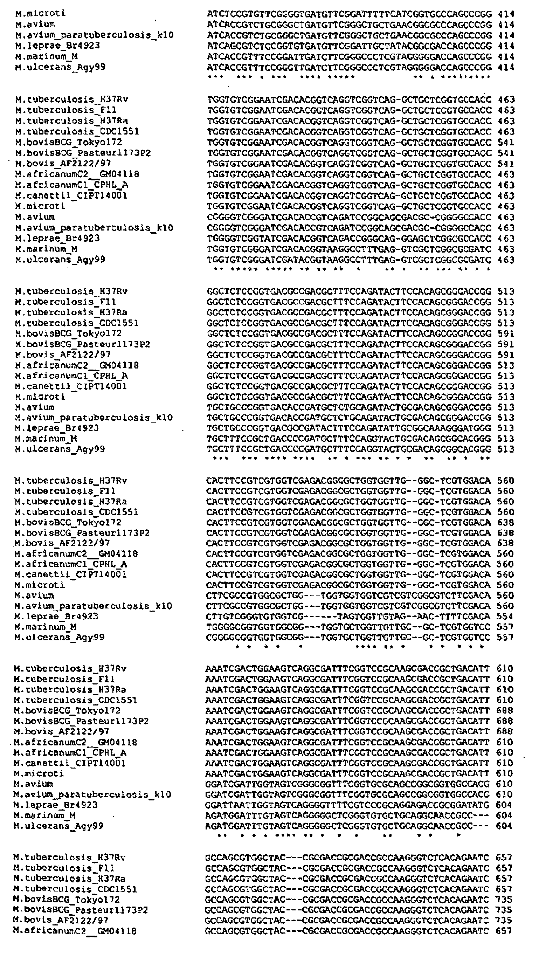

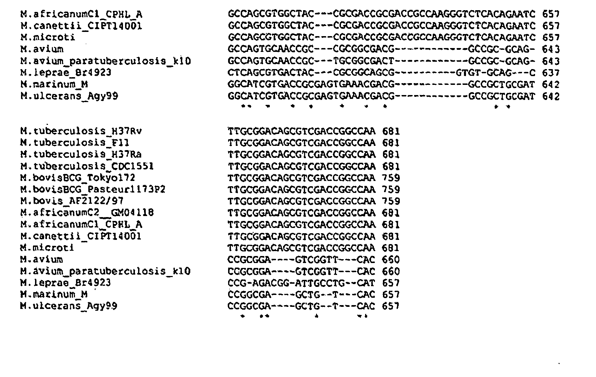

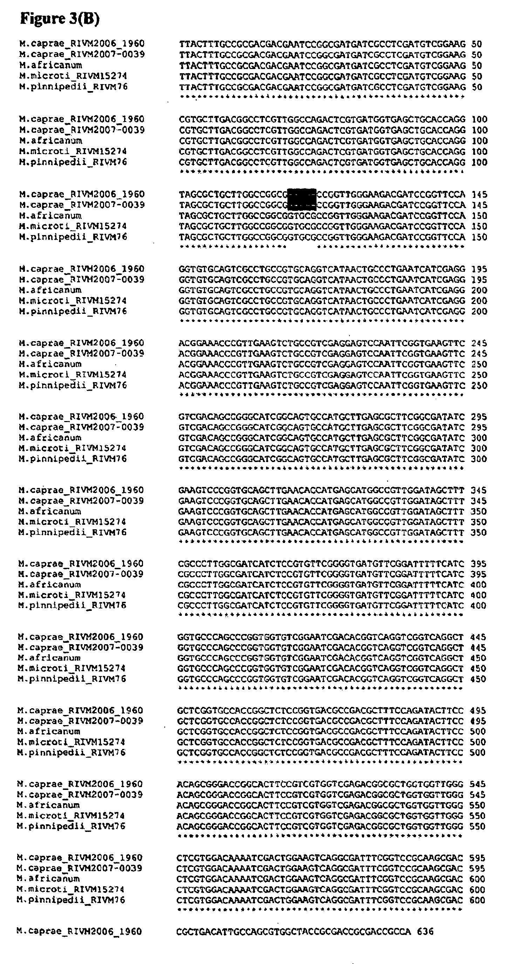

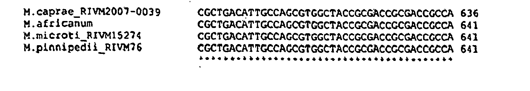

*lpqT* (Rv1016c) was selected to identify the presence of *M. bovis, M. bovis BCG* and *M. caprae.* While the *lpgT* gene is present in all members of the MTC, it was surprising to observe from *in silico* analysis, that a 5 bp deletion was present in *M. bovis* and *M. bovis* BCG. Sequence analysis of nucleotide sequence generated in this study revealed that this deletion was also present in the phylogenetically related *M. caprae. lpgT* belongs to a group of proteins known al lipoproteins (Garnier *et al.,* 2003) which are present in all bacteria (Rezwan *et al.,* 2007). Figure 3A shows that a region of *lpqT* is deleted in *M*. *bovis, M. bovis BCG and M. caprae.*

### Diagnostic target identification - RD1

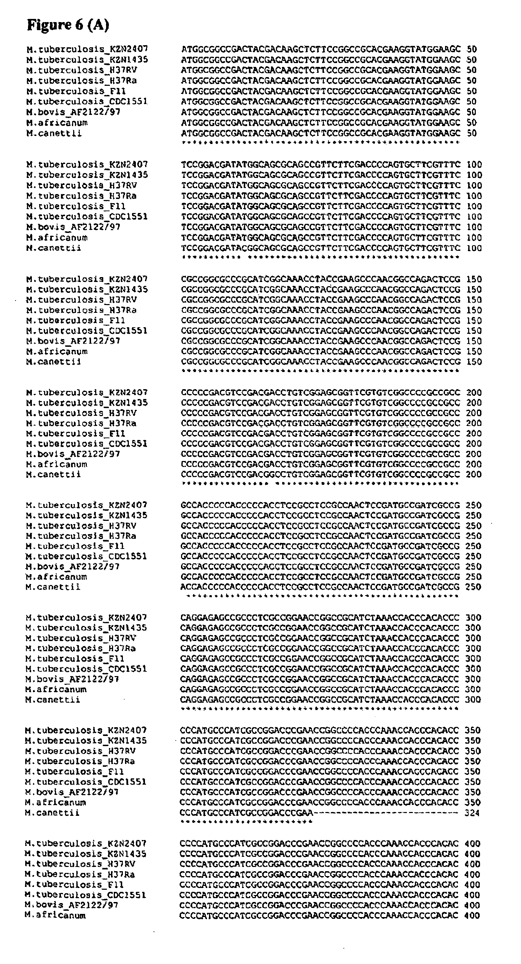

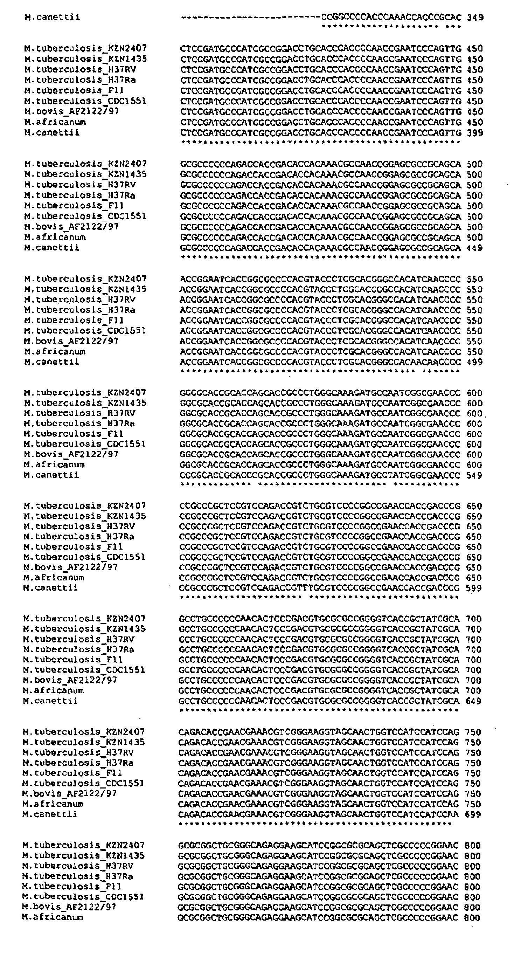

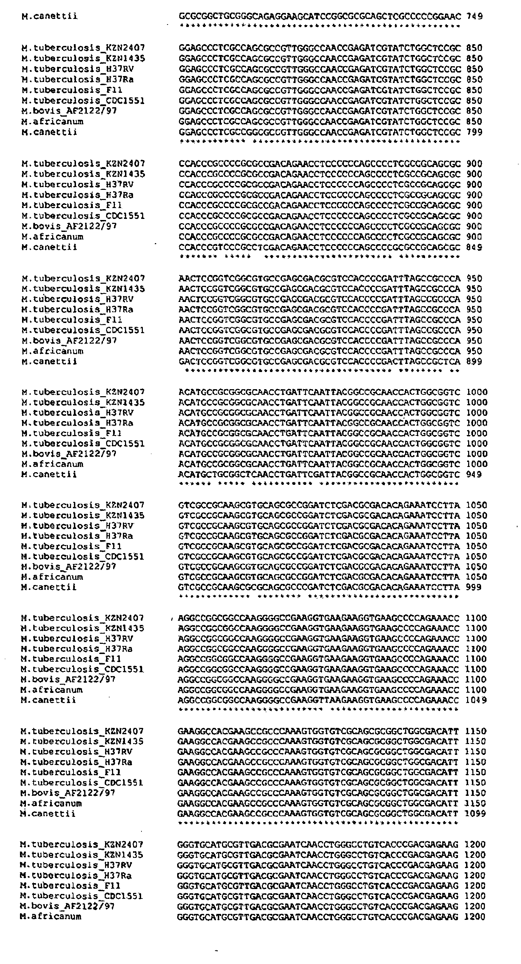

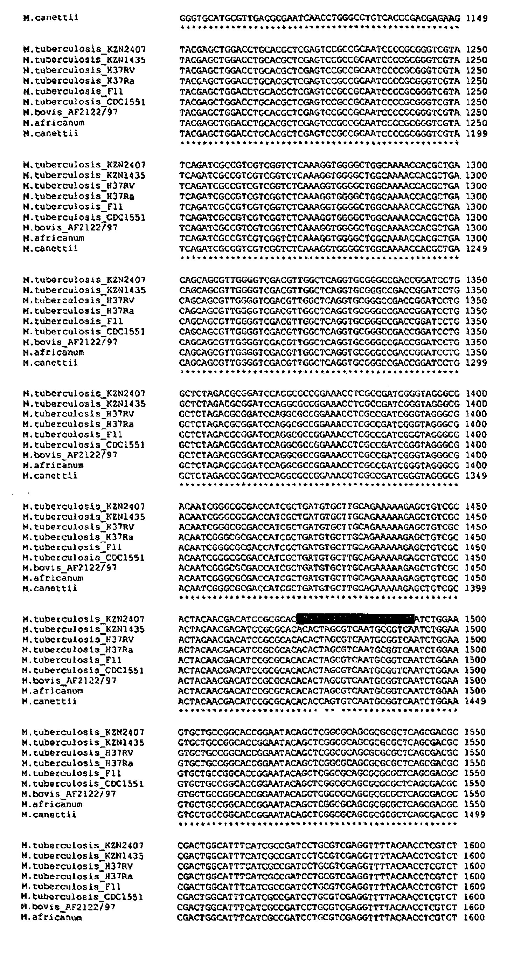

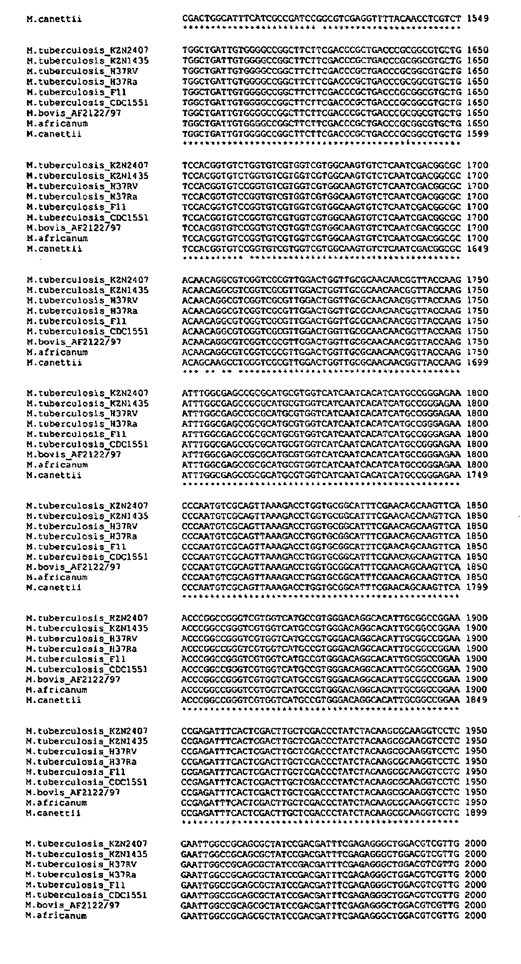

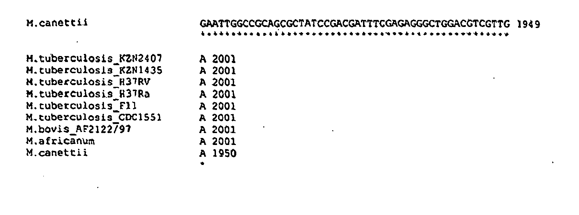

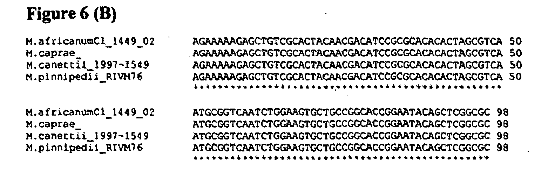

A region of RD1 was selected to identify the presence of *M. bovis BCG* in a sample. In all *M bovis* BCG strains RD1, which contains the genes Rv 3871-3879c, is deleted (Behr *et al.,* 1999). The loss of RD1 has been proposed to be associated with the attenuation of this strain (Pym *et al.,* 2002). Interestingly, regions of RD1 are also deleted in *M. microti,* such as the region targeted in this invention to differentiate *M. bovis* and *M. caprae* from *M. bovis* BCG. RD1 is present in all other members of the MTC, however sequence heterogeneity is observed from the publicly available *M. canetti* sequence as demonstrated in Figure 6 (A). Figure 6 (A) shows a region of RD1 which is present in *M caprae* and *M. bovis* but not in *M. bovis* BCG.

### Diagnostic target identification - RD701

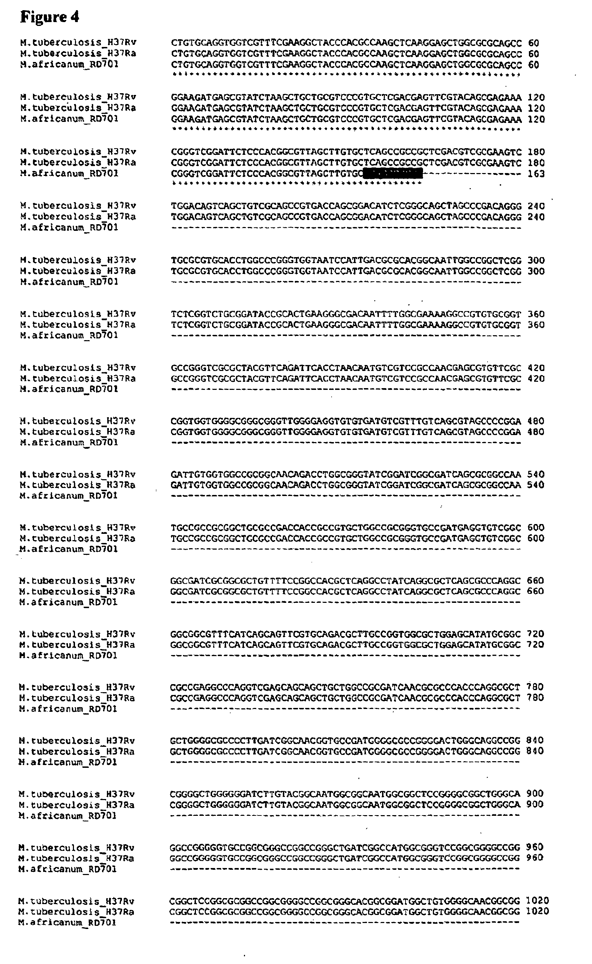

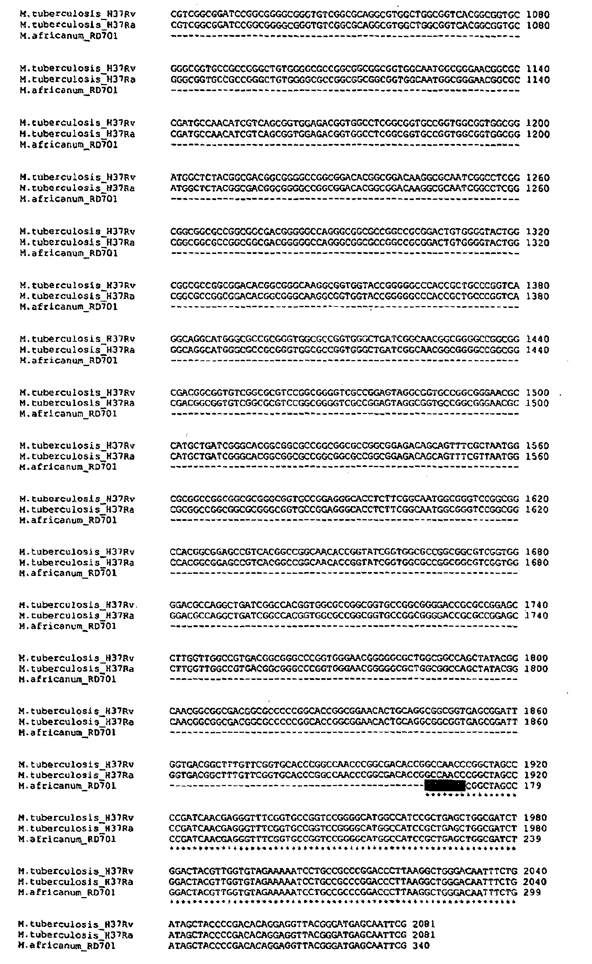

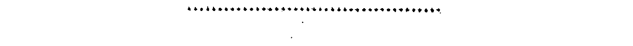

For the specific detection of *M. africanum* clade 2 isolates, two RDs could potentially have be used, namely RD701 and RD702, RD701 was selected to identify the presence of *M. africanum* clade 2. Where intact this is a 2081bp region of sequence. In *M. africanum* clade 2 where the deletion is present this is 320bp region. Where RD702 is intact a PCR product of 2101 bp is observed in members of the MTC, whereas this region is 732 bp in *M. africanum* clade 2 (Vasconcellos *et al.,* 2010). In this invention RD701 was chosen for the specific detection of *M. africanum* clade 2 isolates as a previous study has shown an *M. africanum* like organism which has RD702, RD711 and RD713 present (Vasconcellos *et al.,* 2010). In this instance the use of RD702 may in fact misclassify the *M. africanum* clade present. Figure 4 highlights a region of RD701 which is uniquely deleted from *M. africanum* clade 2.

### Example 2 - Assay design and development for multiplex 1

Nucleotide sequences generated in-house and publicly available sequences from GenBank were aligned for primer and probe design for real-time PCR assays.

TaqMan probes were designed to be specific for each assay following design guidelines.

### MTC assay - lepA

For the MTC assay, PCR primers MTC_Fw (SEQ ID NOs: 164) and MTC_Rv (SEQ ID NO: 165), were designed to amplify a 155 bp fragment of the *lepA* gene for all members of the MTC. The MTC_Fw primer was located at positions 618-634 bp and the MTC_Rv primer located at positions 755-772 bp of the *M*. *tuberculosis* H37RV *lepA* gene.

### M. tuberculosis, M. canetlii and M. africanum clade 1 assay - wbbll

For the *M. tuberculosis, M. canettii* and *M. africanum* clade 1 assay, *wbbl1*_Fw (SEQ ID NO: 97) and *wbbl1*_Rv (SEQ ID NO: 99) were designed to amplify a 146 bp fragment of the *wbbl1* gene. The *wbbl1*_Fw primer was located at positions 16-34 bp and the *wbbl1*_Rv primer located at positions 141-161 bp of the *M tuberculosis* H37RV *wbbl1* gene.

### M. canettii assay - RD^{canettii1}

The *M. canettii* specific assay was designed to amplify a 128 bp fragment of a 2869 bp region of the genome identified in this study as specific to *M. canettii.* This 2869 bp region of the genome has been mapped to the *M. tuberculosis* H37Rv genome and is inserted between Rv0150c (hypothetical protein) and Rv0151c (gene name *PE1*, a PE family protein) at position 177,445 bp on the *M. tuberculosis* H37Rv genome.

### M. africanum clade 1 assay - RD713

For the *M. africanum* clade 1 specific assay, PCR primers RD713_Fw (SEQ ID NO: 167) and RD713_Rv (SEQ ID NO: 168), were designed to amplify a 138bp fragment of RD713 (2800bp in *M. africanum* clade 1 and 4248bp in *M. tuberculosis*_H37Rv). Based on the publicly available *M africanum* clade 1 RD713 nucleotide sequence the RD713_Fw primer is located at positions 2326-2342bp and the RD713_RV primer is located at positions 2446-2463 bp.

All real-time PCR assays were initially tested in a monoplex format, to evaluate their specificity, using probes labelled with FAM and Black Hole Quencher 1 (BHQ1). All primers used in this study were designed to have a melting temperature (Tm) of between 58-61°C, with all probes designed to have a Tm of 4-7 degrees higher. These parameters were adhered to during the design of monoplex assays so that the assays could be easily multiplexed after specificity and sensitivity testing was complete. After the monoplex real-time PCR assays were optimised, four of the five assay probes were labelled with different fluorescent dyes to allow for multiplex real-time PCR. The MTC probe was labelled with HEX and BHQ1, the *M. canettii* specific probe with ROX and BHQ2, the *M. africanum* clade 1 with Cyan 500 and BBQ and the IAC probe with Cy5 and BHQ2. While the guidelines for primer and probe design were adhered to as closely as possible, the high GC content (60-65%) of the Mycobacterium species did have an impact on assay design. The *wbbl1* specific probe was based on a region present in *M. tuberculosis, M. canettii* and *M. africanum* clade 1 which is deleted in other MTC, that is very G/C rich making probe design difficult. This probe was labelled with FAM and double the standard probe concentration (0.4µM/reaction) was used to improve the endpoint fluorescence, sensitivity and robustness of the assay. For optimal performance of the multiplex the half the standard probe concentration (0.1µM/reaction) was required for the HEX labelled MTC assay.

### Example 3 - Assay design and development for multiplex 2

Nucleotide sequences generated in-house and publicly available sequences from GenBank were aligned for primer and probe design for real-time PCR assays.

TaqMan probes were designed to be specific for each assay following design guidelines.

### M. caprae, M. bovis & M. bovis BCG assay - lpqT

lpqT_FW (SEQ ID NO: 158) and IpqT_RV (SEQ ID NO: 159) were designed to amplify a 141 bp fragment of the *lpqT* gene for the identification of *M. bovis, M. bovis* BCG and *M. caprae* (positions 100-117 bp and 224-240 bp of the *M. bovis* AF2122/97 *lpgT* gene).

### M. caprae assay - lepA

The *M. caprae* specific assay PCR primers, MTC_Fw (SEQ ID NO: 164; position 618-634 bp of the *lepA* gene of *M. tuberculosis* H37Rv) and MTC_Rv (SEQ ID NO: 165; position 754-772 bp), were designed to amplify a 155 bp fragment of the *lepA* gene.

### M. bovis BCG assay - RD1

The RD1 assay was designed to amplify a 117 bp region of the Rv3876 gene, a conserved hypothetical protein, part of RD1, absent in all *M. bovis* BCG strains. The RD1_Fw primer (SEQ ID NO: 161) was located at position 1416-1433 bp and the reverse primer RD1_Rv (SEQ ID NO: 162) between 1516-1532 bp of the *M. tuberculosis* H37 Rv 3876 gene.

### M. africanum clade 2 assay - RD701

The *M. africanum* clade 2 specific assay was designed to amplify a 81 bp region of the publicly available RD701 nucleotide sequence (320 bp region in *M. africanum* clade 2, 2081bp region in *M. tuberculosis*_H37Rv). Based on the publicly available *M. africanum* clade 2 RD701 nucleotide sequence the RD701_Fw primer (SEQ ID NO: 170) is located at positions 119-135bp and the RD701_RV (SEQ ID NO: 172) primer is located at positions 182-199 bp.

While the guidelines for primer and probe design were adhered to as closely as possible, the high G/C content *Mycobacterium* species had an impact on assay design. The *lpqT* specific probe was designed spanning the deletion junction of a region deleted in *M. bovis. M. bovis* BCG and *M. caprae* and present in the other members of the MTC, that was very G/C rich, making probe design difficult. The *lpqT* probe, therefore, had a relatively high Tm, but this did not impact on assay performance. The *M. caprae* specific probe targeted an SNP in the *lepA* gene. Avoiding cross reaction of the *M. caprae* probe with other members of the MTC proved challenging. A number of probes were designed and tested and the optimum probe was chosen empirically based on specificity and sensitivity results. The optimum probe was designed complementary to the + strand of the *lepA* gene as the resulting G/A mismatch, that occurred in the presence of non-target MTC DNA, was more destabilising to the probe than the C/T mismatch, hence improving specificity. The probe was designed to have a Tm of 60.1°C, only slightly above the annealing temperature of the assay (60°C), allowing the probe to hybridise to exactly matched sequence only, therefore maximising the specificity effect of the SNP. This did, however, slightly reduce probe binding efficiency, leading to a small reduction in sensitivity.

### Example 3 - Internal control (IAC)

An internal control was designed and incorporated in both of the multiplex assays. It was designed to monitor for PCR inhibition and PCR efficiency. The *lepA* gene was chosen as the target for the IAC because enough sequence heterogeneity exists between the *M smegmatis* and MTC *lepA* gene sequences for the design of independent, specific probes. There was also enough homology present, flanking these probe regions, to design one set of primers to amplify both MTC and IAC targets. This resulted in less primer pairs being required in the multiplex PCR reducing assay complexity.

For the IAC assay, PCR primers, IAC_Fw (SEQ ID NO: 155) and IAC_Rv (SEQ ID NO: 156), were designed to amplify a 157 bp region of the *M. smegmatis* MSMEG_0660 gene. The IAC_Fw primer was located at positions 497-513 bp and the reverse primer between positions 636-653 bp of the publicly available *M*. *smegmatis_*MC2_155 MSMEG_0660 gene.

Titrations of MTC and IAC DNA were performed to determine the optimum concentration of IAC target per reaction such that it is always detected without impacting on detection of the primary MTC target. An internal control concentration of 500 genome equivalents per reaction allowed for the detection of the IAC at low concentrations or the absence of primary target.

In order for a result to be considered valid using this assay, a positive signal must be obtained in at least one of the four detection channels on the LightCycler 480. If none of the assay targets or the internal control are detected, the result is considered invalid and must be repeated (Hoorfar *et al.,* 2004; O' Grady *et al.,* 2008). *M*. *smegmatis* could also be used as a process control to monitor for DNA extraction efficiency from biological samples.

### Example 4 - Sensitivity and specificity of the assays

The specificity of each real-time PCR assay was confirmed both in monoplex and multiplex formats using the specificity panel listed in Tables 2 and 3. Using multiplex 1, the 119 MTC strains were all detected in the MTC assay, a representation of this can be seen in Figure 7 (C) and 44 NTM and 17 other bacterial species were not detected. The *wbbl1* assay was specific for the detection of the 60 *M. tuberculosis* the 8 *M. canettii* and 5 *M*. *africanum* clade 1 strains. A representation of this can be seen in Figure 7 (B), with 8 *M*. *tuberculosis* strains (triangle) 3 *M. canettii* strains (rectangle) and 4 *M. africanum* clade 1 (circle). The remaining members of the MTC, the NTM and closely related species were not detected. The *M. canettii* assay was specific for the *M. canettii* isolates and did not cross-react with the specificity panel. A representation of this can be seen in Figure 7 (D) with 3 *M. canettii* represented with rectangles. The *M. africanum* clade 1 specific assay targeting a region of RD 713 was specific for the detection of *M. africanum* clade 1 isolates. A representation of this can be seen in Figure 7 (A) with 4 *M. africanum* isolates represented with circles. The specificity of the IAC assay was tested using the full specificity panel and was specific for *M. smegmatis* DNA. As the IAC assay is designed using a non-competitive approach, when spiked into the master mix a positive signal should always be observed in the Cy5 channel. A representation of this can be seen in Figure 7 (E) with the no template control highlighted with stars.

Using multiplex 2, the 5 *M. caprae* isolates were detected using the *M. caprae lepA* assay. The remaining members of the MTC, NTM and other bacteria tested for were not detected, a representation of this can bee seen in Figure 8 (A). The *lpqT* assay was specific for the detection of 14 *M. bovis,* 7 *M, bovis* BCG and 5 *M. caprae.* A representation of this can be seen in Figure 8 (B) with 3 *M. bovis* (triangle) and 3 *M caprae* (circles) highlighted. The remaining members of the MTC, the NTM and other bacteria were not detected. For the purpose of multiplex 2, the RD1 assay detected the 14 *M. bovis* and 5 *M. caprae* isolates but not the 7 *M. bovis* BCG. A representation pf this can be seen in Figure 8 (C) with 3 *M*. *caprae* depicted with circles and 2 *M. bovis* depicted with triangles. Additionally the 5 *M. pinnipedii* strains tested for are detected by the RD1 based assay, whereas the 5 *M*. *microti* strains were not. A representation of this is seen in Figure 8 (C) with 1 *M. pinnipedii* (star) and 1 *M. microti* (diamond). The IAC was the same as that used in multiplex 1, a representation can be seen in Figure 8 (E) with the no template control again highlighted with stars.

The limit of detection (LOD) of each assay was evaluated in a monoplex real-time PCR format. Genomic DNA was quantified and serial dilutions were prepared from 200,000 to 2 genome equivalents of *M. canettii, M. africanum* clade 1, *M. caprae* and *M. africanum* clade 2, equating to approximately 5 fg DNA per cell. These members of the MTC were required to evaluate the sensitivity of all assays described.

In a monoplex format, the dilution series was run in duplicate and a sensitivity of 2- 20 genome equivalents was determined for each assay. In multiplex format, multiple sensitivity experiments were performed to optimise primer, probe and IAC concentrations. After optimisation of the multiplex, the lower limit of detection was established using probit regression analysis. In multiplex 1, 12 replicates of each of 20, 15, 12, 10, 7.5,4, 2, 0.2 genome equivalents of *M. canettii* and *M*. *africanum* clade 1 were evaluated. For ease of use and to avoid the possibility of cross talk between channels, a manual bandwidth was set at 1.2 fluorescent units for the primary assays. LOD's of 5.89, 9.04, 0.4 and 5.09 genome equivalents for the *M. canetti*/*M. tuberculosis*/*M. africanum* clade 1, MTC and *M. canettii* specific and *M. africanum* clade 1 assays respectively were determined with 95 % probability. The IAC at a concentration of 100 genome equivalents per reaction was detected in all samples tested.

In multiplex 2, 12 replicates of each of 20, 15, 12, 10, 7.5, 4, 2, 0.2 genome equivalents of *M. caprae* and *M. africanum* clade 2 were evaluated. For ease of use and to avoid the possibility of cross talk between channels, a manual bandwidth was set at 1.3 fluorescent units for the primary assays. LOD's of 5.66, 6.05, 24.9 genome equivalents for the *M. bovis*/*M. bovis* BCG/*M. caprae ,* the *M* bovis/*M. caprae* and *M. africanum* clade 2 assays respectively were determined with 95 % probability. The IAC at a concentration of 100 genome equivalents per reaction was detected in all samples tested. For the *M. caprae* specific assay the dilution series above was not sufficient for analysis, a further dilution series of 200, 100, 80, 60, 50, 40, 20 and 10 genome equivalents of *M. caprae* were evaluated. An LOD of 98.28 genome equivalents was determined with 95 % probability.

### Example 5 - Diagnostics algorithm

For determination of the identification of each specific member of the MTC using the two multiplex real-time PCR diagnostics assays outlined the user must take into account the combination of results observed for each channel of the real-time *in vitro* amplification instrument. This are set out in Table 1 below and explained below.

**Table 1- Result of multiplex PCRs associated with each diagnosis**

| **Test** | **Multiplex 1** | | | | | |
|---|---|---|---|---|---|---|
| Analysis Channel (Target) | Cyan 500 **(RD713)** | FAM (*wbbl1*) | HEX (MTC *lepA*) | ROX (RD^{canettii1}) | Cy5 (IAC MSMEG_0660) | **Result Interpretation** |
| | X | ● | ● | X | ● | *M. tuberculosis* |
| | X | ● | ● | ● | ● | *M. canettii* |
| | ● | ● | ● | X | ● | *M. africanum* clade 1 |
| | X | X | ● | X | ● | MTC - perform second multiplex |
| | X | X | X | X | ● | Not member of MTC |
| | X | X | X | X | X | Result invalid, test must be repeated |

| **Test** | **Multiplex 2 (taking multiplex 1 result into account)** | | | | | |
|---|---|---|---|---|---|---|
| Analysis Channel (Target) | Cyan 500 (*M. caprae lepA*) | FAM (*lpqT*) | HEX HEW (RD1) | ROX (RD701) | Cy5 (IAC MSMEG 0660) | **Result Interpretation** |
| | X | ● | ● | X | ● | *M. bovis* |
| | X | ● | X | X | ● | *M. bovis BCG* |
| | ● | ● | ● | X | ● | *M. caprae* |
| | X | X | ● | ● | ● | *M. africanum* clade 2 |
| | X | X | ● | X | ● | *M. pinnipedii* |
| | X | X | X | X | ● | *M. microli* |
| | X | X | X | X | X | Result invalid, test must be repeated |

| | | | | | | |
|---|---|---|---|---|---|---|
| • Positive signal obtained in this channel X Negative result in this channel | | | | | | |

### Multiplex 1

### Result scenario for the identification of M. tuberculosis

Using multiplex 1, if the HEX labelled MTC *lepA,* the FAM labelled *wbbl1* and the Cy5 labelled IAC MSMEG_0660 diagnostics assays generate a positive signal in each of these channels, but the Cyan 500 labelled RD713 and the ROX labelled RD^{canettiil} diagnostics assays do not generate positive signals in these channels the result indicates *M. tuberculosis* is present in the sample.

### Result scenario for the identification of M. canettii

Using multiplex 1, if the HEX labelled MTC *lepA,* the FAM labelled *wbbl1,* the ROX labelled RD^{canettiil} and the Cy5 labelled IAC MSMEG_0660 diagnostics assays generate a positive signal in each of these channels, but the Cyan 500 labelled RD713 diagnostics assay does not generate a positive signal in this channel, the result indicates *M. canettii* is present in the sample.

### Result scenario for the identification of M. africanum clade 1

Using multiplex 1, if the HEX labelled MTC *lepA,* the FAM labelled *wbbl1,* the Cyan 500 labelled RD713 and the Cy5 labelled IAC MSMEG_0660 diagnostics assay generate a positive signal in each of these channels, but the ROX labelled RD^{canettiil} diagnostics assay does not generate a positive signal in this channel, the result indicates that *M. africanum* clade 1 is present in the samples.

### Result scenario for other members of the MTC other than M. tuberculosis, M. canettii and M africanum clade 1

Using multiplex 1, if the HEX labelled MTC *IepA* and Cy5 labelled IAC MSMEG_0660 diagnostics assays generate a positive signal in each of these channels, but the FAM labelled *wbbl1,* the ROX labelled RD^{canettiil} and the Cyan 500 labelled RD713 diagnostics assays do not generate positive signals in each of these channels, the result indicates that a member of the MTC other than *M tuberculosis, M. canettii* and *M. africanum* clade 1 is present in the sample and the user of the test should now proceed to the second multiplex real-time PCR disclosed in this invention.

### Result scenario if no member of MTC not present

Using multiplex 1, if a positive signal observed in the Cy5 labelled IAC MSMEG_0660 diagnostics assay, but the HEX labelled MTC *lepA,* the FAM labelled *wbbl1,* the ROX labelled RD^{cancttiil} and the Cyan 500 labelled RD713 diagnostics assays do not generate positive signals in each of these channels, the result indicates that a member of the MTC is not present in the sample being tested for.

### Result scenario for invalid result

Using multiplex 1, if no positive signal is observed for any diagnostics assay tested for, including the Cy5 labelled IAC MSMEG_0660 diagnostics assay, the result is considered invalid and must be repeated.

### Multiplex 2

If from multiplex 1 it is known that a member of the MTC is present, other than *M*. *tuberculosis, M. canettii* or *M. africanum* clade 1, the user will perform the second multiplex real-time PCR diagnostics assay disclosed in this invention.

### Result scenario for the identification of M. caprae

Using multiplex 2, if a positive signal is observed for the Cyan 500 labelled *M. caprae lepA,* the FAM labelled *lpqT,* the HEX labelled RD1 and the Cy5 labelled IAC MSMEG_0660 diagnostics assay, but the ROX labelled RD701 diagnostics assay does not generate a positive signal in this channel, the result indicates that *M. caprae* is present in the sample.

### Result scenario for the identification of M. bovis

Using multiplex 2, if a positive signal is observed in the FAM labelled *IpqT,* the HEX labelled RD1 and the Cy5 labelled IAC MSMEG_0660 diagnostics assay, but the Cyan500 labelled *M. caprae lepA* and the ROX labelled RD701 diagnostics assays do not generate positive signals in these channels, the result indicated that *M. bovis* is present in the sample.

### Result scenario for the identification of M. bovis BCG

Using multiplex 2, if a positive signal is observed in the FAM labelled *IpqT* and the Cy5 labelled IAC MSMEG_0660 diagnostics assays, but the Cyan 500 labelled *M*. *caprae lepA,* the HEX labelled RD1 and the ROX labelled RD701 diagnostics assays do not generate positive signals in these channels, the result indicates that *M. bovis.* BCG is present in the sample.

### Result scenario for the identification of M. africanum clade 2

Using multiplex 2, if a positive signal is observed in the ROX labelled RD701, the HEX labelled RD1 and the Cy5 labelled IAC MSMEG_0660 diagnostics assays, but the Cyan500 labelled *M. caprae lepA* and the FAM labelled *lpqT* diagnostics assays do not generate positive signals in these channels, the result indicates that *M*. *africanum* clade 2 is present in the sample.

### Result scenario for invalid result

Using multiplex 2, if no positive signal is observed for any assay tested in this multiplex, the result is considered invalid and must be repeated.

### Combined results of Multiplex 1 and Multiplex 2

If the results from both multiplex real-time PCR assays described are taken into account it is also possible to accurately identify the remaining members of the MTC, namely *M. microti* and *M. pinnipedii.*

### Result scenario for the Identification of M. microti

If a positive signal is observed in the HEX labelled MTC *lepA* and the Cy5 labelled IAC MSMEG_0660 diagnostics assay in multiplex 1 and no other positive signal is observed in any of the other diagnostics assays channels in multiplex 1 and multiplex 2 with the exception of a positive signal in the Cy5 labelled IAC MSMEG_0660 diagnostics assay in multiplex 2 the result indicates that *M. microti* is present in the sample.

### Result scenario for the identification of M. pinnipedii

If taking the results from both multiplex real-time PCR assays described into account and a positive signal is observed in the HEX labelled MTC *lepA* and the Cy5 labelled IAC MSMEG_0660 diagnostics assay in multiplex 1 and no positive signal is observed for all other assays in multiplex 1 and multiplex 2, with the exception of a positive signal in the HEX labelled RD1 and the Cy5 labelled MSMEG_0660 diagnostics assays in multiplex 2 the result indicates that *M. pinnipedii* is present in the sample.

**Table 2: Mycobacterium tuberculosis complex isolates used in this study**

| Species | Strain | Country of Isolation | Origin | Remark |
|---|---|---|---|---|
| *M. tuberculosis* | 22 | Mongolia | RIVM | Beijing lineage |
| *M. tuberculosis* | 53 | Argentina | RIVM | Haarlem lineage |
| *M*. *tuberculosis* | 112 | The Netherlands | RIVM | CAS lineage |
| *M*. *tuberculosis* | 67 | Comoro Islands | RIVM | EAI lineage |
| *M. tuberculosis* | 41 | Chile | RIVM | LAM lineage |
| *M. tuberculosis* | 103 | China | RIVM | T-family lineage |
| *M. tuberculosis* | 12594_02 | Former Soviet Union | Borstel | Beijing lineage |
| *M. tuberculosis* | 1500_03 | Former Soviet Union | Borstel | Beijing lineage |
| *M. tuberculosis* | 1934_03 | Former Soviet Union | Borstel | Beijing lineage |
| *M. tuberculosis* | 1428_02 | Ghana | Borstel | Cameroon lineage |
| *M. tuberculosis* | 5390_02 | Ghana | Borstel | Cameroon lineage |
| *M*. *tuberculosis* | 5400_02 | Ghana | Borstel | Cameroon lineage |
| *M. tuberculosis* | 2637_02 | Germany | Borstel | Delhi/CAS lineage |
| *M. tuberculosis* | 7936_01 | Germany | Borstel | Delhi/CAS lineage |
| *M*. *Tuberculosis* | 1797_03 | Germany | Borstel | EAI lineage |
| *M. tuberculosis* | 4850_03 | Germany | Borstel | EAI lineage |
| *M. tuberculosis* | 947_01 | Germany | Borstel | EAI lineage |
| *M*. *tuberculosis* | 2336_02 | Germany | Borstel | Haarlem lineage |
| *M*. *tuberculosis* | 9S32_03 | Germany | Borstel | Haarlem lineage |
| *M. tuberculosis* | 7968_03 | Germany | Borstel | LAM lineage |
| *M. tuberculosis* | 8885_03 | Germany | Borstel | LAM lineage |
| *M. tuberculosis* | 946_03 | Germany | Borstel | LAM lineage |
| *M. tuberculosis* | 2151_03 | Germany | Borstel | S-type lineage |
| *M. tuberculosis* | 2318_06 | Germany | Borstel | S-type lineage |
| *M. tuberculosis* | 10469_01 | NA^{b} | Borstel | Ghana lineage |
| *M. tuberculosis* | 10493_01 | NA^{b} | Borstel | Ghana lineage |
| *M. tuberculosis* | 2570_02 | NA^{b} | Borstel | Ghana lineage |
| *M. tuberculosis* | 2201_99 | Uganda | Borstel | Uganda I lineage |
| *M. tuberculosis* | 2333_99 | Uganda | Borstel | Uganda I lineage |
| *M. tuberculosis* | 2176_99 | Uganda | Borstel | Uganda II lineage |
| *M. tuberculosis* | 2191_99 | Uganda | Borstel | Uganda II lineage |
| *M. tuberculosis* | 4412_04 | Germany | Borstel | X-type lineage |
| *M. tuberculosis* | 9953_04 | Germany | Borstel | X-type lineage |
| *M. tuberculosis* | 11313_03 | Germany | Borstel | Tur lineage |
| *M*. *tuberculosis* | 1657_03 | Germany | Borstel | Ural lineage |
| *M*. *tuberculosis '* | 10264_03 | Germany | Borstel | Tur lineage |
| *M. tuberculosis* | 10529_03 | Germany | Borstel | Tur lineage |
| *M*. *tuberculosis* | 8431_03 | Germany | Borstel | Ural lineage |
| *M*. *tuberculosis* | 3493_07 | | Borstel | Hamburg lineage |
| *M. tuberculosis* | 10707_07 | | Borstel | Hamburg lineage |
| *M. tuberculosis* | 9679_00 | NA^{b} | Borstel | Laboratory strain ATCC |
| H37Rv | | | | |
| *M. tuberculosis* | - | NA^{b} | Mario | Clinical isolates |
| (*19 clinical isolates*) | | | Vaneechoutte | |
| *M*. *canettii* | 116 | Somalia | RIVM | Smooth growing strain described by van Soolingen *et al.* 1997 |
| *M. canettii* | 1997-1549 | Switzerland | RIVM | Swiss isolate described in Pfyffer *et al.* 1998 |
| *M. canettii* | NLA000701671 - | Somalia | RIVM | Characterised on the basis of their spoligotype, IS6110 RFLP type and smooth growth as |
| *M. canettii* | NLA000200937 | Eritrea | RIVM | Characterised on the basis of their spoligotype, IS6110 RFLP type and smooth growth |
| *M. canettii* | 1996-46 | France | RIVM | Canetti strain |
| *M*. *canettii* | 3040_99 | The Netherlands | Borstel | |
| *M. canettii* | 3151_08 | NA^{b} | Borstel | |
| *M. canettii* | 3041_99 | The Netherlands | Borstel | |
| *M. bovis* | 117 | Argentina | RIVM | See Kremer *et al*. 2005 |
| *M. bovis* | 126 | Argentina | RIVM | See Kremer *et al.* 2005 |
| *M. bovis* | 73 | The Netherlands | RIVM | See Kremer *et al.* 2005 |
| *M. bovis* | 130 | The Netherlands | RIVM | See Kremer *et al.* 2005 |
| *M. bovis* | 24 | Saudi Arabia | RIVM | Isolated from an oryx, Antelope clade, see also Smith *et al.* 2006 |
| *M. bovis* | 4258_00 | Germany | Borstel | |
| *M. bovis* | 751_01 | Germany | Borstel | |
| *M. bovis* | 7540_01 | Germany | Borstel | |
| *M*. bovis (6 isolates) | - | NA^{b} | Mario Vaneecchoutte | Clinical isolates |
| *M. bovis* BCG | 48 (2) | The Netherlands | RIVM | See Kremer *et al.* 2005 |
| *M. bovis* BCG | 71 | Japan | RIVM | See Kremer *et al.* 2005 |
| *M. bovis* BCG | 83 | Russia | RIVM | See Kremer *et al.* 2005 |
| *M. bovis* BCG | 2008-714^{a} | NA^{b} | RIVM | Identified on basis ofcharacteristic IS6110/IS1081 RFLP patterns according to van Soolingen *et al.* 1992 |
| *M. bovis* BCG | 2008-1601^{a} | NA^{b} | RIVM | Identified on basis of characteristic IS6110/IS1081 RFLP patterns according to van Soolingen *et al.* 1992 |
| *M. bovis* BCG | DSM 43990 | NA^{b} | DSMZ | *Mycobacterium bovis* Karlson and Lessel 1970 BCG, Chicago I |
| *M. bovis* BCG | DSM 45071 | NA^{b} | DSMZ | *Mycobacterium bovis* Karlson and Lessel 1970 |
| *M. caprae* | 2006-1960^{a} | The Netherlands | RIVM | Characterised using Hain genotype MTBC kit |
| *M. caprae* | 2007-0039^{a} | The Netherlands | RIVM | Characterised using Hain genotype MTBC kit |
| *M. caprae* | 1694_00 | Germany | Borstel | |
| *M. caprae* | 8986_99 | Germany | Borstel | |
| *M. caprae* | 9577_99 | Germany | Borstel | |
| *M. microti* | 62 | United Kingdom | RIVM | see van Soolingen *et al.* 1998 |
| *M. microti* | 25 | United Kingdom | RIVM | see van Soolingen *et al.* 1998 |
| *M. microti* | 15274^{a} | United Kingdom | RIVM | see van Soolingen *et al.* 1998 |
| *M. microti* | 15912^{a} | Belgium | RIVM | see van Soolingen *et al.* 1998 |
| *M. microti* | 15911^{a} | Netherlands | RIVM | see van Soolingen *et al.* 1998 |
| *M. microti* | 417/01 | Germany | | Llama lineage |
| *M. pinnipedii* | 76 | Argentina | RIVM | See Kremer *et al.* 2005 |
| *M*. *pinnipedii* | 81 | Argentina | RIVM | See Kremer *et al.* 2005 |
| *M. pinnipedii* | 101 | Argentina | RIVM | See Kremer *et al*. 2005 |
| *M. pinnipedii* | 7011_02 | Germany | Borstel | |
| *M. pinnipedii* | 7739_01 | Germany | Borstel | |
| *M*. *africanum* | 6 | The Netherlands | RIVM | *M. africanum* clade 2 |
| *M. africanum* | 128 (85) | The Netherlands | RIVM | *M. africanum* clade 2 |
| *M. africanum* | 2007-1386^{a} | The Netherlands | RIVM | *M. africanum* clade 2 |
| *M. africanum* | 2007-1154^{a} | The Netherlands | RIVM | *M. africanum* clade 2 |
| *M. africanum* | 2007-1073^{a} | The Netherlands | RIVM | *M. africanum* clade 2 |
| *M. africanum* | 1449_02 | Ghana | Borstel | *M. africanum* clade 1 |
| *M. africanum* | 1473_02 | Ghana | Borstel | *M. africanum* clade I |
| *M. africanum* | 10473_01 | Ghana | Borstel | *M. africanum* clade I |
| *M*. *africanum* | 10494_01 | Ghana | Borstel | *M. africanum* clade 1 |
| *M. africanum* | 1443_02 | Ghana | Borstel | *M. africanum* clade 1 |
| *M. africanum* | 10476_01 | Ghana | Borstel | *M. africanum* clade 2 |
| *M. africanum* | 10514_01 | Ghana | Borstel | *M. africanum* clade 2 |
| *M. africanum* | 5468_02 | Ghana | Borstel | *M. africanum* clade 2 |
| *M*. *africanum* | 9550_99 | Ghana | Borstel | *M. africanum* clade 2 ATCC |

| | | | | |
|---|---|---|---|---|
| ^{a}Represent RIVM strains not previously described in literature, however have been characterised to the species level using techniques outlined in references supplied as remark. ^{b}This information was not available. | | | | |

**Table 3: Non-tuberculosis Mycobacterium and Non-Mycobacterium species used in this study**

| **Non *tuberculosis* mycobacteria** | **Strain designation^{a}** | **Remark** |
|---|---|---|
| *Mycobacterium aichiense* | DSM 44147 | Type strain, isolated from soil |
| *Mycobacterium alvei* | DSM 44176 | Type strain, isolated from water sample |
| *Mycobacterium arupense* | DSM 44942 | Type strain, isolated from a tendon |
| *Mycobacterium asiaticum* | ITG 8182 | |
| *Mycobacrerium avium* | ITG 7886 | |
| *Mycobacterium boenickei* | DSM 44677 | Type strain, isolated from a leg wound |
| *Mycobacterium branderi* | DSM 44624 | Type strain, isolated from human sputum |
| *Mycobacterium brisbanense* | DSM 44680 | Type strain, isolated from a sinus |
| *Mycobacterium brumae* | DSM 44177 | Type strain, isolated from water sample |
| *Mycobacterium canariasense* | DSM 44828 | Type strain, isolated from human blood |
| *Mycobacterium celatum* | ITG 6147 | |
| *Mycobacterium chelonae* | ITG 4975 | |
| *Mycobacterium chelonae subsp abscessus* | DSM 44196 | Type strain |
| *Mycobacterium confluentis* | DSM 44017 | Type strain, isolated from human sputum |
| *Mycobacterium conspicuum* | DSM 44136 | Type strain, isolated from patient with disseminated infection |
| *Mycobacterium flavescens* | VUB A016 | |
| *Mycobacterium fortuitum* | ITG 8020 | |
| *Mycobacterium genavense* | ITG 97-102 | |
| *Mycobacterium gilvum* | DSM 9487 | Isolated from soil |
| *Mycobacterium goodii* | DSM 44492 | Type strain |
| *Mycobacterium gordonae* | ITG 7704 | |
| *Mycobacterium heckeshornense* | DSM 44428 | Type strain, isolated from human respiratory tract |
| *Mycobacterium houstonense* | DSM 44676 | Type strain, isolated from a facial abscess |
| *Mycobacterium intracellulare* | DSM 43223 | Type strain |
| *Mycobacterium kansasti* | ITG 7727 | |
| *Mycobacterium kubiciae* | DSM 44627 | Type strain, isolated from human sputum |
| *Mycobacterium lacus* | DSM 44577 | Type strain, isolated from human elbow |
| *Mycobacterium mageritense* | DSM 44476 | Type strain, isolated from human sputum |
| *Mycobacterium malmoense* | ITG 940611 | |
| *Mycobacterium marinum* | ITG 1727 | |
| *Mycobacterium massiliense* | DSM 45103 | Type strain, isolated from human blood |
| *Mycobacterium moriokaense* | DSM 44221 | Type strain, isolated from soil |
| *Mycobacterium mucogenicum* | DSM 44625 | Type strain, isolated from human cyst |
| *Mycobacterium nebraskense* | DSM 44803 | Type strain, isolated from human sputum |
| *Mycobacterium neworleansense* | DSM 44679 | Type strain, isolated from human scalp |
| *Mycobacterium paratuberculosis* | ITG 2666 | |
| *Mycobacterium scrofulaceum* | DSM 43992 | Type strain, isolated from human cervical lymph node |
| *Mycobacterium shimoidei* | DSM 44152 | Type strain, isolated from sputum of patient with tuberculosis-like disease |
| *Mycobacterium simiae* | ITG 4485 | |
| *Mycobacterium smegmatis* | DSM 43756 | Type strain |
| *Mycobacterium sculgai* | ITG 4979 | |
| *Mycobacterium tusciae* | DSM 44338 | Type strain, isolated from human cervical lymph node |
| *Mycobacterium ulcerans* | ITG 96-1439 | |
| *Mycobacterium xenopi* | ITG 4986 | |

| **Other bacteria** | **Strain designation** | **Remark** |
|---|---|---|
| *Staphylococcus aureus* | DSM 20231 | Type strain, isolated from human pleural fluid |
| *Listeria monocytogenes* | DSM 20600 | Type strain, isolated from a rabbit |
| *Escherichia coli* | DSM 301 | Disinfectant test strain |
| *Klebsiella oxytoca* | ATCC 43086 | |
| *Enterococcus faecalis* | DSM 20371 | Isolated from pleural fluid |
| *Proteus mirabilis* | DSM 4479 | Type strain |
| *Bacillus cereus* | DSM 31 | Type strain |
| *Bordetella pertussis* | CCUG 13475 | Isolated from patient suffering from whooping cough |
| *Streptococcus agalactiae* | DSM 2134 | Type strain |
| *Rhodococcus equi* | DSM 20307 | Type strain, isolated from lung abscess of foal |
| *Streptomyces albidoflavus* | DSM 40455 | Type strain |
| *Nocardioides sp.* | DSM 17401 | Proposed type strain, isolated from marine sediment |
| *Nocardia salmonicida* | DSM 40472 | Type strain, isolated from bluebock salmon |
| *Nocardia asiatica* | clinical isolate | Isolated from human wound |
| *Nacardia nova* | clinical isolate | Isolated from human abscess |
| *Nocardia cyriacigeorgica* | clinical isolate | isolated from human bronchial aspirate |
| *Nocardia farcinica* | clinical isolate | Isolated from human abscess |

| | | |
|---|---|---|
| ^{a} RIVM = National Tuberculosis Reference Laboratory, National institute for Public Health and the Environment, Bilthoven, The Netherlands; *Borstel = National Reference Center for Mycobacteria, Borstel, Germany; *DSM = The German Collection of Microorganisms: *ATCC = American Type Culture Collection; *ITG = Institute of Tropical Medicine, Antwerp, Germany; *CCUG = Culture Collection, University of Göteborg, Sweden; *VUB = Department of Microbiology. Academic Hospital of the Free University of Brussels, Brussels, Belgium. ^{b} This information was not available (NA) for this study. | | |

**Table 4: Oligonucleotide primers and probes used in this study**

| **Name** | **Function** | **Sequence 5'→3'** |
|---|---|---|
| MTC_IAC Fw | Forward Sequencing primer, forward MTC and internal control real- time PCR assay primer | AGACCGTGCGGATCTTG (SEQ ID NO: 100/106) |
| MTC_IAC Rv | Reverse Sequencing primer, Reverse MTC and internal control real-time PCR assay primer | CATGGAGATCACCCGTGA (SEQ ID NO: 102/108) |
| MTC Probe | MTC probe | HEX-ACGGATTGGTCACCCGGATT-BHQI (SEQ ID NO: 101) |
| IAC probe | Internal control probe | CY5-ACGACCTTCTCGGAACCGT-BHQ2 (SEQ ID NO:107) |
| *wbbII*_Fw | Forward sequencing primer, Forward real-time PCR assay primer | TACCAGCTTCAGTTTCCGT (SEQ ID NO: 97) |
| *wbbII*_Rv | Reverse sequencing primer, Reverse real-time PCR assay primer | GCACCTATATCTTCTTAGCCG (SEQ ID NO: 99) |
| *wbbII* probe | *wbbII* probe | FAM-ATGGTGCGCAGTTCACTGC-BHQI (SEQ ID NO: 98) |
| *M. canetti* sp Fw | Forward *M*. *canetti* specific primer | ATGTGGTTTCAGTACGACTTC (SEQ ID NO: 103) |
| *M. canetti* sp Rv | Reverse *M*. *canetti* specific primer | GATGGCAGTGTCTTATCCAA (SEQ ID NO: 105) |
| *M. canetti* sp probe | *M. canetti specific* probe | ROX-TGAGAGGTGTTGGCACGCAA-BHQ2 (SEQ ID NO:104) |
| *M. canetti* seq 1.a | Forward sequencing primer 1 | TGTCGGCGGCCACGT (SEQ ID NO: 89) |
| *M. canett* seq 1.b | Reverse sequencing primer I | GAAGTCCAGCATCTTGGCGTT (SEQ ID NO: 90) |
| *M. canetti* seq 2.a | Forward sequencing primer I | TGTCGGCGGCCACGT (SEQ ID NO: 91) |
| *M. canetti* seq 2.b | Reverse sequencing primer 2 | ATCGTGCAGTGCGGCCA (SEQ ID NO: 92) |
| *M. canetti* seq 3.a | Forward sequencing primer 3 | GCAGCATTGTGGTTGACCGA (SEQ ID NO: 93) |
| *M. canetti* seq 3.b | Reverse sequencing primer 3 | TCCCAGCGTTGCGCCTT (SEQ ID NO: 94) |
| *M. canetti* seq 4.a | Forward sequencing primer 4 | TGATGCGGCTGCTCAAGC (SEQ ID NO: 95) |
| *M. canetti* seq 4.b | Reverse sequencing primer 4 | TGTCAAGGGACATGGGGAACT(SEQ ID NO: 96) |
| lpqT_FW' | Forward sequencing primer, Forward real-time PCR assay primer | ACCAATCCGGCGATGATC (SEQ ID NO: 158) |
| *lpqT_Rv* | Reverse sequencing primer, Reverse real-time PCR assay primer | CGACTGCACACCTGGAA (SEQ ID NO: 159) |
| *lpqT* probe | *lpqT* Probe | FAM-TTGGCCGOCOCCGGTT-BHQI (SEQ ID NO:160) |
| RDI_Fw | Forward sequencing primer. Forward real-time PCR assay primer | CATCGCTGATGTGCTTCC (SEQ ID NO: 161) |
| RDI_RV | Reverse sequencing primer, Reverse real-time PCR assay primer | TGCGCCOAGCTGTATTC (SEQ ID NO: 162) |
| RDI_probe | RDI Probe | ROX-ACACTAGCGTCAATGCGGTCA-BHQ2 (SEQ ID NO: 163) |
| *M. caprae lepA*_Fw | Forward sequencing primer, Forward real-time PCR assay primer | AGACCGTGCGGATCTTG (SEQ ID NO: 164) |
| *M. caprae lepA_Rv* | Reverse sequencing primer, Reverse real-time PCR assay primer | CATGGAGATCACCCGTGA (SEQ ID NO: 165) |
| *M. caprae lepA* probe | *M. caprae lepA*Probe | Cyan 500-TATCGGGTACACAAAGACGA - BBQ (SEQ ID NO: 166) |
| R0713_Fw | Forward sequencing primer, Forward real-time PCR assay primer | ACGGAACGGTCAAGAAC (SEQ ID NO: 167) |
| RD713_Rv | Reverse sequencing primer, Reverse real-time PCR assay primer | GCTCAAGAATCGTCGCTA (SEQ ID NO: 168) |
| RD713 probe | RD 713 Probe | Cyan 500-ACGTCCTTGTGACCGCGAC- BBQ (SEQ ID NO: 169) |
| RD701_Fw | Forward sequencing primer, Forward real-time PCR assay primer | AACGGGTCGGATTCTCC (SEQ ID NO: t70) |
| RD701_Rv | Reverse sequencing primer | CCGAAACCCTCGTTGATC (SEQ ID NO: 171) |
| RD701 probe | RD 701 Probe | ROX-TCAGCCGCCGGCCAACC-BHQ2 (SEQ ID NO: 172) |
| MTC_FW | Forward sequencing primer | AGACCGTGCGGATCTTG (SEQ ID NO: 164) |
| MTC_Rv | Reverse sequencing primer | CATGGAGATCACCCGTGA (SEQ ID NO: 165) |
| MTC probe | MTC *lepA* Probe | HEX-ATTGGTCACCCGGATTTCGGT-BHQ1 (SEQ ID NO: 173) |
| IAC | Forward sequence primer, Forward real-time | TCACCGACCATGTCCAG(SEQ ID NO: 155) |
| MSMEG_0660_Fw | PCR assay primer | |
| IAC | Reverse sequencing primer, Reverse real-lime | CGTTGCCCAATCCGTATG (SEQ ID NO: 156) |
| MSMEG 0660_Rv | PCR assay primer | |
| IAC MSMEG_0660 probe | IAC MSMEG_0660 probe | Cy5-CAGCAGTACCATCGCCATCG-BHQ2 (SEQ ID NO: 157) |

### References

Al-Attiyah, R. & Mustafa, A. S. (2008). Characterization of Human Cellular Immune Responses to Novel Mycobacterium tuberculosis Antigens Encoded by Genomic Regions Absent in Mycobacterium bovis BCG. Infect Immun 76,4190-4198.
Arya, M., Shergill, I. S., Williamson, M., Gommersall, L., Arya, N. & Patel, H. R. (2005). Basic principles of real-time quantitative PCR. Expert Review of Molecular Diagnostics 5, 209-219.
Behr, M. A., Wilson, M. A., Gill, W. P., Salamon, H., Schoolnik, G. K., Rane, S. & Small, P. M. (1999). Comparative Genomics of BCG Vaccines by Whole-Genome DNA Microarray. Science 284, 1520-1523.
Brosch, R., Gordon, S. V., Pym, A., Eiglmeier, K., Garnier, T. & Cole, S. T. (2000). Comparative genomics of the mycobacteria. Int J Med Microbiol 290, 143-152.
Brosch, R., Gordon, S. V., Marmiesse, M. & other authors (2002). A new evolutionary scenario for the Mycobacterium tuberculosis complex. Proceedings of the National Academy of Sciences of the United States of America 99, 3684-3689.
Das, S., Das, S. C. & Verma, R. (2007). Occurrence of RD9 region and 500 bp fragment among clinical isolates of Mycobacterium tuberculosis and Mycobacterium bovis. Microbiol Immunol 51, 231-234.
de Jong, B. C., Antonio, M. & Gagneux, S. (2010). Mycobacterium africanum-Review of an Important Cause of Human Tuberculosis in West Africa. PLoS Negl Trop Dis 4, e744.
Dille, B. J., Butzen, C. C. & Birkenmeyer, L. G. (1993). Amplification of Chlamydia trachomatis DNA by ligase chain reaction. J Clin Microbiol 31, 729-731.
Djelouadji, Z., Raoult, D., Daffé, M. & Drancourt, M. (2008). A Single-Step Sequencing Method for the Identification of Mycobacterium tuberculosis Complex Species. PLoS Negl Trop Dis 2, e253.
Dorak, M. T. (2006).In M. T. Dorak (ED.), Real-time PCR, <http://www.dorak.info/genetics/realtime.html>.
Flint, J. L., Kowalski, J. C., Karnati, P. K. & Derbyshire, K. M. (2004). The RD1 virulence locus of Mycobacterium tuberculosis regulates DNA transfer in Mycobacterium smegmatis. Proceedings of the National Academy of Sciences of the United States of America 101, 12598-12603.
Garnier, T., Eiglmeier, K., Camus, J.-C. & other authors (2003). The complete genome sequence of Mycobacterium bovis. Proceedings of the National Academy of Sciences of the United States of America 100, 7877-7882.
Goh, K. S., Legrand, E., Sola, C. & Rastogi, N. (2001). Rapid Differentiation of "Mycobacterium canettii" from Other Mycobacterium tuberculosis Complex Organisms by PCR-Restriction Analysis of the hsp65 Gene. J Clin Microbiol 39, 3705-3708.
Halse, T. A., Edwards, J., Cunningham, P. L., Wolfgang, W. J., Dumas, N. B., Escuyer, V. E. & Musser, K. A. Combined Real-Time PCR and rpoB Gene Pyrosequencing for Rapid Identification of Mycobacterium tuberculosis and Determination of Rifampin Resistance Directly in Clinical Specimens. J Clin Microbiol 48, 1182-1188.
Hoorfar, J., Malorny, B., Abdulmawjood, A., Cook, N., Wagner, M. & Fach, P. (2004). Practical Considerations in Design of Internal Amplification Controls for Diagnostic PCR Assays. J Clin Microbiol 42, 1863-1868.
Huard, R. C., de Oliveira Lazzarini, L. C., Butler, W. R., van Soolingen, D. & Ho, J. L. (2003). PCR-Based Method To Differentiate the Subspecies of the Mycobacterium tuberculosis Complex on the Basis of Genomic Deletions. J Clin Microbiol 41, 1637-1650.
Huard, R. C., Fabre, M., de Haas, P., Claudio Oliveira Lazzarini, L., van Soolingen, D., Cousins, D. & Ho, J. L. (2006). Novel Genetic Polymorphisms That Further Delineate the Phylogeny of the Mycobacterium tuberculosis Complex. J Bacteriol 188, 4271-4287.
Kiers, A., Klarenbeek, A., Mendelts, B., Van Soolingen, D., Ko & ter, G. (2008a). Transmission of Mycobacterium pinnipedii to humans in a zoo with marine mammals. The International Journal of Tuberculosis and Lung Disease 12, 1469-1473.
Kiers, A., Klarenbeek, A., Mendelts, B., Van Soolingen, D. & Koëter, G. (2008b). Transmission of Mycobacterium pinnipedii to humans in a zoo with marine mammals. The International Journal of Tuberculosis and Lung Disease 12, 1469-1473.
Ma, Y., Pan, F. & McNeil, M. (2002). Formation of dTDP-Rhamnose Is Essential for Growth of Mycobacteria. J Bacteriol 184, 3392-3395.
Malhotra-Kumar, S., Haccuria, K., Michiels, M., leven, M., Poyart, C., Hryniewicz, W., Goossens, H. & on behalf of the MOSAR WP2 Study Team (2008). Current Trends in Rapid Diagnostics for Methicillin-Resistant Staphylococcus aureus and Glycopeptide-Resistant Enterococcus Species. J Clin Microbiol 46, 1577-1587.
Miller, M. B. & Tang, Y.-W. (2009). Basic Concepts of Microarrays and Potential Applications in Clinical Microbiology. Clin Microbiol Rev 22, 611-633.
Nallur, G., Luo, C., Fang, L. & other authors (2001). Signal amplification by rolling circle amplification on DNA microarrays. Nucl Acids Res 29, e118-.
Niemann, S., Richter, E. & Rusch-Gerdes, S. (2000). Differentiation among Members of the Mycobacterium tuberculosis Complex by Molecular and Biochemical Features: Evidence for Two Pyrazinamide-Susceptible Subtypes of M. bovis. J Clin Microbiol 38, 152-157.
O' Grady, J., Sedano-Balbás, S., Maher, M., Smith, T. & Barry, T. (2008). Rapid real-time PCR detection of Listeria monocytogenes in enriched food samples based on the ssrA gene, a novel diagnostic target. Flood Microbiology 25, 75-84.
Panteix, G., Gutierrez, M. C., Boschiroli, M. L. & other authors (2010). Pulmonary tuberculosis due to Mycobacterium microti: a study of six recent cases in France. J Med Microbiol 59, 984-989.
Parsons, L. M., Brosch, R., Cole, S. T., Somoskovi, A., Loder, A., Bretzel, G., van Soolingen, D., Hale, Y. M. & Salfinger, M. (2002). Rapid and Simple Approach for Identification of Mycobacterium tuberculosis Complex Isolates by PCR-Based Genomic Deletion Analysis. J Clin Microbiol 40, 2339-2345.
Pfyffer, G. E., Auckenthaler, R., van Embden, J. D. & van Soolingen, D. (1998). Mycobacterium canettii, the smooth variant of M. tuberculosis, isolated from a Swiss patient exposed in Africa. Emerg Infect Dis 4, 631-634.
Pinsky, B. A. & Banaei, N. (2008). Multiplex Real-Time PCR Assay for Rapid Identification of Mycobacterium tuberculosis Complex Members to the Species Level. J Clin Microbiol 46, 2241-2246.
Pym, A. S., Brodin, P., Brosch, R., Huerre, M. & Cole, S. T. (2002). Loss of RD1 contributed to the attenuation of the live tuberculosis vaccines Mycobacterium bovis BCG and Mycobacterium microti. Molecular Microbiology 46, 709-717.
Qin, Y., Polacek, N., Vesper, O., Staub, E., Einfeldt, E., Wilson, D. N. & Nierhaus, K. H. (2006). The Highly Conserved LepA Is a Ribosomal Elongation Factor that Back-Translocates the Ribosome. Cell 127, 721-733.
Rezwan, M., Grau, T., Tschumi, A. & Sander, P. (2007). Lipoprotein synthesis in mycobacteria. Microbiology 153, 652-658.
Robertson, J. M. & Walsh-Weller, J. (1998). An Introduction to PCR Primer Design and Optimization of Amplification Reactions. In Forensic DNA Profiling Protocols, pp. 121-154.
Rodriguez-Lázaro, D., Lloyd, J., Herrewegh, A., Ikonomopoulos, J., D'Agostino, M., Pla, M. & Cook, N. (2004). A molecular beacon-based real-time NASBA assay for detection of <i>Mycobacterium avium<li> subsp. <i>paratuberculosis</i> in water and milk. FEMS Microbiology Letters 237, 119-126.
Scheler, O., Glynn, B., Parkel, S., Palta, P., Toome, K., Kaplinski, L., Remm, M., Maher, M. & Kurg, A. (2009). Fluorescent labeling of NASBA amplified tmRNA molecules for microarray applications. BMC Biotechnology 9, 45.
Somoskovi, A., Dormandy, J., Parsons, L. M., Kaswa, M., Goh, K. S., Rastogi, N. & Salfinger, M. (2006). Sequencing of the pncA gene in members of the Mycobacterium tuberculosis complex has important diagnostic applications: Identification of a species-specific pncA Mutation in Mycobacterium canettii, and the Reliable and Rapid Predictor of Pyrazinamide Resistance. J Clin Microbiol, JCM.01454-01406.
Somoskovi, A., Dormandy, J., Mayrer, A. R., Carter, M., Hooper, N. & Salfinger, M. (2009). "Mycobacterium canettii" Isolated from a Human Immunodeficiency Virus-Positive Patient: First Case Recognized in the United States. J Clin Microbiol 47, 255-257.
Tortoli, E., Lavinia, F. & Simonetti, M. (1997). Evaluation of a commercial ligase chain reaction kit (Abbott LCx) for direct detection of Mycobacterium tuberculosis in pulmonary and extrapulmonary specimens. J Clin Microbiol 35, 2424-2426.
van Soolingen, D., Hoogenboezem, T., De Haas, P. E. W. & other authors (1997). A Novel Pathogenic Taxon of the Mycobacterium tuberculosis Complex, Canetti: Characterization of an Exceptional Isolate from Africa. Int J Syst Bacteriol 47, 1236-1245.
Vasconcellos, S., Huard, R., Niemann, S., Kremer, K., Santos, A., Suffys, P. & Ho, J. (2010). Distinct genotypic profiles of the two major clades of Mycobacterium africanum. BMC Infectious Diseases 10, 80.
Voelkerding, K. V., Dames, S. A. & Durtschi, J. D. (2009). Next-Generation Sequencing: From Basic Research to Diagnostics. Clin Chem 55, 641-658.
Warren, R. et al. (2006). Differentiation of Mycobacterium tuberculosis complex by PCR amplification of genomic regions of difference. The International Journal of Tuberculosis and Lung Disease: The Official Journal of the International Union against Tuberculosis and Lung Disease vol. 10, no. 7, 818-822.
Yang, S. & Rothman, R. E. (2004). PCR-based diagnostics for infectious diseases: uses, limitations, and future applications in acute-care settings. The Lancet Infectious Diseases 4, 337-348.

## Claims

1. A multiplex *in vitro* nucleic acid amplification method for identifying *Mycobacterium canettii* present in a sample, wherein the method comprises detecting the presence or absence of a plurality of nucleic acid molecule targets in the sample in one reaction, which, in combination, are unique in their presence or absence to *Mycobacterium canettii,* wherein the method comprises the use of primers or probes specific for a nucleic acid sequence that is present in *M. canettii,* but is not present in *M. tuberculosis* and comprises a region of RD^{canetti1}, SEQ ID NO: 78.

2. The multiplex *in vitro* nucleic acid amplification method of claim 1 wherein the method comprises a multiplex PCR.

3. The method of claim 1 or 2 wherein:
a) the primers comprise SEQ ID NO: 103 and SEQ ID NO: 105; and/or
b) the probe comprises SEQ ID NO: 104.

4. The method of any preceding claim, further comprising the use of primers or probes specific for a nucleic acid sequence that is present in both *M. tuberculosis* and *M. canettii.*

5. The method of claim 4 wherein the nucleic acid sequence that is present in both *M. tuberculosis* and *M. canettii* comprises a region of *wbbl1*, SEQ ID NO: 1 and preferably wherein:
a) the primers comprise SEQ ID NO: 97 and SEQ ID NO: 99; and/or
b) the probe comprises SEQ ID NO: 98.

6. The method of any preceding claim, further comprising the use of primers or probes specific for a nucleic acid sequence that is present in *M. africanum* clade 1 but is not present in *M. tuberculosis* or *M. canettii.*

7. The method of claim 6 wherein the nucleic acid sequence that is present in *M. africanum* clade 1 but is not present in *M. tuberculosis* or *M. canettii* comprises a region of RD713, SEQ ID NO: 137, and preferably wherein:
a) the primers comprise SEQ ID NOs: 167 and 168; and/or
b) the probe comprises SEQ ID NO: 169.

8. The method of any preceding claim further comprising the use of primers or probes specific for *lepA*, SEQ ID NO: 47 to detect the presence or absence of the *Mycobacterium tuberculosis* complex, and optionally (i) wherein more than one probe specific for *lepA* is used, preferably wherein at least one of the probes specific for *lepA* does not significantly bind to nucleic acid amplified from a member of the *Mycobacterium tuberculosis* complex, and/or (ii) wherein the probe comprises SEQ ID NO: 173.

9. The method of any one of claims 1 to 8 further comprising a *multiplex in vitro* nucleic acid amplification detecting a plurality of nucleic acid molecules which, in combination, are unique in their presence or absence to *Mycobacterium bovis,* detecting a plurality of nucleic acid molecules which, in combination, are unique in their presence or absence to *Mycobacterium bovis BCG,* detecting a plurality of nucleic acid molecules which, in combination, are unique in their presence or absence to *Mycobacterium caprae*, detecting a plurality of nucleic acid molecules which, in combination, are unique in their presence or absence to *Mycobacterium africanum* clade 2, detecting a plurality of nucleic acid molecules which, in combination, are unique in their presence or absence to *Mycobacterium pinnipedii* or detecting a plurality of nucleic acid molecules which, in combination, are unique in their presence or absence to *Mycobacterium microti*, wherein:
a) the primers or probes that are used are specific for a nucleic acid sequence that is present in all three of *M. bovis, M. bovis BCG* and *M. caprae,* optionally wherein the nucleic acid sequence comprises a region of *lpgT*, SEQ ID NO: 109, preferably wherein:
(i) the primers comprise SEQ ID NOs: 158 and 159; and/or
(ii) the probe comprises SEQ ID NO: 160;
b) the primers or probes that are used are specific for a nucleic acid sequence that is present in *M. caprae* but is not present in *M. bovis* or *M. bovis BCG,* optionally wherein the nucleic acid sequence comprises a region of *M. caprae lepA*, SEQ ID NO: 76, preferably wherein:
(i) the primers comprise SEQ ID NOs: 164 and 165; and/or
(ii) the probe comprises SEQ ID NO: 166;
c) the primers or probes that are used are specific for a nucleic acid sequence that is deleted in *M. bovis BCG* and *M. microti* but is present in *M. bovis, M. caprae* and *M. pinnipedii,* optionally wherein the nucleic acid comprises a region of RD1, SEQ ID NO: 141, preferably wherein:
(i) the primers comprise SEQ ID NOs: 161 and 162; and/or
(ii) the probe comprises SEQ ID NO: 163; and/or
d) the primers or probes that are used are specific for a nucleic acid sequence that is present in *M. africanum* clade 2 but is not present in *M. bovis, M. bovis BCG, M. caprae, M. microti* or *M. pinnipedii,* optionally wherein the nucleic acid comprises a region of RD701, SEQ ID NO: 132, preferably wherein:
(i) the primers comprise SEQ ID NOs: 170 and 171; and/or
(ii) the probe comprises SEQ ID NO: 172.

10. The method of claim 9 comprising performing the method of any of claims 1 to 8 and subsequently performing the multiplex *in vitro* nucleic acid amplification as defined in claim 9.

11. The method of any one of any one of the preceding claims including the use of primers or probes specific for an IAC, preferably comprising a region of:
a) *lepA*, SEQ ID NO: 84; or
b) comprising a region of MSMEG_0660, SEQ ID NO: 135, more preferably wherein:
(i) the primers comprise SEQ ID NOs: 155 and 156; and/or
(ii) the probe comprises SEQ ID NO: 157.

12. A kit comprising a set of primers and probes which are specific for a plurality of nucleic acid molecule targets, which in combination are unique in their presence or absence to *Mycobacterium canettii* and wherein the kit comprises primers or probes specific for a nucleic acid that is present in *M. canettii* but is not present in *M. tuberculosis* and that are specific for a region of RD^{canetti1}, SEQ ID NO: 78, preferably wherein:
a) the primers comprise SEQ ID NOs 103 and 105; and/or
b) wherein the probe comprises SEQ ID NO: 104.

13. The kit of claim 12 further comprising:
(I)
a) primers or probes specific for a nucleic acid sequence that is present in both *M. tuberculosis* and *M. canettii,* preferably comprising primers or probes specific for a region of *wbbll,* SEQ ID NO: 1, more preferably wherein:
(i) the primers comprise SEQ ID NOs: 97 and 99; and/or
(ii) the probe comprises SEQ ID NO: 98;
b) primers or probes specific for a nucleic acid sequence that is present in *M. africanum* clade 1 but is not present in *M. tuberculosis* or *M. canettii,* preferably comprising a region of RD713, SEQ ID NO: 137, more preferably wherein:
(i) the primers comprise SEQ ID NOs: 167 and 168; and/or
(ii) the probe comprises SEQ ID NO: 169;
c) primers or probes specific for MTC *lepA*, SEQ ID NO: 47, preferably wherein the probe comprises SEQ ID NO: 173, wherein when the probe comprises SEQ ID NO: 173, the primers optionally comprise SEQ ID NOs: 164 and 165;
d) primers or probes specific for a nucleic acid sequence that is present in all three of *M. bovis, M. bovis BCG* and *M. caprae,* preferably comprising a region of *lpqT*, SEQ ID NO: 109, more preferably wherein:
(i) the primers comprise SEQ ID NOs: 158 and 159; and/or
(ii) the probe comprises SEQ ID NO: 160;
e) primers and probes specific for a nucleic acid sequence that is present in *M. caprae* but is not present in *M. bovis* or *M. bovis BCG,* preferably a region *of M. caprae lepA*, SEQ ID NO: 76, more preferably wherein the probe comprises SEQ ID NO: 166 and when the probe comprises SEQ ID NO: 166, the primers optionally comprise SEQ ID NOs: 164 and 165;
f) primers or probes specific for a nucleic acid sequence that is deleted in *M. bovis BCG* and *M. microti* but is present in *M. bovis, M. caprae* and *M. pinnipedii,* preferably a region of RD1, SEQ ID NO: 141, more preferably wherein:
(i) the primers comprise SEQ ID NOs: 161 and 162; and/or
(ii) the probe comprises SEQ ID NO: 163; and/or
g) primers or probes specific for a nucleic acid sequence that is present in *M. africanum* clade 2 but is not present in *M. bovis, M. bovis BCG, M. caprae, M. microti* or *M. pinnipedii,* preferably a region of RD701, SEQ ID NO: 132, more preferably wherein:
(i) the primers comprise SEQ ID NOs: 170 and 171; and/or
(ii) the probe comprises SEQ ID NO: 172;
and/or
(II) primers or probes specific for an IAC, preferably comprising a region of:
a) *lepA*, SEQ ID NO: 68, more preferably wherein the probe comprises SEQ ID NO: 107; or
b) MSMEG_0660, SEQ ID NO: 135, more preferably wherein:
(i) the primers comprise SEQ ID NOs: 155 and 156; and/or
(ii) the probe comprises SEQ ID NO: 157.

14. A diagnostic technique for identifying *Mycobacterium canettii* comprising hybridising sample nucleic acid molecules to one or more nucleic acid molecules which comprise or are complementary to a region of RD^{canetti1} (SEQ ID NO: 78), and which are specific for a nucleic acid sequence that is present in *M. canettii* but is not present in *M. tuberculosis* and optionally:
a) wherein the sample nucleic acid molecules are hybridised to at least one nucleic acid molecule that comprises or is complementary to a region of *wbbl1* (SEQ ID NOs: 1-46), and which is present in both *M. tuberculosis* and *M. canetti i;*
b) wherein the sample nucleic acid molecules are hybridised to at least one nucleic acid molecule that comprises or is complementary to a region of *lepA* (SEQ ID NOs: 47-77);
c) wherein the sample nucleic acid molecules are hybridised to at least one nucleic acid molecule that comprises or is complementary to a region of RD713 (SEQ ID NO: 137), and which is present in *M. africanum* clade 1 but is not present in *M. tuberculosis* or *M. canettii;*
d) wherein the sample nucleic acid molecules are hybridised to nucleic acid molecules that comprise or are complementary to a region of *lpqT* (SEQ ID NO: 109), and which are present in all three of *M. bovis, M. bovis BCG* and *M. caprae;*
e) wherein the sample nucleic acid molecules are hybridised to nucleic acid molecules that comprise or are complementary to a region of *M. caprae lepA* (SEQ ID NO: 76) and which are specific for a nucleic acid sequence that is present in *M. caprae* but is not present in *M. bovis* or *M. bovis BCG;*
f) wherein the sample nucleic acid molecules are hybridised to nucleic acid molecules that comprise or are complementary to a region of RD1 (SEQ ID NO: 141) and which are specific for a nucleic acid sequence that is deleted in *M. bovis BCG* and *M. microti* but present in *M. bovis, M. caprae* and *M. pinnipedii*; and/or
g) wherein the sample nucleic acid molecules are hybridised to nucleic acid molecules that comprise or are complementary to a region of RD701 (SEQ ID NO: 132) and which are specific for a nucleic acid sequence that is present in *M. africanum* clade 2 but is not present in *M. bovis, M. bovis BCG, M. caprae, M. microti or M. pinnipedii*;
and optionally wherein the hybridisation occurs on a microarray.

15. The method of any one of claims 1-11, the kit of any one of claims 12-13 or the diagnostic technique of claim 14, for use in monitoring disease progression, or monitoring response to treatment, preferably wherein the disease is tuberculosis.

## Patentansprüche

1. *In vitro*-Multiplex-Nukleinsäureamplifikationsverfahren zum Identifizieren von in einer Probe vorliegendem *Mycobacterium canettii,* wobei das Verfahren das Nachweisen des Vorliegens oder Fehlens einer Mehrzahl von Nukleinsäuremolekülzielen, die in Kombination hinsichtlich ihres Vorliegens oder Fehlens für *Mycobacterium canettii* einzigartig sind, in einer Reaktion in der Probe umfasst, wobei das Verfahren die Verwendung von Primern oder Sonden umfasst, die spezifisch für eine Nukleinsäuresequenz sind, die in *M. canettii* vorliegt, aber nicht in *M. tuberculosis* vorliegt und eine Region von RD^{canetti1}, SEQ ID NO: 78, umfasst.

2. *In vitro*-Multiplex-Nukleinsäureamplifikationsverfahren nach Anspruch 1, wobei das Verfahren eine Multiplex-PCR umfasst.

3. Verfahren nach Anspruch 1 oder 2, wobei
a) die Primer SEQ ID NO: 103 und SEQ ID NO: 105 umfassen und/oder
b) die Sonde SEQ ID NO: 104 umfasst.

4. Verfahren nach einem vorhergehenden Anspruch, das zudem die Verwendung von Primern oder Sonden, die für eine Nukleinsäuresequenz spezifisch sind, die sowohl in *M. tuberculosis* als auch *M*. *canettii* vorliegt, umfasst.

5. Verfahren nach Anspruch 4, wobei die Nukleinsäuresequenz, die sowohl in *M*. *tuberculosis* als auch *M. canettii* vorliegt, eine Region von *wbbl1*, SEQ ID NO: 1, umfasst und wobei vorzugsweise:
a) die Primer SEQ ID NO: 97 und SEQ ID NO: 99 umfassen und/oder
b) die Sonde SEQ ID NO: 98 umfasst.

6. Verfahren nach einem vorhergehenden Anspruch, das zudem die Verwendung von Primern oder Sonden umfasst, die spezifisch für eine Nukleinsäuresequenz sind, die in *M. africanum* Clade 1 vorliegt, aber nicht in *M*. *tuberculosis* oder *M. canettii* vorliegt.

7. Verfahren nach Anspruch 6, wobei die Nukleinsäuresequenz, die in *M*. *africanum* Clade 1 vorliegt, aber nicht in *M*. *tuberculosis* oder *M*. *canettii* vorliegt, eine Region von RD713, SEQ ID NO: 137, umfasst und wobei vorzugsweise:
a) die Primer die SEQ ID NO: 167 und 168 umfassen und/oder
b) die Sonde SEQ ID NO: 169 umfasst.

8. Verfahren nach einem vorhergehenden Anspruch, das zudem die Verwendung von Primern oder Sonden, die spezifisch für *lepA*, SEQ ID NO: 47, sind, zum Nachweisen des Vorliegens oder Fehlens von *Mycobacterium tuberculosis*-Komplex umfasst und wobei gegebenenfalls (i) mehr als eine für *lepA* spezifische Sonde verwendet wird, wobei vorzugsweise mindestens eine der für *lepA* spezifischen Sonden nicht signifikant an Nukleinsäure bindet, die von einem Mitglied des *Mycobacterium tuberculosis*-Komplexes amplifiziert wurde, und/oder (ii) wobei die Sonde SEQ ID NO: 173 umfasst.

9. Verfahren nach einem der Ansprüche 1 bis 8, das zudem eine *in vitro*-Multiplex-Nukleinsäureamplifikation umfasst, die eine Mehrzahl von Nukleinsäuremolekülen nachweist, die in Kombination in ihrem Vorliegen oder Fehlen einzigartig für *Mycobacterium bovis* sind, die eine Mehrzahl von Nukleinsäuremolekülen nachweist, die in Kombination in ihrem Vorliegen oder Fehlen einzigartig für *Mycobacterium bovis BCG* sind, eine Mehrzahl von Nukleinsäuremolekülen nachweist, die in Kombination in ihrem Vorliegen oder Fehlen für *Mycobacterium caprae* einzigartig sind, eine Mehrzahl von Nukleinsäuremolekülen nachweist, die in Kombination in ihrem Vorliegen oder Fehlen für *Mycobacterium africanum* Clade 2 einzigartig sind, eine Mehrzahl von Nukleinsäuremolekülen nachweist, die in Kombination in ihrem Vorliegen oder Fehlen für *Mycobacterium pinnipedii* einzigartig sind, oder eine Mehrzahl von Nukleinsäuremolekülen nachweist, die in Kombination in ihrem Vorliegen oder Fehlen für *Mycobacterium microti* einzigartig sind, wobei
a) die Primer oder Sonden, die verwendet werden, spezifisch für eine Nukleinsäuresequenz sind, die in allen drei von *M*. *bovis, M. bovis BCG* und *M*. *caprae* vorliegt, wobei gegebenenfalls die Nukleinsäuresequenz eine Region von *lpqT*, SEQ ID NO: 109, umfasst, wobei vorzugsweise:
(i) die Primer die SEQ ID NO: 158 und 159 umfassen und/oder
(ii) die Sonde SEQ ID NO: 160 umfasst,
b) die Primer oder Sonden, die verwendet werden, spezifisch für eine Nukleinsäuresequenz sind, die in *M*. *caprae* vorliegt, aber nicht in *M*. *bovis* oder *M. bovis BCG* vorliegt, wobei gegebenenfalls die Nukleinsäuresequenz eine Region von *M*. *caprae-lepA,* SEQ ID NO: 76, umfasst, wobei vorzugsweise:
(i) die Primer die SEQ ID NO: 164 und 165 umfassen und/oder
(ii) die Sonde SEQ ID NO: 166 umfasst,
c) die Primer oder Sonden, die verwendet werden, spezifisch für eine Nukleinsäuresequenz sind, die in *M*. *bovis BCG* und *M*. *microti* deletiert ist, aber in *M*. *bovis, M. caprae* und *M*. *pinnipedii* vorliegt, wobei gegebenenfalls die Nukleinsäure eine Region von RD1, SEQ ID NO: 141, umfasst, wobei vorzugsweise:
(i) die Primer die SEQ ID NO: 161 und 162 umfassen und/oder
(ii) die Sonde SEQ ID NO: 163 umfasst, und/oder
d) die Primer oder Sonden, die verwendet werden, spezifisch für eine Nukleinsäuresequenz sind, die in *M. africanum* Clade 2 vorliegt, aber nicht in *M. bovis, M. bovis BCG, M. caprae, M. microti* oder *M. pinnipedii* vorliegt, wobei gegebenenfalls die Nukleinsäure eine Region von RD701, SEQ ID NO: 132, umfasst, wobei vorzugsweise:
(i) die Primer die SEQ ID NO: 170 und 171 umfassen und/oder
(ii) die Sonde SEQ ID NO: 172 umfasst.

10. Verfahren nach Anspruch 9, umfassend das Durchführen des Verfahrens nach einem der Ansprüche 1 bis 8 und anschließend das Durchführen der *in vitro-*Multiplex-Nukleinsäureamplifikation nach Anspruch 9.

11. Verfahren nach einem von einem der vorhergehenden Ansprüche, das die Verwendung von Primern oder Sonden beinhaltet, die spezifisch für eine IAC sind, vorzugsweise umfassend eine Region von:
a) *lepA,* SEQ ID NO: 84, oder
b) umfassend eine Region von MSMEG_0660, SEQ ID NO: 135, wobei stärker bevorzugt:
(i) die Primer die SEQ ID NO: 155 und 156 umfassen und/oder
(ii) die Sonde SEQ ID NO: 157 umfasst.

12. Kit, umfassend einen Satz von Primern und Sonden, die spezifisch für eine Mehrzahl von Nukleinsäuremolekülzielen sind, die in Kombination hinsichtlich ihres Vorliegens oder Fehlens für *Mycobacterium canettii* einzigartig sind, und wobei das Kit Primer und Sonden umfasst, die für eine Nukleinsäure spezifisch sind, die in *M. canettii* vorliegt, aber nicht in *M. tuberculosis* vorliegt, und die spezifisch für eine Region von RD^{canetti1}, SEQ ID NO: 78, sind, wobei vorzugsweise:
a) die Primer die SEQ ID NO 103 und 105 umfassen und/oder
b) wobei die Sonde SEQ ID NO: 104 umfasst.

13. Kit nach Anspruch 12, das zudem umfasst:
(I)
a) Primer oder Sonden, die für eine Nukleinsäure spezifisch sind, die sowohl in M. *tuberculosis* als auch *M. canettii* vorliegt, vorzugsweise umfassend Primer oder Sonden, die für eine Region von *wbbll,* SEQ ID NO: 1, spezifisch sind, wobei stärker bevorzugt:
(i) die Primer die SEQ ID NO: 97 und 99 umfassen und/oder
(ii) die Sonde SEQ ID NO; 98 umfasst,
b) Primer oder Sonden, die spezifisch für eine Nukleinsäuresequenz sind, die in *M. africanum* Clade 1 vorliegt, aber nicht in *M. tuberculosis* oder *M. canettii* vorliegt, vorzugsweise umfassend eine Region von RD713, SEQ ID NO: 137, wobei stärker bevorzugt:
(i) die Primer die SEQ ID NO: 167 und 168 umfassen und/oder
(ii) die Sonde SEQ ID NO: 169 umfasst,
c) Primer oder Sonden, die spezifisch für MTC *lepA*, SEQ ID NO: 47, sind, wobei vorzugsweise die Sonde SEQ ID NO: 173 umfasst, wobei, wenn die Sonde SEQ ID NO: 173 umfasst, die Primer gegebenenfalls die SEQ ID NO: 164 und 165 umfassen,
d) Primer oder Sonden, die für eine Nukleinsäuresequenz spezifisch sind, die in allen drei von *M. bovis, M. bovis BCG* und *M. caprae* vorliegt, vorzugsweise umfassend eine Region von *lpqT*, SEQ ID NO: 109, wobei stärker bevorzugt:
(i) die Primer die SEQ ID NO: 158 und 159 umfassen und/oder
(ii) die Sonde SEQ ID NO: 160 umfasst,
e) Primer und Sonden, die für eine Nukleinsäuresequenz spezifisch sind, die in *M. caprae* vorliegt, aber nicht in *M. bovis* oder *M. bovis BCG* vorliegt, vorzugsweise eine Region von *M. caprae-lepA,* SEQ ID NO: 76, wobei stärker bevorzugt die Sonde SEQ ID NO: 166 umfasst, und wenn die Sonde SEQ ID NO: 166 umfasst, die Primer gegebenenfalls die SEQ ID NO: 164 und 165 umfassen,
f) Primer oder Sonden, die für eine Nukleinsäuresequenz spezifisch sind, die in *M. bovis BCG* und *M. microti* deletiert ist, aber in *M. bovis, M. caprae* und *M. pinnipedii* vorliegt, vorzugsweise eine Region von RD1, SEQ ID NO: 141, wobei stärker bevorzugt:
(i) die Primer die SEQ ID NO: 161 und 162 umfassen und/oder
(ii) die Sonde SEQ ID NO: 163 umfasst, und/oder
g) Primer oder Sonden, die für eine Nukleinsäuresequenz sind spezifisch, die in *m. africanum* Clade 2 vorliegt, aber nicht in *M. bovis, M. bovis BCG, M. caprae, M. microti* oder *M. pinnipedii* vorliegt, vorzugsweise eine Region von RD701, SEQ ID NO: 132, wobei stärker bevorzugt:
(i) die Primer die SEQ ID NO: 170 und 171 umfassen und/oder
(ii) die Sonde SEQ ID NO: 172 umfasst und/oder
(II) Primer oder Sonden, die spezifisch für eine IAC sind, vorzugsweise umfassend eine Region von:
a) *lepA*, SEQ ID NO: 68, wobei stärker bevorzugt die Sonde SEQ ID NO: 107 umfasst, oder
b) MSMEG_0660, SEQ ID NO: 135, umfassen, wobei stärker bevorzugt:
(i) die Primer die SEQ ID NO: 155 und 156 umfassen und/oder
(ii) die Sonde SEQ ID NO: 157 umfasst.

14. Diagnosetechnik zur Identifizierung von *Mycobacterium canettii,* umfassend
Hybridisieren von Proben-Nukleinsäuremolekülen an ein oder mehrere Nukleinsäuremoleküle, die eine Region von RD^{canetti1} (SEQ ID NO: 78) umfassen oder dazu komplementär sind und die für eine Nukleinsäuresequenz spezifisch sind, die in *M. canettii* vorliegt, aber nicht in *M. tuberculosis* vorliegt, und wobei gegebenenfalls:
a) die Proben-Nukleinsäuremoleküle an mindestens ein Nukleinsäuremolekül hybridisiert werden, das eine Region von *wbbl1* (SEQ ID NO: 1-46) umfasst oder komplementär dazu ist und das sowohl in *M. tuberculosis* als auch in *M. canettii* vorliegt,
b) die Proben-Nukleinsäuremoleküle an mindestens ein Nukleinsäuremolekül hybridisiert werden, das eine Region von *lepA* (SEQ ID NO: 47-77) umfasst oder komplementär dazu ist,
c) die Proben-Nukleinsäuremoleküle an mindestens ein Nukleinsäuremolekül hybridisiert werden, das eine Region von RD713 (SEQ ID NO: 137) umfasst oder komplementär dazu ist und das in *M. africanum* Clade 1 vorliegt, aber nicht in *M. tuberculosis* oder *M. canettii* vorliegt,
d) die Proben-Nukleinsäuremoleküle an Nukleinsäuremoleküle hybridisiert werden, die eine Region von *lpqT* (SEQ ID NO: 109) umfassen oder komplementär dazu sind und die in allen drei von *M*. *bovis, M. bovis BCG* und *M. caprae* vorliegen,
e) die Proben-Nukleinsäuremoleküle an Nukleinsäuremoleküle hybridisiert werden, die eine Region von *M. caprae-lepA* (SEQ ID NO: 76) umfassen oder dazu komplementär dazu sind und die für eine Nukleinsäuresequenz spezifisch sind, die in *M. caprae* vorliegt, aber nicht in *M. bovis* oder *M. bovis BCG* vorliegt,
f) die Proben-Nukleinsäuremoleküle an Nukleinsäuremoleküle hybridisiert werden, die eine Region von RD1 (SEQ ID NO: 141) umfassen oder komplementär dazu sind und die für eine Nukleinsäuresequenz spezifisch sind, die in *M. bovis BCG* und *M. microti* deletiert ist, aber in *M. bovis, M. caprae* und *M. pinnipedii* vorliegt, und/oder
g) die Proben-Nukleinsäuremoleküle an Nukleinsäuremoleküle hybridisiert werden, die eine Region von RD701 (SEQ ID NO: 132) umfassen oder komplementär dazu sind und die spezifisch für eine Nukleinsäuresequenz sind, die in *M. africanum* Clade 2 vorliegt, aber nicht in *M. bovis, M. bovis BCG, M. caprae, M. microti* oder *M. pinnipedii* vorliegt,
und wobei gegebenenfalls die Hybridisierung an einem Mikroarray erfolgt.

15. Verfahren nach einem der Ansprüche 1-11, Kit nach einem der Ansprüche 12-13 oder Diagnosetechnik nach Anspruch 14 für die Verwendung zur Überwachung der Krankheitsprogression oder zur Überwachung der Reaktion auf eine Behandlung, wobei die Krankheit vorzugsweise Tuberkulose ist.

## Revendications

1. Procédé *in vitro* d'amplification multiplexe d'acide nucléique destiné à identifier *Mycobacterium canettii* présent dans un échantillon, dans lequel le procédé comprend la détection de la présence ou l'absence d'une pluralité de cibles moléculaires d'acide nucléique dans l'échantillon en une réaction, lesquelles sont, en combinaison, uniques pour *Mycobacterium canettii* de par leur présence ou absence, dans lequel le procédé comprend l'utilisation d'amorces ou de sondes spécifiques d'une séquence d'acide nucléique qui est présente dans *M. canettii* mais qui n'est pas présente dans *M*. *tuberculosis* et qui comprend une région de RD^{canetti1}, SEQ ID n° : 78.

2. Procédé *in vitro* d'amplification multiplexe d'acide nucléique selon la revendication 1, dans laquelle le procédé comprend une ACP multiplexe.

3. Procédé selon la revendication 1 ou la 2, dans lequel :
a) les amorces comprennent les SEQ ID n° : 103 et SEQ ID n° : 105 ; et/ou
b) la sonde comprend la SEQ ID n° : 104.

4. Procédé, selon l'une quelconque des revendications précédentes, comprenant en outre l'utilisation d'amorces ou de sondes spécifiques d'une séquence d'acide nucléique qui est présente à la fois dans *M. tuberculosis* et *M. canettii.*

5. Procédé selon la revendication 4, dans laquelle la séquence d'acide nucléique qui est présente à la fois dans *M. tuberculosis* et *M. canettii* comprend une région de *wbbl1*, SEQ ID n° : 1, et dans laquelle de préférence :
a) les amorces comprennent les SEQ ID n° : 97 et SEQ ID n° : 99 ; et/ou
b) la sonde comprend la SEQ ID n° : 98.

6. Procédé, selon l'une quelconque des revendications précédentes, comprenant en outre l'utilisation d'amorces ou de sondes spécifiques d'une séquence d'acide nucléique qui est présente dans *M*. *africanum* clade 1 mais qui n'est pas présente dans *M*. *tuberculosis* ou *M*. *canettii.*

7. Procédé selon la revendication 6, dans laquelle la séquence d'acide nucléique qui est présente dans *M. africanum* clade 1 mais qui n'est pas présente dans *M. tuberculosis* ou *M. canettii* comprend une région de RD713, SEQ ID n° : 137, et dans laquelle de préférence :
a) les amorces comprennent les SEQ ID n° : 167 et 168 ; et/ou
b) la sonde comprend la SEQ ID n° : 169.

8. Procédé, selon l'une quelconque des revendications précédentes, comprenant en outre l'utilisation d'amorces ou de sondes spécifiques de *lepA,* SEQ ID n° : 47, pour détecter la présence ou l'absence du complexe de *Mycobacterium tuberculosis* et, en option, (i) dans laquelle plus d'une sonde spécifique de *lepA* est utilisée, de préférence dans laquelle au moins l'une des sondes spécifiques de *lepA* ne se lie pas, de manière significative, à un acide nucléique amplifié à partir d'un membre du complexe de *Mycobacterium tuberculosis*, et/ou (ii) dans laquelle la sonde comprend la SEQ ID n° : 173.

9. Procédé, selon l'une quelconque des revendications 1 à 8, comprenant en outre une amplification *in vitro* multiplexe d'acide nucléique détectant une pluralité de molécules d'acide nucléique qui, en combinaison, sont uniques pour *Mycobacterium bovis* de par leur présence ou absence détectant une pluralité de molécules d'acide nucléique qui, en combinaison, sont uniques pour *Mycobacterium bovis BCG* de par leur présence ou absence, détectant une pluralité de molécules d'acide nucléique qui, en combinaison, sont uniques pour *Mycobacterium caprae* de par leur présence ou absence, détectant une pluralité de molécules d'acide nucléique qui, en combinaison, sont uniques pour *Mycobacterium africanum* clade 2 de par leur présence ou absence, détectant une pluralité de molécules d'acide nucléique qui, en combinaison, sont uniques pour *Mycobacterium pinnipedii* de par leur présence ou absence, ou détectant une pluralité de molécules d'acide nucléique qui, en combinaison, sont uniques pour *Mycobacterium microti* de par leur présence ou absence, dans laquelle :
a) les amorces ou sondes qui sont utilisées sont spécifiques d'une séquence d'acide nucléique qui est présente dans l'ensemble des trois *M. bovis, M. bovis BCG* et *M. caprae,* facultativement dans laquelle la séquence d'acide nucléique comprend une région de *lpq*T, SEQ ID n° : 109, dans laquelle de préférence :
(i) les amorces comprennent les SEQ ID n° : 158 et 159 ; et/ou
(ii) la sonde comprend la SEQ ID n° : 160 ;
b) les amorces ou sondes qui sont utilisées sont spécifiques d'une séquence d'acide nucléique qui est présente dans *M. caprae* mais qui n'est pas présente dans *M. bovis* ou *M. bovis BCG,* facultativement dans laquelle la séquence d'acide nucléique comprend une région de *lepA* de *M. caprae,* SEQ ID n° : 76, dans laquelle de préférence :
(i) les amorces comprennent les SEQ ID n° : 164 et 165 ; et/ou
(ii) la sonde comprend la SEQ ID n° : 166 ;
c) les amorces ou sondes qui sont utilisées sont spécifiques d'une séquence d'acide nucléique qui est supprimée dans *M. bovis BCG* et *M. microti* mais qui est présente dans *M. bovis, M. caprae* et *M. pinnipedii,* facultativement dans laquelle l'acide nucléique comprend une région de RD1, SEQ ID n° : 141, dans laquelle de préférence :
(i) les amorces comprennent les SEQ ID n° : 161 et 162 ; et/ou
(ii) la sonde comprend la SEQ ID n° : 163 ; et/ou
d) les amorces ou sondes qui sont utilisées sont spécifiques d'une séquence d'acide nucléique qui est présente dans *M. africanum* clade 2 mais qui n'est pas présente dans *M. bovis, M. bovis BCG, M. caprae, M. microti* ou *M*. *pinnipedii,* facultativement dans laquelle l'acide nucléique comprend une région de RD701, SEQ ID n° : 132, dans laquelle de préférence :
(i) les amorces comprennent les SEQ ID n° : 170 et 171 ; et/ou
(ii) la sonde comprend la SEQ ID n° : 172.

10. Procédé, selon la revendication 9, comprenant l'exécution du procédé, selon l'une quelconque des revendications 1 à 8, et l'exécution ultérieure de l'amplification *in vitro* multiplexe d'acide nucléique telle que définie dans la revendication 9.

11. Procédé, selon l'une quelconque des revendications précédentes, comprenant l'utilisation d'amorces ou de sondes spécifiques d'un IAC, de préférence comprenant une région de :
a) *lepA*, SEQ ID n° : 84 ; ou
b) comprenant une région de MSMEG_0660, SEQ ID n° : 135, mieux préféré dans laquelle :
(i) les amorces comprennent les SEQ ID n° : 155 et 156 ; et/ou
(ii) la sonde comprend la SEQ ID n° : 157.

12. Trousse comprenant un ensemble d'amorces et de sondes qui est spécifique d'une pluralité de cibles moléculaires d'acide nucléique lesquelles sont, en combinaison, uniques pour *Mycobacterium canettii* de par leur présence ou absence, et dans laquelle la trousse comprend des amorces ou sondes spécifiques d'un acide nucléique qui est présent dans *M. canettii* mais qui n'est pas présent dans *M*. *tuberculosis* et qui sont spécifiques d'une région de RD^{canetti1}, SEQ ID n° : 78, dans laquelle de préférence :
a) les amorces comprennent les SEQ ID n° : 103 et 105 ; et/ou
b) dans laquelle la sonde comprend la SEQ ID n° : 104.

13. Trousse, selon la revendication 12, comprenant en outre :
(I)
a) des amorces ou sondes spécifiques d'une séquence d'acide nucléique qui est présente à la fois dans *M. tuberculosis* et *M. canettii,* de préférence comprenant des amorces ou sondes spécifiques d'une région de *wbbl1*, SEQ ID n° : 1, mieux préféré dans laquelle :
(i) les amorces comprennent les SEQ ID n° : 97 et 99 ; et/ou
(ii) la sonde comprend la SEQ ID n° : 98 ;
b) des amorces ou sondes spécifiques d'une séquence d'acide nucléique qui est présente dans M. *africanum* clade 1 mais qui n'est pas présente dans M. *tuberculosis* ou *M. canettii,* comprenant de préférence une région de RD713, SEQ ID n° : 137, mieux préféré dans laquelle :
(i) les amorces comprennent les SEQ ID n° : 167 et 168 ; et/ou
(ii) la sonde comprend la SEQ ID n° : 169 ;
c) des amorces ou sondes spécifiques d'un MTC *lepA,* SEQ ID n° : 47, de préférence dans laquelle la sonde comprend la SEQ ID n° : 173 dans laquelle, quand la sonde comprend la SEQ ID n° : 173, les amorces comprennent facultativement les SEQ ID n° : 164 et 165 ;
d) des amorces ou sondes spécifiques d'une séquence d'acide nucléique qui est présente dans l'ensemble des trois *M. bovis, M. bovis BCG* et *M. caprae*, de préférence comprenant une région de *lpq*T, SEQ ID n° : 109, mieux préféré dans laquelle :
(i) les amorces comprennent les SEQ ID n° : 158 et 159 ; et/ou
(ii) la sonde comprend la SEQ ID n° : 160 ;
e) des amorces ou sondes spécifiques d'une séquence d'acide nucléique qui est présente dans *M. caprae* mais qui n'est pas présente dans *M. bovis* ou *M*. *bovis BCG,* de préférence une région de *lep*A de *M. caprae,* SEQ ID n° : 76, mieux préféré dans laquelle la sonde comprend la SEQ ID n° : 166 et, quand la sonde comprend la SEQ ID n° : : 166, les amorces comprennent facultativement les SEQ ID n° : 164 et 165 ;
f) des amorces ou sondes spécifiques d'une séquence d'acide nucléique qui est supprimée dans *M. bovis BCG* et *M. microti* mais qui est présente dans *M*. *bovis, M. caprae* et *M. pinnipedii,* de préférence une région de RD1, SEQ ID n° : 141, mieux préféré dans laquelle :
(i) les amorces comprennent les SEQ ID n° : 161 et 162 ; et/ou
(ii) la sonde comprend la SEQ ID n° : 163 ;
et/ou
g) des amorces ou sondes spécifiques d'une séquence d'acide nucléique qui est présente dans *M. africanum* clade 2 mais qui n'est pas présente dans *M. bovis, M. bovis BCG, M. caprae, M. microti* ou *M. pinnipedii,* de préférence une région de RD701, SEQ ID n° : 132, mieux préféré dans laquelle :
(i) les amorces comprennent les SEQ ID n° : 170 et 171 ; et/ou
(ii) la sonde comprend la SEQ ID n° : 172 ;
et/ou
(II) des amorces ou sondes spécifiques d'un IAC, de préférence comprenant une région de :
a) *lepA,* SEQ ID n° : 68, mieux préféré dans laquelle la sonde comprend la SEQ ID n° : 107 ; ou
b) MSMEG_0660, SEQ ID n° : 135, mieux préféré dans laquelle :
(i) les amorces comprennent les SEQ ID n° : 155 et 156 ; et/ou
(ii) la sonde comprend la SEQ ID n° : 157.

14. Technique diagnostique destinée à identifier *Mycobacterium canettii,* comprenant l'hybridation de molécules échantillons d'acide nucléique à une ou plusieurs molécule(s) d'acide nucléique, qui comprennent ou sont complémentaires à une région de RD^{canetti1} (SEQ ID n° : 78), et qui sont spécifiques d'une séquence d'acide nucléique qui est présente dans *M. canettii* mais qui n'est pas présente dans *M. tuberculosis* et, en option :
a) dans laquelle les molécules échantillons d'acide nucléique sont hybridées à au moins une molécule d'acide nucléique qui comprend ou qui est complémentaire à une région de *wbbl1* (SEQ ID n° : 1 à 46), et qui est présente à la fois dans *M. tuberculosis* et *M. canettii* ;
b) dans laquelle les molécules échantillons d'acide nucléique sont hybridées à au moins une molécule d'acide nucléique qui comprend ou qui est complémentaire à une région de *lep*A (SEQ ID n° : 47 à 77) ;
c) dans laquelle les molécules échantillons d'acide nucléique sont hybridées à au moins une molécule d'acide nucléique qui comprend ou qui est complémentaire à une région de RD713 (SEQ ID n° : 137), et qui est présente dans *M. africanum* clade 1 mais qui n'est pas présente dans *M*. *tuberculosis* ou M. *canettii* ;
d) dans laquelle les molécules échantillons d'acide nucléique sont hybridées à des molécules d'acide nucléique qui comprennent ou qui sont complémentaires à une région de *lpq*T (SEQ ID n° : 109), et qui sont présentes dans l'ensemble des trois *M. bovis, M. bovis BCG* et *M. caprae ;*
e) dans laquelle les molécules échantillons d'acide nucléique sont hybridées à des molécules d'acide nucléique qui comprennent ou qui sont complémentaires à une région de *lep*A de *M. caprae* (SEQ ID n° : 76) et qui sont spécifiques d'une séquence d'acide nucléique qui est présente dans *M. caprae* mais qui n'est pas présente dans *M. bovis* ou *M. bovis BCG* ;
f) dans laquelle les molécules échantillons d'acide nucléique sont hybridées à des molécules d'acide nucléique qui comprennent ou qui sont complémentaires à une région de RD1 (SEQ ID n° : 141) et qui sont spécifiques d'une séquence d'acide nucléique qui est supprimée dans *M. bovis BCG* et *M. microti* mais qui est présente dans *M. bovis, M. caprae* et *M. pinnipedii ;* et/ou
g) dans laquelle les molécules échantillons d'acide nucléique sont hybridées à des molécules d'acide nucléique qui comprennent ou qui sont complémentaires à une région de RD701 (SEQ ID n° : 132) et qui sont spécifiques d'une séquence d'acide nucléique qui est présente dans *M. africanum* clade 2 mais qui n'est pas présente dans *M. bovis, M. bovis BCG, M. caprae, M. microti* ou *M. pinnipedii ;*
et, en option, dans laquelle l'hybridation a lieu sur un micro-arrangement.

15. Procédé selon l'une quelconque des revendications 1 à 11, trousse selon l'une quelconque des revendications 12 à 13, ou technique diagnostique selon la revendication 14, destiné(e) à être utilisé(e) dans le suivi de la progression d'une maladie ou le suivi de la réponse à un traitement, de préférence dans laquelle la maladie est la tuberculose.
